# EUROPEAN PATENT APPLICATION

(11) **EP 1 816 139 A1**
(43) Date of publication of application: **08.08.2007**
(21) Application number: 07000032.8
(22) Date of filing: 20.11.2003
(51) Int. Cl.: C07K 14/705, C07K 16/00, C12N 5/10, C12N 15/11, C12N 15/63, G01N 33/53

(54) **Nact as a target for lifespan expansion and weight reduction**

(30) Priority: 22.11.2002 US 428469 P; 01.04.2003 US 459441 P
(62) Divisional of application: 03796430.1
(71) Applicant: Medical College Of Georgia Research Institute, Inc., Augusta, GA 30912-4810 (US); GANAPATHY, Vadivel, Martinez, GA 30907 (US); INOUE, Katsuhisa, Nagoya 467-0056 (JP); FEI, You-Jun, North Augusta, SC 29841 (US)
(72) Inventor: Ganapathy, Vadivel, Martinez, GA 30907 (US); Inoue, Katsuhisa, Nagoya 467-0056 (JP); Fei, You-Jun, North Augusta, SC 29841 (US)
(74) Representative: Vossius & Partner

(57) **Abstract**

The present invention provides the identfication and characterization of a novel transmembrane transporter, a Na'-coupled citrate transporter ("NaCT"). Isolated polynucleotides encoding the transmembrane transporter, the transmembrane transporter polypeptide itself, antibodies thereto, and methods of use, are provided.

## Description

### CONTINUING APPLICATION DATA

This application claims the benefit of U.S. Provisional Application Serial No. 60/428,469, filed November 22, 2002, and U.S. Provisional Application Serial No. 60/459,441, filed April 1, 2003, each of which is incorporated by reference herein.

### GOVERNMENT FUNDING

The present invention was made with government support under National Institutes of Health Grant No. DA10045, Grant No. HD 33347, Grant No. HL64196, Grant No. HD44404, and Grant No. AI49849. The Government may have certain rights in this invention.

### BACKGROUND

Na⁺-coupled dicarboxylate transporters (NaDCs) mediate the cellular entry of a variety of citric acid cycle intermediates in mammalian tissues. There are two different isoforms of NaDC in mammals, namely NaDC1 and NaDC3 (Pajor, J. Membrane Biol. (2000);175: 1-8). While both are sodium-coupled transporters for succinate and other dicarboxylate intermediates of citric acid cycle (Pajor, Annu. Rev. Physiol. (1999);61: 663-682), these transporters can be distinguished from one another primarily based on their substrate affinity. NaDC1 is a low affinity transporter with a Michaelis-Menten constant (K*ₜ*) for succinate in the millimolar range, whereas NaDC3 is a high affinity transporter with a K*ₜ* for succinate in the micromolar range. NaDC2, identified in *Xenopus laevis* intestine, is functionally and structurally related to the mammalian NaDCs, but may represent a species-specific ortholog of NaDC1 (Bai and Pajor, Am. J. Physiol. (1997);273: G267-G274).

The mammalian NaDCs have been cloned from different species and their functional characteristics have been elucidated in different heterologous expression systems. See, for example, Pajor, J. Biol. Chem. (1995);270: 5779-5785; Pajor, Am. J. Physiol. (1996);270: F642-F648; Chen et al., J. Biol. Chem. (1998);273: 20972-20981; Kekuda et al., J. Biol. Chem. (1999);274: 3422-3429; Wang et al., Am. J. Physiol. (2001);278: C1019-C1030; Chen et al., J. Clin. Invest. (1999);103: 1159-1168; Pajor et al., Am. J. Physiol. (2001);280: C1215-C1223; and Pajor and Sun, Am. J. Physiol. (2000);279: F482-F490. The Na⁺-coupled dicarboxylate transporters ceNaDC1 and ceNaDC2 from the nematode *C. elegans* have also been cloned. See, Fei et al., (2003) J Biol Chem 278, 6136-6144.

Recently, Rogina et al. (Rogina et al., Science (2000);290: 2137-2140) reported on the identification of a gene in *Drosophila melanogaster* which, when mutated, confers life span extension to the organism. Interestingly, the predicted protein product of this gene, known as Indy (for *I'*m *N*ot *D*ead *Y*et), shows significant homology to mammalian NaDCs. It was therefore suggested that Indy is the *Drosophila* ortholog of either NaDC1 or NaDC3. However, even though it was assumed, based on the structural similarity, that *Drosophila* Indy is a sodium-coupled transporter for dicarboxylates similar to mammalian NaDCs, its transport identity has not been established.

NaDC1 is expressed primarily in the intestine and kidney, whereas NaDC3 is expressed, not only in the intestine and kidney, but also in the brain, liver, and placenta (Pajor, Annu. Rev. Physiol. (1999);61: 663-682). A unique feature of both of these transporters is that they interact with dicarboxylates with greater preference than with citrate, a tricarboxylate at physiological pH. Furthermore, even though NaDC1 and NaDC3 are able to transport citrate to some extent, only the dianionic form of citrate is recognized as the substrate by these transporters. Thus, NaDC1 and NaDC3 are specific for dicarboxylates. NaDCs are structurally related to the Na⁺-coupled sulfate transporters NaSi and SUT1 (Pajor, Annu. Rev. Physiol.(1999);61: 663-682). Together, these transporters constitute the NaDC/NaSi gene family.

While NaDC1, as well as NaDC3, can transport citrate to some extent, the efficiency of transport is low because these transporters recognize only the dicarboxylate form of citrate as substrate. Since the dicarboxylates, such as succinate, malate, fumarate, oxaloacetate, and α-ketoglutarate, are present in blood at low levels, the high-affinity transporter NaDC3 is ideally suited for efficient transport of these substrates under physiological conditions. However, the circulating levels of citrate are several-fold greater than the combined levels of the dicarboxylates. The concentration of citrate in blood is approximately 135 µM. In contrast, the concentration of succinate is approximately 40 µM and the concentrations of other dicarboxylates are even lower. The divalent form of citrate, which is recognized by NaDC3 as a substrate, is present only at low concentrations (approximately 10 µM) in blood at physiological pH. Therefore NaDC3 does not provide an efficient mechanism for the cellular utilization of citrate present in the circulation.

### SUMMARY OF THE INVENTION

The present invention includes the identification and characterization of a novel transmembrane transporter, a Na⁺-coupled citrate transporter ("NaCT"), which recognizes the tricarboxylate citrate with higher affinity than the dicarboxylates, such as succinate, malate, fumarate, and 2-oxo-glutarate. The present invention also demonstrates, for the first time, that the Na⁺-coupled citrate transporter is a target for lithium action, with the action of lithium on NaCT varying depending on the animal species. Human NaCT is activated by lithium, while rodent NaCTs are inhibited by lithium.

The present invention includes isolated polynucleotides encoding a polypeptide having at least 35% sequence identity to SEQ ID NO:8, wherein the polynucleotide encodes a polypeptide demonstrating Na⁺-dependent transmembrane transport of citrate. In some embodiments, the polynucleotide includes SEQ ID NO:3, SEQ ID NO:5, SEQ ID NO:7, SEQ ID NO:9, or SEQ ID NO:11.

The present invention also includes isolated polynucleotides that hybridize to SEQ ID NO:1 under stringent hybridization conditions, wherein the polynucleotide encodes a polypeptide demonstrating transmembrane transport of citrate. In some embodiments, the polynucleotide includes SEQ ID NO:1. In some embodiments, the polynucleotide does not include SEQ ID NO:1.

The present invention includes isolated polynucleotides that hybridize to SEQ ID NO:3 under stringent hybridization conditions, wherein the polynucleotide encodes a polypeptide demonstrating Na⁺-dependent transmembrane transport of citrate. In some embodiments, the polynucleotide includes SEQ ID NO:3.

The present invention includes isolated polynucleotides that hybridize to SEQ ID NO:5 under stringent hybridization conditions, wherein the polynucleotide encodes a polypeptide demonstrating Na⁺-dependent transmembrane transport of citrate. In some embodiments the isolated polynucleotide includes SEQ ID NO:5 .

The present invention also includes isolated polynucleotides that hybridize to SEQ ID NO:7 under stringent hybridization conditions, wherein the polynucleotide encodes a polypeptide demonstrating Na⁺-dependent transmembrane transport of citrate. In some embodiments, the isolated polynucleotide includes SEQ ID NO:7.

The present invention also includes isolated polynucleotides that hybridize to SEQ ID NO:9 under stringent hybridization conditions, wherein the polynucleotide encodes a polypeptide demonstrating Na⁺-dependent transmembrane transport of citrate. In some embodiments, the isolated polynucleotide includes SEQ ID NO:9.

The present invention also includes isolated polynucleotides that hybridize to SEQ ID NO:11 under stringent hybridization conditions, wherein the polynucleotide encodes a polypeptide demonstrating Na⁺-dependent transmembrane transport of citrate. In some embodiments, the isolated polynucleotide includes SEQ ID NO:11.

Also included in the present invention are isolated polynucleotides encoding a polypeptide having at least 35% sequence identity to SEQ ID NO:6, wherein the polynucleotide encodes a polypeptide demonstrating Na⁺-dependent transmembrane transport of citrate. In some embodiments, the isolated polynucleotide includes SEQ ID NO:3, SEQ ID NO:5, SEQ ID NO:7, SEQ ID NO:9, or SEQ ID NO:11. In some embodiments, the Na⁺-dependent transmembrane transport of citrate is modulated by Li⁺. In some embodiments, the Na⁺-dependent transmembrane transport of citrate demonstrates a requirement for multiple Na⁺ions for transport coupling. In some embodiments, the transmembrane transport of citrate is electrogenic.

Also included in the present invention are plasmids with a polynucleotide encoding a polypeptide demonstrating transmembrane transport of citrate. In some embodiments, the plasmid includes an expression vector.

The present invention also includes host cells including a polynucleotide encoding a Na⁺-dependent transmembrane citrate transporter. In some embodiments, the host cell demonstrates transient expression of the encoded Na⁺-dependent transmembrane citrate transporter. In some embodiments, the host cell demonstrates stable expression of the encoded Na⁺-dependent transmembrane citrate transporter. In some embodiments, the encoded Na⁺-dependent transmembrane transport of citrate is modulated by Li⁺. In some embodiments, the isolated host cell may be a human cell, an insect cell, a *Xenopus* oocyte, or a yeast cell.

The present invention also includes isolated polypeptides having at least 35% identity with SEQ ID NO:2, wherein the polypeptide is a transmembrane transporter of citrate. In various embodiments, the isolated polypeptide includes SEQ ID NO:2, SEQ ID NO:4, SEQ ID NO:6, SEQ ID NO:8, SEQ ID NO:10, or SEQ ID NO:12. In some embodiments, the polypeptide demonstrates Na⁺-dependent transmembrane transport of citrate. In some embodiments, the Na⁺-dependent transmembrane transport of citrate is modulated by Li⁺.

The present invention also includes isolated polypeptides, wherein the polypeptide is encoded by a polynucleotide that hybridizes to SEQ ID NO:1 under stringent hybridization conditions and wherein the polypeptide demonstrates transmembrane transport of citrate.

The present invention includes isolated polypeptides having at least 35% sequence identity to SEQ ID NO:6, wherein the polypeptide demonstrates Na⁺-dependent transmembrane transport of citrate. In some embodiments, the Na⁺-dependent transmembrane transport of citrate is modulated by Li⁺. In some embodiments, the Na⁺-dependent transmembrane transport of citrate demonstrates a requirement for multiple Na⁺ions for transport coupling. In some embodiments, the transmembrane transport of citrate is electrogenic.

The present invention also includes isolated polypeptides having at least 35% sequence identity to SEQ ID NO:8, wherein the polypeptide demonstrates Na⁺-dependent transmembrane transport of citrate.

Also included in the present invention are antibodies that specifically bind to a polypeptide that demonstrates Na⁺-dependent transmembrane transport of citrate. In some embodiments, the antibody is monoclonal or polyclonal. In some embodiments, the antibody is derived from a mouse, rat, rabbit, hamster, goat, horse, or human. In some embodiments, the antibody is produced recombinantly. In some embodiments, one or more variable regions from the antibody are included in a chimeric protein. In some embodiments, the antibody is linked to a detectable marker.

The present invention also includes a method of identifying an agent that modifies transmembrane citrate transporter activity, the method including contacting a host cell expressing a transmembrane citrate transporter polypeptide having at least 35% identity with SEQ ID NO:2 with an agent; measuring citrate transport into the host cell in the presence of agent; and comparing citrate transport into the host cell in the presence of the agent to citrate transport into the host cell in the absence of the agent; wherein a decreased transport of citrate into the host cell in the presence of the agent indicates the agent is an inhibitor of transmembrane citrate transporter activity; wherein an increased transport of citrate into the host cell in the presence of the agent indicates the agent is a stimulator of transmembrane citrate transporter activity.

The present invention also includes a method of identifying an agent that modifies transmembrane citrate transporter activity, the method including contacting a host cell expressing a transmembrane citrate transporter polypeptide having at least 35% sequence identity to SEQ ID NO:8, wherein the transmembrane citrate transporter polypeptide demonstrates Na⁺-dependent transmembrane transport of citrate; measuring citrate transport into the host cell in the presence of agent; and comparing citrate transport into the host cell in the presence of the agent to citrate transport into the host cell in the absence of the agent; wherein a decreased transport of citrate into the host cell in the presence of the agent indicates the agent is an inhibitor of transmembrane citrate transporter activity; wherein an increased transport of citrate into the host cell in the presence of the agent indicates the agent is a stimulator of transmembrane citrate transporter activity.

The present invention includes a method of identifying an agent that modifies transmembrane citrate transporter activity, the method including contacting a host cell expressing a transmembrane citrate transporter polypeptide having at least 35% sequence identity to SEQ ID NO:6, wherein the transmembrane citrate transporter polypeptide demonstrates Na⁺-dependent transmembrane transport of citrate and wherein the encoded Na⁺-dependent transmembrane transport of citrate is stimulated by Li⁺; measuring citrate transport into the host cell in the presence of agent; and comparing citrate transport into the host cell in the presence of the agent to citrate transport into the host cell in the absence of the agent; wherein a decreased transport of citrate into the host cell in the presence of the agent indicates the agent is an inhibitor of transmembrane citrate transporter activity; wherein an increased transport of citrate into the host cell in the presence of the agent indicates the agent is a stimulator of transmembrane citrate transporter activity. In some embodiments, the transmembrane citrate transporter polypeptide includes SEQ ID NO:6. In some embodiments, the present invention includes a modifier of a transmembrane citrate transporter, as identified by the method.

The present invention also includes a modifier of a transmembrane citrate transporter. In some embodiments, the transmembrane citrate transporter has SEQ ID NO:6. In some embodiments, the modifiers may be included in a composition, including compositions that include a pharmaceutically acceptable carrier. In some embodiments, the composition may include an additional therapeutic agent, including, for example, lithium.

The present invention includes a method of extending the lifespan in a subject by administering an inhibitor of a transmembrane citrate transporter to a subject.

The present invention includes a method of weight reduction in a subject by administering an inhibitor of a transmembrane citrate transporter to a subject.

The present invention includes a method of preventing weight gain in a subject by administering an inhibitor of a transmembrane citrate transporter to a subject. In some embodiments, the subject may be a human subject or a domestic pet.

The present invention includes a method of lowering blood cholesterol levels in a subject by administering an inhibitor of a transmembrane citrate transporter to a subject.

The present invention includes a method of lowering blood triglyceride levels in a subject by administering an inhibitor of a transmembrane citrate transporter to a subject.

The present invention includes a method of lowering blood LDL levels in a subject by administering an inhibitor of a transmembrane citrate transporter to a subject.

The present invention includes a method of lowering blood glucose levels in a subject by administering an inhibitor of a transmembrane citrate transporter to a subject. In some embodiments, the subject is a diabetic.

The present invention includes a method of identifying an agent that modifies Na⁺-dependent transmembrane citrate transporter activity, the method including contacting a host cell expressing a Na⁺-dependent transmembrane citrate transporter selected from the group consisting of SEQ ID NO:4, SEQ ID NO:6, SEQ ID NO:8, SEQ ID NO:10, and SEQ ID NO:12 with an agent; measuring the citrate-induced inward electrical current into the host cell in the presence of agent; and comparing the citrate-induced inward electrical current into the host cell in the presence of the agent to the citrate-induced inward electrical current into the host cell in the absence of the agent; wherein a decrease in the inward electrical current into the host cell in the presence of the agent indicates the agent is a blocker of Na⁺-dependent transmembrane citrate transporter activity; wherein an increase in the inward electrical current into the host cell in the presence of the agent indicates the agent is a stimulator of Na⁺-dependent transmembrane citrate transporter activity.

The present invention includes a method of identifying an agent that serves as a substrate of a Na⁺-dependent transmembrane citrate transporter, the method including contacting a host cell expressing a Na⁺-dependent transmembrane citrate transporter selected from the group consisting of SEQ ID NO:4, SEQ ID NO:6, SEQ ID NO:8, SEQ ID NO:10, and SEQ ID NO:12 with an agent; and determining the entry of the agent into the cell via the Na⁺-dependent transmembrane citrate transporter in the presence of agent; wherein entry of the agent via the Na⁺-dependent transmembrane citrate transporter indicates the agent is a substrate of a Na⁺-dependent transmembrane citrate transporter.

### Definitions

As used herein, the term "isolated" means that a polynucleotide or polypeptide is either removed from its natural environment or synthetically derived, for instance by recombinant techniques, or chemically or enzymatically synthesized. An isolated polynucleotide denotes a polynucleotide that has been removed from its natural genetic milieu and is thus free of other extraneous or unwanted coding sequences, and is in a form suitable for use within genetically engineered protein production systems. Isolated polynucleotides of the present invention are free of other coding sequences with which they are ordinarily associated, but may include naturally occurring 5' and 3' untranslated regions such as promoters and terminators. Preferably, the polynucleotide or polypeptide is purified, i.e., essentially free from any other polynucleotides or polypeptides and associated cellular products or other impurities.

"Polynucleotide" and "nucleic acid sequences" are used interchangeably to refer to a linear polymeric form of nucleotides of any length, either ribonucleotides or deoxynucleotides, and includes both double- and single-stranded DNA and RNA. A polynucleotide can be linear or circular in topology. A polynucleotide can be obtained using any method, including, without limitations, common molecular cloning and chemical nucleic acid synthesis. A polynucleotide may include nucleotide sequences having different functions, including for instance coding sequences, and non-coding sequences.

As used herein "coding sequence," "coding region," and "open reading frame" are used interchangeably and refer to a polynucleotide that encodes a polypeptide, usually via mRNA, when placed under the control of appropriate regulatory sequences. The boundaries of the coding region are generally determined by a translation start codon at its 5' end and a translation stop codon at its 3' end.

As used herein, "stringent hybridization conditions" refer to hybridization conditions such as 6X SSC, 5X Denhardt, 0.5% sodium dodecyl sulfate (SDS), and 100 µg/ml fragmented and denatured salmon sperm DNA hybridized overnight at 65°C and washed in 2X SSC, 0.1% SDS at least one time at room temperature for about 10 minutes followed by at least one wash at 65°C for about 15 minutes followed by at least one wash in 0.2X SSC, 0.1% SDS at room temperature for at least 3-5 minutes. Typically, a 20X SSC stock solution contains about 3M sodium chloride and about 0.3M sodium citrate.

As used herein, "complement" and "complementary" refer to the ability of two single stranded polynucleotides to base pair with each other, where an adenine on one polynucleotide will base pair to a thymine on a second polynucleotide and a cytosine on one polynucleotide will base pair to a guanine on a second polynucleotide. Two polynucleotides are complementary to each other when a nucleotide sequence in polynucleotide can base pair with a nucleotide sequence in a second polynucleotide. For instance, 5'-ATGC and 5'-GCAT are complementary. Typically two polynucleotides are complementary if they hybridize under the standard conditions referred to herein.

"Polypeptide," as used herein, refers to a polymer of amino acids and does not refer to a specific length of a polymer of amino acids. Thus, for example, the terms peptide, oligopeptide, protein, and enzyme are included within the definition of polypeptide, whether naturally occurring or synthetically derived, for instance, by recombinant techniques or chemically or enzymatically synthesized. This term also includes post-expression modifications of the polypeptide, for example, glycosylations, acetylations, phosphorylations, and the like. The following abbreviations are used throughout the application:

| | |
|---|---|
| A = Ala = Alanine | T = Thr = Threonine |
| V = Val = Valine | C = Cys = Cysteine |
| L = Leu = Leucine | Y = Tyr = Tyrosine |
| I = Ile = Isoleucine | N = Asn = Asparagine |
| P = Pro = Proline | Q = Gln = Glutamine |
| F = Phe = Phenylalanine | D = Asp = Aspartic Acid |
| W = Trp = Tryptophan | E = Glu = Glutamic Acid |
| M = Met = Methionine | K = Lys = Lysine |
| G = Gly = Glycine | R = Arg = Arginine |
| S = Ser = Serine | H = His = Histidine |

A "subject" or an "individual" is an organism, including, for example, a microbe, a plant, an invertebrate, or a vertebrate, such as, but not limited to, an animal. An animal may include, for example, a bird, a fish, a rat, a mouse, a domestic pet, such as, but not limited to, a dog or a cat, livestock, such as, but not limited to, a cow, a horse, or a pig, a primate, or a human. Subject also includes model organisms, including, for example, *Drosophila,* the nematode *C. elegans*, or animal models used, for example, for the study of NaCT structure or function, life span and weight gain. A "non-human animal" refers to any animal that is not a human and includes vertebrates such as rodents, non-human primates.

A "control" sample or subject is one in which a NaCT polypeptide has not been manipulated in any way.

As used herein *in vitro* is in cell culture, *ex vivo* is a cell that has been removed from the body of a subject and *in vivo* is within the body of a subject.

Unless otherwise specified, "a," "an," "the," and "at least one" are used interchangeably and mean one or more than one.

### BRIEF DESCRIPTION OF THE DRAWINGS

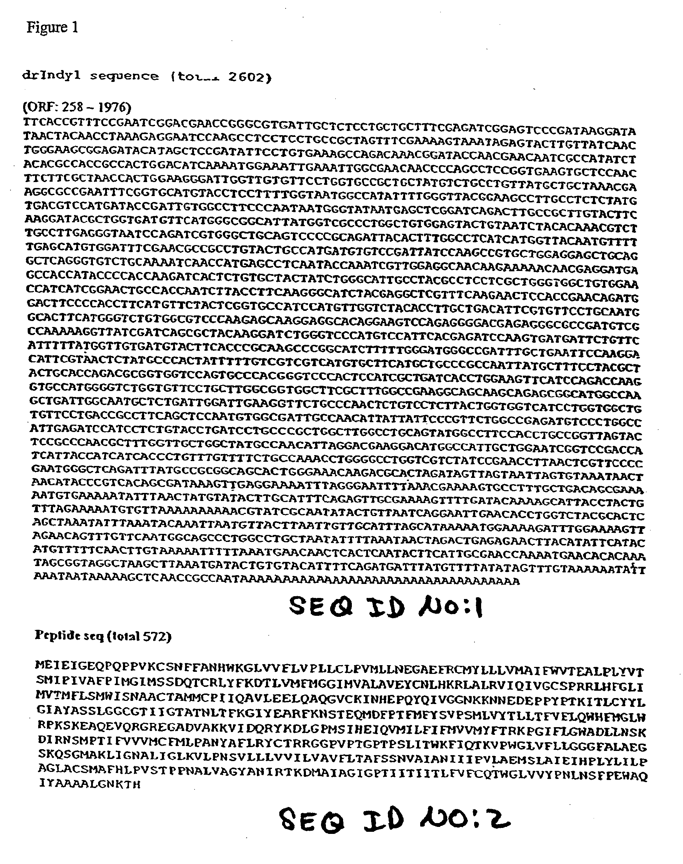

Figure 1. The full-length cDNA nucleotide sequence (SEQ ID NO:1) and translated amino acid sequence (SEQ ID NO:2) of *D*. *melanogaster* Indy.

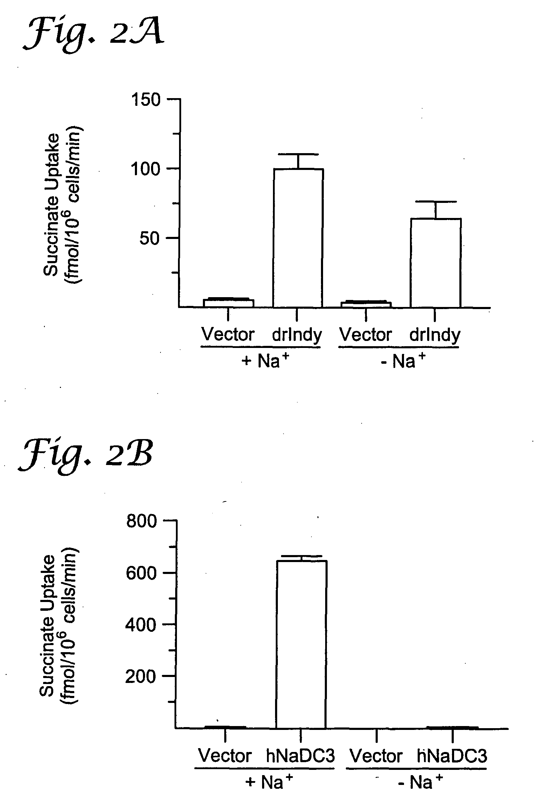

Figure 2. Comparison of succinate uptake by drIndy (Fig. 2A) and hNADC3 (Fig. 2B) in the presence or absence of Na⁺. HRPE cells were transfected with vector alone, drIndy cDNA or hNaDC3 cDNA. Uptake of succinate (40 nM) was measured in the presence of either NaCl (+Na⁺) or *N-*methyl-D-glucamine chloride (-Na⁺). Data (means ± S.E.M.) are from nine independent measurements.

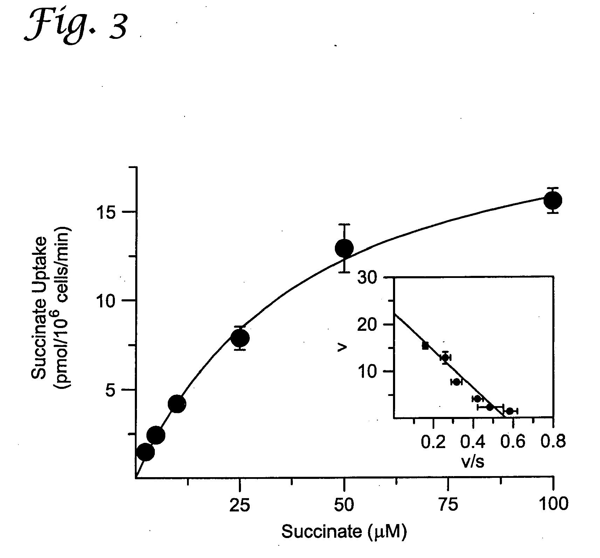

Figure 3. Saturation kinetics of succinate uptake via drIndy measured in the presence of Na⁺. Data (means ± S.E.M.) represent only the cDNA-specific uptake and are from four independent measurements. Inset: Eadie-Hofstee plot [succinate uptake/succinate concentration (v/s) versus succinate uptake (v)].

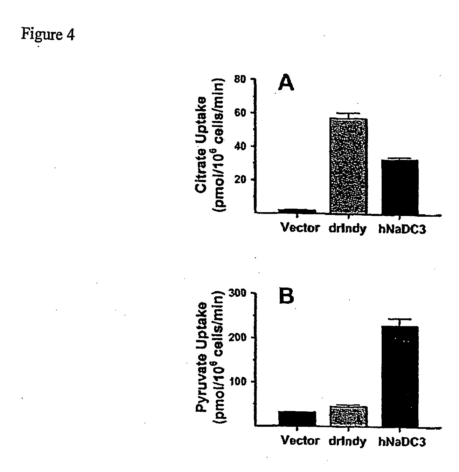

Figure 4. Comparison of the ability of drIndy and hNaDC3 to transport citrate and pyruvate. Uptake of citrate (35 µM) (Fig. 4A) or pyruvate (135 µM) (Fig. 4B) was measured in the presence of NaCl in HRPE cells transfected with vector alone, drIndy cDNA, or hNaDC3 cDNA. Data (means ± S.E.M.) are from three independent measurements.

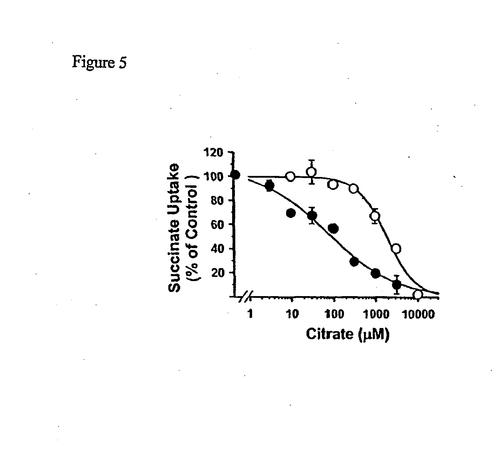

Figure 5. Comparison of affinity for citrate for the transport process mediated by drIndy and hNaDC3. HRPE cells were transfected with vector alone, drIndy cDNA (●) or hNaDC3 cDNA (○). Uptake of succinate (80 nM) was measured in the presence of NaCl with or without increasing concentrations of citrate. Data (means ± S.E.M.) represent only the cDNA-specific uptake and are from three independent measurements.

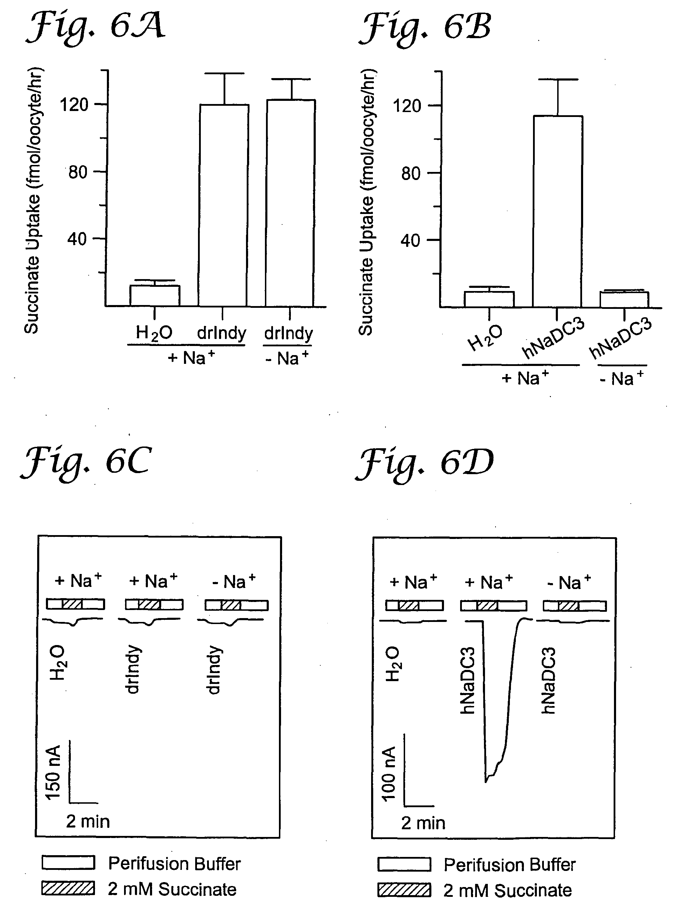

Figure 6. Transport characteristics of drIndy and hNaDC3 in *X*. *laevis* oocytes. drIndy and hNaDC3 were expressed functionally in oocytes by injection of the respective cRNA. Water-injected oocytes served as control (Fig. 6A and Fig. 6B). Uptake of succinate (0.1 µM) was measured in the presence of either NaCl (+Na⁺) or choline chloride (-Na⁺). Data (means ± S.E.M.) are from ten oocytes (Fig. 6C and Fig. 6D). Succinate (2 mM)-induced inward currents were monitored using the two-micro-electrode voltage-clamp method in drIndy- and hNaDC3-expressing oocytes or in water-injected oocytes. The membrane potential was maintained at -50 mV. Perfusion buffers contained either NaCl (+Na⁺) or choline chloride (-Na⁺).

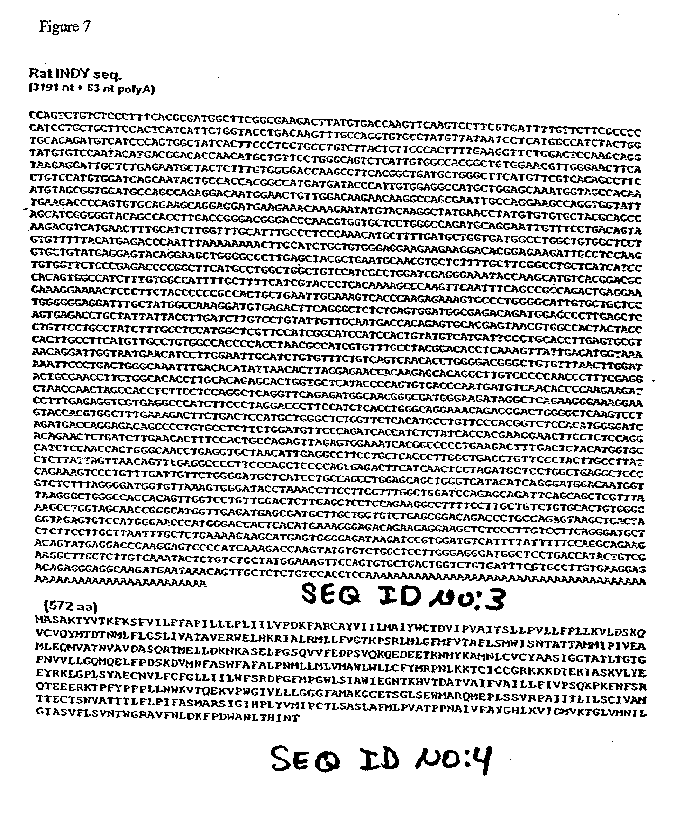

Figure 7. The full-length cDNA nucleotide sequence (SEQ ID NO:3) and translated amino acid sequence (SEQ ID NO:4) of rat NaCT transporter.

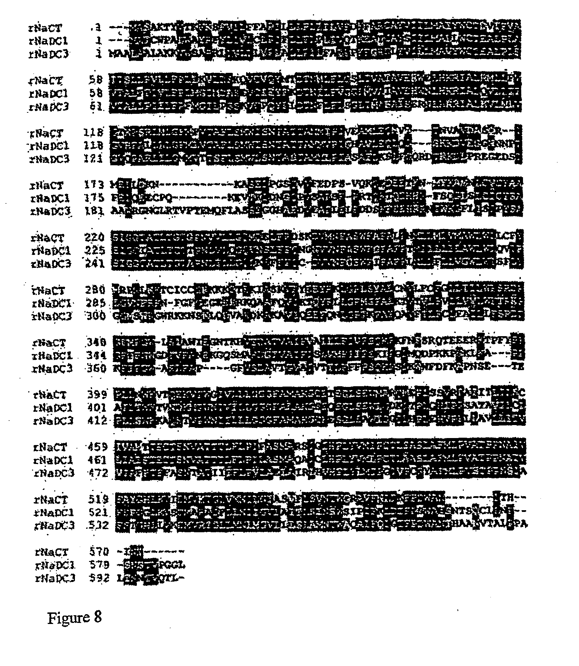

Figure 8. Alignment of amino acid sequence of rat NaCT (SEQ ID NO:4) with that of rat NaDC1 (SEQ ID NO:13) and rat NaDC3 (SEQ ID NO:14). Regions of similarity are shaded.

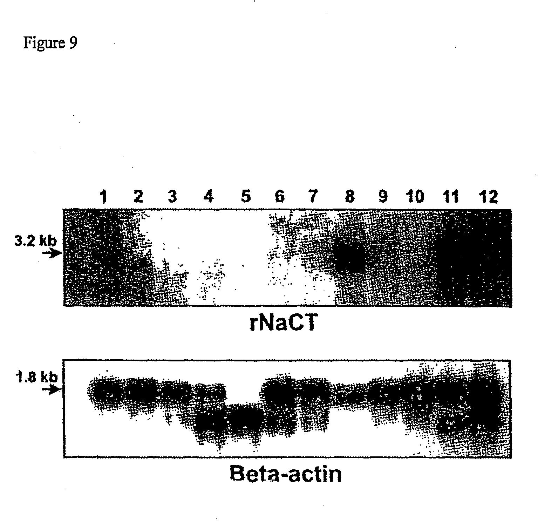

Figure 9. Tissue expression pattern of NaCT mRNA in rat by Northern analysis. A commercially available rat multiple tissue blot was hybridized sequentially first with a rat NaCT-specific probe and then with a rat β-actin-specific probe under high stringency conditions. Lanes 1-12 present brain, thymus, lung, heart, skeletal muscle, stomach,
   small intestine, liver, kidney, spleen, testis, and skin, respectively.

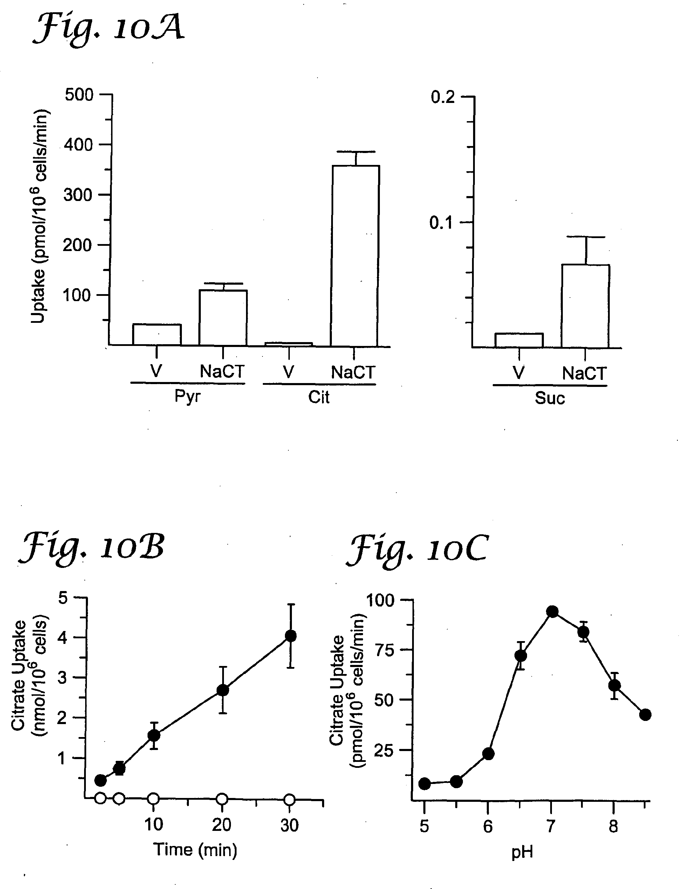

Figure 10. Uptake of citrate, succinate, and pyruvate by rat NaCT. Fig. 10A shows the uptake of [³H]succinate (80 nM), [¹⁴C]pyruvate (135 µM), and [¹⁴C]citrate (35 µM) in vector-transfected (V) and rat NaCT cDNA-transfected (NaCT) HRPE cells. Fig. 10B is a time course of [¹⁴C]citrate (18 µM) uptake in vector-transfected (O) and rat NaCT cDNA-transfected (●) HRPE cells. Fig. 10C shows the influence of extracellular pH on the uptake of [¹⁴C]citrate (7 µM) that was mediated specifically via rat NaCT.

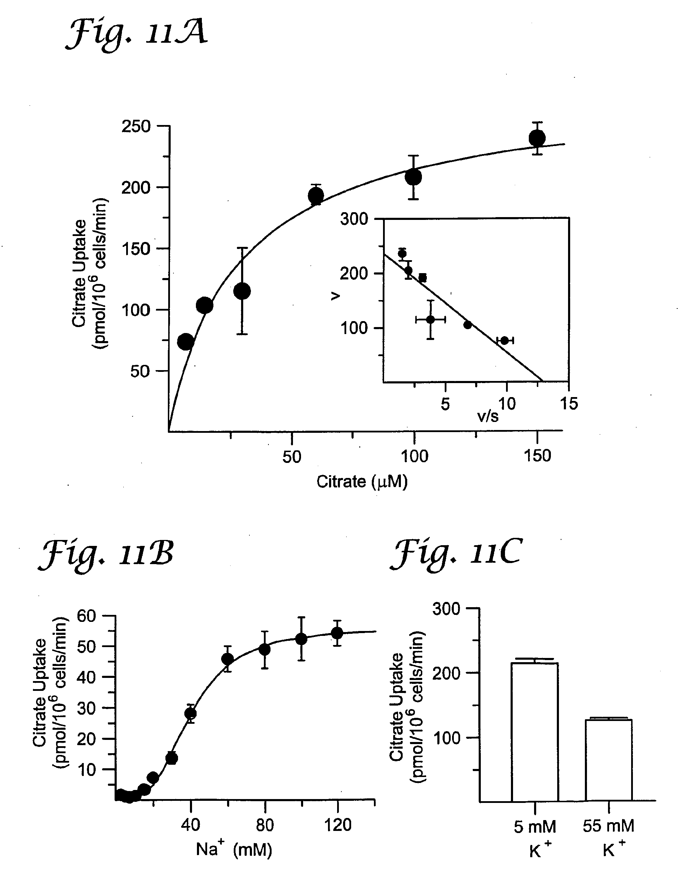

Figure 11. Influence of substrate concentration, sodium concentration, and membrane potential on citrate uptake mediated by rat NaCT. Fig. 11A shows saturation kinetics of citrate uptake via rat NaCT. Inset shows Eadie-Hofstee plot (v, citrate uptake in pmol/10⁶ cells/minute; s, citrate concentration in µM). Fig. 11B shows the dependence of rat NaCT-mediated citrate (7 µM) uptake on Na⁺ concentration. Fig. 11C shows uptake of citrate (20 µM) by rat NaCT under normal (5 mM K⁺) and membrane depolarizing (55 mM K⁺) conditions.

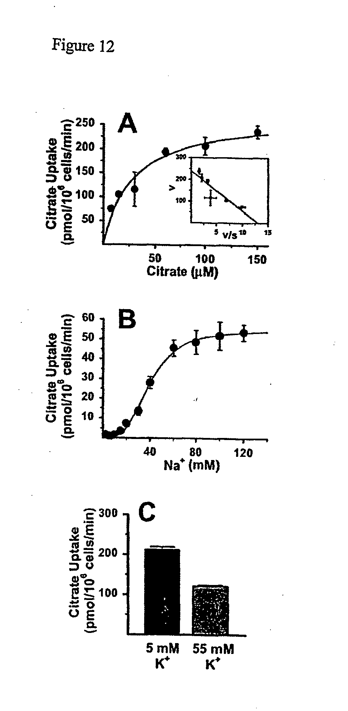

Figure 12. Substrate selectivity of rat NaCT and NaDC3. Fig. 12A shows inhibition of rat NaCT-mediated [¹⁴C]citrate (14 µM) uptake by increasing concentrations of citrate (●), succinate (○), cis-aconitate (▼), fumarate (□), α-ketoglutarate (△), and isocitrate (■). Uptake measured in the absence of inhibitors was taken as 100%. Fig. 12B shows inhibition of rat NaDC3- mediated [³H]succinate (80 nM) uptake by increasing concentrations of succinate (O), fumarate, (D), α-ketoglutarate (Δ), citrate (●), isocitrate **(■),** and cis-aconitate (▼). Uptake measured in the absence of inhibitors was taken as 100%.

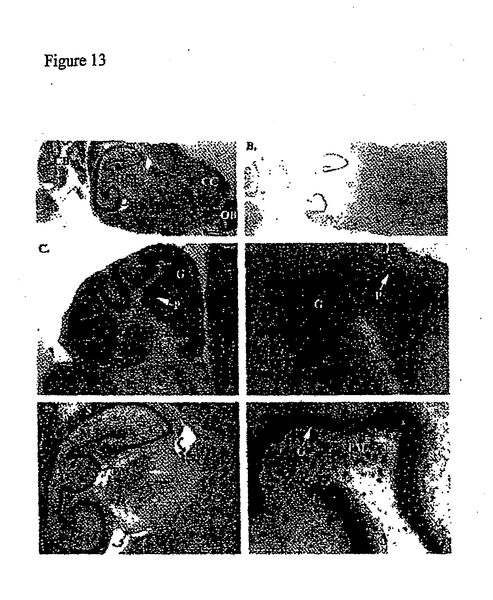

Figure 13. Analysis of expression pattern of NaCT mRNA in mouse brain by *in situ* hybridization. Fig. 13A, hybridization with an antisense riboprobe specific for mouse NaCT. CC, cerebral cortex; OB, olfactory bulb; HCF, hippocampal formation; CB, cerebellum. Fig. 13B, hybridization with a sense riboprobe specific for mouse NaCT (negative control). Fig. 13C and Fig. 13D, higher power magnification of cerebellar region hybridized with the antisense probe. M, molecular layer with stellate and basket cells; P, Purkinje cell layer; G, granular layer; W, white matter; DCN, deep cerebellar nuclei. Fig. 13E and Fig. 13F, higher power magnification of hippocampal formation region hybridized with the antisense probe. M, molecular layer of dentate gyrus; G, granulate layer of dentate gyrus; PM, polymorphic layer of dentate gyrus; CA, cornu ammonis neurons; S, subiculum.

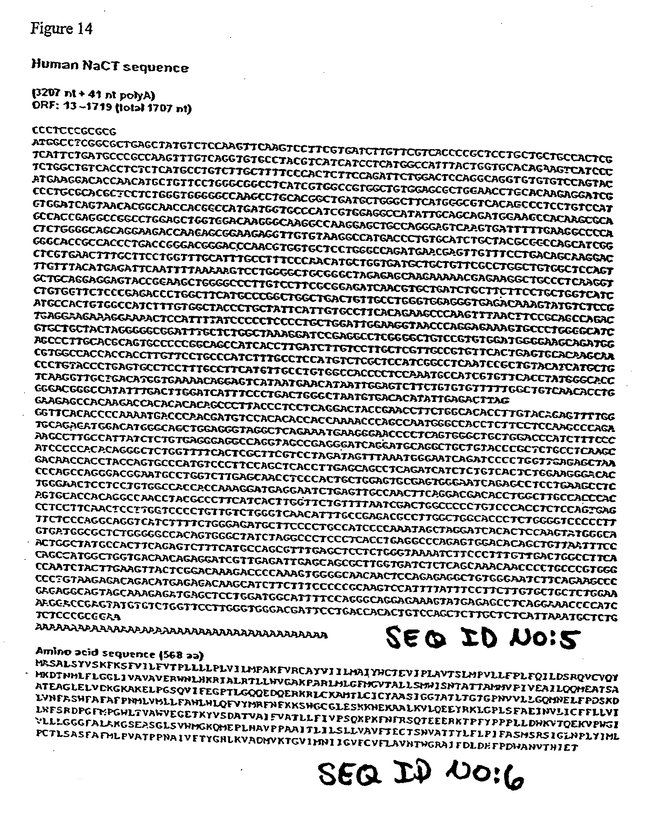

Figure 14. The full-length cDNA nucleotide sequence (SEQ ID NO:5) and translated amino acid sequence (SEQ ID NO:5) of human NaCT transporter.

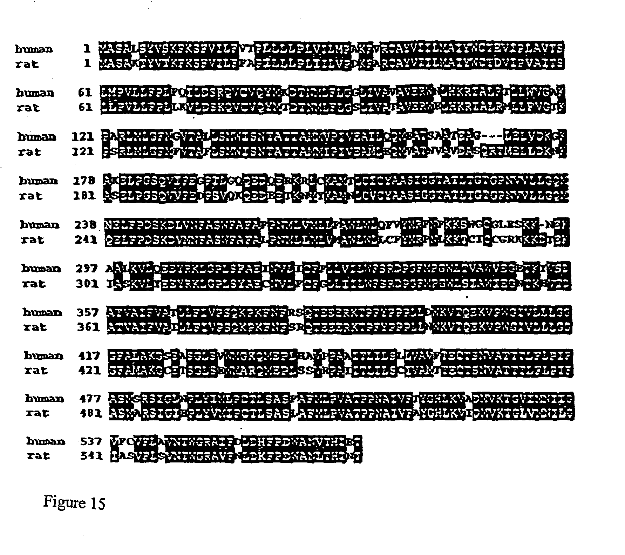

Figure 15. Alignment of amino acid sequence of human NaCT (SEQ ID NO:6) with that of rat NaCT (SEQ ID NO:4). Regions of sequence similarity are shaded.

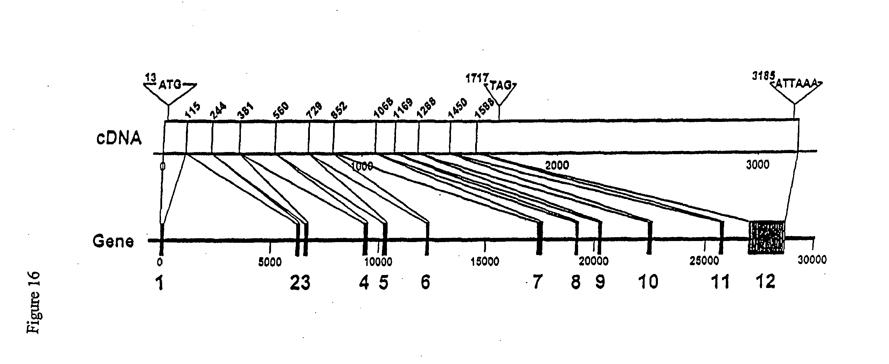

Figure 16. Exon-intron organization of the human *nact* gene. Exons are numbered in bold in the gene. The other numbers in the gene show the relative positions of the exons and introns in the approximately 30 kb gene. The shaded areas in exon 1 and exon 12 denote the 5'- and 3'-untranslated regions.
   Numbers in the cDNA indicate the nucleotide positions of the splice junctions. The exact length of the first exon is not known because of lack of information on the transcription start site.

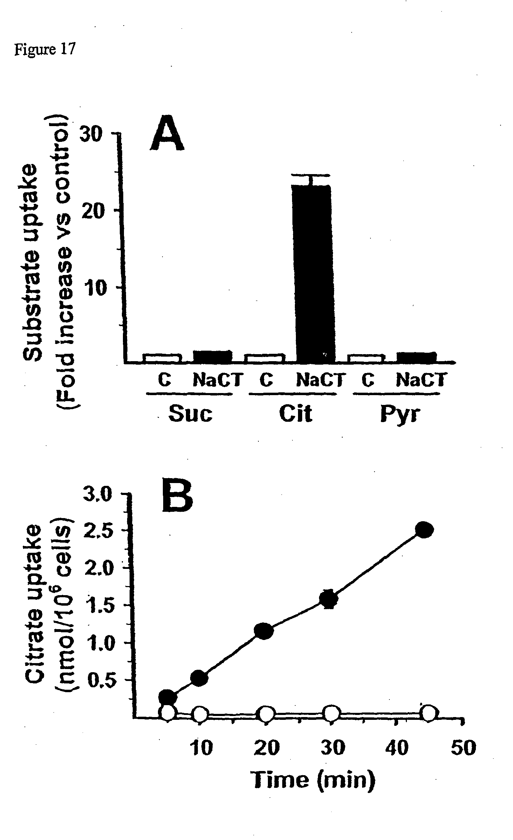

Figure 17. Transport of monocarboxylates, dicarboxylates, and tricarboxylates by NaCT. Figure 17A represents Relative abilities of human NaCT to transport monocarboxylates, dicarboxylates, and tricarboxylates. Uptake of [¹⁴C]-pyruvate (100 µM), [³H]-succinate (80 nM), and [¹⁴C]-citrate (20 µM) was measured in vector-transfected (V) and human NaCT cDNA-transfected (NaCT) HRPE cells. Figure 17B is a time course of citrate uptake mediated by human NaCT. Uptake of [¹⁴C]-citrate (20 µM) was measured in vector-transfected (O) and human NaCT cDNA-transfected (●) HRPE cells.

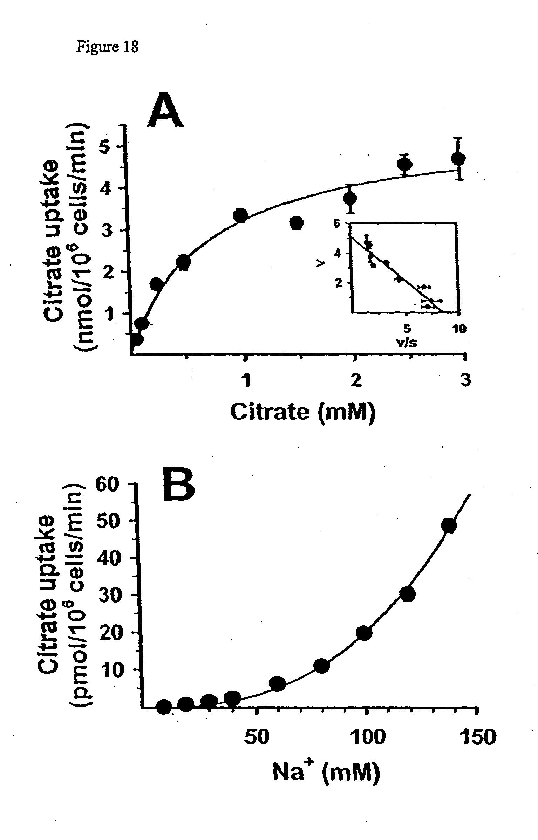

Figure 18. Kinetics of citrate transport. Figure 18A shows saturation kinetics of citrate uptake via human NaCT. Inset: Eadie-Hofstee plot (v, citrate uptake in nmol/10⁶ cells/min; s, citrate concentration in mM). Figure 18B shows dependence of human NaCT-mediated citrate (20 µM) uptake on Na⁺ concentration.

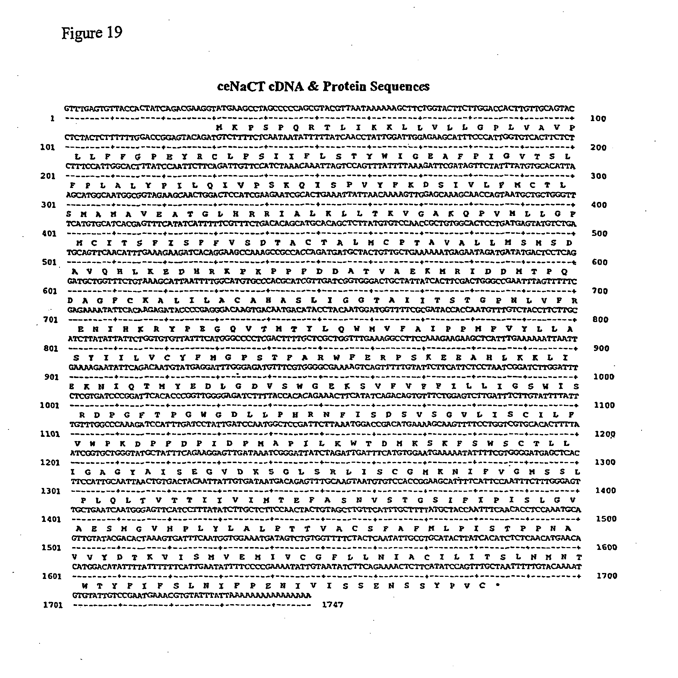

Figure 19. The full-length cDNA nucleotide sequence (SEQ ID NO:7) and translated amino acid sequence (SEQ ID NO:8) of *C*. *elegans* NaCT.

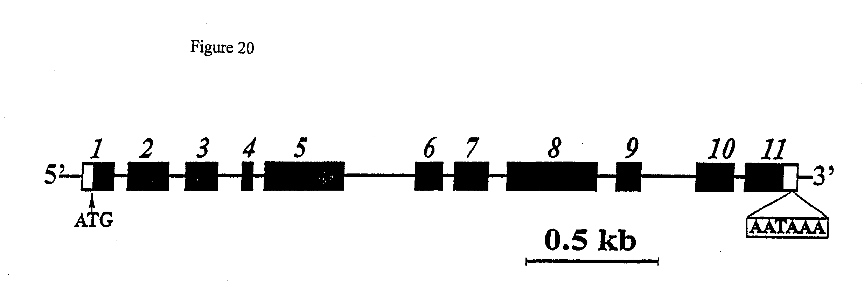

Figure 20. Structure of the *C*. *elegans nact* gene. Exons are indicated by filled boxes and numbered accordingly; introns, by solid lines. The untranslated regions in exon 1 and exon 11 are indicated by blank boxes. The consensus polyadenylation signal AATAAA is also shown. Sizes and positions of the exons and the introns are drawn to the exact scale.

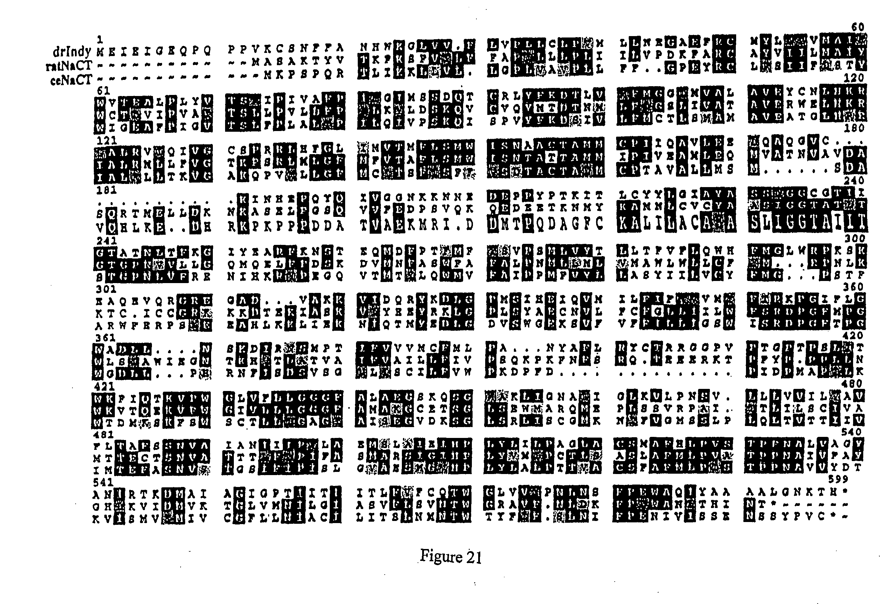

Figure 21. Amino acid sequence similarity among NaCTs from *Drosophila* (SEQ ID NO:2), *C. elegans* (SEQ ID NO:8), and rat (SEQ ID NO:4).

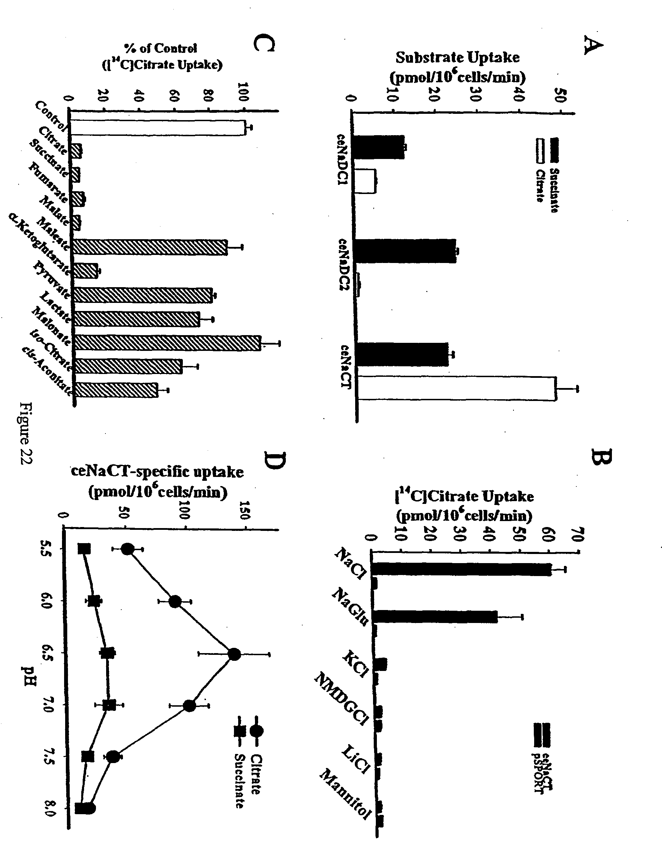

Figure 22. Functional characteristics of *C*. *elegans* NaCT in a mammalian cell expression system. Fig. 22A is a comparison of the transport activities of NaDC1, NaDC2 and NaCT from C. *elegans.* Uptake of 10 µM citrate or 10 µM succinate was measured in HRPE cells transfected with the transporter cDNAs. Uptake measured in the vector (pSPORT)-transfected cells served as a control for endogenous uptake activity. Values (cDNA-specific activity) represent means ± S.E. for four determinations. Fig. 22B shows ion-dependence of *C. elegans* NaCT-mediated citrate uptake in HRPE cells. Uptake of 10 µM citrate was measured in buffers containing 140 mM Na⁺, Li⁺, K⁺, and NMDG (as chloride salts), or 300 mM mannitol. Values represent means ± S.E. for four determinations. Fig. 22C shows substrate specificity of ceNaCT-mediated uptake. Uptake of 10 µM [¹⁴C]citrate was measured in the absence or presence of potential inhibitors (2.5 mM) in cells transfected with vector alone or ceNaCT cDNA. The cDNA-specific uptake was calculated by adjusting for the uptake in vector-transfected cells. The cDNA-specific uptake in the absence of inhibitors was taken as the control (100%) and the uptake in the presence of inhibitors is given as percent of this control value. Fig. 22D shows influence of extracellular pH on *C. elegans* NaCT-mediated citrate or succinate (10 µM) uptake in HRPE cells.

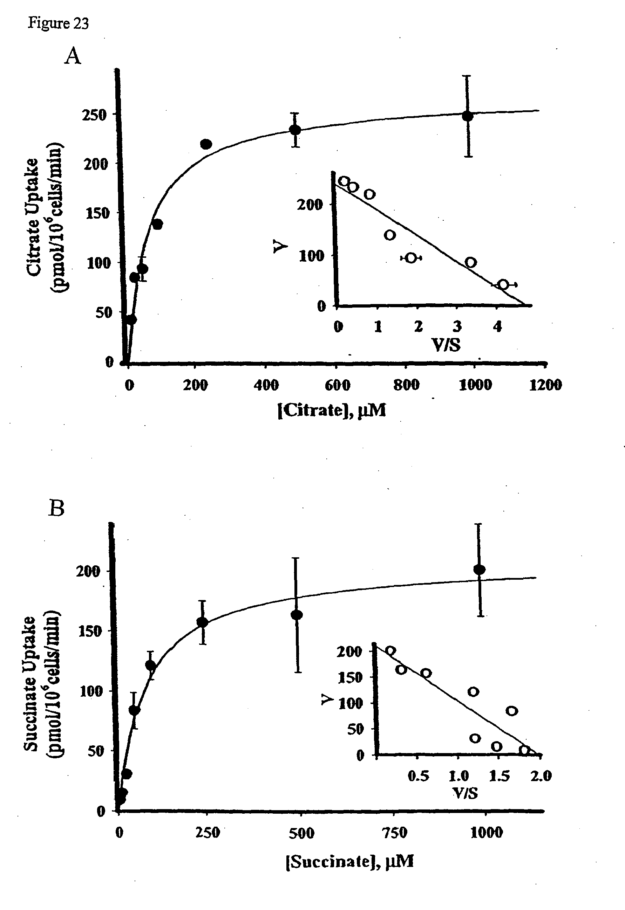

Figure 23. Saturation kinetics of citrate and succinate uptake mediated by *C*. *elegans* NaCT in HRPE cells. Uptake of citrate (Fig. 23A) or succinate (Fig. 23B) was measured in a NaCl-containing medium (pH 7.5) over a substrate concentration range of 10-1000 µM in cells transfected with vector or *C. elegans* NaCT cDNA. The cDNA-specific uptake was calculated by adjusting for the endogenous uptake measured in vector-transfected cells. Values represent means ± S.E. for four determinations. Insets are the Eadie-Hofstee transformation of the data.

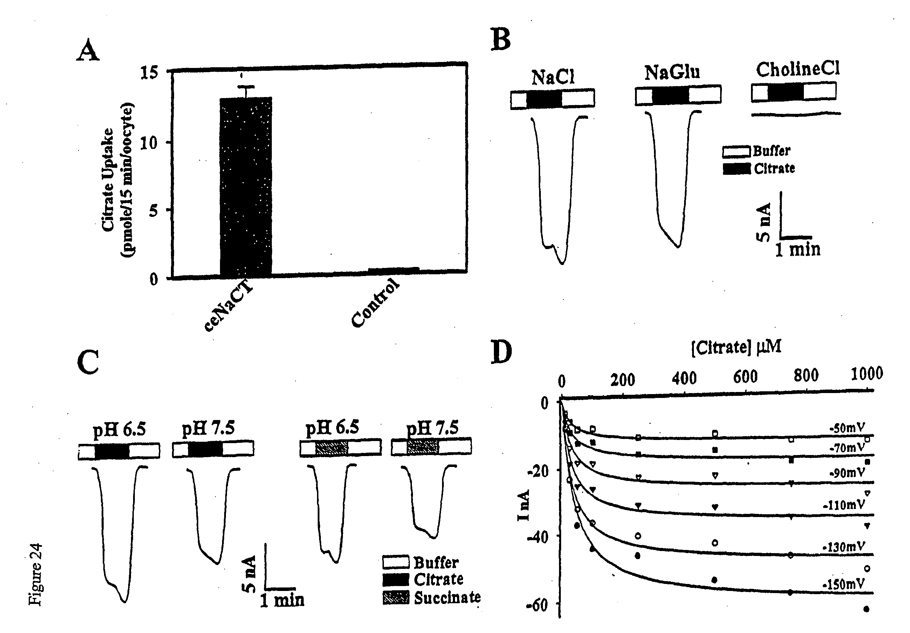

Figure 24. Functional characteristics of *C. elegans* NaCT in *Xenopus* oocyte expression system. In Fig. 24A uptake of [¹⁴C]-citrate (40 µM) was measured in control (water-injected) oocytes and in oocytes injected with *C. elegans* NaCT cRNA at pH 6.5 in the presence of NaCl. Values represent mean ± S.E (*n* =8-10 oocytes). Fig. 24B shows ion-dependence of the citrate-evoked currents under voltage-clamp conditions in oocytes expressing *C. elegans* NaCT. Oocytes were sequentially superfused with 250 µM of citrate in a Na⁺-containing buffer (NaCl), or in a chloride-free buffer (NaGlu) in which NaCl was replaced isoosmatically with sodium gluconate or in a Na⁺-free buffer (CholineCi) in which NaCl was replaced isoosmotically with choline chloride. Fig. 24C shows the effects of pH on substrate (250 µM)-induced currents in oocytes expressing *C. elegans* NaCT. Fig. 24D is a kinetic analyses of citrate-evoked inward currents in oocytes expressing *C. elegans* NaCT at different testing membrane potentials. The perifusion buffer (pH 6.5) contained NaCl.

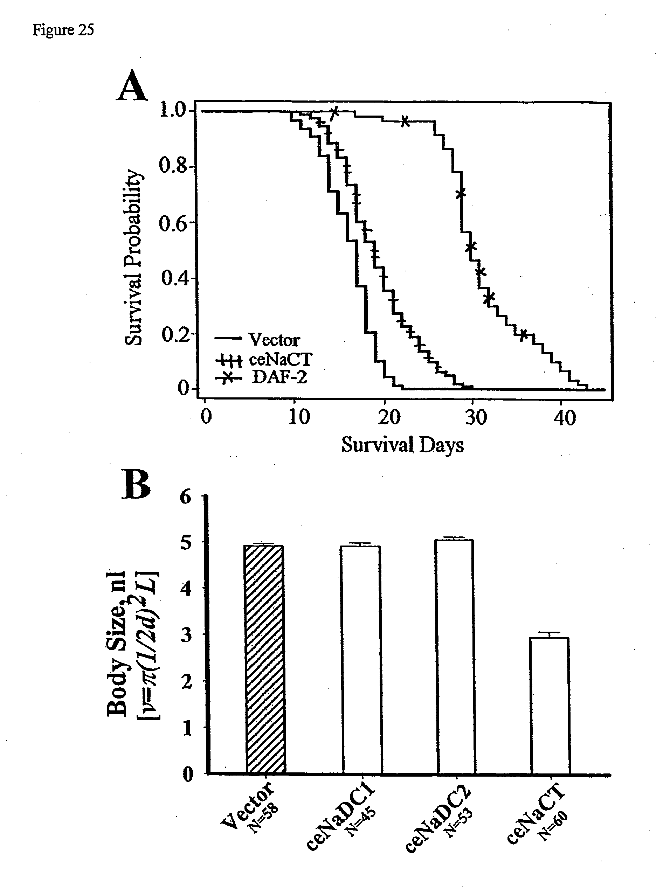

Figure 25. Effect of the knockdown of NaCT by RNAi on life span and body size in *C*. *elegans*. Fig. 25A shows the effect of the knockdown of NaCT by RNAi on life span in *C*. *elegans*. The knockdown of NaCT was done by feeding the worms with bacteria producing NaCT-specific dsRNA. The knockdown of DAF-2 was included as a positive control. Worms fed on bacteria carrying the empty vector pPD129 served as the wild type control. The curves show the survival probability of the worms in different experimental groups at a given day after hatching under the influence of the gene-specific dsRNAs. Fig. 25B is a graphical representation of the effect of the knockdown of NaCT by RNAi on body size in *C*. *elegans.* Results from gene-specific RNAi for ceNaDC1 and ceNaDC2 have been included.

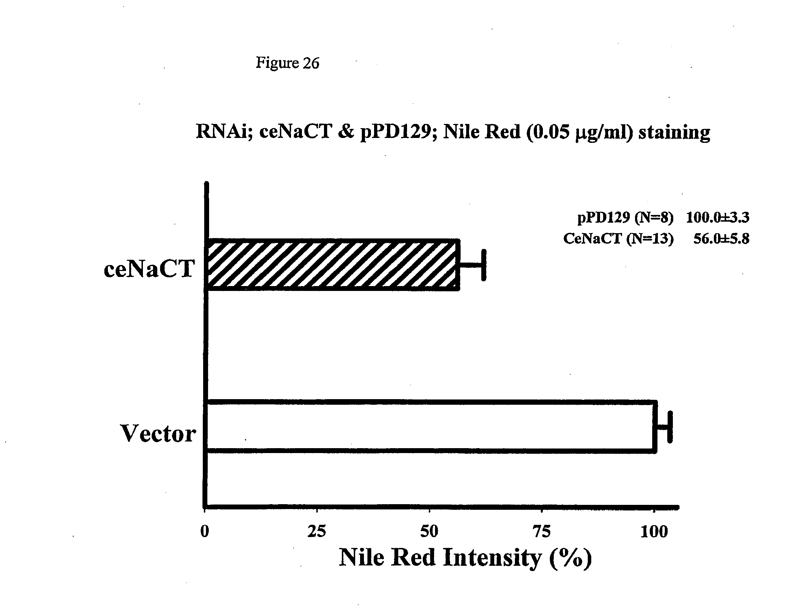

Figure 26. Effect of NaCT knockdown on fat deposition in *C*. *elegans.* Comparison of the fluorescence intensity of the Nile red staining between worms with NaCT knockdown by RNAi (hatched bar, N=13) and control worms (empty bar, N=80). The intensity in control worms was taken as 1.

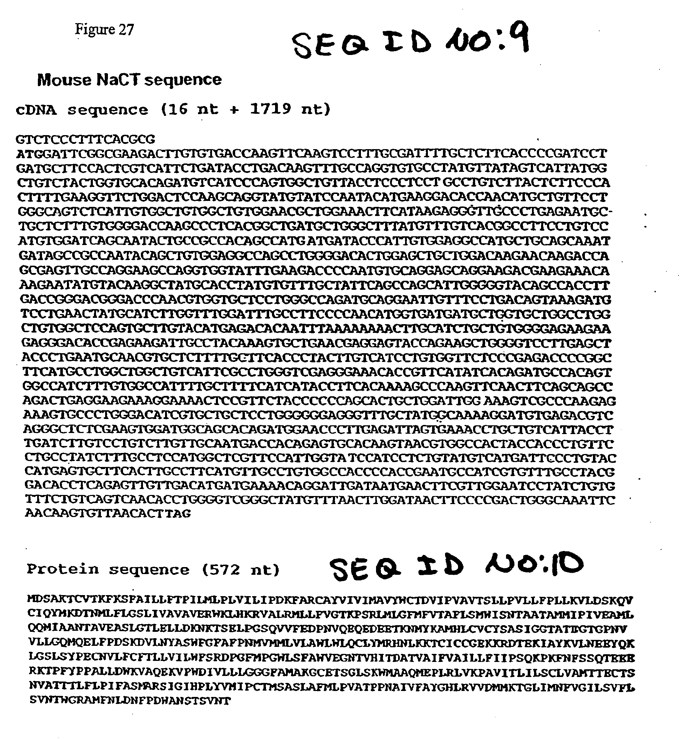

Figure 27. The full-length cDNA nucleotide sequence (SEQ ID NO:9) and translated amino acid sequence (SEQ ID NO:10) of mouse NaCT.

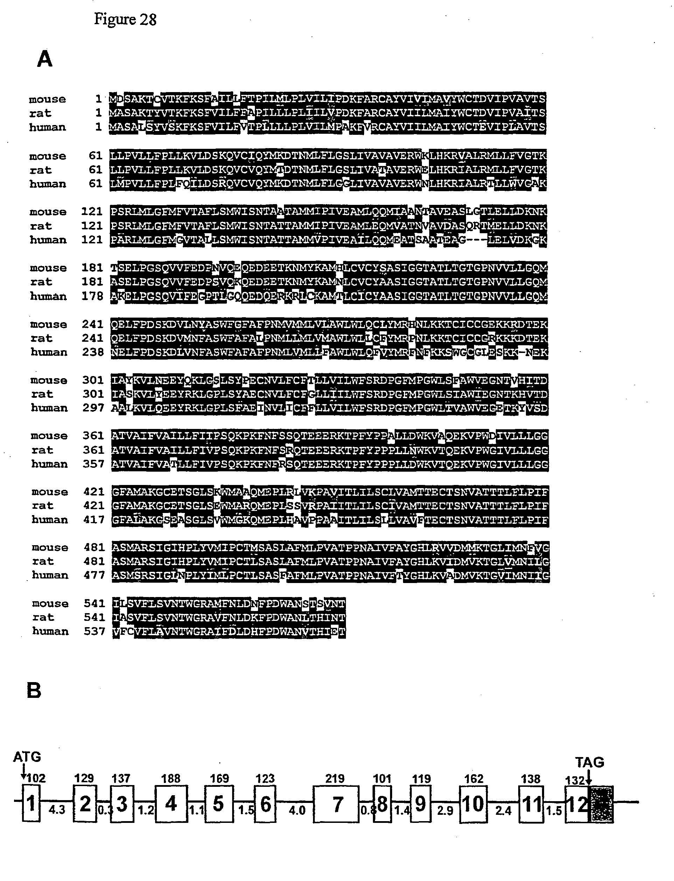

Figure 28. Amino acid sequence of mouse NaCT and the exon-intron organization of murine *nact* gene. Fig. 28A shows a comparison of the primary structure of mouse NaCT (SEQ ID NO:10) with that of rat (SEQ ID NO:4) and human (SEQ ID NO:6) NaCTs. Identical amino acids are indicated by dark shading and conserved amino acid substitutions are indicated by light shading. In Fig. 28B, exons, identified by boxes, are numbered in the gene and numbers above the exon boxes indicate the number of base pairs in respective exons. The numbers associated with the introns indicate the size of the respective introns in kilobase pairs. The shaded region in exon 12 denotes the 3'-untranslated region.

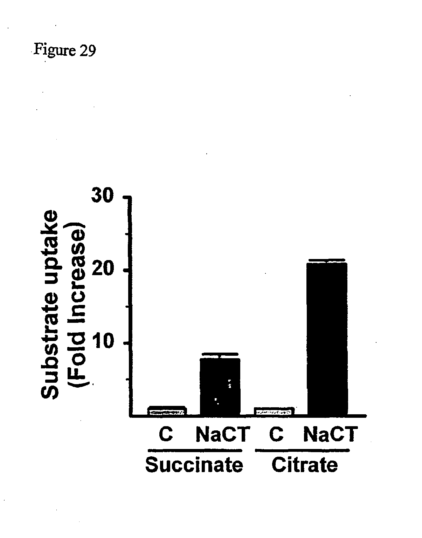

Figure 29. Uptake of succinate and citrate via mouse NaCT. HRPE cells were transfected with either vector alone (C) or mouse NaCT cDNA (NaCT). Uptake of [³H]-succinate (50 nM) and [¹⁴C]-citrate (20 µM) was measured in transfected cells. The uptake of each substrate measured in control cells was taken as 1 and the corresponding uptake in cDNA-transfected cells is given as a ratio (-fold increase) in comparison with this control uptake.

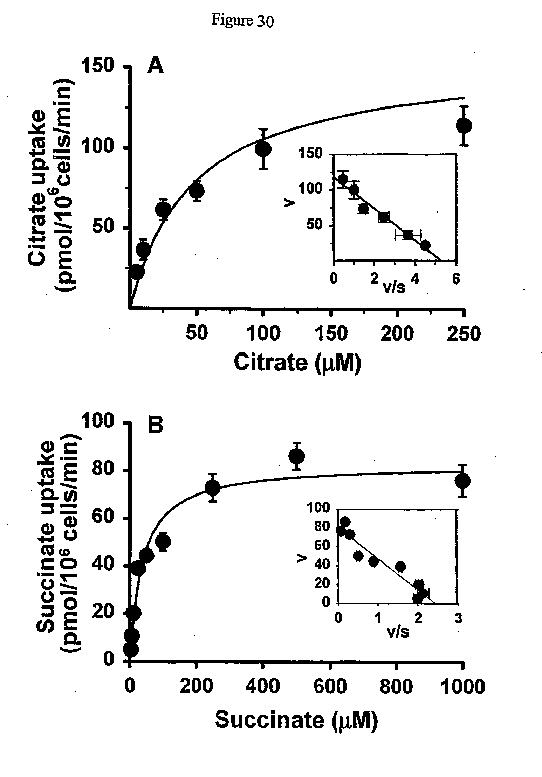

Figure 30. Kinetics of mouse NaCT-mediated citrate and succinate uptake. HRPE cells were transfected with either vector alone or mouse NaCT cDNA and uptake of citrate (Fig. 30A) and succinate (Fig. 30B) was measured in these cells. The concentration range was 5-250 µM for citrate and 2.5-1000 µM for succinate. The uptake measured in vector-transfected cells was subtracted from the corresponding uptake measured in cDNA-transfected cells to determine the cDNA-specific uptake. Only uptake values that are specific for mouse NaCT were used in kinetic analysis. Insets, Eadie-Hosftee plots: *V*/*S* (uptake rate/substrate concentration) *versus V* (uptake rate).

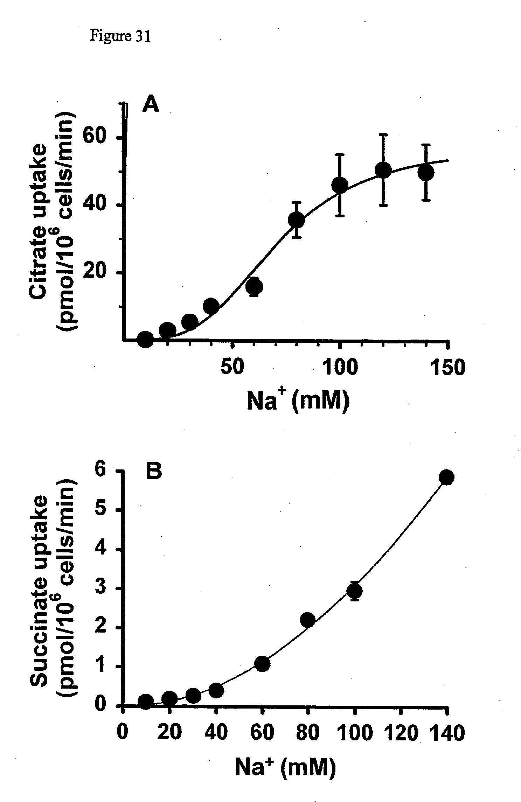

Figure 31. Na⁺-activation kinetics of citrate and succinate uptake mediated by mouse NaCT. HRPE cells were transfected with either vector alone or mouse NaCT cDNA and uptake of citrate (20 µM) (Fig. 31A) and succinate (2.5 µM) (Fig. 31B) was measured in these cells. Concentration of Na⁺ was varied over the range of 10-140 mM by adjusting the concentrations of NaCl and *N*-methyl-D-glucamine chloride appropriately to maintain the osmolality. The uptake measured in vector-transfected cells was subtracted from the corresponding uptake measured in cDNA-transfected cells to determine the cDNA-specific uptake. Only the uptake values specific for mouse NaCT were used in kinetic analysis.

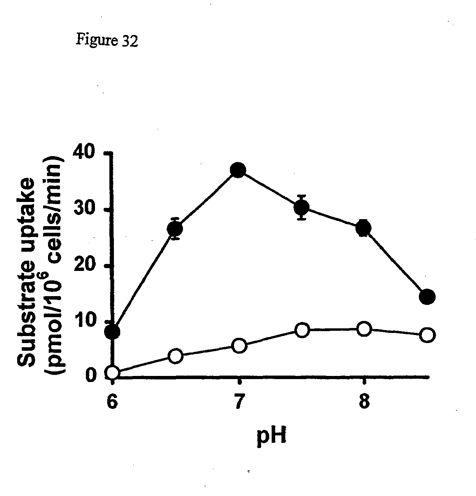

Figure 32. Influence of extracellular pH on mouse NaCT-mediated uptake of citrate and succinate. HRPE cells were transfected with either vector alone or mouse NaCT cDNA and uptake of citrate (10 µM) (●) and succinate (10 µM) (O) was measured in these cells. The pH of the uptake buffer was varied by appropriately adjusting the concentrations of Mes, Hepes, and Tris. The uptake measured in vector-transfected cells was subtracted from the corresponding uptake measured in cDNA-transfected cells to determine the cDNA-specific uptake. Data represent only the cDNA-specific uptake.

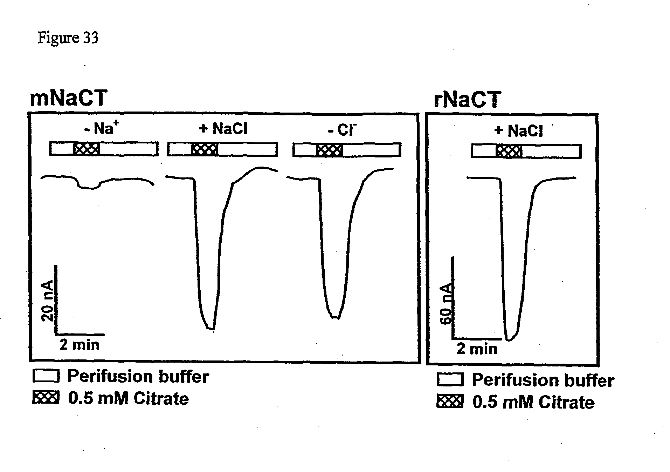

Figure 33. Electrogenicity of mouse and rat NaCTs. Mouse and rat NaCTs were expressed functionally in *X*. *laevis* oocytes by injection of respective cRNAs. Citrate-induced currents were monitored in these oocytes using the two-microelectrode voltage-clamp technique. The membrane potential was clamped at - 50 mV. The perfusion buffer contained *N*-methyl-D-glucamine chloride (-Na⁺), NaCl, or sodium gluconate (-Cl⁻).

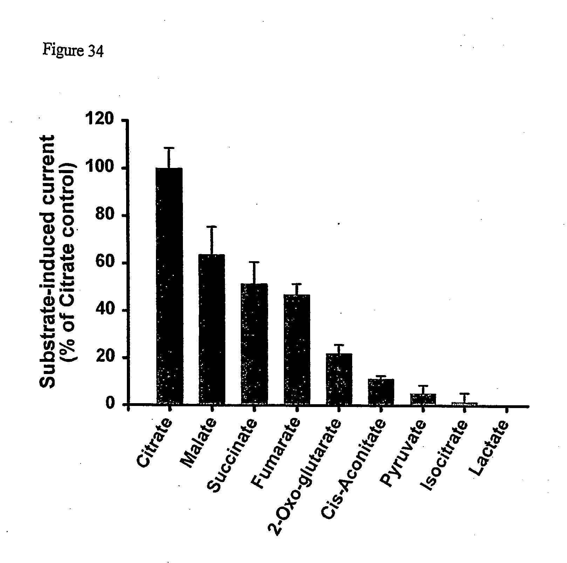

Figure 34. Relative ability of rat NaCT to transport various citric acid cycle intermediates and other related compounds. Rat NaCT was expressed functionally in *X. laevis* oocytes by injection of cRNA. The oocytes were perifused with various monocarboxylates, dicarboxylates, and tricarboxylates (0.5 mM) and the substrate-induced inward currents were monitored using the two-microelectrode voltage-clamp technique. The currents induced by various substrates are given as percent of the current induced by citrate. The data are from three different oocytes and the citrate-induced current in each oocyte was normalized by taking this value as 100%. The value for citrate-induced current in three different oocytes was 87 ± 8 nA.

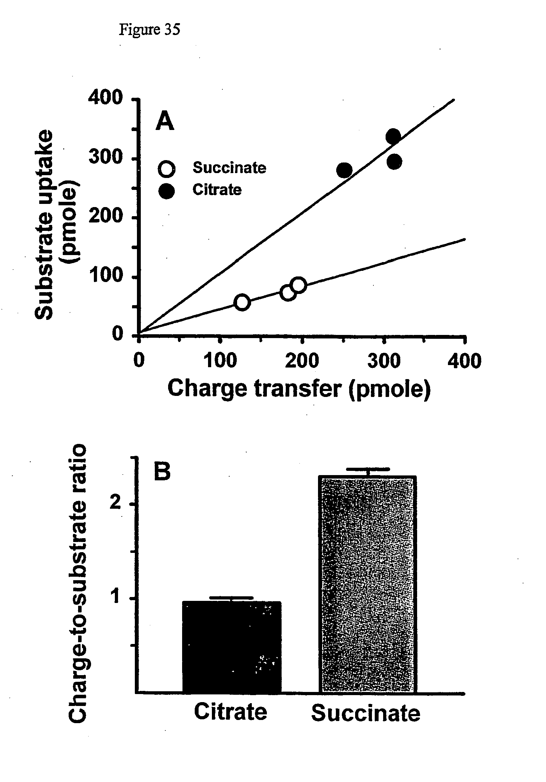

Figure 35. Determination of charge-to-substrate ratio for rat NaCT with citrate and succinate as substrates. Rat NaCT was expressed functionally in *X. laevis* oocytes by injection of cRNA. The oocytes were perifused with 50 µM citrate or succinate (radiolabeled plus unlabeled substrates) for 10 minutes and the substrate-induced currents were monitored in these oocytes using the two-microelectrode voltage-clamp technique. The membrane potential was clamped at - 50 mV. At the end of the experiment, the oocytes were washed with the perfusion buffer and the radioactivity associated with the oocytes was determined. The quantity of charge transferred into the oocytes during perfusion with the substrates was determined from the integration of the area covered by the time *versus* inward current curves and the quantity of the substrates transferred into the oocytes was determined from the radioactivity associated with the oocytes. Fig. 35A shows the relationship between substrate uptake and charge transfer for citrate and succinate in three different oocytes. Fig. 35B shows the charge-to-substrate ratio for citrate and succinate.

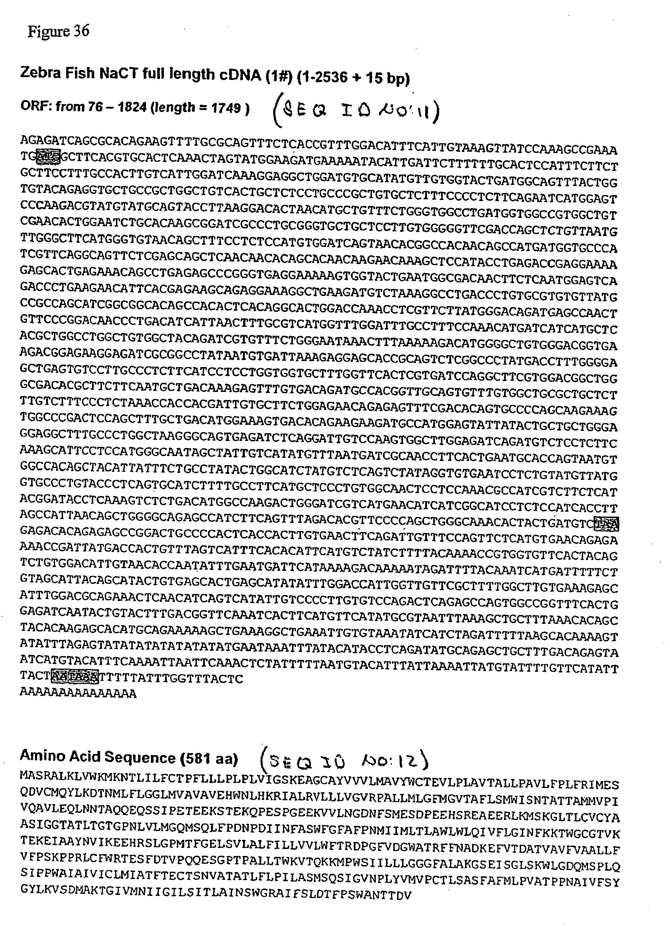

Figure 36. The full-length cDNA nucleotide sequence (SEQ ID NO:11) and translated amino acid sequence (SEQ ID NO:12) of zebrafish NaCT.

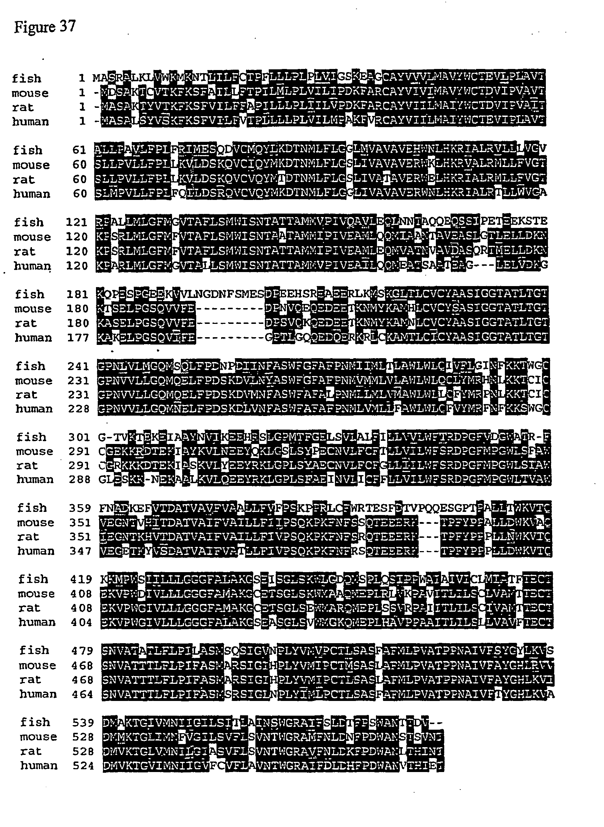

Figure 37. Comparison of the amino acid sequence of zebrafish NaCT (SEQ ID NO:12) with that of rat (SEQ ID NO:4), mouse (SEQ ID NO:10), and human (SEQ ID NO:6).

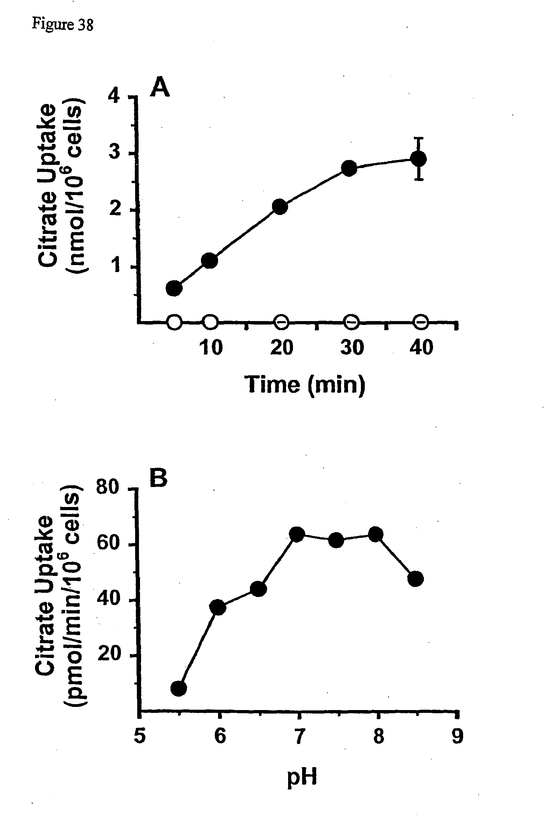

Figure 38. Citrate uptake by cells transfected with zebrafish NaCT. Fig. 38A shows a time course of citrate (2 µM) uptake in cells transfected with either vector alone (O) or zebrafish NaCT cDNA (●). Fig. 38B demonstrates the influence of pH on citrate (1 µM) uptake mediated by zebrafish NaCT.

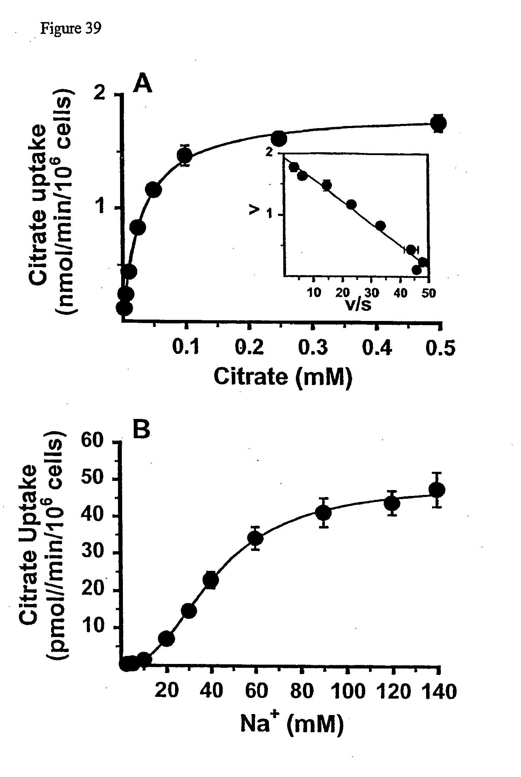

Figure 39. Saturation kinetics (Fig. 39A) and Na⁺-activation kinetics (Fig. 39B) of citrate uptake mediated by zebrafish NaCT. The Michaelis constant for citrate uptake is 40 ± 4 µM. The value for Hill coefficient for the activation of uptake is 26 ± 0.2.

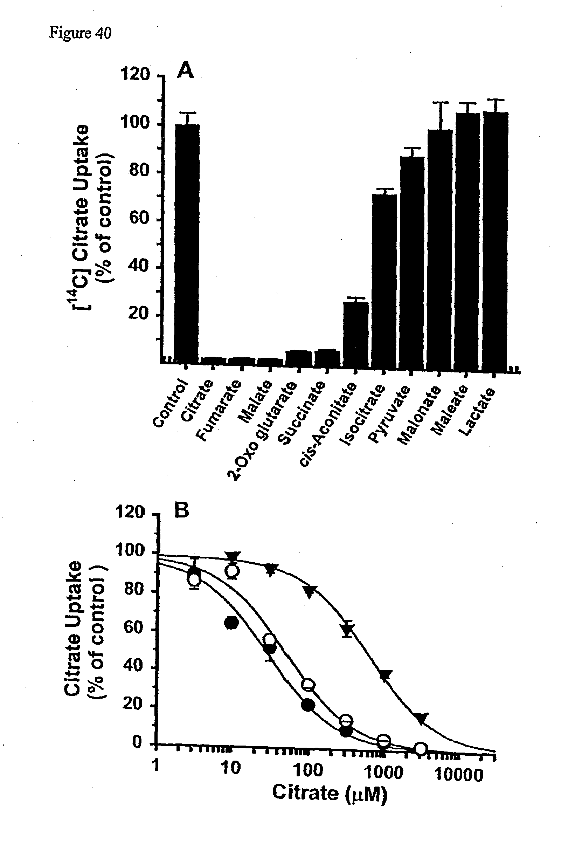

Figure 40. Inhibition of citrate uptake. Fig. 40A shows the inhibition of zebrafish NaCT-mediated [¹⁴C]-citrate (1 µM) uptake by various structural analogs (2mM). Fig. 40B demonstrates dose-response relationships for inhibition of zebrafish NaCT-mediated [¹⁴C]-citrate (1 µM) uptake by citrate (●), succinate (○), and cis-aconitate (▼). The IC₅₀ values for the inhibition are 30 ± 4,51 ± 9, and 624 ± 45 µM, respectively, for citrate, succinate , and cis-aconitate.

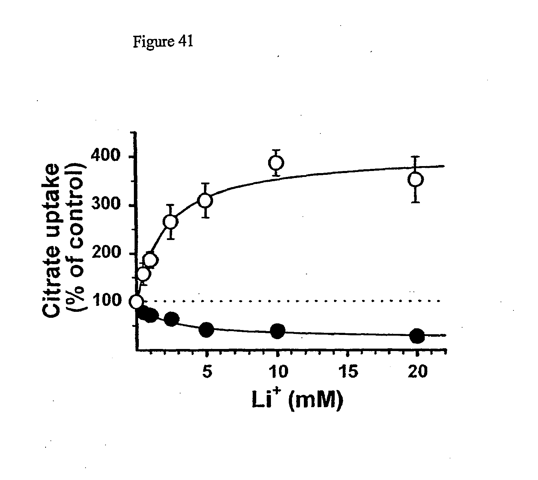

Figure 41. Differential effect of Li⁺ on the uptake of citrate (20 µM) via rat NaCT (●) and human NaCT (O).

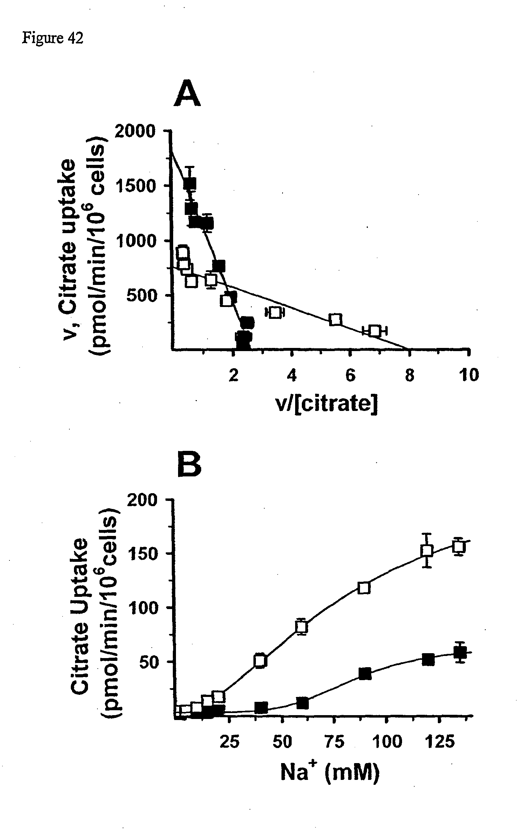

Figure 42. Substrate saturation kinetics and Na⁺-activation kinetics for human NaCT. Fig. 42A shows substrate saturation kinetics of human NaCT in the absence (■) and presence (□) of 10 mM Li⁺. Fig. 42B shows Na⁺-activation kinetics of human NaCT in the absence (■) and presence (□) of 10 mM Li⁺.

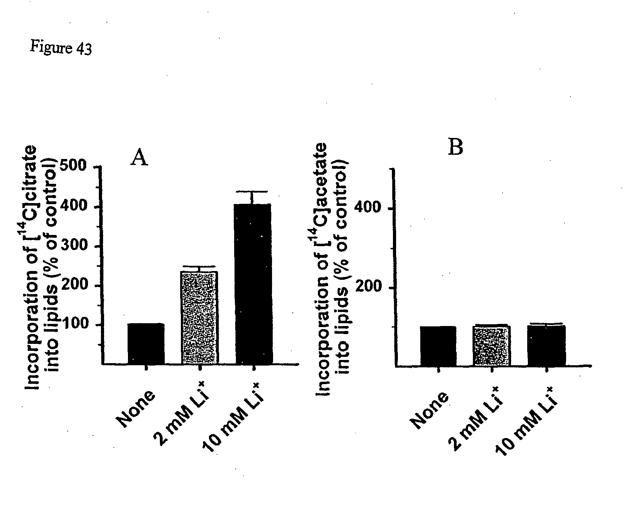

Figure 43. Incorporation of citrate and acetate into lipids in HepG2 cells in the absence and presence of Li⁺. Fig. 43A is a histogram showing the incorporation of [¹⁴C]citrate in HepG2 cells in the absence of Li⁺, or in the presence of 2 or 10 mM Li⁺. Fig. 43B is a histogram showing the incorporation of [¹⁴C]acetate into lipids for the same concentrations of Li⁺.

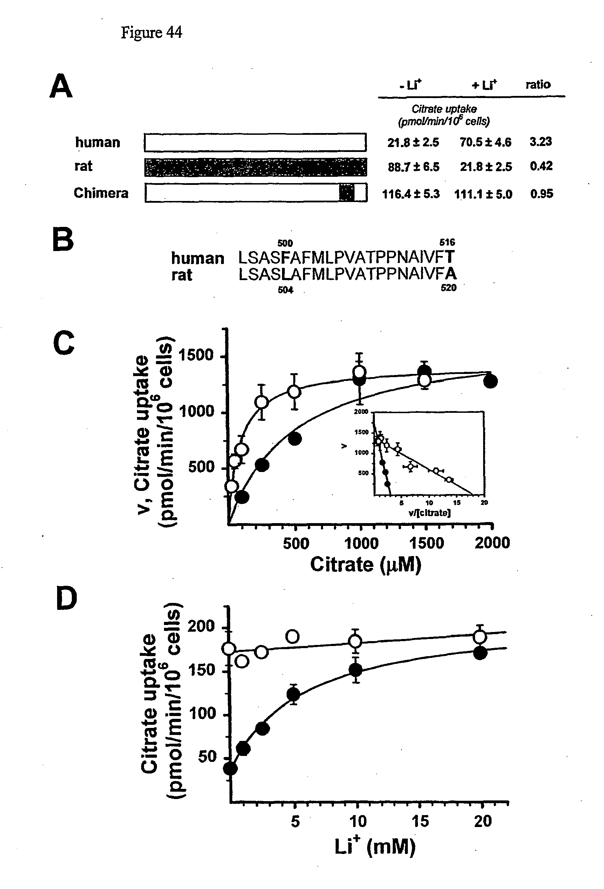

Figure 44. Structure-function relationship for NaCT. Fig. 44A demonstrates the influence of Li⁺ (10 mM) on the uptake of citrate (20 µM) via wild type human and rat NaCTs and the chimeric transporter in which the region containing the amino acids 496-516 in human NaCT has been replaced with the corresponding region from rat NaCT. Fig. 44B compares the amino acid sequences between human NaCT (amino acids 496-516) and rat NaCT (amino acids 500-520). The amino acids that are different between human and rat NaCTs are identified in bold. Fig. 44C shows substrate saturation kinetics of wild type human NaCT (●) and the Phe→Leu mutant of human NaCT (O). Fig. 44D demonstrates the influence of increasing concentrations of Li⁺ on the uptake of citrate (20 µM) via wild type human NaCT (●) and the Phe→Leu mutant of human NaCT (O).

### DETAILED DESCRIPTION OF ILLUSTRATIVE EMBODIMENTS OF THE INVENTION

With the present invention, a new transmembrane citrate transporter has been identified and functionally characterized. This transporter efficiently transports citrate. As citrate transport by this transporter is Na⁺-couple, this transmembrane transporter is herein designated as "NaCT" for "Na⁺-coupled citrate transporter." The NaCT polypeptide of the present invention is involved in the utilization of extracellular citrate for the synthesis of fatty acids and cholesterol. The NaCT polypeptide of the present invention will serve as a drug target for the treatment of obesity, hyperlipidemia, and hypercholesterolemia.

The present invention also provides the functional characterization of *Drosophila* Indy (DrIndy) as a citrate transporter and identifies DrIndy as the *Drosophila* ortholog of mammalian NaCT.

### Polypeptides:

As used herein, a "NaCT polypeptide" demonstrates one or more of the functional activities of a Na⁺-coupled transmembrane citrate transporter. Each of these functional activities of a NaCT polypeptide, and the assays for measuring these functional activities, are described in more detail herein. Briefly, the functional activities of a NaCT polypeptide include, but are not limited to, one or more of the following. A NaCT polypeptide may demonstrate the transmembrane transport of citrate. A NaCT polypeptide may demonstrate Na⁺-dependent transmembrane transport of citrate. A NaCT polypeptide may demonstrate Na⁺-dependent transmembrane transport of citrate that is modulated by Li⁺. Such modulation includes, but is not limited to, the stimulation of citrate transport and the inhibition of citrate transport. The Na⁺-dependent transmembrane transport of citrate by a NaCT polypeptide may demonstrate a requirement for multiple Na⁺ ions for transport coupling. The stoichiometry of this coupling may be, for example, 2:1, 3:1, 4:1, or 5:1. A NaCT polypeptide may demonstrate transmembrane transport of citrate that is electrogenic.

A NaCT polypeptide may include a sodium symporter family signature motif. The consensus pattern for such a sodium symporter family signature motif is: (S)SXXFXXP(V)(G)XXXNX(I)V (SEQ ID NO:29), wherein X denotes any amino acid residue, (S) denotes serine or other related amino acids, such as alanine, cysteine, threonine, or proline, (V) denotes valine or other related amino acids, such as leucine, isoleucine or methionine, (G) denotes glycine or other related amino acids, such as serine or alanine, and (I) denotes isoleucine or other related amino acids, such as leucine, valine, or methionine. The sodium symporter family is a group of integral membrane proteins that mediate the cellular uptake of a wide variety of molecules including di- or tricarboxylates and sulfate by a transport mechanism involving sodium cotransport (Pajor, Annu Rev Physiol (1999);61: 663-682 and Pajor, J Membr Biol (2000);175:1-8)).

As used herein, a "citrate transporter polypeptide" demonstrates transmembrane transport of citrate. The transmembrane transport of citrate by a citrate transporter polypeptide need not be Na⁺-coupled.

The NaCT polypeptides of the present invention may be derived from a variety of species, including, but not limited to, human, primate, rat, mouse, *C. elegans,* and zebrafish. For example, the NaCT polypeptides of the present invention include, but are not limited to, rat NaCT (SEQ ID NO:4), human NaCT (SEQ ID NO:6), *C. elegans* NaCT (SEQ ID NO:8), mouse NaCT (SEQ ID NO:10), and zebrafish NaCT (SEQ ID NO:12). A citrate transporter polypeptide of the present invention may be derived from a variety of species. One example of a citrate transporter polypeptide is *Drosophila* Indy (DrIndy), having SEQ ID NO:2, as described in more detail in Example 1.

The polypeptides of the present invention also include "biologically active analogs" of naturally occurring polypeptides. For example, the NaCT polypeptides of the present invention include, but are not limited to, biologically active analogs of rat NaCT (SEQ ID NO:4), human NaCT (SEQ ID NO:6), *C*. *elegans* NaCT (SEQ ID NO:8), mouse NaCT (SEQ ID NO:10), and zebrafish NaCT (SEQ ID NO:12). The citrate transport polypeptides of the present invention includes, but is not limited to, biologically active analogs of DrIndy (SEQ ID NO:2).

As used herein, a "biologically active analog" demonstrates one or more of the following functional activities; demonstrate the transmembrane transport of citrate; demonstrate Na⁺-dependent transmembrane transport of citrate; demonstrate Na⁺-dependent transmembrane transport of citrate that is modulated by Li⁺, with such modulation including, but is not limited to, the stimulation of citrate transport and the inhibition of citrate transport; demonstrate a requirement for multiple Na⁺ ions for transport coupling, where the stoichiometry of this coupling may be, for example, 2:1, 3:1, 4:1, or 5:1; and demonstrate transmembrane transport of citrate that is electrogenic. Functional activity of a NaCT polypeptide can be easily assessed using the various assays described herein as well as other assays well known to one with ordinary skill in the art. A modulation in functional activity, including the stimulation or the inhibition of functional activity, can be readily ascertained by the various assays described herein, and by assays known to one of skill in the art.

A modulation in a functional activity can be quantitatively measured and described as a percentage of the functional activity of a comparable control. The functional activity of the present invention includes a modulation that is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, at least 100%, at least 110%, at least 125%, at least 150%, at least 200%, or at least 250% of the activity of a suitable control.

For example, the stimulation of a functional activity can be quantitatively measured and described as a percentage of the functional activity of a comparable control. The functional activity of the present invention includes a stimulation that is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, at least 100%, at least 110%, at least 125%, at least 150%, at least 200%, or at least 250% of the activity of a suitable control.

For example, inhibition of a functional activity can be quantitatively measured and described as a percentage of the functional activity of a comparable control. The functional activity of the present invention includes an inhibition that is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 99%, at least 100%, at least 110%, at least 125%, at least 150%, at least 200%, or at least 250% of the activity of a suitable control.

A "biologically active analog" of a polypeptide includes polypeptides having one or more amino acid substitutions that do not eliminate a functional activity. Substitutes for an amino acid in the polypeptides of the invention may be selected from other members of the class to which the amino acid belongs. For example, it is well-known in the art of protein biochemistry that an amino acid belonging to a grouping of amino acids having a particular size or characteristic (such as charge, hydrophobicity and hydrophilicity) can be substituted for another amino acid without altering the activity of a protein, particularly in regions of the protein that are not directly associated with biological activity. Substitutes for an amino acid may be selected from other members of the class to which the amino acid belongs. For example, nonpolar (hydrophobic) amino acids include alanine, leucine, isoleucine, valine, proline, phenylalanine, tryptophan, and tyrosine. Polar neutral amino acids include glycine, serine, threonine, cysteine, tyrosine, asparagine and glutamine. The positively charged (basic) amino acids include arginine, lysine and histidine. The negatively charged (acidic) amino acids include aspartic acid and glutamic acid. Examples of such preferred conservative substitutions include Lys for Arg and vice versa to maintain a positive charge; Glu for Asp and vice versa to maintain a negative charge; Ser for Thr so that a free -OH is maintained; and GIn for Asn to maintain a free NH2. Likewise, biologically active analogs of a NaCT polypeptide containing deletions or additions of one or more contiguous or noncontiguous amino acids that do not eliminate a functional activity of a NaCT polypeptide are also contemplated.

A "biologically active analog" of a NaCT polypeptide includes "fragments" and "modifications" of a NaCT polypeptide. As used herein, a "fragment" of a NaCT polypeptide means a NaCT polypeptide that has been truncated at the N-terminus, the C-terminus, or both. A fragment may range from about 5 to about 250 amino acids in length. For example it may be about 5, about 10, about 20, about 25, about 50, about 75, about 100, about 125, about 150, about 175, about 200, about 225, or about 250 amino acids in length. Fragments of a NaCT polypeptide with potential biological activity can be identified by many means. One means of identifying such fragments of a NaCT polypeptide with biological activity is to compare the amino acid sequences of a NaCT polypeptide from rat, mouse, human and/or other species to one another. Regions of homology can then be prepared as fragments. Fragments of a polypeptide also include a portion of the polypeptide containing deletions or additions of one or more contiguous or noncontiguous amino acids such that the resulting polypeptide still retains a biological activity of the full-length polypeptide.

A "modification" of a NaCT polypeptide includes NaCT polypeptides or fragments thereof chemically or enzymatically derivatized at one or more constituent amino acid, including side chain modifications, backbone modifications, and N- and C- terminal modifications including acetylation, hydroxylation, methylation, amidation, and the attachment of carbohydrate or lipid moieties, cofactors, and the like. Modified polypeptides of the invention may retain the biological activity of the unmodified polypeptide or may exhibit a reduced or increased biological activity.

The polypeptides and biologically active analogs thereof of the present invention include native (naturally occurring), recombinant, and chemically or enzymatically synthesized polypeptides. For example, the NaCT polypeptides of the present invention may be prepared by isolation form naturally occurring tissues or prepared recombinantly, by well known methods, including, for example, preparation as fusion proteins in bacteria and insect cells.

The polypeptides of the present invention include polypeptides with "structural similarity" to naturally occurring polypeptides, such as *Drosophila* drIndy (SEQ ID NO:2), rat NaCT (SEQ ID NO:4), human NaCT (SEQ ID NO:6), *C*. *elegans* NaCT (also referred to as CeNaCT) (SEQ ID NO:8), mouse NaCT (SEQ ID NO:10), or zebrafish NaCT (SEQ ID NO:12).

As used herein, "structural similarity" refers to the identity between two polypeptides. For polypeptides, structural similarity is generally determined by aligning the residues of the two polypeptides (for example, a candidate polypeptide and the polypeptide of SEQ ID NO:2, SEQ ID NO:4, SEQ ID NO:6, SEQ ID NO:8, SEQ ID NO:10, or SEQ ID NO:12) to optimize the number of identical amino acids along the lengths of their sequences; gaps in either or both sequences are permitted in making the alignment in order to optimize the number of identical amino acids, although the amino acids in each sequence must nonetheless remain in their proper order. A candidate polypeptide is the polypeptide being compared to the polypeptide of SEQ ID NO:2, SEQ ID NO:4, SEQ ID NO:6, SEQ ID NO:8, SEQ ID NO:10, or SEQ ID NO:12. A candidate polypeptide can be isolated, for example, from an animal or a microbe, or can be produced using recombinant techniques, or chemically or enzymatically synthesized.

A pair-wise comparison analysis of transporter protein sequences can carried out using the BESTFIT algorithm in the GCG package (version 10.2, Madison WI). Alternatively, polypeptides may be compared using the Blastp program of the BLAST 2 search algorithm, as described by Tatiana et al., (FEMS Microbiol Lett, 174, 247-250 (1999)), and available on the world wide web at ncbi.nlm.nih.gov/BLAST/. The default values for all BLAST 2 search parameters may be used, including matrix = BLOSUM62; open gap penalty = 11, extension gap penalty = 1, gap x_dropoff = 50, expect = 10, wordsize = 3, and filter on.

In the comparison of two amino acid sequences, structural similarity may be referred to by percent "identity" or may be referred to by percent "similarity." "Identity" refers to the presence of identical amino acids and "similarity" refers to the presence of not only identical amino acids but also the presence of conservative substitutions.

The NaCT polypeptides of the present invention include polypeptides with at least 35%, at least 40%, at least 45%, at least 50%, at least 55%, at least 60%, at least 65%, at least 70%, at least 75%, at least 80%, at least 85%, at least 90%, at least 95%, or at least 99% sequence similarity to a known NaCT polypeptide, including, but not limited to, SEQ ID NO:2, SEQ ID NO:4, SEQ ID NO:6, SEQ ID NO:8, SEQ ID NO:10, or SEQ ID NO:12.

The NaCT polypeptides of the present invention also include polypeptides with at least 35%, at least 40%, at least 45%, at least 50%, at least 55%, at least 60%, at least 65%, at least 70%, at least 75%, at least 80%, at least 85%, at least 90%, at least 95%, or at least 99% sequence identity to a known NaCT polypeptide, including, but not limited to, SEQ ID NO:2, SEQ ID NO:4, SEQ ID NO:6, SEQ ID NO:8, SEQ ID NO:10, or SEQ ID NO:12.

Some structural similarities between NaCT polypeptides of the present invention are as follows. Rat NaCT (SEQ ID NO:4) compared to rat NaDC1 (SEQ ID NO:13) demonstrates 62% amino acid sequence similarity and 50% amino acid sequence identity. Rat NaCT (SEQ ID NO:4) compared to rat NaDC3 (SEQ ID NO:14) demonstrates 59% amino acid sequence similarity and 48% amino acid sequence identity.

CeNaCT (SEQ ID NO:8) compared to DrIndy (SEQ ID NO:2) demonstrates 48% amino acid sequence similarity and 35% amino acid sequence identity. CeNaCT (SEQ ID NO:8) compared to rat NaCT (SEQ ID NO:4) demonstrates 49% amino acid sequence similarity and 36% amino acid sequence identity. CeNaCT (SEQ ID NO:8) compared to mouse NaCT (SEQ ID NO:10) demonstrates 48% amino acid sequence similarity and 35% amino acid sequence identity. CeNaCT (SEQ ID NO:8) compared to human NaCT (SEQ ID NO:6) demonstrates 49% amino acid sequence similarity and 37% amino acid sequence identity.

DrIndy (SEQ ID NO:2) compared to rat NaCT (SEQ ID NO:4) demonstrates 51% amino acid sequence similarity and 37% amino acid sequence identity. DrIndy (SEQ ID NO:2) compared to mouse NaCT (SEQ ID NO:10) demonstrates 49% amino acid sequence similarity and 36% amino acid sequence identity. DrIndy (SEQ ID NO:2) compared to human NaCT (SEQ ID NO:6) demonstrates 52% amino acid sequence similarity and 40% amino acid sequence identity. DrIndy (SEQ ID NO:2) compared to human NaDC1 (Genbank Accession No. 26209) demonstrates 35% amino acid sequence identity. DrIndy (SEQ ID NO:2) compared to human NaDC3 (Genbank Accession No. AF154121) demonstrates 34% amino acid sequence identity.

Human NaCT (SEQ ID NO:6) compared to rat NaCT (SEQ ID NO:4) demonstrates 87% amino acid sequence similarity and 77% amino acid sequence identity. Human NaCT (SEQ ID NO:6) compared to human Na⁺-coupled sulfate transporter NaSi (GenBank Accession No. AF260824) demonstrates 43% amino acid sequence identity. Human NaCT (SEQ ID NO:6) compared to human sulfate transporter SUT-1 (GenBank Accession No. AF169301) demonstrates 40% amino acid sequence identity.

Mouse NaCT (SEQ ID NO:10) compared to rat NaCT (SEQ ID NO:4) demonstrates 93% amino acid sequence similarity and 86% amino acid sequence identity. Mouse NaCT (SEQ ID NO:10) compared to human NaCT (SEQ ID NO:6) demonstrates 85% amino acid sequence similarity and 74% amino acid sequence identity. Mouse NaCT (SEQ ID NO:10) demonstrates 50% amino acid sequence identity compared to mouse NaDC1; 44% amino acid sequence identity compared to mouse NaDC3; 40% amino acid sequence identity compared to mouse Na⁺-coupled sulfate transporter NaSi1; and 39% amino acid sequence identity compared to the mouse sulfate transporter SUT1.

Zebrafish NaCT (SEQ ID NO:12) compared to rat NaCT (SEQ ID NO:4) demonstrates 72% amino acid sequence similarity and 57% amino acid sequence identity. Zebrafish NaCT (SEQ ID NO:12) compared to human NaCT (SEQ ID NO:6) demonstrates 77% amino acid sequence similarity and 61% amino acid sequence identity. Zebrafish NaCT (SEQ ID NO:12) compared to mouse NaCT (SEQ ID NO:10) demonstrates 74% amino acid sequence similarity and 57% amino acid sequence identity.

The polypeptides of the present invention can also be designed to provide additional sequences, such as, for example, the addition of coding sequences for added C-terminal or N-terminal amino acids that would facilitate purification by trapping on columns or use of antibodies. Such tags include, for example, histidine-rich tags that allow purification of polypeptides on nickel columns. Such gene modification techniques and suitable additional sequences are well known in the molecular biology arts.

Amino acids essential for the function of NaCT polypeptides can be identified according to procedures known in the art, such as site-directed mutagenesis or alanine-scanning mutagenesis (Cunningham and Wells, Science 244: 1081-1085, 1989; Bass et al., Proc. Natl. Acad. Sci. USA 88: 4498-4502, 1991).

The polypeptides of the present invention may be formulated in a composition along with a "carrier." As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

By "pharmaceutically acceptable" is meant a material that is not biologically or otherwise undesirable, i.e., the material may be administered to an individual along with a NaCT polypeptide without causing any undesirable biological effects or interacting in a deleterious manner with any of the other components of the pharmaceutical composition in which it is contained.

### Polynucleotides:

The present invention provides isolated polynucleotides encoding NaCT polypeptides. As used herein a NaCT polypeptide is a polypeptide having one or more of the functional activities that are described herein. Examples of the present invention include an isolated polynucleotide having the nucleotide sequence of SEQ ID NO:1, SEQ ID NO:3, SEQ ID NO:5, SEQ ID NO:7, SEQ ID NO:9, SEQ ID NO:11, and the complements thereof. Also included in the present invention are polynucleotides hybridizing to one or more of SEQ ID NO:1, SEQ ID NO:3, SEQ ID NO:5, SEQ ID NO:7, SEQ ID NO:9, SEQ ID NO:11, or a complement thereof, under standard hybridization conditions, that encode a polypeptide that exhibits one or more of the functional activities of a NaCT polypeptide. Also included in the present invention are polynucleotides having a sequence identity of at least 50%, at least 55%, at least 60%, at least 65%, at least 70%, at least 75%, at least 80%, at least 85%, at least 90%, at least 95%, at least 96%, at least 97%, at least 98%, or at least 99% with the nucleotide sequence of SEQ ID NO:1, SEQ ID NO:3, SEQ ID NO:5, SEQ ID NO:7, SEQ ID NO:9, or SEQ ID NO:11, where the polynucleotide encodes a polypeptide that exhibits one or more of the functional activities of a NaCT polypeptide.

As used herein, "sequence identity" refers to the identity between two polynucleotide sequences. Sequence identity is generally determined by aligning the residues of the two polynucleotides (for example, aligning the nucleotide sequence of the candidate sequence and the nucleotide sequence of SEQ ID NO:1, SEQ ID NO:3, SEQ ID NO:5, SEQ ID NO:7, SEQ ID NO:9, or SEQ ID NO:11) to optimize the number of identical nucleotides along the lengths of their sequences; gaps in either or both sequences are permitted in making the alignment in order to optimize the number of shared nucleotides, although the nucleotides in each sequence must nonetheless remain in their proper order. A candidate sequence is the sequence being compared to a known sequence, such as SEQ ID NO:1, SEQ ID NO:3, SEQ ID NO:5, SEQ ID NO:7, SEQ ID NO:9 or SEQ ID NO:11. For example, two polynucleotide sequences can be compared using the Blastn program of the BLAST 2 search algorithm, as described by Tatiana et al., FEMS Microbiol Lett., 1999;174: 247-250, and available on the world wide web at ncbi.nlm.nih.govBLAST/. The default values for all BLAST 2 search parameters may be used, including reward for match = 1, penalty for mismatch = -2, open gap penalty = 5, extension gap penalty = 2, gap x_dropoff = 50, expect = 10, wordsize = 11, and filter on.

In some aspects of the present invention, the polynucleotides of the present invention include nucleotide sequences having a sequence identity with SEQ ID NO:1, SEQ ID NO:3, SEQ ID NO:5, SEQ ID NO:7, SEQ ID NO:9, or SEQ ID NO:11, or at least 50%, at least 55%, at least 60%, at least 65%, at least 70%, at least 75%, at least 80%, at least 85%, at least 90%, at least 95%, at least 96%, at least 97%, at least 98%, or at least 99% identity with SEQ ID NO:1, SEQ ID NO:3, SEQ ID NO:5, SEQ ID NO:7, SEQ ID NO:9, or SEQ ID NO:11.

Also included in the present invention are polynucleotide fragments. A polynucleotide fragment is a portion of an isolated polynucleotide as described herein. Such a portion may be several hundred nucleotides in length, for example about 100, about 200, about 300, about 400, about 500, about 600, about 700, about 800, about 900 or about 1000 nucleotides in length. Such a portion may be about 10 nucleotides to about 100 nucleotides in length, including but not limited to, about 14 to about 40 nucleotides in length.

The polynucleotides of the present invention may be formulated in a composition along with a "carrier." As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

By "pharmaceutically acceptable" is meant a material that is not biologically or otherwise undesirable, i.e., the material may be administered to an individual along with a NaCT polynucleotide without causing any undesirable biological effects or interacting in a deleterious manner with any of the other components of the pharmaceutical composition in which it is contained.

Polynucleotides of the present invention can be inserted into a vector. Construction of vectors containing a polynucleotide of the invention employs standard ligation techniques known in the art. See, for instance, Sambrook et al, "Molecular Cloning: A Laboratory Manual," Cold Spring Harbor Laboratory Press, 1989. The term vector includes, but is not limited to, plasmid vectors, viral vectors, cosmid vectors, or artificial chromosome vectors. Typically, a vector is capable of replication in a bacterial host, for instance, *E. coli.* Selection of a vector depends upon a variety of desired characteristics in the resulting construct, such as a selection marker, vector replication rate, and the like. A vector can provide for further cloning (amplification of the polynucleotide), e.g., a cloning vector, or for expression of the polypeptide encoded by the coding sequence, e.g., an expression vector. Suitable host cells for cloning or expressing the vectors herein are prokaryote or eukaryotic cells.

As used herein, an "expression vector" is a DNA molecule, linear or circular, that includes a segment encoding a polypeptide of interest operably linked to additional segments that provide for its transcription. Such additional segments may include promoter and terminator sequences, and optionally one or more origins of replication, one or more selectable markers, an enhancer, a polyadenylation signal, and the like. Expression vectors are generally derived from plasmid or viral DNA, or may contain elements of both.

By "host cell" is meant a cell that supports the replication or expression of an expression vector. Host cells may be bacterial cells, including, for example, *E. coli* and *B. subtilis,* or eukaryotic cells, such as yeast, including, for example, *Saccharomyces* and *Pichia,* insect cells, including, for example, *Drosophila* cells and the Sf9 host cells for the baculovirus expression vector, amphibian cells, including, for example, *Xenopus* oocytes and mammalian cells, such as CHO cells, HeLa cells, human retinal pigment epithelial (RPE) cells, human hepatoma HepG2 cells, and plant cells.

An expression vector optionally includes regulatory sequences operably linked to the coding sequence. The invention is not limited by the use of any particular promoter, and a wide variety of promoters are known. Promoters act as regulatory signals that bind RNA polymerase in a cell to initiate transcription of a downstream (3' direction) coding sequence. The promoter used can be a constitutive or an inducible promoter. It can be, but need not be, heterologous with respect to the host cell.

The transformation of a host cell with an expression vector may be accomplished by a variety of means known to the art, including, but not limited to, calcium phosphate-DNA co-precipitation, DEAE-dextran-mediated transfection, polybrene-mediated transfection, electroporation, microinjection, liposome fusion, lipofection, protoplast fusion, retroviral infection, biolistics (i.e., particle bombardment) and the like.

Transformation of a host cell may be stable or transient. The term "transient transformation" or "transiently transformed" refers to the introduction of one or more transgenes into a cell in the absence of integration of the transgene into the host cell's genome. Transient transformation may be detected by, for example, enzyme-linked immunosorbent assay (ELISA) that detects the presence of a polypeptide encoded by one or more of the transgenes. Alternatively, transient transformation may be detected by detecting the activity of the protein encoded by the transgene. The term "transient transformant" refers to a cell that has transiently incorporated one or more transgenes. In contrast, the term "stable transformation" or "stably transformed" refers to the introduction and integration of one or more transgenes into the genome of a cell. The term "stable transformant" refers to a cell that has stably integrated one or more transgenes into the genomic DNA. Thus, a stable transformant is distinguished from a transient transformant in that, whereas genomic DNA from the stable transformant contains one or more transgenes, genomic DNA from the transient transformant does not contain a transgene. Methods for both transient and stable expression of coding regions are well known in the art.

Among the known methods for expressing transporter genes is expression in a *Xenopus* oocyte system. A cDNA encoding the open reading frame of a citrate transporter polypeptide or portions thereof can be incorporated into commercially available bacterial expression plasmids such as the pGEM (Promega) or pBluescript (Stratagene) vectors or one of their derivatives. After amplifying the expression plasmid in bacterial (*E*. *coli*) cells the DNA is purified by standard methods. The incorporated transporter sequences in the plasmid DNA are then transcribed in vitro according to standard protocols, such as transcription with SP6 or T7 RNA polymerase. The RNA thus prepared is injected into *Xenopus* oocytes where it is translated and the resulting transporter polypeptides are incorporated into the plasma membrane. The functional properties of these transporters can then be investigated by electrophysiological, biochemical, pharmacological, and related methods.

The polynucleotides of the present invention may be inserted into a recombinant DNA vector for the production of products including, but not limited to, mRNA, antisense oligonucleotides, and polynucleotides for use in RNA interference (RNAi) (see, for example, Cheng et al., Mol Genet Metab. (2003);80: 121-28). For example, for the production of mRNA, a cDNA comprising, for example, SEQ ID NO:1, SEQ ID NO:3, SEQ ID NO:5, SEQ ID NO:7, SEQ ID NO:9, SEQ ID NO:11, or a fragments thereof, may be inserted into a plasmid containing a promoter for either SP6 or T7 RNA polymerase. The plasmid is cut with a restriction endonuclease to allow run-off transcription of the mRNA, and the RNA is produced by addition of the appropriate buffer, ribonucleotides, and polymerase. The RNA is isolated by conventional means such as ethanol precipitation. The mRNA can be capped or polyadenylated, for example, prior to injection into a cell such as a *Xenopus* oocyte, for expression.

The NaCT polypeptide transports citrate, an important metabolic intermediate with multiple metabolic functions, including, for example, lipid and cholesterol synthesis. Thus, the present invention also includes transgenic and knockout animal models, useful in studies to further understand the physiological functions of this transporter. Such animals may be constructed using standard methods known in the art and as set forth, for example, in U.S. Pat. Nos. 5,614,396 5,487,992, 5,464,764, 5,387,742, 5,347,075, 5,298,422, 5,288,846,5,221,778,5,175,384,5,175,383,4,873,191, and 4,736,866.

### Antibodies:

Included in the present invention are antibodies that specifically bind to one or more of the polypeptides described herein. Such antibodies include, but are not limited to, antibodies that specifically bind to *Drospohila* Indy (SEQ ID NO:2), rat NaCT (SEQ ID NO:4), human NaCT (SEQ ID NO:6), *C. elegans* NaCT (SEQ ID NO:8), mouse NaCT (SEQ ID NO:10), zebrafish NaCT (SEQ ID NO:12) and variants thereof. Such antibodies include, but are not limited to, polyclonal antibodies, affinity-purified polyclonal antibodies, monoclonal antibodies, humanized antibodies, chimeric antibodies, anti-idiotypic antibodies, single chain antibodies, and antigen-binding fragments thereof, such as F(ab')₂ and Fab proteolytic fragments and fragments produced from an Fab expression library. The term "polyclonal antibody" refers to an antibody produced from more than a single clone of plasma cells; in contrast "monoclonal antibody" refers to an antibody produced from a single clone of plasma cells.

As used herein, "antibodies" or "antibody" refers to an immunoglobulin molecule or immunologically active antigen-binding portion thereof. In preferred embodiments, an antibody has at least one, and preferably two, heavy (H) chain variable regions (abbreviated herein as VH), and at least one and preferably two light (L) chain variable regions (abbreviated herein as VL). The VH and VL regions can be further subdivided into regions of hypervariability, termed "complementarity determining regions" ("CDR"), interspersed with regions that are more conserved, termed "framework regions" (FR). The extent of the framework region and CDR's has been precisely defined (see, Kabat, E. A., et al. (1991) Sequences of Proteins of Immunological Interest, Fifth Edition, U.S. Department of Health and Human Services, NIH Publication No. 91-3242, and Chothia, C. et al., J. Mol. Biol. 1987;196: 901-917). Each VH and VL is composed of three CDR's and four FRs, arranged from amino-terminus to carboxy-terminus in the following order: FR1, CDR1, FR2, CDR2, FR3, CDR3, FR4.

The phrase "specifically binds" or "specifically immunoreactive with," when referring to an antibody, refers to a binding reaction that is determinative of the presence of a protein in a heterogeneous population of proteins and other biologics. Thus, under designated immunoassay conditions, the specified antibodies bind to a particular protein at least two times the background and do not substantially bind in a significant amount to other proteins present in the sample. Typically a specific or selective reaction will be at least twice background signal or noise and more typically more than 10 to 100 times background. Specific binding to an antibody under such conditions may require an antibody that is selected for its specificity for a particular protein

Antibodies of the present invention can be prepared using the intact polypeptide or fragments thereof as the immunizing agent. If a polypeptide fragment is used as an immunizing agent, a preferred fragment is about 15 to about 30 contiguous amino acids of SEQ ID NO:2, SEQ ID NO:4, SEQ ID NO:6, SEQ ID NO:8, SEQ ID NO:10, or SEQ ID NO:12. For example, contiguous amino acid fragments of about 14, about 15, about 16, about 17, about 18, about 19, about 20, about 21, about 22, about 23, about 24, about 25, about 26, about 27, about 28, about 29, about 30, about 31, or about 32 amino acids may be used. The polypeptide fragment may be selected from a non-transmembrane domain of a NaCT polypeptide, for example, in an extracellular or intracellular loop. A preferred antibody binds to an extracellular epitope and alters the functional ability of the transporter to transport citrate. Such antibodies may be identified using any of the methods for assaying transporters described herein.

In addition to specifically binding to a citrate transporter polypeptide, the antibodies may have additional binding specificities. For example, an antibody may bind to the C terminus or the N terminus of a citrate transport polypeptide. Or, an antibody may be selected that demonstrates limited cross reactivity. For example, an antibody may bind to a human NaCT polypeptide, but not to a rat NaCT polypeptide or mouse NaCT polypeptide; or an antibody may bind to a NaCT polypeptide of a given species, such as human, rat, mouse, zebrafish, or *C. elegans,* but not bind to the NADC1, NADC2 or NADC3 polypeptides of the same species.

The preparation of polyclonal antibodies is well known. Polyclonal antibodies may be obtained by immunizing a variety of warm-blooded animals such as horses, cows, goats, sheep, dogs, chickens, rabbits, mice, hamsters, guinea pigs and rats as well as transgenic animals such as transgenic sheep, cows, goats or pigs, with an immunogen. The resulting antibodies may be isolated from other proteins by using an affinity column having an Fc binding moiety, such as protein A, or the like.

Monoclonal antibodies can be obtained by various techniques familiar to those skilled in the art. Briefly, spleen cells from an animal immunized with a desired antigen are immortalized, commonly by fusion with a myeloma cell (see, for example, Kohler and Milstein, Eur. J. Immunol. (1976);6: 511-519; J. Goding (1986) In "Monoclonal Antibodies: Principles and Practice," Academic Press, pp 59-103; and Harlow et al., Antibodies: A Laboratory Manual, page 726 (Cold Spring Harbor Pub. 1988). Monoclonal antibodies can be isolated and purified from hybridoma cultures by techniques well known in the art.

In some embodiments, the antibody can be recombinantly produced, for example, produced by phage display or by combinatorial methods. Phage display and combinatorial methods can be used to isolate recombinant antibodies that bind to a NaCT polypeptide or fragments thereof (see, for example, U.S. Pat. No. 5,223,409; WO 92/18619; WO 91/17271; WO 92/20791; WO 92/15679; WO 93/01288; WO 92/01047; WO 92/09690; WO 90/02809; Fuchs et al., Bio/Technology (1991);9: 1370-1372; Huse et al., Science (1989);246: 1275-1281; Griffths et al., EMBO J. (1993);12: 725-734; Hawkins et al., J Mol Biol (1992);226: 889-896; Clackson et al., Nature (1991);352: 624-628; Gram et al., PNAS (1992);89:3576-3580; Garrad et al., Bio/Technology (1991);9: 1373-1377; Hoogenboom et al., Nuc Acid Res (1991);19: 4133-4137; and Barbas et al., PNAS (1991);88: 7978-7982). Such methods can be used to generate human monoclonal antibodies.

Human monoclonal antibodies can also be generated using transgenic mice carrying the human immunoglobulin genes rather than the mouse system. Splenocytes from these transgenic mice immunized with the antigen of interest are used to produce hybridomas that secrete human mAbs with specific affinities for epitopes from a human protein (see, for example, WO 91/00906; WO 91/10741; WO 92/03918, Lonberg et al., Nature (1994);368: 856-859; Green et al., Nature Genet. (1994);7: 13-21; Morrison et al., PNAS (1994);81: 6851-6855; Tuaillon et al., PNAS (1993);90:3720-3724; Bruggeman et al., Eur J Immunol (1991);21:1323-1326).

A therapeutically useful antibody may be derived from a "humanized" monoclonal antibody. Humanized monoclonal antibodies are produced by transferring one or more CDRs from the heavy and light variable chains of a mouse (or other species) immunoglobulin into a human variable domain, then substituting human residues into the framework regions of the murine counterparts. The use of antibody components derived from humanized monoclonal antibodies obviates potential problems associated with immunogenicity of murine constant regions. Techniques for producing humanized monoclonal antibodies can be found, for example, in Jones et al., Nature (1986);321: 522 and Singer et al., J. Immunol., (1993);150: 2844.

In addition, chimeric antibodies can be obtained by splicing the genes from a mouse antibody molecule with appropriate antigen specificity together with genes from a human antibody molecule of appropriate biological specificity; see, for example, Takeda et al., Nature (1985);314: 544-546. A chimeric antibody is one in which different portions are derived from different animal species.

Antibody fragments can be generated by techniques well known in the art. Such fragments include Fab fragments produced by proteolytic digestion, and Fab fragments generated by reducing disulfide bridges.

Antibodies, or fragments thereof, may be coupled directly or indirectly to a detectable marker by techniques well known in the art. A detectable marker is an agent detectable by spectroscopic, photochemical, biochemical, immunochemical, or chemical means. For example, useful detectable markers include fluorescent dyes, chemiluminescent compounds, radioisotopes, electron-dense reagents, enzymes, colored particles, biotin, or dioxigenin. A detectable marker often generates a measurable signal, such as radioactivity, fluorescent light, color, or enzyme activity.

When used for immunotherapy, antibodies, or fragments thereof, may be unlabelled or labeled with a therapeutic agent. These agents can be coupled directly or indirectly to the monoclonal antibody by techniques well known in the art, and include such agents as drugs, radioisotopes, lectins and toxins.

Antibodies can be used alone or in combination with additional therapeutic agents, such as those described above. Preferred combinations include monoclonal antibodies with modifiers of citrate transporters or other biological response modifiers. The dosage administered may vary with age, condition, weight, sex, age and the extent of the condition to be treated, and can readily be determined by one skilled in the art. Dosages can be about 0.1 mg/kg to about 2000 mg/kg. The monoclonal antibodies can be administered intravenously, intraperitoneally, intramuscularly, subcutaneously, intracavity, or transdermally, alone or with effector cells.

Antibodies that are both specific for a citrate transporter polypeptide and interfere with its activity may be used to inhibit polypeptide function. Such antibodies may be generated using standard techniques, against the proteins themselves or against peptides corresponding to portions of the proteins. In some embodiments, it is preferred to use fragments of the antibody, as the smallest inhibitory fragment which binds to the target protein's binding domain. For example, peptides having an amino acid sequence corresponding to the domain of the variable region of the antibody that binds to the target polypeptide may be used. Such peptides may be synthesized chemically or produced via recombinant DNA technology using methods well known in the art (e.g., see Sambrook et al., Eds., Molecular Cloning: A Laboratory Manual, 2nd ed., Cold Spring Harbor Laboratory Press, 1989, or Ausubel, F. M. et al., eds. Current Protocols in Molecular Biology, 1994).

### Modulation of NaCT Polypeptides:

The present invention includes methods for identifying agents that serve as substrates, modifiers, stimulators or inhibitors for one or more functional activities of a NaCT polypeptide.

As used herein a "substrate" of a NaCT polypeptide is an agent that is taken up into the cells via the Na⁺-coupled citrate transporter.

As used herein a "modifier" or "modulator" of a NaCT polypeptide is an agent that alters the entry of citrate into the cell via a Na⁺-coupled citrate transporter.

A modifier includes activator and stimulators of a NaCT polypeptide. As used herein an "activator" or "stimulator" of a NaCT polypeptide is an agent that increases or enhances the entry of citrate into the cell via a Ne⁺-coupled citrate transporter. "Activators" are agents that increase, open, activate, facilitate, enhance activation, agonize, or up regulate a Na⁺-coupled citrate transporter. Examples of a stimulator of a NaCT polypeptide, include, for example, lithium and lithium-related salts.

A modifier includes inhibitors of a NaCT polypeptide. As used herein an "inhibitor" of a NaCT polypeptide is an agent that decreases or reduces the entry of citrate into the cell via a Na⁺-coupled citrate transporter. Inhibitors are agents that, partially or totally block activity, decrease, prevent, delay activation, inactivate, or down regulate the activity or expression of a Na⁺-coupled citrate transporter. Examples of an inhibitor of a NaCT polypeptide include, for example, hydroxycitrate and other citrate analogs.

A modifier includes blockers of a NaCT polypeptide. As used herein a "blocker" of a NaCT polypeptide is an agent that binds to the NaCT polypeptide and blocks the entry of citrate into the cell via the Na⁺-coupled citrate transporter but is itself not transported into the cell via the Na⁺-coupled citrate transporter.

Suitable agents can include citrate analogs, naturally occurring and synthetic ligands, antagonists, agonists, antibodies, antisense molecules, ribozymes, small chemical molecules and the like. Suitable agents can also include modified versions of a NaCT polypeptide itself; for example, versions with altered activity.

Substrates, modifiers, stimulators, inhibitors, and blockers of a NaCT polypeptide may be identified using a variety of assays, including the various *in vitro* and *in vivo* assays described herein. Assays for such agents can include, for example, expressing a NaCT polypeptide protein *in vitro,* in cells, in cell membranes, or in vivo, applying putative modulator compounds, and then evaluating the functional effects on activity, as described above.

Samples or assays of a NaCT polypeptide that are treated with a potential activator, inhibitor, or modulator can be compared to control samples without the inhibitor, activator, or modulator to examine the extent of modification. Untreated control samples can be assigned a relative protein activity value of 100%. Inhibition of a Na⁺-coupled citrate transporter, for example, is achieved when the activity value relative to the control is about 80%, preferably 50%, more preferably 25-0%. Activation of a Na⁺-coupled citrate transporter, for example, is achieved when the activity value relative to the control (untreated with activators) is 110%, more preferably 150%, more preferably 200-500% (i.e., two to five fold higher relative to the control), more preferably 1000-3000% higher.

An agent that modulates one or more functional activities of a NaCT polypeptide may be formulated as a composition. The compositions of the present invention may be formulated in a variety of forms adapted to the chosen route of administration. The formulations may be conveniently presented in unit dosage form and may be prepared by methods well known in the art of pharmacy. Formulations of the present invention may include, for instance, a pharmaceutically acceptable carrier. The formulations of this invention may include one or more accessory ingredients including diluents, buffers, binders, disintegrants, surface active agents, thickeners, lubricants, preservatives (including antioxidants) and the like. In addition, the formulations of this invention may further include additional therapeutic agents.

Agents of the present invention, that disrupt one or more functions of a Na⁺-coupled citrate transporter have a variety of therapeutic applications. Such agents may modify the availability of di- and tricarboxylates for cellular production of metabolic energy. Agents that disrupt the function of Na⁺-coupled citrate transporter may create a biological state similar to that of caloric restriction. Such agents may consequently lead to life span extension. Agents that disrupt the function of Na⁺-coupled citrate transporter may be useful in body weight control, the treatment of body weight disorders or the treatment of diabetes.

The present invention is illustrated by the following examples. It is to be understood that the particular examples, materials, amounts, and procedures are to be interpreted broadly in accordance with the scope and spirit of the invention as set forth herein.

### EXAMPLES

### Example 1

### Functional Identity of Drosophila melanogaster Indy as a Cation-independent, Electroneutral Transporter for Tricarboxylic acid-cycle Intermediates

*Indy* (an acronym for "*I*"m *n*ot *d*ead *y*et") is a gene in *Drosophila melanogaster* which, when made dysfunctional, leads to an extension of the average adult life span of the organism. Rogina et al., Science (2000);290: 2137-2140. In this example, the *Indy* gene-product was cloned and its functional identity established. A full-length Indy cDNA (SEQ ID NO:1) from a *D. melanogaster* cDNA library was isolated and the nucleotide sequence determined, as shown in Fig. 1. The cDNA codes for a protein of 572 amino acids (SEQ ID NO:2) (Fig. 1), that is called "*Drosophila* Indy" or "drIndy." In its amino acid sequence, drIndy exhibits comparable similarity to the two known Na⁺-coupled dicarboxylate transporters in mammals; namely, NaDC1 (35% identity) and NaDC3 (34% identity). In this example, the functional characteristics of drIndy were elucidated in two different heterologous expression systems by using mammalian cells and *Xenopus laevis* oocytes. These studies showed that drIndy is a cation-independent electroneutral transporter for a variety of tricarboxylic acid-cycle intermediates, with preference for citrate compared with succinate. These characteristics of drIndy differ markedly from those of NaDC1 and NaDC3, indicating that neither of these latter transporters is the mammalian functional counterpart of drIndy. Since drIndy is a transporter for tricarboxylic acid-cycle intermediates, dysfunction of the *Indy* gene may lead to decreased production of metabolic energy in cells, analogous to caloric restriction, providing a molecular basis for the observation that disruption of the Indy gene function in Drosophila leads to extension of the average adult life span of the organism.

The protein product of the *Indy* gene is most closely related in amino acid sequence to mammalian Na⁺-coupled dicarboxylate transporters, known as NaDCs. NaDCs are secondary active transporters for dicarboxylate intermediates of the tricarboxylic acid cycle. Two different NaDCs have been identified so far in mammalian tissues. Pajor, Annu. Rev. Physiol (1999);61: 663-682. These are NaDC1 and NaDC3 (a unique NaDC identified in *Xenopus laevis* is currently referred to as NaDC2). Therefore the question arises as to which one of the two NaDCs is the mammalian counterpart of *Drosophila* Indy (drIndy) in terms of biological function.

NaDC1 is Na⁺-coupled, electrogenic and exhibits a low affinity for its dicarboxylate substrates. The Michaelis-Menten constant for the prototypical substrate succinate is in the range of 0.1-4.0 millimolar (mM) (Pajor, J. Biol. Chem. (1995);270: 5779-5785; Pajor, Am. J. Physiol. (1996);270: F642-F648; and Chen et al., J. Biol. Chem. (1998);273: 20972-20981. This isoform is expressed primarily in the brush-border membrane of intestinal and renal epithelial cells.

NaDC3 is also Na⁺-coupled and electrogenic but exhibits relatively higher affinity for its dicarboxylate substrates compared with NaDC1. The Michaelis-Menten constant for succinate is in the range of 2-50 micromolar (µM) (Kekuda et al., J. Biol. Chem. (1999);274: 3422-3429; Wang et al., Am. J. Physiol. (2000);278: C1019-C1030; Chen et al., J. Clin. Invest. (1999);103: 1159-1168; and Huang et al., J. Pharmacol. Exp. Ther. (2000);295: 392-403). This isoform is expressed primarily in the basolateral membrane of intestinal and renal epithelial cells, sinusoidal membrane of hepatocytes, brush-border membrane of placental trophoblasts and in the plasma membrane of neurons and glial cells.

Since NaDC1 and NaDC3 differ significantly in transport characteristics and tissue-expression pattern, it is important to identify the isoform of NaDC that is the mammalian functional counterpart of drIndy. This cannot be achieved without information on the functional nature of drIndy. While Rogina et al. (Science (2000);290: 2137-2140) derived the amino acid sequence of a putative Indy protein on the basis of the genomic sequence and expressed sequence tags ('ESTs'), prior to the work of the present example, the full-length Indy cDNA had not been cloned nor had the transport function of Indy been established. It was not known whether drIndy is actually a Na⁺-coupled transporter for dicarboxylate anions. Therefore the present studies were undertaken to clone the full-length drIndy cDNA and identify its transport function.

### MATERIALS AND METHODS

Materials. [³H]Succinate (specific radioactivity, 40 Ci/mmol), [¹⁴C]citrate (specific radioactivity, 55 mCi/mmol), and [¹⁴C]pyruvate (specific radioactivity, 15 mCi/mmol) were purchased from Moravek Biochemicals (Brea, CA, U.S.A.). The human retinal pigment epithelial (HRPE) cell line, used in functional expression studies, was routinely maintained in Dulbecco's modified Eagle's medium/F-12 medium supplemented with 10% (v/v) fetal bovine serum, 100units/ml penicillin and 100µg/ml streptomycin. Frogs (*Xenopus* species) were purchased from Nasco (Fort Atkinson, WI, U.S.A.).

Cloning of the drIndy cDNA. The nucleotide sequence of the putative mRNA coding for the Indy protein was first deduced from the *Drosophila* gene sequence (GenBank Accession No. AE003519; reverse complement). This sequence was used to design primers for reverse transcriptase (RT)-PCR to obtain a cDNA probe specific for Indy. The forward primer was 5'-CTCCAACTTCTTCGCTAACC-3' (SEQ ID NO:15) and the reverse primer was 5'-CTAGTGCGTCTTGTTTCCC-3' (SEQ ID NO:16). The predicted size of the RT-PCR product was 1675 basepairs (bp). This primer pair was used to obtain a fragment of Indy cDNA by using the commercially available polyadenylated (poly(A)+) RNA from adult *D*. *melanogaster* (ClonTech, Palo Alto, CA). This yielded a RT-PCR product of expected size. The product was subcloned in pGEM-T vector and sequenced to establish its molecular identity. A unidirectional *Drosophila* cDNA library was then established using the commercially available poly(A)+ RNA. The SuperScriptTM plasmid system (Life Technologies, Gaithersburg, MD) was employed for this purpose. The Indy-specific cDNA probe derived from RT-PCR was labeled with [α-³²P]dCTP and used to screen the *Drosophila* cDNA library under high-stringency conditions, as described in Kekuda et al., J. Biol. Chem. (1996);271: 18657-18661, and Prasad et al., J. Biol. Chem. (1998); 273: 7501-7506.

DNA sequencing. Both the sense and antisense strands of the cDNA were sequenced by primer walking. Sequencing was performed by Taq DyeDeoxy terminator cycle sequencing using an automated PerkinElmer Applied Biosystems 377 Prism DNA sequencer. The sequence was analyzed using the National Center for Biotechnology Information server, available on the world wide web at ncbi.nlm.nih.gov.

Functional expression of drIndy cDNA in mammalian cells. The functional expression of drIndy cDNA was accomplished in HRPE cells using the vaccinia virus expression system, as described in Blakely et al., Anal. Biochem. (1991);194: 302-308; Rajan et al., J. Biol. Chem. (1999);274: 29005-29010; and Kekuda et al., J. Biol. Chem. (1998);273: 15971-15979. Subconfluent HRPE cells grown on 24-well plates were first infected with a recombinant (VTF7-3) vaccinia virus encoding T7 RNA polymerase and then transfected with the plasmid carrying the full-length drIndy cDNA. After 12 to 15 hours post-transfection, uptake measurements were made at 37° C with radiolabelled succinate, citrate, or pyruvate. The uptake medium was 25 mM Hepes/Tris, pH7.5, containing 140 mM NaCl, 5.4 mM KCl, 1.8 mM CaCl₂, 0.8 mM MgSO₄ and 5mM glucose. The time of incubation was 15 minutes, a time period representing initial-transport rates as determined from time course studies. Endogenous transport was always determined in parallel using cells transfected with pSPORT1 vector alone. The transport activity in cDNA-transfected cells was adjusted for the endogenous activity to calculate the cDNA-specific transport activity. Experiments were performed in triplicate, and each experiment was repeated at least three times. Results are expressed as the means ± S.E.M. Since infection with vaccinia virus 'shuts off' host-cell proliferation, the cell number of HRPE cells determined immediately prior to infection with the virus was used for calculation of transport activity.

Functional expression of drIndy cRNA in *X. laevis* oocytes. Capped cRNA from the cloned drIndy cDNA was synthesized using the MEGAscript kit (Ambion, Austin, TX, U.S.A.). Mature oocytes from *X*. *laevis* were isolated by treatment with collagenase A (1.6mg/ml), manually defolliculated and maintained at 18° C in modified Barth's medium supplemented with 10mg/l gentamicin, following procedures of Parent et al., J. Membr. Biol. (1992);125: 49-62. On the following day, oocytes were injected with 50 nanograms (ng) of cRNA in 50 nanoliters (nl) of water. Oocytes injected with 50 nanoliters of water served as a control. The oocytes were used for electrophysiological studies 6 days after cRNA injection. Electrophysiological studies were performed by the conventional two-micro-electrode voltage-clamp method, following procedures described in Wang et al., Am. J. Physiol. (2000);278: C1019-C1030; Huang et al., J. Pharmacol. Exp. Ther. (2000);295: 392-403; and Kekuda et al., J. Biol. Chem. (1998);273: 15971-15979. Oocytes were superfused with a NaCl-containing transport buffer (100 mM NaCl, 2 mM KCl, 1 mM MgCl₂, 1 mM CaCl₂, 3 mM Hepes, 3 mM Mes and 3 mM Tris, pH7.5) followed by the same buffer containing 2mM succinate. The membrane potential was clamped at -50mV. The dependence of succinate-induced currents on Na⁺ was assessed by comparing the succinate-induced currents in the Na⁺-containing transport buffer with those in a Na⁺-free transport buffer (NaCl in the transport buffer was replaced iso-osmotically by choline chloride). Oocytes injected with human NaDC3 (hNaDC3) cRNA (Wang et al., *Am*. *J. Physiol.* (2000);278: C1019-C1030) were used as a positive control for succinate-induced currents.

Uptake of succinate in water-injected and cRNA-injected oocytes was measured as described previously by Fei et al., Biochemistry (1995);34: 8744-8751 and Nakanishi et al., N. Am. J. Physiol (2001);281: C1757-C1768. At 6 days after injection with water or cRNA, oocytes were incubated with [³H]succinate (7.5 µCi/ml; succinate concentration, 0.1 µM) in a NaCl-containing transport buffer at room temperature for 1hour. After the incubation, oocytes were washed with fresh transport buffer in the absence of radiolabelled succinate four times, and then each oocyte was transferred individually into scintillation vials for determination of radioactivity.

### RESULTS

Structural features of drIndy. The cloned drIndy cDNA (SEQ ID NO:1), available as GenBank Accession No. AF509505, is 2602bp long with an open reading frame (258-1976bp) coding for a protein of 572 amino acids. When compared with the amino acid sequences of hNaDC1 (Genebank Accession No. U26209) (592 amino acids) and hNaDC3 (Genebank Accession No.AF154121) (602 amino acids), there is significant similarity among the three proteins. The sequence identity between drIndy and hNaDC1 is 35%, and that between drIndy and hNaDC3 is 34%.

Functional features of drIndy. To establish the functional identity of drIndy, the cloned cDNA was expressed heterologously in mammalian cells and assessed the ability of the clone to transport succinate. When measured in the presence of Na⁺, the uptake of succinate (40 nM) in HRPE cells transfected with drIndy cDNA was 20-fold higher than in cells transfected with vector alone (Figure 2A). This shows that drIndy indeed possesses the ability to transport the dicarboxylate succinate. However, surprisingly, drIndy was able to transport succinate not only in the presence of Na⁺, but also in the absence of Na⁺. When measured in the absence of Na⁺, the uptake of succinate in cells transfected with drIndy cDNA was still 15-fold higher than in cells transfected with vector alone. These results are in contrast with those obtained with hNaDC3 under identical conditions (Figure 2B). The uptake of succinate (40 nM), when measured in the presence of Na⁺, increased 200-fold in HRPE cells as a result of transfection with hNaDC3 cDNA. This cDNA-induced uptake was, however, completely abolished when Na⁺ was omitted in the uptake medium. Similar is the case with NaDC3s from other animal species. See, Kekuda et al., J. Biol. Chem. (1999);274: 3422-3429 and Chen et al., J. Clin. Invest. (1999); 103: 1159-1168. Studies by Pajor, J. Biol. Chem. (1995);270: 5779-5785; Pajor, Am. J. Physiol. (1996);270: F642-F648; and Chen et al., J. Biol. Chem. (1998);273: 20972-20981 have shown that the uptake of succinate mediated by NaDC1 from different animal species is also obligatorily dependent on the presence of Na⁺. Thus, although the mammalian NaDC1 and NaDC3 are Na⁺-dependent succinate transporters, drIndy is an Na⁺-independent succinate transporter.

The ability of drIndy to transport succinate remained almost the same even when Na⁺ in the uptake medium was replaced with K⁺, Li⁺, N-methyl- D-glucamine or mannitol, suggesting that drIndy is a cation-independent succinate transporter (Table 1). Whether the drIndy-mediated transport process is dependent on an H+ gradient was then tested by measuring the uptake of succinate at different pH values between pH 5 and 8. The uptake of succinate (40 nM) mediated by drIndy decreased gradually from 127 ± 5 to 52 ± 2 fmol/10⁶ cells per minute (means±S.E.M.) when the pH of the uptake medium was reduced from 8 to 5. These data show that drIndy is not a H⁺-coupled succinate transporter either.

**TABLE 1**

| *Ion-dependence of drIndy-mediated succinate transport* | | | |
|---|---|---|---|
| Uptake of succinate (40 nM) was measured in HRPE cells transfected with either vector alone or drIndy cDNA. Uptake buffer (25 mM Hepes/Tris), pH 7.5, contained one of the indicated salts (140 mM) or mannitol (280 mM). In addition, all buffers contained 5.4 mM KCl, 1.8 mM CaCl₂, 0.8 mM MgSO₄ and 5 mM glucose. Data (means±S.E.M.) are from six independent measurements. NMDG, *N*-methyl-D-glucamine. | | | |
| Succinate uptake (fmol/10⁶ cells per min) | | | |
| Salt | Vector | drIndy | Fold Increase |
| NaCI | 5.9±0.8 | 118.0±7.8 | 20.0 |
| KCl | 7.0±1.2 | 111.0±8.5 | 15.9 |
| LiCl | 5.0±0.6 | 122.3±4.3 | 24.5 |
| NMDG chloride | 4.9±0.7 | 84.7±10.0 | 17.3 |
| Sodium gluconate | 6.0±1.0 | 76.6±2.6 | 12.9 |
| Mannitol | 5.3±0.4 | 83.3±7.1 | 15.7 |

The substrate specificity of drIndy was then studied by assessing the ability of various monocarboxylate, dicarboxylate and tricarboxylate compounds (at a concentration of 2.5 mM) to compete with succinate (40 nM) for the transport process mediated by drIndy (Table 2). The dicarboxylate compounds 2-oxoglutarate, malate, fumarate and dimethylsuccinate were the most potent inhibitors of succinate transport mediated by drIndy. The dicarboxylate compounds maleate and malonate, and the monocarboxylate compounds lactate and β-hydroxybutyrate, were not effective. Surprisingly, the monocarboxylate and tricarboxylate compounds (pyruvate and citrate respectively) were very potent in competing with succinate for transport via drIndy. The amino acid derivative N-acetyl aspartate was moderately effective in inhibiting succinate transport. Kinetic analysis revealed that the transport of succinate via drIndy was saturable (Figure 3). The Michaelis-Menten constant (Km) for the transport process was 40 ± 4 µM.

**TABLE 2**

| **Substrate specificity of drIndy** | | |
|---|---|---|
| Uptake of [³H]succinate (40 nM) was measured in HRPE cells transfected with either vector alone or drIndy cDNA. The uptake buffer was 25 mM Hepes/Tris, pH 7.5, containing 140 mM NaCl, 5.4 mM KCl, 1.8 mM CaCl₂, 0.8 mM MgSO₄ and 5 mM glucose. Concentration of the inhibitors was 2.5 mM. Uptake in vector-transfected cells was subtracted from uptake in cDNA-transfected cells to determine drIndy cDNA-specific uptake. Data (means ± S.E.M.) are from four independent measurements. | | |
| Inhibitor | cDNA-specific [³H]succinate uptake (fmol/106 cells per min) | % Control |
| Control | 143.0±10.8 | 100 |
| Succinate | 4.6±0.4 | 6 |
| 2-Oxoglutarate | 3.0±1.0 | 5 |
| Malate | 6.8±2.7 | 8 |
| Fumarate | 4.8±0.3 | 6 |
| Dimethylsuccinate | 14.1±0.8 | 13 |
| N-Acetylaspartate | 58.4±1.5 | 43 |
| Maleate | 123.1±7.6 | 85 |
| Malonate | 98.8±7.1 | 71 |
| Pyruvate | 28.1±3.0 | 21 |
| Lactate | 111.2±3.2 | 77 |
| β-Hydroxybutyrate | 134.0±9.0 | 91 |
| Citrate | 18.6±3.0 | 13 |

Since the potent inhibition of drIndy-mediated, succinate transport by pyruvate and citrate was a surprise finding, the ability of drIndy to transport these two compounds was assessed directly by using radiolabelled pyruvate and citrate (Figure 4). The ability of hNaDC3 to transport these two compounds was also assessed under identical conditions, for the purposes of comparison. These experiments showed that drIndy possesses a marked ability to transport citrate (Figure 4A). The uptake of citrate (35 µM) in cells transfected with drIndy cDNA was approximately 30-fold higher than in cells transfected with vector alone. The uptake of pyruvate was also stimulated in these cells as a result of transfection with drIndy cDNA, but the magnitude of stimulation was comparatively much smaller. The increase in pyruvate (135 µM) uptake as a result of transfection with drIndy cDNA was only 1.5-fold compared with transfection with vector alone, but this increase was statistically significant (P<0.05). hNaDC3 differed markedly from drIndy in terms of transport of these two compounds. hNaDC3 exhibited a much higher ability to transport pyruvate than to transport citrate (compare Figures 4B and 4A). These studies show a significant difference between drIndy and hNaDC3 in their relative abilities (i.e. the fold increase in transport in cDNA-transfected cells compared with vector-transfected cells) to transport pyruvate, succinate and citrate when measured under identical conditions (for drIndy: citrate > succinate>pyruvate; and for hNaDC3: succinate >> pyruvate > citrate).

Since drIndy transports succinate in a cation-independent manner, whether the transport of citrate mediated by the transporter is also cation-independent was investigated. The results of these studies show that citrate transport via drIndy is also cation-independent, as is the transport of succinate (Table 3). There was, however, an interesting difference between the transport of these two substrates. While the transport of succinate was not influenced by chloride, the transport of citrate was enhanced markedly when chloride was absent. Thus chloride has differential influence on the transport of succinate and citrate mediated by drIndy.

**TABLE 3**

| *Ion-dependence of drIndy-mediated citrate transport* | | | |
|---|---|---|---|
| Uptake of citrate (35µM) was measured in HRPE cells transfected with either vector alone or drIndy cDNA. The composition of the uptake buffers was as described in Table 1. Data (means±S.E.M.) are from six independent measurements. NMDG, *N*-methyl D-glucamine. | | | |
| Citrate uptake (pmol/10⁶ cells per min) | | | |
| Salt | Vector | drIndy | Fold Increase |
| NaCl | 2.2±0.4 | 57.4±3.3 | 26.1 |
| KCl | 1.7±0.1 | 33.8±2.7 | 19.9 |
| LiCl | 1.9±0.1 | 52.1±2.2 | 27.4 |
| NMDG chloride | 1.9±0.1 | 25.4±1.8 | 13.4 |
| Sodium gluconate | 2.2±0.1 | 144.2±1.6 | 65.6 |
| Mannitol | 1.7±0.1 | 26.3±0.3 | 15.5 |

The intracellular levels of various tricarboxylic acid-cycle intermediates in HRPE cells are not known. Since the influx of succinate in these cells was enhanced by drIndy, whether this influx was coupled with efflux of tricarboxylic acid-cycle intermediates from the cells was investigated. Control cells and drIndy-expressing cells were first incubated in a Na⁺-containing medium for 30 minutes in the absence or presence of 0.1mM succinate, fumarate, malate or 2-oxoglutarate. The cells were then washed, and the influx of [³H]succinate was determined. These studies showed that pre-loading of the cells with these compounds did not facilitate the influx of succinate, suggesting that drIndy-mediated succinate influx does not involve counter-transport of intracellular tricarboxylic acid-cycle intermediates.
drIndy and hNaDC3 exhibit similar affinities for succinate, the Kₘ values for the two transporters being 40±4 (as determined by the present study) and 20±µM (as determined by Wang et al., Am. J. Physiol. (2000);278: C1019-C1030), respectively. The preferential ability of drIndy to transport citrate compared with hNaDC3 indicated that there may be a significant difference in the affinities of these two transporters for citrate. Therefore, the potency of citrate to inhibit the transport of succinate mediated by drIndy and hNaDC3 was compounded under identical conditions (Figure 5). Citrate inhibited the drIndy-mediated transport of succinate with a Kᵢ of 105±35 µM. The corresponding value for hNaDC3 was 2.1±0.3 mM. Thus hNaDC3 exhibits a 20-fold lower affinity than drIndy for citrate.

It has been well established that NaDC1 and NaDC3 mediate the Na⁺-coupled transport of succinate by an electrogenic mechanism. See, for example, Pajor, J. Biol. Chem.(1995);270: 5779-5785; Pajor, Am. J. Physiol. (1996);270: F642-F648; Chen et al., J. Biol. Chem. (1998);273: 20972-20981; Kekuda et al., J. Biol. Chem. (1999);274: 3422-3429; Wang et al., Am. J. Physiol. (2000);278: C1019-C1030; and Chen et al., J. Clin. Invest. (1999);103: 1159-1168. In contrast, the transport process mediated by drIndy occurs via a Na⁺-independent mechanism. This raises the question as to whether or not the drIndy-mediated transport process is electrogenic. To address this issue, the cloned drIndy was expressed in *X. laevis* oocytes and transport function assessed by measuring the uptake of radiolabelled succinate, as well as by monitoring the succinate-induced changes in membrane potential by the two-micro-electrode voltage-clamp method. Water-injected oocytes served as the control. For comparison, these experiments were also performed under identical conditions with oocytes expressing hNaDC3. The results are shown in Figure 6. The uptake of radiolabelled succinate in oocytes injected with drIndy cRNA was 12-fold higher than in oocytes injected with water. This drIndy-induced uptake of succinate was, however, not influenced by the absence of Na⁺ (Figure 6A). In contrast, even though the induction of radiolabelled succinate uptake by hNaDC3 in oocytes was similar to the induction caused by drIndy when measured in the presence of Na⁺, the hNaDC3-induced uptake was abolished completely when Na⁺ was absent in the uptake medium (Figure 6B). The changes in membrane potential were then monitored in oocytes expressing drIndy or hNaDC3 in response to succinate in the medium. Even though drIndy induced the uptake of radiolabelled succinate in oocytes, there was no detectable change in membrane potential associated with the transport process (Figure 6C). This was the case irrespective of whether Na⁺ was present or absent in the medium. In contrast, the presence of succinate in the medium induced marked inward currents in oocytes expressing hNaDC3, and this current was obligatorily dependent on the presence of Na⁺ (Figure 6D). There was no detectable current in oocytes expressing hNaDC3 in response to succinate in the medium when Na⁺ was absent. These data show that the transport process mediated by drIndy is electroneutral.

### DISCUSSION

The drIndy cDNA (SEQ ID NO:1) is 2602 base pairs (bp) long with a poly(A)+ tail. This cDNA is longer than the drIndy mRNA sequence (1872 bp long) reported by Rogina et al. (Science (2000);290: 2137-2140) (GenBank Accession No. NM_079426), which was derived from the *Drosophila* genomic sequence (GenBank Accession No. AE003519). The additional sequence is located in the 5'-untranslated region, as well as in the 3'-untranslated region of the cloned drIndy cDNA. Comparison of the nucleotide sequence of the cloned cDNA (SEQ ID NO:1) with that of the genomic clone reveals that the *Indy* gene consists of nine exons, as deduced by Rogina et al., except that the first exon is 97 bp longer than that reported in this example. Rogina et al. (Science (2000); 290: 2137-2140) predicted this 97 bp sequence to be a part of the first intron, but this portion of the gene is indeed expressed in mRNA, as evidenced from the sequence of the cloned cDNA (SEQ ID NO:1). The start codon is within exon 2 and the stop codon is within exon 9. The drIndy mRNA reported by Rogina et al. provides sequence information only up to the stop codon. The cloned cDNA (SEQ ID NO:1) provides the sequence information on the 3'-untranslated region located in exon 9.

The mammalian proteins most similar in amino acid sequence to drIndy are the Na⁺-coupled dicarboxylate ion transporters NaDC1 and NaDC3. The sequence identity between drIndy and NaDC1 or NaDC3 is 34-35%. Thus, on the basis of the primary structure alone, one cannot predict which one of these two transporters is the mammalian functional counterpart of drIndy. Therefore the functional characterization of drIndy was carried out. This was done in an attempt to establish the functional identity of drIndy, and also to determine which one of the two mammalian Na⁺-coupled dicarboxylate transporters is similar to drIndy in functional characteristics. These studies have led to an unexpected conclusion. Even though drIndy is indeed a succinate transporter, neither NaDC1 nor NaDC3 is the mammalian functional counterpart of drIndy.

There are three important functional differences between drIndy and the two mammalian Na⁺-coupled dicarboxylate transporters. The first notable difference is in the ion-dependence of succinate transport mediated by the three proteins. NaDC1 and NaDC3 are strictly Na⁺-coupled succinate transporters. See, for example, Pajor, J. Biol. Chem.(1995);270: 5779-5785; Pajor, Am. J. Physiol.(1996);270: F642-F648; Chen et al., J. Biol. Chem. (1998);273: 20972-20981; Kekuda et al., J. Biol. Chem. (1999);274: 3422-3429; Wang et al., Am. J. Physiol. (2000);278: C1019-C1030; and Chen et al., J. Clin. Invest. (1999);103: 1159-1168. In the absence of Na⁺, the mammalian transporters do not exhibit any detectable ability to transport succinate. In contrast, Na⁺ is not essential for the transport of succinate via drIndy. The ability of drIndy to mediate the transport of succinate remains the same even when Na⁺ in the medium is replaced iso-osmotically by other univalent inorganic cations, such as K⁺ or Li⁺, or by the non-ionizable organic solute mannitol. The second notable difference is in substrate selectivity. drIndy transports the tricarboxylate citrate much more efficiently than the dicarboxylate succinate.

This is not the case with NaDC1 and NaDC3. These two mammalian proteins transport succinate much more efficiently than citrate. With respect to pyruvate, drIndy possesses a small, but detectable, ability to transport this monocarboxylate. Surprisingly, NaDC3 shows a much higher ability to transport pyruvate. The third important difference is in the electrogenic nature of these transporters. NaDC1 and NaDC3 are electrogenic transporters for which the transport function is associated with a net transfer of positive charge into the cells. In contrast, drIndy is electroneutral. The transport function of drIndy is not associated with membrane depolarization, as evidenced from the absence of substrate- induced inward currents in oocytes functionally expressing drIndy. Thus, whereas the mammalian NaDCs are Na⁺-coupled electrogenic transporters with preference towards dicarboxylate groups, drIndy is a cation-independent electroneutral transporter with preference for the tricarboxylate groups of citrate.

### Example 2

### Structure, Function, and Expression Pattern of a Novel Sodium-coupled Citrate Transporter (NaCT) Cloned from Mammalian Brain

Citrate plays a pivotal role not only in the generation of metabolic energy but also in the synthesis of fatty acids, isoprenoids, and cholesterol in mammalian cells. Plasma levels of citrate are the highest (approximately 135 µM) among the intermediates of the tricarboxylic acid cycle. This example reports on the cloning and functional characterization of a plasma membrane transporter (NaCT for Na⁺-coupled citrate transporter) from rat brain that mediates uphill cellular uptake of citrate coupled to an electrochemical Na⁺ gradient. NaCT consists of 572 amino acids and exhibits structural similarity to the members of the Na⁺-dicarboxylate cotransporter/Na⁺-sulfate cotransporter (NaDC/NaSi) gene family including the recently identified *Drosophila* Indy. In rat, the expression of NaCT is restricted to liver, testis, and brain. When expressed heterologously in mammalian cells, rat NaCT mediates the transport of citrate with high affinity (Michaelis-Menten constant, approximately 20 µM) and with a Na⁺:citrate stoichiometry of 4:1. The transporter does interact with other dicarboxylates and tricarboxylates but with considerably lower affinity. In mouse brain, the expression of NaCT mRNA is evident in the cerebral cortex, cerebellum, hippocampus, and olfactory bulb. NaCT represents the first transporter to be identified in mammalian cells that shows preference for citrate over dicarboxylates. This transporter is likely to play an important role in the cellular utilization of citrate in blood for the synthesis of fatty acids and cholesterol (liver) and for the generation of energy (liver and brain). NaCT thus constitutes a potential therapeutic target for the control of body weight, cholesterol levels, and energy homeostasis.

The cloning of *Drosophila* Indy, presented in Example 1, produced unexpected results. *Drosophila* Indy does possess the ability to transport succinate as do mammalian NaDCs but the transport is Na⁺-independent. Furthermore, unlike mammalian NaDCs, *Drosophila* Indy transports citrate very effectively. The affinity for citrate is several fold greater in the case of *Drosophila* Indy than in the case of mammalian NaDCs. These findings suggested that neither NaDC 1 nor NaDC3 is the mammalian ortholog of *Drosophila* Indy. Therefore, the Genbank database was searched to see if there are additional transporters in mammals that are structurally related to NaDC1 and NaDC3. This search led to the identification of a novel mammalian transporter that is structurally similar to mammalian NaDCs as well as to *Drosophila* Indy. Interestingly, this new transporter transports citrate much more effectively than succinate, a characteristic more similar to that of *Drosophila* Indy than to that of mammalian NaDCs. However, the transport process is coupled to Na⁺, a feature distinct from that of *Drosophila* Indy but similar to that of mammalian NaDCs. Based on these functional characteristics, this novel transporter has been designated NaCT (for Na⁺-coupled citrate transporter). This represents the first sodium-coupled transporter in mammals to be identified that shows preference for citrate as a substrate.

### EXPERIMENTAL PROCEDURES

Materials. [¹⁴C]Citrate (specific radioactivity, 55 mCi/mmol), [³H]succinate (specific radioactivity, 40 Ci/mmol), and [¹⁴C]pyruvate (specific radioactivity, 15 mCi/mmol) were purchased from Moravek Biochemicals (Brea, CA). The human retinal pigment epithelial (HRPE) cell line was maintained in Dulbecco's minimum essential medium/F-12 medium supplemented with 10% fetal bovine serum, 100 units/ml penicillin, and 100 µg/ml streptomycin. Lipofectin was purchased from Invitrogen. Restriction enzymes were obtained from New England Biolabs (Beverly, MA). Magna nylon transfer membranes used in library screening were purchased from Micron Separations (Westboro, MA). Unlabeled monocarboxylates, dicarboxylates, and tricarboxylates were obtained from Sigma.

Cloning of NaCT from Rat Brain. A search of the Genbank database for murine established sequence tags (ESTs) with the amino acid sequence of *Drosophila* Indy as a query identified several ESTs whose predicted amino acid sequences showed significant similarity to that of mammalian sodium-coupled dicarboxylate transporters NaDC1 and NaDC3. Many of these ESTs were identical to murine NaDC1 and NaDC3, which have been already cloned and functionally characterized. Pajor et al., Am. J. Physiol. (2001);280: C1215-C1223; and Pajor and Sun, Am. J. Physiol. (2000);279: F482-F490. However, there were three ESTs (Genbank Accession Nos. BB261903, BB393630, and BB641100) with overlapping nucleotide sequences, and the predicted amino acid sequence from these ESTs did not correspond to that of murine NaDC1 or NaDC3. However, there was significant similarity between this sequence and that of NaDC1, NaDC3, and *Drosophila* Indy. There is no information in the literature on the functional identity of this new putative mammalian transporter. The sequence similarity however suggested that it might represent a new member of the sodium/dicarboxylate cotransporter gene family. The overlapping nucleotide sequences of two of these ESTs were used to design primers for RT-PCR to obtain a cDNA probe that is specific for this transporter. The forward primer was 5'-TCTTTTCTCCCTCCAGTCAGT-3' (SEQ ID NO:17) and the reverse primer was 5'-GGCAATCTTCTCGGTGTC-3' (SEQ ID NO:18). The predicted size of the RT-PCR product, based on the positions of the primers, was 943 bp. Since both of these ESTs were identified from a mouse cerebral cortex cDNA library, poly(A) RNA from mouse brain was used as the template for RT-PCR to obtain the probe. This yielded a cDNA product of expected size. The product was subcloned in pGEM-T vector and sequenced to establish its molecular identity. The cDNA was labeled with [α-³²P]dCTP by random priming using the ready-to-go oligolabeling beads (Amersham Biosciences). This probe was used to screen a rat brain cDNA library under medium stringency conditions. The screening of the library was as described by Helfand and Rogina (Cell Differ. (2000);29: 67-80) and Kekuda et al. (J. Biol. Chem. (1998);273: 15971-15979). Positive clones were purified by secondary or tertiary screening. The library used here was originally established in pSPORT1 vector at SalI/NotI site using RNA isolated from rat total brain (Rajan et al., J. Biol. Chem.(1999);274: 29005-29010; Seth et al., J. Neurochem. (1998);70: 922-931; Wang et al., Am. J. Physiol.(1998); 275: C967-C975; and Huang et al., J. Pharmacol. Exp. Ther. (2000);295: 392-403). The cDNA inserts in the vector were under the control of T7 promoter.

DNA Sequencing. Both sense and antisense strands of the cDNA were sequenced by primer walking. Sequencing was performed by Taq DyeDeoxy terminator cycle sequencing using an automated PE Applied Biosystems 377 Prism DNA sequencer. The sequence was analyzed using the National Center for Biotechnology Information server on the world wide web at ncbi.nlm.nih.gov.

Northern Analysis. The expression pattern of NaCT mRNA in rat tissues was analyzed by Northern hybridization using a commercially available rat multiple tissue blot. The blot was hybridized with a ³²P-labeled rat NaCT-specific cDNA probe under high stringency conditions. The same blot was then hybridized with a cDNA probe specific for rat -actin as an internal control to detect the presence of RNA in each lane.

Functional Expression in HRPE Cells. The cloned transporter was expressed heterologously in HRPE cells using the vaccinia virus expression technique as described by Kekuda et al., J. Biol. Chem. (1998);273: 15971-15979; Rajan et al., J. Biol. Chem. (1999);274: 29005-29010; Seth et al., J. Neurochem. (1998);70: 922-931; Wang et al., Am. J. Physiol. (1998); 275: C967-C975; Huang et al., J. Pharmacol. Exp. Ther. (2000);295: 392-403; and Nakanishi et al., Am. J. Physiol. (2001);281: C1757-C1768). This is a transient expression system. Functional analysis of the cloned transporter was carried out with this experimental system. Briefly, subconfluent HRPE cells grown on 24-well plates were first infected with a recombinant (VTF7-3) vaccinia virus encoding T7 RNA polymerase and then transfected with the plasmid carrying the full-length rat NaCT cDNA. For comparison with the newly cloned transporter, rat NaDC3 cDNA isolated from a placental cDNA library (Kekuda et al., J. Biol. Chem. (1999);274: 3422-3429) was used in some of the functional studies. After 12-15 hours post-transfection, uptake measurements were made at 37° C with radiolabeled citrate, succinate, or pyruvate. The uptake medium in most experiments was 25 mM Hepes/Tris (pH 7.5), containing 140 mM NaCl, 5.4 mM KCI, 1.8 mM CaCl₂,0.8 mM MgSO₄ and 5 mM glucose. The time of incubation was 15 minutes for citrate and 1 minute for succinate. These time periods were chosen from time course studies with respective substrates to represent linear uptake conditions. Endogenous uptake was always determined in parallel using the cells transfected with pSPORT1 vector alone. The uptake activity in cDNA-transfected cells was adjusted for the endogenous uptake activity to calculate the cDNA-specific activity. In experiments in which the cation and anion dependence of the uptake process was investigated, NaCl was replaced isoosmotically with LiCl, KCl, N-methyl-D-glucamine (NMDG) chloride, sodium gluconate, or mannitol. When the influence of pH on the uptake process was investigated, uptake buffers of different pH values were prepared by appropriately altering the concentrations of Tris, Mes, and Hepes. Saturation kinetics was evaluated by non-linear regression analysis, and the kinetic parameters derived from this method were confirmed by linear regression analysis. The Na⁺:citrate stoichiometry was calculated by measuring citrate uptake with varying concentrations of Na⁺ and then by analyzing the data according to Hill equation. The concentrations of Na⁺ were varied by appropriately changing the concentrations of NaCl and NMDG chloride without changing the osmolality. The influence of membrane potential on the uptake process was investigated by measuring the uptake in the presence of high concentrations of K⁺, a condition, which leads to depolarization of the membrane. Experiments were done in duplicate or triplicate, and each experiment was repeated two or three times. Results are expressed as means ± standard error (S.E.).

*In Situ* Hybridization. The expression pattern of NaCT mRNA in mouse brain was investigated by *in situ* hybridization as previously described in Huang et al., J. Pharmacol. Exp. Ther. (2000);295: 392-403; Wu et al., J. Biol. Chem. (1998);273: 32776-32786; Wu et al., J. Pharmacol. Exp. Ther. (1999);290: 1482-1492; Wu et al., Biochim. Biophys. Acta (2000);1466: 315-327; Seth et al., Biochim. Biophys. Acta (2001);1540: 59-67; Bridges et al., J. Biol. Chem. (2000);275: 20676-20684; and Ola et al., Brain Res. Mol. Brain Res. (2001);95: 86-95. For the preparation of the mouse NaCT-specific riboprobe, as the template a 363 bp cDNA derived by PCR from the approximately 0.9 kilobasepair (kbp) RT-PCR product that was generated with mouse brain RNA was used. This cDNA was subcloned in pGEM-T vector, and T7 RNA polymerase and SP6 RNA polymerase were used to prepare the sense and antisense riboprobes after linearization of the plasmid with appropriate restriction enzymes. The riboprobes were labeled using a digoxigenin-labeling kit (Roche Molecular Biochemicals).

Whole brains from mice were frozen immediately in Tissue-Tek OCT, sectioned at 10-µm thickness, and fixed in 4% paraformaldehyde. Sections were rinsed in ice-cold phosphate-buffered saline and treated with 1% diethylpyrocarbonate. Permeabilization of the sections was carried out with proteinase K (1 µg/ml) in phosphate-buffered saline for 4 minutes. Proteinase K activity was terminated by rinsing the sections with glycine (2 mg/ml) in phosphate-buffered saline. Sections were then washed in phosphate-buffered saline, equilibrated in 5× SSC (100 mM NaCl/15 mM sodium citrate), and prehybridized for 2 hours at 58° C in 50% (v/v) formamide, 5× SSC, 2% (w/v) blocking agent (provided with the digoxigenin nucleic acid detection kit), 0.1% (w/v) N-laurylsarcosine, and 0.02% (w/v) sodium dodecyl sulfate. Sections were hybridized with digoxigenin-UTP-labeled antisense or sense (negative control) riboprobes (1 µg/ml) and were incubated overnight at 58° C. Post-hybridization washings were done twice in 2× SSC at 58° C, twice in 1× SSC at 55° C, and twice in 0.1× SSC at 37° C. Hybridization signals were then detected immunologically by using an antibody specific for digoxigenin. This was done by first washing the sections in a buffer containing 0.1 M maleic acid and 0.15 M NaCl (pH 7.5) and then exposing the washed sections to 1% blocking agent in the same buffer. Sections were then incubated with alkaline phosphatase-coupled anti-digoxigenin antibody (1 in 5000 dilution) overnight at 4° C. Following this, sections were washed in the preceding wash buffer containing levamisol (0.2 mg/ml) twice for 10 minutes and equilibrated with 100 mM Tris/HCl buffer (pH 9.5) containing 100 mM NaCl and 50 mM MgCl₂. The color reaction was developed in NBT/BCIP (4-nitroblue tetrazolium chloride/5-bromo-4-chloro-3-indolyl phosphate). Slides were washed in distilled water and coverslipped but not counterstained so that the purplish-red colored precipitate, indicative of a positive reaction, could be visualized in the sections.

### RESULTS

Structural Features of Rat NaCT. As shown in Figure 7, the cloned rat NaCT cDNA (SEQ ID NO:3) is 3254 bp long with a poly(A) tail and an open reading frame (24-1742 bp) coding for a protein of 572 amino acids (SEQ ID NO:4). The nucleotide sequence of rat NaCT cDNA is also available as Genbank Accession No. AF522186. This rat transporter is new and has not been reported previously in the literature. Based on the amino acid sequence, rat NaCT belongs to the NaDC/NaSi (sodium-dicarboxylate cotransporter/sodium-sulfate cotransporter) gene family which, to date, consists of three sodium-coupled dicarboxylate transporters (NaDC1, NaDC2, and NaDC3) and two sodium-coupled sulfate transporters (NaSi and SUT1) (Pajor, Annu. Rev. Physiol. (1999);61: 663-682). However, the rat NaCT is structurally more closely related to sodium-coupled dicarboxylate transporters than to sodium-coupled sulfate transporters. It shows a 44-50% sequence identity with rat NaDCs (Fig. 8) (Inoue et al., J. Biol. Chem. (2002);277: 39469-39476).

It also shows significant structural similarity to *Drosophila* Indy (34% identity). Hydropathy analysis suggests that rat NaCT possesses eleven putative transmembrane domains, similar to the previously known sodium-coupled dicarboxylate transporters. A search of the Genbank database using the rat NaCT amino acid sequence as a query has revealed that a putative human ortholog of rat NaCT is located on chromosome 17 upstream of the gene coding for NaDC1 (17p13 in the case of the gene coding for NaCT and 17p11.1-q11.1 in the case of the gene coding for NaDC1) (Pajor, Am. J. Physiol. (1996);270: F642-F648).

Tissue Expression Pattern of NaCT mRNA in the Rat. Northern blot analysis shows that NaCT mRNA (approximately 3.2 kb) is expressed in a restricted manner in rat tissues. The expression is evident only in the liver, testis, and brain (Fig. 9). The level of expression in the liver and testis is much higher than in the brain. There is only a single transcript detectable.

Functional Features of Rat NaCT. The transport function of rat NaCT was assessed in a heterologous expression system in a mammalian cell line (HRPE) using the vaccinia virus expression technique. Since rat NaCT shows high structural similarity to the sodium-coupled dicarboxylate transporters, the ability of rat NaCT to transport succinate in the presence of a Na⁺ gradient was first tested (Fig. 10A). The uptake of succinate (80 nM) in cells transfected with rat NaCT cDNA was about 7-fold higher than in cells transfected with vector alone, indicating that the cloned transporter does possess the ability to mediate the uptake of this dicarboxylate. It was then tested whether pyruvate (a monocarboxylate) and citrate (a tricarboxylate) are recognized as transport substrates by rat NaCT. Surprisingly, the uptake of both of these compounds (pyruvate, 135 µM; citrate, 35 µM) was higher in cDNA-transfected cells than in vector-transfected cells. The cDNA-induced increase in pyruvate uptake was marginal (approximately 3-fold) whereas the increase was much more marked in the case of citrate (approximately 90-fold). This was very interesting because the previously described NaDCs possess much lower ability to transport citrate than to transport succinate (Pajor, Annu- Rev. Physiol. (1999);61: 663-682). Since rat NaCT was able to transport citrate preferentially, this tricarboxylate was used as a substrate for subsequent functional characterization of the transporter. The cDNA-mediated uptake of citrate was linear even up to 30 minutes (Fig. 10B). The uptake process operated maximally at pH 7 (Fig. 10C).

The involvement of Na⁺ in the uptake process mediated by rat NaCT was evaluated by monitoring the uptake of citrate in vector-transfected cells and in rat NaCT cDNA-transfected cells in the presence and absence of Na. This was done by isoosmotically replacing NaCl in the uptake medium with NMDG chloride, KCl, LiCl, or mannitol (Table 4). The cDNA-specific uptake was almost completely abolished when Na⁺ was removed from the medium. The uptake process was however not dependent on Cl because the replacement of Cl with gluconate had only a small effect on the cDNA-specific citrate uptake.

The substrate specificity of rat NaCT was examined by competition studies in which the ability of various unlabeled compounds (2.5 mM) to compete with [¹⁴C]citrate (7 µM) for uptake via rat NaCT (Table 5) was assessed. Unlabeled citrate was the most potent inhibitor of [¹⁴C]citrate uptake mediated by rat NaCT. Among the dicarboxylates, succinate and malate were very potent inhibitors. Fumarate and α-ketoglutarate were comparatively less effective. Maleate, the cis isomer of fumarate, was one of the least potent inhibitors, showing stereoselectivity of the transporter. The monocarboxylates pyruvate and lactate were not effective in inhibiting [¹⁴C]citrate uptake.

**TABLE 4**

| *Ion dependence of citrate uptake via rat NaCT* | | | | |
|---|---|---|---|---|
| Uptake of [¹⁴C]citrate (7 µM) was measured in vector-transfected HRPE cells and in rat NaCT cDNA-transfected HRPE cells at pH 7.5 in the presence of various inorganic salts or mannitol. Values represent means ± S.E. | | | | |
| Citrate Uptake | | | | |
| Salt | Vector | cDNA | cDNA-specific | |
| | | *pmol*/*10⁶ cells*/*min* | | % |
| NaCl | 0.84 ± 0.14 | 38.8 ± 3.3 | 38.0 ± 3.3 | 100 |
| NMDG chloride | 1.22 ± 0.22 | 1.6 ± 0.3 | 0.4 ± 0.3 | 1 |
| KCI | 0.49 ± 0.03 | 0.6 ± 0.1 | 0.1 ± 0.1 | 0 |
| LiCl | 0.51 ± 0.02 | 1.6 ± 0.1 | 1.1 ± 0.1 | 3 |
| Sodium gluconate | 0.48 ± 0.03 | 29.6 ± 2.3 | 29.1 ± 2.3 | 77 |
| Mannitol | 0.59 ± 0.03 | 0.7 ± 0.1 | 0.1 ± 0.1 | 0 |

Kinetic Features of Rat NaCT. Citrate uptake mediated by rat NaCT was saturable with a K*ₜ* of 18 ± 4 µM (Fig. 11A). Kinetic analysis of Na⁺ dependence of citrate uptake via rat NaCT showed that the relationship between the uptake and Na⁺ concentration was sigmoidal, indicating the involvement of multiple Na⁺ ions per transport cycle (Fig. 11B). The data were analyzed by Hill equation to determine the Na⁺:citrate stoichiometry. This analysis gave a value between 3 and 4 (3.3 ± 0.3) for the Hill coefficient (nH). Since citrate exists predominantly as a trivalent anion under the experimental conditions (i.e. pH 7.5), cotransport of citrate with 3 Na⁺ ions will render the transport process electrically silent whereas cotransport with 4 Na⁺ ions will render the process electrogenic. Therefore, whether or not the rat NaCT-mediated citrate uptake in the presence of Na⁺ is influenced by membrane potential was investigated. For this purpose, the cDNA-specific citrate uptake between control conditions and membrane depolarizing conditions (i.e. high extracellular K⁺ (Fig. 11C) was compared. The uptake was inhibited significantly (43 ± 2%) when membrane was depolarized, indicating that the uptake process is influenced by membrane potential. Since depolarization inhibits the uptake, one can conclude that the uptake process mediated by rat NaCT is electrogenic associated with a net transfer of positive charge into the cells. This would then suggest that at least 4 Na⁺ are cotransported with one citrate in the transport process.

**TABLE 5**

| *Substrate specificity of rat NaCT* | | |
|---|---|---|
| Uptake of [¹⁴C]citrate (14 µM) was measured in vector-transfected HRPE cells and in rat NaCT cDNA-transfected HRPE cells in the absence or presence of various monocarboxylates, dicarboxylates, or tricarboxylates (2.5 mM). Data (means ± S.E.) represent only cDNA-specific uptake. | | |
| Unlabeled compound | [¹⁴C]Citrate uptake | |
| | *Pmol*/*10⁶ cells*/*min* | % |
| None | 108.3 ± 5.5 | 100 |
| Citrate | 1.5 ± 0.1 | 1 |
| Succinate | 5.7 ± 0.2 | 5 |
| Malate | 7.8 ± 0.1 | 7 |
| Fumarate | 17.7 ± 1.4 | 16 |
| α-Ketoglutarate | 36.5 ± 2.1 | 34 |
| Maleate | 78.8 ± 1.5 | 73 |
| Pyruvate | 89.4 ± 7.2 | 83 |
| Lactate | 89.6 ± 2.0 | 83 |

The affinities of rat NaCT for different tricarboxylates (citrate, isocitrate, and cis-aconitate) and dicarboxylates (succinate, fumarate, and α-ketoglutarate) was then compared by monitoring the potencies of these compounds to inhibit the uptake of [¹⁴C]citrate (14 µM) mediated by rat NaCT (Fig. 12A). Among these compounds, citrate was the most potent inhibitor of rat NaCT-mediated [¹⁴C]citrate uptake. The inhibitory potencies of other compounds were in the following order: succinate > fumarate = cis-aconitate > α-ketoglutarate. While citrate was the most potent inhibitor, isocitrate had no detectable inhibitory effect. The IC₅₀ values (i.e. concentrations of inhibitors causing 50% inhibition) calculated for citrate and succinate, the two most potent inhibitors, from these dose-response curves are 28 ± 4 and 172 ± 24 µM, respectively. The IC₅₀ for other compounds are several fold higher than these values. Using the IC₅₀ values for citrate and succinate, the corresponding inhibition constants was calculated by the method of Cheng and Prusoff (Cheng et al., Biochem Pharmacol.(1973);22: 3099-3108). The inhibition constants for these two compounds are 16 ± 2 and 98 ± 14 µM, respectively. These data show that, among the compounds tested, rat NaCT possesses the highest affinity for citrate. Thus, the affinity of rat NaCT for succinate is 6-fold lower than that for citrate. The affinities for other dicarboxylates and tricarboxylates are even lower. Thus, citrate is relatively a specific substrate for rat NaCT.

For comparison, the relative affinity of rat NaDC3 for these compounds was analyzed. This was done in a similar way by assessing the potencies of these compounds to inhibit the uptake of [³H]succinate (80 nM) mediated by rat NaDC3 cloned from placenta using the same heterologous expression system (Fig. 12B). The results show that rat NaDC3 interacts with dicarboxylates (succinate, fumarate, and α-ketoglutarate) much more preferentially than with tricarboxylates (citrate, isocitrate, and cis-aconitate). The inhibition constants for the three dicarboxylates are in the range of 15-60 µM whereas the corresponding values for the three tricarboxylates are in the range of 2-4 mM. Thus, rat NaDC3 recognizes a number of dicarboxylates as preferential substrates and has very low affinity for tricarboxylates. This qualifies rat NaDC3 to be recognized as a sodium-coupled dicarboxylate transporter. On the contrary, rat NaCT transports citrate but interacts with other tricarboxylates very poorly. It has to be noted that this transporter does recognize succinate as a substrate, but its affinity for succinate is several fold lower than for citrate.

Furthermore, other dicarboxylates interact with the transporter very poorly. Based on these data, the newly cloned transporter was identified as a sodium-coupled citrate transporter.

Next the physiological relevance of the differential substrate specificity of rat NaCT and rat NaDC3 was assessed. Plasma contains significant levels of citrate (approximately 25 mg/liter) and succinate (approximately 5 mg/liter). A-ketoglutarate, oxaloacetate, fumarate, and malate are also present in the plasma but at much lower levels. Normal fasting levels of citrate and succinate in the plasma are approximately 135 and approximately 40 µM, respectively. The plasma levels of citrate increase by 25-30% above the fasting levels after meal. To investigate the physiological relevance of the substrate specificity of NaCT and NaDC3, the ability of these two transporters to mediate the transport of citrate and succinate in the presence of a Na⁺ gradient when the medium contained physiological concentrations of these two compounds (i.e., 135 µM citrate and 40 µM succinate) was assessed. Under these conditions, the values for succinate uptake mediated by rat NaCT and rat NaDC3 were 0.005 ± 0.001 and 0.48 ± 0.01 nmol/10⁶ cells/minute, respectively. In contrast, the values for citrate uptake mediated by these two transporters were 0.24 ± 0.01 and 0.04 ± 0.01 nmol/10⁶ cells/minute, respectively. These data show that, at physiological concentrations of succinate and citrate found in plasma, NaDC3 functions primarily to transport succinate whereas NaCT functions primarily to transport citrate.

Analysis of Expression Pattern of NaCT mRNA in Mouse Brian by *In Situ* Hybridization. To determine the expression pattern of NaCT mRNA in mouse brain, sagittal sections of mouse brain were subjected to *in situ* hybridization with digoxigenin-labeled riboprobes specific for mouse NaCT. Hybridization with antisense probe has revealed that the NaCT mRNA is expressed widely in the mouse brain, primarily in the neurons of the cerebral cortex, hippocampal formation, cerebellum, and olfactory bulb (Fig. 13A). The signals are specific as evidenced from markedly reduced signals with sense probe (Fig. 13B). In the cerebellum, the hybridization signals with antisense probe are most intense in the Purkinje cell bodies, followed by the neurons in the granular layer (Fig. 13C and 13D). Expression is also evident in the interneurons of the molecular layer, namely the stellate cells located in the outer portion of the molecular layer and the basket cells located close to the border between the molecular and Purkinje layers. While the white matter is negative for hybridization signal, the deep cerebellar nuclei are intensely positive for expression. In the hippocampal formation (Fig. 13E and 13F), the pyramidal cells in the cornus ammonis regions CA3, CA2, and CA1 are highly positive for mRNA expression, followed by neurons in the subiculum. Granule cells, which are the projection neurons of the dentate gyrus, are intensely positive for the hybridization signal. Similarly, the interneurons of the polymorphic layer in the dentate gyrus are also positive for expression. The molecular layer that has very few cell bodies is largely negative. In the cerebral cortex, the expression is widespread. Based on the expression pattern in the hippocampal formation and cerebellum, the observed expression in the cerebral cortex is likely to be restricted primarily to neurons.

### DISCUSSION

Functional Differences between NaCT and NaDC1/NaDC3. NaCT represents the newest member of the NaDC/NaSi gene family. Structurally and functionally, NaCT is closely related to NaDC1 and NaDC3. But, there are significant differences among these three transporters in terms of substrate specificity, substrate affinity, and tissue expression pattern. NaCT is Na⁺-coupled and exhibits much higher affinity for citrate than for succinate. This is in contrast to NaDC1 and NaDC3 that, though Na⁺-coupled as NaCT, exhibit much higher affinity for succinate than for citrate. It has to be pointed out here that NaDC1 and NaDC3 do transport citrate, but only the divalent form of citrate is recognized as a substrate by these two transporters (Pajor, Annu. Rev. Physiol. (1999);61: 663-682).

In contrast, it is the trivalent form of citrate that serves as the substrate for NaCT. This conclusion is supported by the differential influence of pH on citrate uptake via NaCT, NaDC1, and NaDC3. Even though a change of pH from 8.5 to 7.0 enhances citrate uptake via NaCT, further decrease in pH actually interferes with uptake. Citrate (pK1, 3.1, pK2, 4.8, and pK3, 6.4) exists predominantly (approximately 75%) as a trivalent anion at pH 7.0 and NaCT-mediated uptake of citrate is maximal under these conditions. The fraction of the divalent form of citrate becomes significantly greater when the pH is made more acidic than pH 7.0. If the divalent form of citrate is the preferred substrate for NaCT, the uptake is expected to increase at pH more acidic than 7.0. Instead, the uptake decreases markedly when the pH is made more acidic. This is in contrast to NaDC3, which transports citrate at a much higher rate at pH 6.0 than at pH 7.5 (Wang et al., Am. J. Physiol. (2001);278: C1019-C1030). Similar results have been obtained with NaDC1 (Pajor, Annu. Rev. Physiol. (1999);61: 663-682). Additional evidence for the transport of citrate via NaCT as a trivalent anion stems from the Na⁺:citrate stoichiometry. The Hill coefficient is greater than 3 and the uptake process at pH 7.5 is electrogenic. Since citrate exists 90% as a trivalent anion at pH 7.5, the electrogenic nature of the uptake process suggests that the number of Na⁺ ions involved in the process is greater than 3. In the case of NaDC1 and NaDC3, the number of Na⁺ ions involved in the uptake process is 3 and the process is still electrogenic, suggesting that these transporters prefer to interact with the divalent anionic forms of their substrates.

Another notable difference between NaCT and the two isoforms of NaDC is in substrate selectivity. NaCT is relatively very specific for citrate. Even though it recognizes the tricarboxylate citrate as its substrate, it interacts very poorly with other structurally related tricarboxylates such as isocitrate and cis-aconitate. NaCT does transport succinate, but its affinity for this dicarboxylate is 6-fold less than for citrate. In contrast, NaDC3 exhibits comparable affinity for several structurally related dicarboxylates such as succinate, malate, fumarate, oxaloacetate, and α-ketoglutarate. Furthermore, NaDC3 does not differentiate among the tricarboxylates citrate, isocitrate, and cis-aconitate though the affinity of the transporter for these tricarboxylates is several fold lower than for the dicarboxylates. This indicates that NaDC3 has broad substrate selectivity among the dicarboxylates and the divalent forms of tricarboxylates. The same is true with NaDC1 (Pajor, Annu. Rev. Physiol. (1999);61: 663-682). This is not the case with NaCT. The substrate selectivity of NaCT is comparatively more restricted. This is particularly evident from the lack of interaction between NaCT and isocitrate. Citrate differs from isocitrate only in the position of the hydroxyl group. Thus, among the tricarboxylates tested, citrate has the highest affinity for NaCT whereas isocitrate has no detectable affinity for the transporter. These data indicate that NaCT is primarily a citrate transporter. This is especially appreciable when the abilities of NaCT and NaDC3 to transport succinate or citrate are compared under physiologcial conditions with plasma concentration of citrate far exceeding the combined concentrations of other potential substrates. Under these conditions, NaCT transports primarily citrate whereas NaDC3 transports primarily succinate. Therefore, NaCT does not possess broad specificity toward structurally related tricarboxylates. Its substrate selectivity is essentially restricted to citrate. On the other hand, NaDC1 and NaDC3 are Na⁺-coupled transporters with broad specificity toward structurally related dicarboxylates.

Differences in Tissue Expression Pattern Between NaCT and NaDC1/NaDC3. In the rat, NaCT mRNA is expressed primarily in the liver, testis, and brain. In contrast, NaDC1 mRNA is expressed mostly in the small intestine and kidney whereas NaDC3 mRNA is expressed primarily in the kidney, small intestine, liver, placenta, and brain (Chen et al., J. Biol. Chem. (1998);273: 20972-20981, and Kekuda et al., J. Biol. Chem. (1999);274: 3422-3429). Thus, these three transporters differ significantly in the expression pattern in rat tissues. Even though NaCT mRNA and NaDC3 mRNA are expressed in rodent brain, the distribution pattern is very different. NaCT mRNA is detectable very widely in the brain. The expression is most abundant in the hippocampal formation, cerebellum, cerebral cortex, and olfactory bulb. On the other hand, NaDC3 mRNA is expressed primarily in the meningeal layers of supporting tissue that surround the brain. Its expression is evident but weak in the cerebral cortex, hippocampus, and cerebellum. Furthermore, NaCT mRNA expression is found mostly in neurons whereas NaDC3 mRNA expression occurs mostly in glial cells.

Physiologic and Therapeutic Significance of NaCT as a Citrate Transporter. NaCT is the first plasma membrane transporter described in mammals which functions primarily in the cellular uptake of citrate. The plasma concentration of citrate is 135 µM. Since the *Kₜ* for the transport of citrate via NaCT is approximately 20 µM, the transporter is likely to play an efficient role in the cellular entry of citrate under physiological conditions. Citrate occupies a pivotal position in cellular metabolism. It is not only an intermediate in the citric acid cycle that is the primary site of biological energy production in most cells, but also is a source of cytosolic acetyl CoA for the synthesis of fatty acids, isoprenoids, and cholesterol and for the elongation of fatty acids. Acetyl CoA present in the cytoplasm originates from citrate produced within mitochondria. A tricarboxylate transporter located in the inner mitochondrial membrane mediates the electroneutral efflux of citrate from the mitochondrial matrix in exchange for cytosolic malate or succinate. Following the entry into the cytoplasm, citrate is cleaved by ATP-citrate lyase to generate acetyl CoA.

The identification of NaCT as a plasma membrane citrate transporter indicates that citrate in the circulation may serve as an important source of cytoplasmic citrate. NaCT is a highly concentrative transporter with a Na⁺:citrate stoichiometry of 4:1. It is electrogenic and thus the cellular entry of citrate via NaCT is energized not only by a Na⁺ gradient but also by the membrane potential. Citrate that enters the cells via NaCT may either serve as a precursor for the biosynthesis of fatty acids and cholesterol or enter the mitochondrial matrix to serve as an intermediate in the citric acid cycle. The choice between these two pathways will depend on the hormonal milieu and metabolic state of the cell. The expression of NaCT in the liver is of physiologic importance in this respect because this organ plays a vital role in the synthesis of fatty acids, isoprenoids, and cholesterol. Therefore, NaCT will be an important therapeutic target for controlling hepatic production of fatty acids and cholesterol, with a selective blocker or inhibitor of this transporter preventing hepatic utilization of citrate in blood in these biosynthetic processes. Since NaCT is expressed in the brain mostly in neurons, this transporter will play an important role in supplying citrate for these cells as a metabolic precursor for production of ATP via citric acid cycle.

Interestingly, even though the mitochondrial tricarboxylate transporter (Palmieri, FEBS Lett. (1994);346: 48-54; Kaplan et al., J. BioL Chem. (1993);268: 13682-13690) and the plasma membrane NaCT transport citrate, there is no structural similarity between these two transporters. The mitochondrial tricarboxylate transporter consists of 298 amino acids and possesses six putative transmembrane domains (Kaplan et al., J. Biol. Chem. (1993);268: 13682-13690). The membrane topology of this transporter is similar to that of several other transporters in the inner mitochondrial membrane (Palmieri, FEBS Lett. (1994);346: 48-54). In contrast, the plasma membrane NaCT is a much larger protein and its membrane topology, with a predicted eleven transmembrane domains, is different from that of the mitochondrial tricarboxylate transporter. Furthermore, these two transporters also differ in energetics. The mitochondrial tricarboxylate transporter is an electroneutral exchanger and there is no role for a Na⁺ gradient in the transport process. In contrast, NaCT is driven by an electrochemical Na⁺ gradient. Some microbial organisms possess a sodium-coupled citrate transporter (van der Rest et al., J. Biol. Chem. (1992);267: 8971-8976), but there is no significant structural homology between this transporter and mammalian NaCT.

Is NaCT the Mammalian Ortholog of *Drosophila* Indy? *Indy* is a gene in *Drosophila* which, when made dysfunctional, leads to an extension of the average life span of the organism (Rogina et al., Science (2000);290: 2137-2140). The putative protein product of this gene is structurally similar to mammalian NaDC1 and NaDC3 and therefore it was suggested that *Drosophila* Indy is most likely the species-specific ortholog of either NaDC1 or NaDC3 (Rogina et al., Science (2000);290: 2137-2140). In Example 1 a functional clone of *Drosophila* Indy was isolated and its transport function characterized. *Drosophila* Indy functions as a Na⁺-independent, electroneutral transporter for a variety of citric acid cycle intermediates. The functional characteristics of *Drosophila* Indy are different from those of NaDC1 and NaDC3. Therefore, neither NaDC1 nor NaDC3 represents the mammalian ortholog of *Drosophila* Indy. The newly identified NaCT is the newest member of the NaDC/NaSi gene family in mammals.

NaCT represents the mammalian ortholog of *Drosophila* Indy. *Drosophila* Indy transports citrate much more effectively than it does succinate. In this respect, the NaCT resembles *Drosophila* Indy. Furthermore, there is also significant similarity between the tissue expression pattern of NaCT in mammals and that of Indy in *Drosophila.* NaCT is expressed abundantly in mammalian liver, a highly metabolic organ involved in fatty acid and cholesterol biosynthesis and energy storage. Similarly, in *Drosophila*, Indy is expressed abundantly in the fat body, an organ of metabolic function compared with that of liver in mammals. However, the two transporters differ in their energetics. While *Drosophila* Indy is a Na⁺-independent transporter for citrate, NaCT is a Na⁺-coupled transporter for citrate. Furthermore, *Drosophila* Indy is electroneutral with no role for membrane potential in the transport process mediated by the transporter. In contrast, NaCT is electrogenic with its transport function associated with membrane depolarization. Thus, the two transporters are similar in substrate selectivity and tissue expression pattern but are different in their transport mechanism.

### Example 3

### Human Na⁺-coupled Citrate Transporter (NaCT): Primary Structure, Genomic Organization, and Transport Function.

This example describes the cloning and functional characterization of the human Na⁺-coupled citrate transporter (NaCT). The cloned human NaCT shows 77% sequence identity with rat NaCT. The *nact* gene is located on human chromosome 17 at p12-13. NaCT mRNA is expressed most predominantly in the liver, with moderate expression detectable in the brain and testis. When functionally expressed in mammalian cells, human NaCT mediates the Na⁺-coupled transport of citrate. Studies with several monocarboxylates, dicarboxylates, and tricarboxylates show that the transporter is selective for citrate with comparatively several-fold lower affinity for other intermediates of citric acid cycle. The Michelis-Menten constant for citrate is approximately 650 µM. The activation of citrate transport by Na⁺ is sigmoidal, suggesting involvement of multiple Na⁺ ions in the activation process. The transport process is electrogenic. This represents the first plasma membrane transporter in humans that mediates the preferential entry of citrate into cells. Citrate occupies a pivotal position in many important biochemical pathways. Among various citric acid cycle intermediates, citrate is present at the highest concentrations in human blood. The selectivity of NaCT towards citrate and its predominant expression in the liver suggest that this transporter may facilitate the utilization of circulating citrate for the generation of metabolic energy and for the synthesis of fatty acids and cholesterol.

Example 2 described a new transporter from rat brain that recognizes citrate as the primary substrate was cloned. This transporter, designated as NaCT (Na⁺-coupled citrate transporter) is structurally homologous to NaDC1 and NaDC3. NaCT transports citrate in a Na⁺-dependent manner and the transport process is electrogenic. It is the trivalent form of citrate rather than the divalent form that is recognized as the substrate by NaCT. NaCT does accept succinate as a substrate but with lower affinity compared to citrate. The tissue expression pattern of NaCT is quite different from that of NaDC1 and NaDC3. NaCT mRNA is expressed primarily in the liver, testis, and brain in the rat. In this example, the human ortholog of this novel transporter has been is cloned and functionally characterized.

### MATERIALS AND METHODS

Materials. [¹⁴C]-Citrate (sp. radioactivity, 55 mCi/mmol), [³H]-succinate (sp. radioactivity, 40 Ci/mmol), and [¹⁴C]-pyruvate (sp. radioactivity, 15 mCi/mmol) were purchased from Moravek Biochemicals (Brea, CA). The human retinal pigment epithelial cell line (HRPE), used in heterologous functional expression studies, was maintained in DMEM/F-12 medium supplemented with 10% fetal bovine serum, 100 U/ml penicillin, and 100 µg/ml streptomycin.

Cloning of human NaCT from a HepG2 cDNA library. A search of the Genbank database for human established sequence tags (ESTs) with the amino acid sequence of *Drosophila* Indy (Rogina et al., Science (2000);290: 2137-2140, Inoue et al., Biochem. J.(2002);367: 313-319) as a query identified several ESTs whose predicted amino acid sequences showed significant similarity to that of human NaDC1 and NaDC3. However, there were four ESTs (Genbank Accession Nos. BI490092, BG616615, BI490615, and R01302) with overlapping nucleotide sequences and the predicted amino acid sequence from these ESTs was significantly different from that of human NaDC1 or NaDC3, indicating that this sequence may correspond to a new member of the NaDC gene family. A comparison of this sequence with that of the recently cloned rat NaCT suggested that the new putative transporter is most likely the human ortholog of NaCT. The overlapping nucleotide sequences of these ESTs were used to design primers for RT-PCR to obtain a cDNA probe that is specific for this transporter. The forward primer was 5'-CTCGGCGCTGAGCTATGTCT- 3' (SEQ ID NO:19) and the reverse primer was 5'-GTTGATCTCCGCGAAGG- 3' (SEQ ID NO:20). The predicted size of the RT-PCR product, based on the positions of the primers, was 949 bp. Since these ESTs were identified from a human liver cDNA library and also because NaCT is expressed abundantly in the rat liver, poly(A) RNA from the human hepatoma cell line HepG2 was used as the template for RT-PCR to obtain the probe. This yielded a cDNA product of expected size. The product was subcloned in pGEM-T vector and sequenced to establish its molecular identity. The cDNA was labeled with [α-³²P]dCTP by random priming using the ready-to-go oligolabeling beads (Amersham Pharmacia Biotech). This probe was used to screen a HepG2 cDNA library under strong stringency conditions. The screening of the library was done as described previously (Kekuda et al., J. Biol. Chem.(1998);273: 15971-15979, Rajan et al., J. Biol. Chem. (1999);274: 29005-29010). Positive clones were isolated by secondary or tertiary screening. The library used here was originally established in pSPORT1 vector at *Sal*I/*Not*I site using poly(A) RNA isolated from HepG2 (Hatanaka et al., Biochim. Biophys. Acta (2000);1467: 1-6, Hatanaka et al., Biochim. Biophys. Acta (2001);1510: 10-17). The cDNA inserts in the vector were under the control of T7 promoter.

DNA sequencing. Sequencing of the sense and antisense strands of the cDNA was performed by *Taq* DyeDeoxy terminator cycle sequencing using an automated Perkin-Elmer Applied Biosystems 377 Prism DNA sequencer. The sequence was analyzed using the National Center for Biotechnology Information server available on the world wide web at ncbi.nlm.nih.gov.

Northern analysis. The expression pattern of NaCT mRNA in a number of human tissues, including brain, colon, heart, kidney, liver, lung, skeletal muscle, placenta, small intestine, spleen, stomach, and testis, was studied by northern analysis using a commercially available human multiple tissue blot (Origene, Rockville, MD). The blot was hybridized with a [³²P]-labeled human NaCT-specific cDNA probe under high stringency conditions. The same blot was then hybridized with a cDNA probe specific for human β-actin as an internal control to detect the presence of RNA in each lane.

Functional expression of human NaCT cDNA in HRPE cells. The functional expression of the cloned transporter was done in HRPE cells using the vaccinia virus expression technique as described previously (Inoue et al., Biochem. J. (2002);367: 313-319). Uptake measurements were made at 37° C with radiolabeled citrate, succinate, or pyruvate. The uptake medium in most experiments was 25 mM Hepes/Tris (pH 7.5), containing 140 mM NaCI, 5.4 mM KCI, 1-8 mM CaCl₂, 0.8 mM MgSO₄, and 5 mM glucose. The time of incubation was 30 minutes for citrate and 15 minutes for succinate and pyruvate. Endogenous uptake was always determined in parallel using the cells transfected with pSPORT1 vector alone. The uptake activity in cDNA-transfected cells was adjusted for the endogenous uptake activity to calculate the cDNA-specific activity. In experiments in which the cation and anion dependence of the uptake process was investigated, NaCI was replaced isoosmotically with LiCI, KCI, *N*-methyl-D-glucamine (NMDG) chloride, and sodium gluconate. Substrate saturation kinetics was evaluated by non-linear regression and the kinetic parameters derived from this method were confirmed by linear regression. In studies involving the dependence of the transport process on Na⁺ concentration, the concentration of Na⁺ was varied by appropriately mixing NaCl and NMDG chloride without altering the osmolality. The influence of membrane potential on the uptake process was investigated by measuring the uptake in the presence of high concentrations of K⁺ (55 mM) in the extracellular medium, a condition that leads to depolarization of the membrane. Experiments were done in duplicate or triplicate and each experiment was repeated two or three times. Results are expressed as means ± S. E.

### RESULTS AND DISCUSSION

Structural features of human NaCT cDNA. The human NaCT cDNA (SEQ ID NO:5), also available as GENBANK Accession No. AY151833, is 3,207 bp-long with a poly(A) tail and a 1,707 bp-long open reading frame (including the stop codon). See Fig. 14. The 5'-untranslated region is 12 bp-long and the 3'-untranslated region is 1,488 bp-long. The cDNA possesses a polyadenylation signal (ATTAAA) upstream of the poly(A) tail, which is a variant of the consensus sequence (AATAAA). The cDNA encodes a protein consisting of 568 amino acids (SEQ ID NO:6) (Fig. 14). At the level of amino acid sequence, human NaCT (SEQ ID NO:6) exhibits a high degree of homology to rat NaCT (SEQ ID NO:4) (77% identity, 87% similarity) (Fig. 15). Human NaCT is however 4 amino acids shorter than rat NaCT. Human NaCT also shows 54% sequence identity with human NaDC1 and 47% sequence identity with human NaDC3. The sequence identity between human NaCT and the human sodium-coupled sulfate transporters NaSi and SUT-1 is comparatively less (43% with human NaSi and 40% with human SUT-1).

Exon-intron organization and chromosomal location of human *nact* gene. A search of the GENBANK database with the nucleotide sequence of human NaCT cDNA as the query identified the chromosomal location and the sequence of the human nact gene. The gene is approximately 30 kb in size and consists of at least 12 exons. Fig. 16 describes the exon-intron organization of the human nact gene. The translation start site resides in exon 1. Exon 12 codes for a small C-terminal region of the protein and for the entire 3'-untranslated region. The polyadenylation signal is also located in this exon. The sizes of the exons and introns and the sequences at the splice junctions are given in Table 6. The *nact* gene is located on human chromosome 17 p12-13. Interestingly, the human gene coding for NaDC1 is also located on the same chromosome (17p11.1-q11.1) close to the location of the *nact* gene (Pajor, Am. J. Physiol. (1996);270: F642-F648). It is also interesting to note that, among the known members of the gene family consisting of the sodium-coupled dicarboxylate and sulfate transporters, human NaCT exhibits the greatest sequence identity with human NaDC1.

Tissue expression pattern of human NaCT mRNA. The expression pattern of NaCT mRNA in human tissues was investigated by northern blot analysis using a commercially available multiple human tissue blot. NaCT mRNA (approximately 3.2 kb) is expressed in a restricted manner in human tissues. The expression is evident only in the liver, testis, and brain. The level of expression in the liver is several-fold higher than in the brain and testis. Kidney and heart show weak, but detectable, hybridization signals. This tissue expression pattern of NaCT is different from that of NaDC1 and NaDC3 (Pajor, J. Membrane Biol. (2000);175: 1-8). NaDC1 mRNA is expressed mostly in the small intestine and kidney whereas NaDC3 mRNA is expressed primarily in the kidney, small intestine, liver, placenta, and brain.

Functional features of human NaCT. The functional characteristics of the cloned human NaCT were investigated using a heterologous expression system in a mammalian cell line (HRPE). The expression of the heterologous cDNA was carried out using the vaccinia virus expression technique. The ability of human NaCT to transport succinate, citrate, and pyruvate in the presence of NaCl was first tested (Fig. 17A). The uptake of citrate (20 µM) in cells transfected with human NaCT cDNA was about 23-fold higher than in cells transfected with vector alone. In contrast, the uptake of succinate (80 nM) and pyruvate (100 µM) was increased only by 20-30% in cDNA-transfected cells compared to control cells. These data show that human NaCT accepts citrate as the most preferred substrate. Succinate, the prototypical substrate for human NaDC1 and NaDC3, is not recognized as well by human NaCT. The recently cloned rat NaCT also prefers citrate as a substrate, but is able to mediate the uptake of succinate and pyruvate to a much greater extent compared to human NaCT. This indicates that human NaCT exhibits comparatively greater selectivity towards citrate than the rat ortholog. Nonetheless, based on the substrate selectivity, rat NaCT as well as human NaCT can be classified as tricarboxylate transporters rather than dicarboxylate transporters.

Since human NaCT was able to transport citrate preferentially, this tracarboxylate was used as the substrate for subsequent functional characterization of the transporter. The cDNA-mediated uptake of citrate was linear even up to 45 minutes (Fig. 17B). Therefore, all subsequent studies were carried out with a 30-minute incubation. The involvement of Na⁺ in the uptake process mediated by human NaCT was evaluated by monitoring the uptake of citrate in vector-transfected cells and in human NaCT cDNA-transfected cells in the presence and absence of Na⁺. This was done by isoosmotically replacing NaCl in the uptake medium with NMDG chloride, KCl, and LiCl (Table 7). The cDNA-specific uptake was completely abolished when Na⁺ was substituted with other monovalent cations. The uptake process was however not dependent on Cl⁻ because replacement of Cl⁻ with gluconate had no effect on the cDNA-specific citrate uptake.

The substrate specificity of human NaCT was examined by competition studies in which the ability of various unlabeled compounds (2.5 mM) to compete with [¹⁴C]-citrate (20 µM) for uptake via human NaCT was assessed (Table 8). Unlabeled citrate was the most potent inhibitor of [¹⁴C]-citrate uptake mediated by human NaCT. Among the dicarboxylates, only succinate and malate showed significant inhibitory effect. Fumarate, α-ketoglutarate, and maleate were unable to inhibit the uptake of [¹⁴C]-citrate. The monocarboxylates pyruvate and lactate were also not effective in inhibiting [¹⁴C]-citrate uptake. Interestingly, isocitrate and cis-aconitate, close structural analogs of citrate, were also excluded as substrates by human NaCT. The affinities of human NaCT for citrate and dicarboxylates (succinate, malate, and fumarate) was then compared by studying the dose-response relationship for the inhibition of [¹⁴C]-citrate uptake mediated by human NaCT. The *IC₅₀* value (i.e., concentration of the inhibitor necessary for 50% inhibition) calculated for citrate from the dose-response relationship was 688 ± 150 µM. The corresponding value for succinate, malate, and fumarate were 1.92 ± 0.48, 3.12 ± 1.34, and 12.6 ± 5.0 mM, respectively. These values for the dicarboxylates are several-fold higher than that for citrate. These results show that, among the various intermediates of the citric acid cycle, citrate is the most preferred substrate for human NaCT.

Kinetic features of human NaCT. Citrate uptake mediated by human NaCT was saturable with a *Kₜ* of 604 ± 73 µM (Fig. 18A). Interestingly, this value is markedly different from the corresponding value for rat NaCT. In the case of rat NaCT, the *Kₜ* value for citrate is 18 ± 4 µM. Thus, the affinity of human NaCT for citrate is about 30-fold less than the affinity of rat NaCT for citrate under identical assay conditions. Kinetic analysis of Na⁺-activation of citrate uptake via human NaCT showed that the uptake process is obligatorily dependent on Na⁺. However, the activation failed to reach the maximum within the physiological concentrations of Na⁺ (Fig. 18B). Nonetheless, the activation response was not hyperbolic in nature. It was sigmoidal, indicating the involvement of multiple Na⁺ ions in the activation process. Since the activation did not reach the maximum within the Na⁺ concentration range tested, the exact number of Na⁺ ions involved in the activation process could not be determined. The concentration range could not be extended beyond 140 mM due to the fact that the extracellular medium would become hyperosmolar when the concentration of NaCl goes beyond 140 mM. But, the inability of Na⁺ to saturate the uptake process within the physiological concentrations indicates that the transporter exhibits low affinity for Na⁺. This characteristic of human NaCT differs from that of rat NaCT. The uptake of citrate mediated by rat NaCT under similar experimental conditions is activated to the maximum within the physiological concentrations of Na⁺. Since the activation reaches the maximum, the Hill coefficient for Na⁺-activation could be calculated for rat NaCT. The value is between 3 and 4. It can be speculated that the Na⁺:citrate stoichiometry for rat NaCT is 4:1. Such a stoichiometry will render the uptake process mediated by rat NaCT electrogenic. This was indeed the case because membrane depolarization was able to inhibit the uptake of citrate mediated by rat NaCT. It is predicted that human NaCT also behaves in a similar manner with respect to the coupling of citrate uptake with Na⁺. This is supported by the electrogenic nature of the transport process mediated by human NaCT. The cDNA-specific citrate uptake between control conditions (concentration of K⁺ in the extracellular medium, 5 mM) and membrane-depolarizing conditions (concentration of K⁺ in the extracellular medium, 55 mM) was compared. The uptake was inhibited significantly (58 ± 2%) when the membrane was depolarized, indicating that the uptake process is influenced by membrane potential. Since depolarization inhibits the uptake, it can be concluded that the uptake process mediated by human NaCT is electrogenic, associated with a net transfer of positive charge into the cells. Therefore, the Na⁺:citrate stoichiometry is 4:1 for human NaCT as is the case for the rat ortholog.

In summary, a human transporter (NaCT) that mediates the cellular entry of citrate by a process energized by the electrochemical Na⁺ gradient has been cloned and characterized. This represents the first human plasma membrane transporter with preferential selectivity towards citrate as a substrate.

**TABLE 6**

| Exon-intron boundaries of the human *nact* gene | | | | | | | | |
|---|---|---|---|---|---|---|---|---|
| 5' Exon | | | Intron ' | | | | 3' Exon | |
| Number | Size (bp) | Sequence | Donor | Size (bp) | Number | Acceptor | Sequence | Number |
| 1 | >114 | ...GCCAAG | gtcagt... | 6075 | 1 | ...cttcag | TTTGTC... | 2 |
| 2 | 129 | ...AGGCAG | gtgagc... | 249 | 2 | ...ctccag | GTGTGT... | 3 |
| 3 | 137 | ...TGCACG | gtaatt... | 2585 | 3 | ...ctgcag | GCTGAT... | 4 |
| 4 | 179 | ...TGCCAG | gtgagc... | 739 | 4 | ...ccacag | GGAGTC... | 5 |
| 5 | 169 | ...GAACGA | gtgagt... | 1843 | 5 | ...ttgtag | GTTGTT... | 6 |
| 6 | 123 | ...ATTCAA | gtaagt... | 5062 | 6 | ...ctccag | TTTTAA... | 7 |
| 7 | 216 | ...GACAAA | gtaagt... | 1528 | 7 | ...acctag | GTATGT... | 8 |
| 8 | 101 | ...AGGAAG | gtaagt... | 934 | 8 | ...tcccag | AAAGGA... | 9 |
| 9 | 119 | ...TCCGAG | gtaact... | 2103 | 9 | ...tcccag | GCCTCG... | 10 |
| 10 | 162 | ...TCCATG | gtaagt... | 3112 | 10 | ...ccgcag | TCTCGC... | 11 |
| 11 | 138 | ...GACATG | gtaaca... | 1190 | 11 | ...ttccag | GTGAAA... | 12 |
| 12 | 1617 | ...CCGCGG | | | | | | |

**TABLE 7**

| Ion dependence of citrate uptake via human NaCT | | | | |
|---|---|---|---|---|
| Uptake of [¹⁴C]-citrate (20 µM) was measured in vector-transfected HRPE cells and in human NaCT cDNA-transfected HRPE cells at pH 7.5 in the presence of various inorganic salts. Values represent means ± S.E. | | | | |
| Salt | Citrate Uptake | | | |
| | Vector | CDNA | cDNA-specific | |
| | pmol/10⁶ cells/min | | | % |
| NaCl | 1.5 ± 0.3 | 59.9 ± 3.9 | 58.4 ± 3.9 | 100 |
| NMDG chloride | 1.4 ± 0.2 | 1.3 ± 0.2 | -0.1 ± 0.2 | 0 |
| KCl | 1.2 ± 0.2 | 1.1 ± 0.1 1 | -0.1 ± 0.1 | 0 |
| LiCI | 1.3 ± 0.1 | 2.0 ± 0.1 | 0.7 ± 0.1 | 1 |
| Sodium gluconate | 1.6 ± 0.3 | 61.7 ± 4.7 | 60.1 ± 4.7 | 103 |

**TABLE 8**

| Substrate specificity of human NaCT | | |
|---|---|---|
| Uptake of [¹⁴C]-citrate (20 µM) was measured in vector-transfected HRPE cells and in human NaCT cDNA-transfected HRPE cells in the absence or presence of various monocarboxylates, dicarboxylates or tricarboxylates (2.5 mM). Data (means ± S.E.) represent only cDNA-specific uptake. | | |
| Unlabeled compound | [¹⁴C]-Citrate Uptake | |
| | pmol/10⁶ cells/min | % |
| None | 47.7 ± 4.8 | 100 |
| Citrate | 7.7 ± 0.7 | 16 |
| Succinate | 32.1 ± 3.6 | 67 |
| Malate | 29.5 ± 1.9 | 62 |
| Fumarate | 43.7 ± 5.0 | 92 |
| oc-Ketoglutarate | 52.9 ± 3.3 | 111 |
| Maleate | 58.0 ± 6.7 | 121 |
| Pyruvate | 51.5 ± 3.8 | 108 |
| Lactate | 61.2 ± 6.0 | 128 |
| Malonate | 57.3 ± 3.2 | 120 |
| Isocitrate | 59.6 ± 1.1 | 125 |
| cis-Aconitate | 55.5 ± 3.3 | 116 |

### Example 4

### Functional Characterization of a Sodium-coupled Citrate Transporter from Caenorhabditis elegans and the Relevance of the Transporter to Body Fat Storage and Life Span

Na⁺-coupled citrate transporter (ceNaCT) from *C*. *elegans* has been cloned and functionally characterized. This transporter shows significant sequence homology with the *Drosophila* Indy and the mammalian Na⁺-coupled citrate transporter NaCT. When heterologously expressed in a mammalian cell line or in *Xenopus* oocytes, the cloned ceNaCT mediates the Na⁺-coupled transport of various intermediates of the citric acid cycle. The substrates for ceNaCT include dicarboxylates such as succinate as well as the tricarboxylate citrate. Monocarboxylates are excluded by the transporter. The substrate specificity of this transporter is different from that of the previously identified ceNaDC1 and ceNaDC2. The transport process is electrogenic as evidenced from the substrate-induced inward currents in oocytes expressing the transporter under voltage-clamp conditions. The *nact* gene is expressed more predominantly during the early stages of development than during adult life of the organism. Tissue specific expression pattern studies using a reporter gene fusion method in transgenic *C*. *elegans* show that the gene is expressed in the intestinal tract, the organ responsible for not only the digestion and absorption of nutrients but also for the storage of energy in this organism. Functional knockdown of the transporter by RNA interference (RNAi) not only leads to a significant increase in life span, but also causes a dramatic decrease in fat storage in the intestinal tract. Citrate occupies a pivotal position in metabolic energy production and in the synthesis of fatty acids and cholesterol and the present data show that the newly cloned transporter plays an obligatory role in the cellular utilization of extracellular citrate for these metabolic functions. Since mammals express an ortholog of ceNaCT in the liver, an organ involved in fatty acid and cholesterol synthesis, the data from the present study suggest that the transporter may play a similar role in the mammalian liver.

Even though the mammalian NaCT is similar to drIndy with respect to the recognition of citrate as a substrate, NaCT differs from drIndy in terms of Na⁺-dependence. This example reports the molecular identification of the NaCT ortholog in *C. elegans*, its structural and functional characteristics, and its relevance to life span. As shown in this example, *C. elegans* NaCT is a Na⁺-coupled transporter that recognizes citrate as a substrate. RNAi-based knockdown of NaCT function leads to a significant increase in the life span of this organism. In *C*. *elegans*, NaCT is expressed primarily in the intestinal tract, an organ with metabolic function similar to that of the liver in mammals. Furthermore, since citrate plays a pivotal role in the synthesis of fatty acids and cholesterol, it was investigated whether the transporter has any functional relevance to the utilization of extracellular citrate in these metabolic functions. This example also shows that RNAi-based knockdown of NaCT function in *C*. *elegans* leads to a marked decrease in the levels of fat stores in the intestinal tract.

### EXPERIMENTAL PROCEDURES

Materials. [¹⁴C]Citrate (specific radioactivity, 55 mCi/mmol) and [³H]succinate (specific radioactivity, 40 Cl/mmol) were purchased from Moravek Biochemicals (Brea, CA). The human retinal pigment epithelial (HRPE) cell line, used routinely for heterologous expression of cloned transporters, was maintained in Dulbecco's minimum essential medium/F-12 medium supplemented with 10% fetal bovine serum, 100 units/ml penicillin, and 100 mg/ml streptomycin. Lipofectin was purchased from Invitrogen. Restriction enzymes were obtained from New England Biolabs (Beverly, MA). Magna nylon transfer membranes used in library screening were purchased from Micron Separations (Westboro, MA). Unlabeled monocarboxylates, dicarboxylates, and ticarboxylates were obtained from Sigma.

Nematode culture and RNA preparation. A wild type nematode strain, *C*. *elegans N2* (Bristol) was obtained from the *Caenorhabditis* Genetics Center (St. Paul, MN). Nematode culture was carried out using a standard procedure with a large-scale liquid cultivation protocol (Fei et al., J Biol Chem (2003); 278: 6136-6144; Wood, (1988) in The nematode Caenorhabditis elegans. (Wood, W.B and the Community of C. elegans Researchers, eds) Cold Spring Harbor Laboratory Press, Cold Spring Harbor, NY. 587-606: Fei et al., Biochem J (1998);332: 565-572; and Fei et al., J Biol Chem (2000);275: 9563-9571). The nematodes were cleaned by sedimentation through 15% (w/v) Ficoll 400 in 0.1 M NaCl. The pellet was then used for total RNA preparation. Total RNA was isolated using the TRIzol reagent (GIBCO-BRL, Gaithersburg, MD). Poly(A)⁺ mRNA was purified by affinity chromatography using oligo(dT)-cellulose.

Reverse transcription polymerase chain reaction (RT-PCR) and hybridization probe preparation. A search in the WormBase (Release WS93 on the worldwide web at wormbase.org) using the drIndy protein sequence as a query revealed that the gene R107.1 located on chromosome III encodes a hypothetical sodium-coupled transporter belonging to the family of sodium-coupled dicarboxylate transporters. The putative protein product of this gene is different from the previously identified NaDC1 and NaDC2 in this organism. This indicated that this gene is a potential candidate for NaCT in *C*. *elegans*. A pair of PCR primers specific for this gene was designed based on the sequence of the cosmid R107.1 (GenBank accession no. Z14092): forward primer, 5'-CTC CAT CGA AGA ATC GCA C-3' (SEQ ID NO:21) and reverse primer, 5'-GAA ATA GCA TAC CCA GCA CC-3' (SEQ ID NO:22). These primers yielded a single RT-PCR product with RNA from *C. elegans.* The size of the product was approximately 1.0 kb which agreed with the predicted size based, on the distance between the two primers in the theoretically derived gene transcript. The RT-PCR product was gel-purified and subcloned into pGEM-T easy vector (Promega Madison, WI). The molecular identity of the insert was established by sequencing. This cDNA fragment was used as a probe to screen a *C. elegans* cDNA library.

Construction of a directional *C. elegans* cDNA library. SuperScript Plasmid System from GIBCO-BRL (Gaithersburg, MD) was used to establish the cDNA library using the poly(A)⁺ RNA from *C. elegans.* The transformation of the ligated cDNA into E. *coli* was performed by electroporation using ElectroMAX DHIOB competent cells. The bacteria plating, the filter lifting, the DNA fragment labeling, and the hybridization methods followed the routine procedure (Sambrook et al., (1989) Molecular Cloning: A Laboratory Manual, 2nd Ed., Cold Spring Harbor Laboratory Press, Cold Spring Harbor, NY. 9.31-9.50).

The DNA sequencing of the full-length ceNaC°T cDNA clone was performed using an automated Perkin-Elmer Applied Biosystems 377 Prism DNA sequencer (Foster City, CA) and the Taq DyeDeoxy teminator cycle sequencing protocol.

Vaccinia/T7 expression system. Functional expression of the ceNaCT cDNA in HRPE cells was done using the vaccinia virus expression system as described previously (Fei et al., J Biol Chem (2003);278: 6136-6144, Kekuda et al., J Biol Chem (1999);274: 3422-3429, Huang et al., J Pharmacol Exp Ther (2000);295: 392-403, Wang et al., Am J Physiol Cell Physiol (2000);278: C1019-1030, Inoue et al., Biochem J, (2002);367: 313-319, Inoue et al., (2002) J Biol Chem (2002);277: 39469-39476, Inoue et al., (2002) Biochem Biophys Res Commun (2002);299: 465- 471). HRPE cells grown in 24-well plates were infected with a recombinant vaccinia virus (VTF₇₋₃) at a multiplicity of 10 plaque-forming units/cell. The virus was allowed to adsorb for 30 minute at 37° C with gentle shaking of the plate. Cells were then transfected with the plasmid DNA (empty vector pSPORT or ceNaCT cDNA constructs) using the lipofection procedure (GIBCO-BRL, Gaithersburg, MD). The cells were incubated at 37° C for 12 hours and then used for determination of transport activity. Uptake of [¹⁴C]-citrate and [³H]-succinate was determined at 37° C as described previously (Inoue et al. (2002) Biochem J (2002);367: 313-319, Inoue et al., (2002) J Biol Chem (2002);277: 39469-39476, Inoue et al., (2002) Biochem Biophys Res Commun (2002);299: 465- 471). In most experiments, the uptake medium was 25 mM Hepes/Tris (pH 7.5) or 25 mM Mes/Tris (pH 6.5), containing 140, mM NaCl, 5.4 mM KCI, 1.8 mM CaCl₂, 0.8 mM MgSO₄, and 5 mM glucose. In experiments in which the cation and anion dependence of the transport process was investigated, NaCl was replaced isoosmotically by LiCI, KCl, sodium gluconate, or N-methyl-D-glucamine (NMDG) chloride. Uptake measurements were routinely made in parallel in control cells transfected with the plasmid alone and in cells transfected with the vector-cDNA construct. The uptake activity in cDNA-transfected cells was adjusted for the endogenous activity measured in control cells to calculate the cDNA-specific activity. Experiments were performed in triplicate and each experiment was repeated at least three times. Results are presented as means ±S.E.

Electrophysiological studies of ceNaCT transport activity. Initially, ceNaCT cRNA prepared from the pSPORT-ceNaCT cDNA construct was used for functional expression of the transporter in *X*. *laevis* oocytes. These initial attempts were unsuccessful. Therefore, a *X. laevis* oocyte expression vector was used. The coding sequence of ceNaCT cDNA was amplified by PCR using the following primers: 5'-CCC GGG TAT GAA GCC TAG CCC CCA GCG TAC GTT AAT AAA A-3' (SEQ ID NO:23) (forward primer) and 5',GCG GAT CCA AAA ATT AGC AAA CTG GAT ATG AAG AGT TTT CTG AAG-3' (SEQ ID NO:24) (reverse primer). The forward primer contained the start codon and also an *Xma I* site introduced at the 5'-end for the purpose of subcloning. The reverse primer contained the termination codon and also α *BamH I* site at the 5'end for the purpose of subcloning. The PCR-derived ceNaCT coding fragment was then inserted into the *Xma* I/*Bam*H I site in the oocyte expression vector pGH19. In this construct, the ceNaCT coding sequence was flanked by a synthetic *Xenopus* (β-globin gene 5'-UTR and 3'-UTR regulatory elements. Plasmid pGH19-ceNaCT cDNA was linearized with *Xho* Land transcribed *in vitro* using the mMESSAGE mMACHINE RNA transcription kit (Ambion, Austin, TX). When the cRNA derived from the pGH19-ceNaCT cDNA construct was injected into the oocytes, the expression of ceNaCT was detected by monitoring the uptake of the [¹⁴C]citrate. The procedures for oocyte manipulation, microinjection and electrophysiological studies using the two-electrode voltage-clamp (TEVC) protocol have been described previously (Fei et al., (1998) Biochem J 332, 565-572, Fei et al. (2000) J Biol Chem 275, 9563-9571*,* Fei et al., (1994) Nature 368, 563-566*,* Mackenzie et al., (1996) Biochim BiophysActa 1284, 125-128).

Semi-quantitative RT-PCR. An RT-PCR assay with the ceNaCT-specific primers described in the previous section was used to study the developmental stage-specific expression pattern of the *nact* gene. A Quantum RNA 18S internal standard (Ambion, Austin, TX) was used for the semi-quantitative RT-PCR. Total RNA (approximately 1.0 µg), isolated from different developmental stages of C. *elegans* (embryo, early larva, late larva, and adult), was taken as template to perform reverse transcription using an RT-PCR kit from Perkin Elmer Corp. (Norwalk, CT) as described previously ( Fei et al., (2003) J Biol Chem 278, 6136-6144, Fei et al., (1998) Biochem J 332, 565-572). Reverse transcription was followed by PCR in a multiplex format. The gene specific primers and the primers for the internal control (18S rRNA) with their competimers were combined at a predetermined ratio. The resultant multiplex PCR products were subjected to electrophoresis on an 1 % agarose gel and the steady state levels of ceNaCT mRNA at different developmental stages were estimated from the relative ratios of the intensity of the ceNaCT specific RT-PCR product to the intensity of the 18S rRNA-specific RT-PCR product at each of these stages.

Analysis of tissue-specific expression pattern of ceNaCT. To study the tissue-specific expression pattern of the *nact* gene in *C. elegans, a* transcriptional *nact::gfp* fusion gene was constructed and transgenic animals expressing these transgenes were developed as described previously (Fei et al., (2003) J Biol Chem 278, 6136-6144, Fei et al. (2000) J Biol Chem 275, 9563-9571). A GFP-expression vector pPD 117.01 was a gift from Dr. A. Fire (Carnegie Institution of Washington, Baltimore, MD). The cosmid R107 containing the *C. elegans nact* gene and its promoter was obtained from the Sanger Center (Cambridge, UK). A DNA fragment containing the *C. elegans nact* gene promoter region was generated by PCR using this cosmid DNA as the template. The sense primer (coordinates in cosmid R107 are 11,660-11,630) with a *Sal* I adapter attached to the 5'-end was 5'-GTC GAC GAG GTG TTA AAC TGT ATA GTC GTG GTG-3' (SEQ ID NO:25) and the reverse primer (coordinates in cosmid R107 are 9,609-9,637) with *a BamH* I adapter attached to the 5'-end was 5'-GCC GGA TCC AAG AAG TAC CAG AAG CTT TTT TAT-3' (SEQ ID NO:26). A recombinant KlenTaq DNA-polymerase (AB Peptides, St. Louis, MO) was used for the long-range PCR. The size of the PCR product was -2.1 kb. The PCR product was digested with *Sal I* and *BamH I* and ligated into the multiple cloning site II in the pPD117.01 vector. Bacterial transformation and plasmid preparation followed a standard protocol (Sambrook et al., (1989) Molecular Cloning: A Laboratory Manual, 2nd Ed., Cold Spring Harbor Laboratory Press, Cold Spring Harbor, NY. 9.31-9.50). The minigene fusion constructs were verified by sequencing. Transgenic lines were established using a standard germ line transformation protocol ( Mello and Fire, Methods Cell Biol (1995);48: 451-482). A cloned mutant collagen gene containing the *rol-6* (plasmid pRF4) was used as a dominant genetic marker for DNA transformation (Kramer et al., Mol Cell Biol (1990);10: 2081-2089, Pettitt and Kingston, Dev Biol (1994);161: 22-29). The F1 rollers were picked up according to their characteristic rolling behavior and cultured individually to establish transformed lines. GFP expression pattern was determined by fluorescence microscopy (Chalfie et al., Science (1994);263: 802-805 and Miller and Shakes, Methods Cell Biol (1995);48 :365-394).

Bacteria-mediated RNA interference (RNAi) and life span measurement. A fragment of the coding region of ceNaCT cDNA was generated by PCR and subcloned into a "double T7" plasmid pPD129.36, which was subsequently transformed into HT115 (DE3) cells. Induction of HT115 cells harboring the double-T7 plasmid to express dsRNA and the bacteria-mediated RNAi procedure were carried out as previously described (Fei et al., J Biol Chem (2003);278: 6136-6144; Fire et al., Nature (1998);391: 806-811); and Timmons et al., Gene (2001);263: 103-112).

Mean life spans from different groups were compared using the nonparametric log-rank analysis. The survival curves were plotted according to the Kaplan-Meier algorithm using Minitab software (version 13, Minitab Inc. State College, PA). Nematode body size measurement was made on day 5. Worms were placed on agar plates and anesthetized using 0.1 M NaN3. To facilitate measurement, animals were laid out straight using a platinum wire. Measurement was carried our under a dissecting microscope, with an eyepiece graticule calibrated with a stage micrometer (Olympus, Melville, NY). To calculate body volume, worms were treated as cylinders (V=p(1/2D)2L), where D is the body width and L is body length.

To serve as a positive control for the bacteria-mediated RNAi in the assessment of the influence of ceNaCT on life span, an HTI 15 strain containing DAF-2specific dsRNA was included. Knockdown of DAF-2 function in *C. elegans* leads to an increase in life span in this organism (Kenyon et al. (1993) Nature 366, 461-464, Wolkow et al., (2000) Science 290,147-150). Life span of age-synchronous nematodes was determined at 20° C as described previously (Fei et al., (2003) J Biol Chem 278, 6136-6144). To avoid any potential subjective errors in the determination of life span, the studies were conducted in a blinded manner in which the person performing the life span analysis was unaware of the identity of the dsRNA used in feeding the nematodes. Statistical analysis was performed using the Microsoft EXCEL 2000 analysis ToolPak. Mean life spans from different groups were compared using the Student's t-test assuming unequal population variances. The survival curves were plotted according to the Kaplan-Meier algorithm using Sigma Plot (version 8.0, SPSS Inc., Chicago IL).

Quantitative fat deposit analysis by laser-scanning confocal microscopy. Fat storage droplets in living *C. elegans* were visualized using the vital dye Nile red (Molecular Probes, Eugene, OR). This dye is a selective stain for intracellular lipid droplets (Greenspan et al., J Cell Biol (1985);100:965-973). Addition of Nile red to *E. coli,* the common laboratory diet of *C. elegans,* resulted in uptake and incorporation of the dye into lipid droplets in intestinal cells, the principal location of fat storage in this organism. It is known that Nile red staining does not affect animals' growth rate, brood size, feeding or life span. (Ashrafi et al., Nature (2003);421: 268-272). Nile red stock solution (500 µg/ml) was prepared in acetone and was diluted in phosphate buffered saline to a final concentration of 0.1 µg/ml (working solution). Eggs, obtained from gravid hermaphrodites using an alkaline hypochlorite treatment procedure, were dispensed on NGM (Nematode Growth Medium) petri dishes with bacteria lawn and allowed to hatch. The IPTG-induced HT 115 bacteria suspension harboring different gene-specific dsRNAs was mixed with an equal volume of the Nile red working solution and fed to the newly hatched worms on individual petri dishes every day until the worms developed into the adult stage (Fei et al., J Biol Chem (2003);278: 6136-6144). Nile redstained worms were placed on 2% agarose pads attached to microscope slides and anesthetized with 0.1 M sodium azide for examination by confocal fluorescence microscope (Zeiss Axioplan2 upright microscope equipped with a Zeiss 510 NLO scan head (Carl Zeiss Microlmaging. Inc., Thornwood, NY). All Nile red images were acquired using identical settings and exposure times. The excitation filter wavelength was 515-560 nm and the emission filter wavelength was >590 nm. Fluorescence intensity was quantified on equal planes (optical section thickness <100 µm), which encompassed the entire worm body. The LSM 510 software (version 3.0 SP3, Carl-Zeiss, Heidelberg, Germany) was used to calculate the total fluorescence intensity for all Nile red stained droplets within the animal body as the product of area multiplied by mean fluorescence. At least 10-15 animals in each group were examined for fluorescence intensity measurements and average intensity for each tested group was compared using a Student's t-test (Sigma Plot version 8.0).

### RESULTS

Molecular cloning and structural characterization of ceNaCT. The *C. elegans nact* gene is localized on chromosome III and its size is at least approximately 2.7 kb, excluding the promoter region. The gene consists of 11 exons as deduced by a comparison between the sequence of the cloned cDNA with that of the GenBank deposit R107.1 from the nematode genome sequence project (WormBase release WS93). The structural organization of the gene is shown in Fig. 20. The ceNaCT cDNA is 1,747 bp long and contains a poly(A) tail. The 5'- and the 3'-untranslated regions are 30 by and 44 by long, respectively. The ceNaCT protein deduced from the cDNA sequence contains 551 amino acids with an estimated molecular size of 61 kDa and an isoelectric point of 7.24. According to the Kyte-Doolittle hydropathy plot, the ceNaCT protein possesses 12 putative transmembrane domains. The ceNaCT cDNA and its encoded transporter protein sequences have been deposited into the GenBank (Accession number: AY090486).

A pair-wise comparison analysis of the transporter protein sequences between ceNaCT and its closely related functional counterparts, the mammalian NaCT and the *Drosophila* Indy, using the BESTFIT algorithm in the GCG package (version 10.2, Madison WI) has shown that ceNaCT is closely related to mammalian NaCT (49% similarity and 36% identity) and to drIndy (48% similarity and 35% identity). The mammalian NaCT and drIndy are also similar to each other (51 % similarity and 37% identity). Following a multiple protein sequence alignment of the three transporters, using the PILEUP program and in combination with the PRETTYBOX program in the GCG package, a sodium symporter family signature motif was identified within these transporter proteins (Fig. 21). A consensus pattern established for the signature sequence is: (S)SXXFXXGP(V)(G)XXXNX(I)V (SEQ ID NO:29), wherein X denotes any amino acid residue, (S) denotes serine or other related amino acids, such as alanine, cysteine, threonine, or proline, (V) denotes valine or other related amino acids, such as leucine, isoleucine or methionine, (G) denotes glycine or other related amino acids, such as serine or alanine, and (I) denotes isoleucine or other related amino acids, such as leucine, valine, or methionine. This sodium symporter family is a group of integral membrane proteins that mediate the cellular uptake of a wide variety of molecules including di- or tricarboxylates and sulfate by a transport mechanism involving sodium cotransport (Pajor, Annu Rev Physiol (1999);61: 663-682 and Pajor, J Membr Biol (2000); 175: 1-8).

Functional characterization of ceNaCT using a heterologous mammalian expression System. The functional analysis of the cloned ceNaCT was carried out by heterologous expression of the cDNA in HRPE cells using the vaccinia virus expression system. Since the ceNaCT transporter protein is structurally similar to drIndy and mammalian NaCT, which preferentially recognize citrate as a substrate, the transport function of ceNaCT with citrate as a potential substrate was tested. The uptake of [¹⁴C]citrate (10 µM) in the presence of extracellular Na⁺ (pH7.5) increased approximately 45-fold in cells expressing ceNaCT (61 ± 5 pmol/10⁶ Cells/min) compared to vector-transfected control cells (1.3±0.1 pmol/10⁶ cells/min). Then, the ability of ceNaCT to transport the dicarboxylate succinate was tested. Surprisingly, the uptake of succinate was also increased markedly in ceNaCT-expressing cells compared to control cells. However, the ability of ceNaCT to transport citrate was much greater than to transport succinate, when measured under identical conditions (10 µM substrate concentration). Since it has already been shown that succinate is a substrate for the previously cloned ceNaDC1 and ceNaDC2, the abilities of all three transporters to transport citrate and succinate under identical conditions was compared. As shown earlier, ceNaDC1 and ceNaDC2 were able to transport succinate very effectively (Fig. 22A). The magnitude of succinate transport was comparable between ceNaDC2 and ceNaCT whereas that of ceNaDC1 was comparatively a little lower. In contrast, the magnitude of citrate transport was the highest for ceNaCT. ceNaDC2 showed only a minimal ability to transport this tricarboxylate. The ability of ceNaDC1 to transport citrate was much higher than that of ceNaDC2 but much lower than that of ceNaCT. These data show that, among these three transporters, ceNaCT is the only transporter that recognizes citrate as the preferred substrate. The ceNaCT-mediated citrate uptake was obligatorily dependent on the presence of Na⁺ because substitution of Na⁺ with Li⁺, K⁺, or NMDG (*N*-methyl-D-glucamine) abolished completely the cDNA-induced increase in citrate uptake. There was no involvement of chloride in the uptake process as indicated by comparable uptake activities in the presence of NaCl or sodium gluconate (Fig. 22B).

The substrate specificity of ceNaCT was studied using a competition analysis by monitoring the ability of various monocarboxylates, dicarboxylates, and tricarboxylates (2.5 mM) to compete with [¹⁴C]citrate (15 µM) for the uptake process. Uptake measurements were made in parallel in vector-transfected cells and in cDNA-transfected cells and the cDNA-specific uptake was calculated by subtracting the uptake in vector-transfected cells from the uptake in cDNA-transfected cells. Only the cDNA-specific uptake was used in the analysis. Unlabeled citrate was a potent inhibitor of [¹⁴C] citrate uptake (Fig. 22C). Interestingly, the two other tricarboxylates tested, namely isocitrate and cis-aconitate, did not compete with [¹⁴C]citrate as effectively as unlabelled citrate. Among the various dicarboxylates tested, the ceNaCT-mediated citrate uptake was inhibited markedly by succinate, α-ketoglutarate, fumarate and malate. In contrast to fumarate, its stereoisomer maleate failed to compete with citrate. Similarly, malonate, a structural homolog of succinate also failed to inhibit the uptake of citrate. The monocarboxylates, pyruvate and lactate, caused only a minimal inhibition.

Citrate uptake mediated by ceNaCT was influenced by extracellular pH (Fig. 22D). The uptake increased markedly when the extracellular pH was changed from 7.5 to 6.5. Further acidification of extracellular pH resulted in a decrease of uptake. The uptake activity at pH 6.5 was 3- to 4-fold higher than that at pH 7.5. These data could be interpreted in two ways. The uptake activity in the pH range 8.0-6.5 might indicate that citrate is recognized by ceNaCT as a substrate only in its dianionic form. Alternatively, the pH-dependence may simply indicate the pH optimum for the catalytic function of the transporter. To differentiate between these two possibilities, the pH-dependence of succinate uptake via ceNaCT in parallel was studied (Fig. 22D). Succinate exists predominantly in its dianionic form over the pH range 6.5 - 8.0; yet the uptake of succinate via ceNaCT was also enhanced when the pH was changed from 8.0 to 6.5. This indicates that the pH-dependence represents the pH optimum for the transporter and that citrate is likely to be recognized as a substrate in its trivalent form. This would mean that ceNaCT is able to recognize a dicarboxylate as well as a tricarboxylate as substrates. Fig. 22D also shows that, under identical conditions, the transport rate via ceNaCT is much higher for citrate than for succinate over the entire pH range studied (5.5-8.0). The transport of citrate via ceNaCT was saturable with a Michaelis-Menten constant (*Kₜ*) of 76 ± 14 µM at pH 7.5 (Fig. 23A). The corresponding value for succinate was 88 ± 13 µM under identical conditions (Fig. 23B). These data show that ceNaCT is a high-affinity Na⁺-coupled transporter for the tricarboxylate citrate and the dicarboxylate succinate. Interestingly, even though the affinities for citrate and succinate are comparable, the transport rate for citrate is much greater than for succinate, suggesting that the transport processes of these two substrates differ in maximal velocity (*Vₘₐₓ*). This is evident from the *Vₘₐₓ*. values for these two substrates (270±13 pmol/10⁶ cells/min for citrate versus 210±8 pmol/10⁶ cells/min for succinate). These characteristics are different from those *of* ceNaDC1 and ceNaDC2. Thus, the transport features of ceNaCT are similar to those of *Drosophila* Indy. However, ceNaCT and *Drosophila* Indy differ in their dependence on Na⁺. ceNaCT is also similar to the recently identified mammalian NaCT not only in Na⁺-dependence but also in the ability to transport citrate much more effectively than succinate.

The effect of Na⁺ on the uptake of citrate and succinate was then investigated by measuring the uptake in the presence of varying concentrations of extracellular Na⁺ in cells transfected with either ceNaCT eDNA or plasmid alone. Again, the uptake values were adjusted for the endogenous uptake activity measured under identical conditions in cells transfected with vector alone. The concentration of Na⁺ in the uptake medium was varied from 0-140 mM. The osmolality of the medium was maintained by adding appropriate concentrations of NMDG chloride as a substitute for NaCl. The relationship between the cDNA-specific uptake and Na⁺ concentration was sigmoidal for citrate as well as succinate, suggesting the involvement of more than one Na⁺ per substrate molecule transported.

Functional characterization of ceNaCT using the *X*. *laevis* oocyte expression system. ceNaCT mediates the Na⁺-coupled transport of several dicarboxylates' as well as the tricarboxylate citrate. To determine whether the transport process is electrogenic, the *X. laevis* oocyte expression system was used to express ceNaCT heterologously. This system is amenable to study the electrogenic characteristics of transport processes by using the two-electrode voltage clamp technique. First, it was tested whether the transporter is expressed in oocytes injected with ceNaCT cRNA by measuring the uptake of [¹⁴C]citrate (40 µM) at pH 6.5. The uptake in cRNA injected oocytes was 12.9 ± 0.8 pmol/oocyte/15 min. This value was about 45-fold higher than the uptake measured under identical conditions in oocytes injected with water (0.3 ± 0.01 pmol/oocyte/15 min) (Fig. 24A). Next, the electrogenic nature of the transport process was examined.

Perifusion of the ceNaCT cRNA-injected oocytes with citrate (250 µM) in the presence of Na⁺ (100 mM NaCl) induced inward currents, detectable by the TEVC method at a holding membrane potential of -50 mV (Fig. 24B). Perifusion of the oocytes with succinate also induced similar inward currents, though the magnitude of the currents was less than that induced by citrate. These data show unequivocally that ceNaCT-mediated transport process is electrogenic irrespective of whether the transported substrate is a dicarboxylate or a tricarboxylate. The citrate- and succinate-induced currents were obligatorily dependent on the presence of Na⁺. In the absence of Na⁺ (NaCl was substituted by choline chloride), perifusion of the oocytes with citrate did not induce any detectable current (Fig. 24B). On the other hand, when chloride in the perifusion buffer was replaced with gluconate, the citrate-induced current remained unaltered. These data show that the transport process mediated by ceNaCT is Na⁺ dependent and that Cl⁻ ions do not have any role in the transport process. Similar results were obtained with succinate in terms of Na⁺-dependence and C1⁻ -independence. The substrate (citrate or succinate)-induced inward currents in ceNaCT cRNA-injected oocytes were influenced by the pH in the perifusion buffer (Fig. 24C). The magnitude of the currents was increased significantly when the pH of the perifusion buffer was changed from 7.5 to 6.5. The same was observed when the buffer pH was changed from 5.5 to 6.5.

The citrate-induced currents of ceNaCT were further analyzed in terms of their dependence on membrane potential. Steady-state currents induced by citrate over a concentration range of 10 µM to 1 mM were measured at different testing membrane potentials (-50 mV to -150 mV). At each of these testing membrane potentials, the citrate-induced currents were saturable with respect to citrate concentration (Fig. 24D). The maximal current induced by citrate however increased with increasing testing membrane potential. The data show that the transport rate increases with increasing membrane potential, suggesting a role for membrane potential as a driving force for the transport process. Thus, the transport process mediated by ceNaCT derives its driving force from the electrochemical Na⁺ gradient. The relationship between the substrate concentration and the induced current was hyperbolic at each of the testing membrane potentials. The data were analyzed at each of the testing membrane potentials according to the MichaelisMenten equation. The Michaelis-Menten constant *(K_{0.5}),* the concentration of citrate needed for the induction of half-maximal current, was 34 ± 8 µM at a testing membrane potential of - 70 mV. This value did not change significantly with different testing membrane potentials. However, the maximal current *(Iₘₐₓ)* increased with increasing testing membrane potential. The value for *Iₘₐₓ* was 12.3 ± 0.6 nA at a testing membrane potential of -50 mV and this value increased gradually to 61.1 ± 2.6 nA as the testing membrane potential increased to - 150 mV. These data show that the membrane potential does not influence the substrate affinity of the transporter but it enhances the maximal velocity of the transport process.

Developmental stage-specific expression pattern of the *nact* gene. To monitor the relative expression levels of NaCT mRNA during different stages of *C. elegans* development, synchronized cultures were obtained and total RNA was isolated at each of the following four stages of development: embryo, early larva (larva stages 1 and 2), late larva (larva stages 3 and 4), and adult. The steady-state levels of mRNA for NaCT were then determined by semi-quantitative RT-PCR with 18S rRNA as an internal control to adjust for variations in RNA input into RT-PCR reactions. The levels of NaCT mRNA were compared at different developmental stages based on relative intensities of the NaCT-specific RT-PCR product. The *nact* gene was expressed at much higher levels during the early embryo stage than during the adult stage. However, the expression was detectable all through the different stages of development. The levels of NaCT mRNA as assessed by the relative band intensities of RT-PCR products for NaCT and 18S rRNA at the stages of embryo, early larva, late larva, and adult were 5.6, 2.1, 0.8, and 1.4.

Tissue-specific expression pattern of the *nact* gene. Tissue expression pattern of the *nact* gene in *C*. *elegans* was investigated using the transgenic GFP fusion technique in which the transgene consisted of the *nact* gene-specific promoter fused with GFP cDNA. The expression of GFP in this fusion gene is controlled by the *nact* gene-specific promoter. Therefore, the expression pattern of GFP in transgenic *C*. *elegans* expressing the fusion gene would match the expression pattern of the native *nact* gene. With this technique, it was found that GFP expression is restricted to the intestinal tract in this organism. This expression pattern is evident from the early larva stage through the adult stage. The GFP fluorescence is detectable throughout the intestinal tract, starting from the pharynx all the way through the anus. The expression level of GFP is significantly greater in the anterior half of the intestine than in the posterior half. This expression pattern was confirmed with at least 10 transgenic animals.

Influence of RNAi-mediated knockdown of the function of NaCT on life span, body size and fat deposit. Knockdown of the function of NaCT by feeding wild type N2 worms with bacteria expressing the ceNaCT-specific dsRNA caused a significant increase in average life span and maximal life span of the organism (Fig. 25A). Average life span of the worms fed on bacteria harboring ceNaCT-specific dsRNA was 19.4 ± 0.3 days (N=211); average life span of the worms fed on bacteria harboring the empty vector pPD129 was the same as that of wild-type N2 worms (16.3 ± 0.2 days; N=180). The increase in average life span induced by ceNaCT knockdown was 19% (p < 0.001). The DAF-2 knockdown was also included as a positive control in these experiments. Worms fed on bacteria expressing DAF-2-specific dsRNA exhibited an average life span of 31.4 ± 0.6 days (N=60), showing that knockdown of the function of DAF-2 doubles average life span. This influence of DAF-2 knockdown on life span is similar to the influence of homozygous knockout of *daf*-2 gene function on life span ((Kenyon et al., Nature (1993); 366: 461-464, Wolkow et al., Science (2000); 290:147-150). A "lean" phenotype was detected when the *cenact* gene was knocked down by bacteria-mediated RNAi approach. Body length and body width were smaller in ceNaCT-RNAi worms than those of control worms. Therefore, the calculated body size was significantly reduced (approximately 40%) in ceNaCT-RNAi worms (2.95±0.13 nl, N=60) in comparison with the control vector fed worms (4.91±0.05 nl, N=58, Fig. 25B). This interesting phenotype was not observed when ceNaDC1 or ceNaDC2 was knocked down by a similar experimental approach (ceNaDC1 knockdown: 4.92±0.07 nl, N=45; ceNaDC2 knockdown: 5.05±0.07 nl, N=53). The intestinal fat content was also analyzed by a laser confocal fluorescence microscopy approach using Nile red (5H-benzo[α]phenoxazine-5-one, 9-diethylamino) to stain intracellular lipid droplets in live *C*. *elegans* under the influence of the ceNaCT gene-specific RNAi (Ashrafi et al., Nature (2003);421: 268-272). The intensity of Nile red staining in this organism was reduced markedly to 56% of the control value when the NaCT was knocked down by RNAi (P < 0.001, N=13; Fig. 26). In these experiments, the control worms (N=8) were fed on bacteria harboring empty vector pPD129. Under the same experimental conditions, the intensity of Nile red staining was not altered when NaDC1 was knocked down by RNA.

Interestingly, the knockdown of NaDC2 caused a significant decrease in the intensity of Nile red staining, but the decrease was much less than that seen with NaCT knockdown. In the case of NaDC2 knockdown, the decrease in fat content was 30% (P < 0.05) (Fig. 8D). These experiments included a group of worms in which DAF-2 was knocked down as a positive control because it has been shown that knockdown of this gene function leads to a significant increase in body fat storage (Ashrafi et al., Nature (2003);421: 268-272). As expected, the knockdown of DAF-2 led to a 12% increase (P < 0.05) in the intensity of Nile red staining (Fig. 26).

### DISCUSSION

This example describes the cloning and functional characterization of a Na⁺-coupled citrate transporter (NaCT) in C. *elegans.* The *C. elegans* NaCT transports the tricarboxylate citrate as well as several dicarboxylates such as succinate and α-ketoglutarate. The pH-dependence of the transport of citrate and succinate via this transporter shows that the trivalent form of citrate and the divalent form of succinate are the transportable substrates at physiological pH. The transport process is Na⁺-dependent, Cl⁻-independent, and electrogenic for both substrates. Functionally, the *C. elegans* NaCT is similar to rat NaCT (Example 2) and human NaCT (Example 3). The C. *elegans* NaCT and the mammalian NaCT are structurally similar to *Drosophila* Indy (Example 1). NaCTs are also similar to *Drosophila* Indy with respect to the ability of these transporters to recognize citrate as a substrate. However, *Drosophila* Indy is a Na⁺-independent citrate transporter. The transport characteristics of *C. elegans* NaCT are distinct from those of NaDC1 and NaDC2, previously identified in this organism. The two NaDCs transport succinate and other dicarboxylates much more effectively than citrate. Thus, the two NaDCs in *C. elegans* correspond to mammalian NaDC1 and NaDC3, whereas the transporter reported in this example corresponds to mammalian NaCT.

The unique functional feature of C. *elegans* NaCT is its ability to transport the tricarboxylate citrate as well as the dicarboxylates such as succinate. Mammalian NaDCs (NaDC1 and NaDC3) are able to transport only dicarboxylates. Even though these transporters can transport citrate, it is only the dianionic form of citrate that is recognized as the substrate. Therefore, mammalian NaDCs are truly Na⁺-coupled dicarboxylate transporters. In contrast, NaCT possess the ability to transport dicarboxylates as well as the tricarboxylate citrate. The pH-dependence of succinate uptake and citrate uptake mediated by *C. elegans* NaCT shows that the transporter recognizes succinate in its divalent form and citrate in its trivalent form as the substrates.

This example also provides information on the electrophysiological characteristics of *C. elegans* NaCT, as an electrogenic transporter. The transport process involves transfer of positive charge across the membrane irrespective of whether the transported substrate is a tricarboxylate or a dicarboxylate. Since the transport of citrate, a tricarboxylate, is an electrogenic process, there should be at least 4 Na⁺ ions involved in the transport process. The predicted Na⁺:substrate stoichiometry of 4:1 makes NaCT a very efficient concentrative transporter. In addition to the chemical Na⁺ gradient, the membrane potential also serves as a driving force for this transporter. The substrate affinity of the transporter is not influenced by membrane potential, but the maximal velocity of the transport process is enhanced markedly by hyperpolarization.

The extracellular pH influences markedly the transport of citrate via *C*. *elegans* NaCT. When the pH was changed from 7.5 to 6.5, the transport rate increased about 3-fold. At pH 7.5, only 7% of citrate exists in its divalent form. The concentration of the divalent form increases six fold to 44% when the pH is changed to 6.5. This pH-dependence of the transport function of *C. elegans* NaCT reflects the influence of pH on the translocation process as well as on the influence of pH on the ionization of the substrates. The pH-dependent stimulation of citrate uptake, when the pH was changes from 7.5 to 6.5, was about 3-fold in the mammalian cell expression system whereas the corresponding value was about 1.25-fold in the *X*. *laevis* oocyte expression system. This difference was most likely due to different citrate concentrations used (10 µM in the case of mammalian cells and 250 µM in the case of oocytes). The concentrations of the trivalent form of citrate relative to the *Kₜ* value were much lower in experiments with mammalian cells than with oocytes.

Irrespective of the charge of the transported substrate, the transport process mediated by *C*. *elegans* NaCT is electrogenic, with at least 4 Na⁺ ions involved in the transport process. If the Na⁺: substrate stoichiometry is 4:1 irrespective of the charge nature of the substrate, the net transfer of charge across the membrane is expected to differ depending on whether the transported substrate is a tricarbboxylate or a dicarboxylate. The transport of citrate, a tricarboxylate, would result in the transfer of only one positive charge whereas the transport of dicarboxylates such as succinate would result in the transfer of two positive charges. The amount of charge that is transferred across the membrane during the transport process can be quantified electrophysiologically in *X. laevis* oocytes by measuring the charge transfer and the substrate transfer simultaneously. The magnitude of currents induced by succinate and citrate in oocytes expressing rat NaCT is at least 10 times higher than that seen in oocytes expressing *C*. *elegans* NaCT. This made the analysis of the charge: substrate transfer ratio possible in the case of rat NaCT using the Fetchex protocol. These studies have shown that the charge: substrate transfer ratio is 1:1 for citrate and 2:1 for succinate.

Since citrate is an excellent substrate for NaCT, the physiological function of this transporter will be related to energy production and synthesis of fatty acids and cholesterol. The successful cloning of this transporter from *C*. *elegans* has provided an opportunity to demonstrate this relationship. Studies of NaCT knockdown using the RNAi technique in *C. elegans* show suppression of NaCT function does lead to a significant increase in the average life span of the organism. Interestingly, previous studies have shown that suppression of NaDC2 function also leads to life span extension. This is not surprising because both NaCT and NaDC2 are functionally similar in terms of transport of succinate and other dicarboxylate intermediates of TCA cycle. Therefore, knockdown of NaCT or NaDC2 impair the utilization of succinate and other TCA cycle intermediates for energy production, leading to a metabolic state similar to that of caloric restriction and hence to life span extension. However, even though NaCT and NaDC2 are similar in their ability to transport succinate and other dicarboxylates, they differ markedly in their ability to transport citrate. This tricarboxylate occupies a unique position in metabolism in that it is not only an intermediate in the TCA cycle but also a precursor for the synthesis of fatty acids and cholesterol.

Therefore, these two transporters differ in their role in body fat storage. The knockdown of NaCT function leads to a marked decrease (65%) in the fat content of the organism. Similar maneuver with NaDC2 also decreases the fat content, but to a much lesser degree (30%). This is because succinate and other dicarboxylate intermediates of the TCA cycle can enter the TCA cycle leading to the conversion of their carbon skeleton to citrate within the mitochondria. The mitochondria-derived citrate can subsequently function as a precursor for fatty acid and cholesterol synthesis in the cytoplasm after being transported out of the mitochondria. Since the influence of NaCT knockdown on body fat content is much higher than that of NaDC2 knockdown, the utilization of extracellular citrate is quantitatively more important than the mitochondria-derived citrate in the biosynthesis of fatty acids. NaDC1, also transports succinate and other dicarboxylate intermediates of the TCA cycle as does NaDC2, but the knockdown of NaDC1 has no detectable influence on body fat storage and on life span. This may be because NaDC1 is a low affinity transporter whereas NaDC2 is a high affinity transporter.

In summary, in this example, a Na⁺-coupled transporter was identified in *C. elegans* which is an ortholog of mammalian NaCT. Similar to the mammalian NaCT, *C. elegans* NaCT transports not only succinate and other dicarboxylate intermediates of the TCA cycle but also the tricarboxylate citrate. This example demonstrates the role of this novel transporter in metabolic functions in *C*. *elegans.* This transporter plays a critical role in life span and body fat storage. The knockdown of the function of the transporter in this organism leads to an increase in life span and a decrease in body fat content.

### Example 5

### Murine Na⁺-coupled Citrate Transporter (NaCT): Primary Structure, Genomic Organization, and Transport Function

This example presents the molecular cloning and functional characterization of mouse NaCT, the murine ortholog of *Drosophila* Indy, and provides evidence for the electrogenic nature of the transport process mediated by the rodent NaCT, irrespective of whether the transported substrate is a tricarboxylate or a dicarboxylate. Mouse NaCT consists of 572 amino acids and is highly similar to rat and human NaCTs in primary sequence. The murine *nact* gene coding for the transporter is approximately 23 kb-long and consists of 12 exons. When expressed in mammalian cells, the cloned transporter mediates the Na⁺-coupled transport of citrate and succinate. Competition experiments reveal that mouse NaCT also recognizes other citric acid cycle intermediates such as malate, fumarate, and 2-oxo-glutarate as excellent substrates. The Michaelis-Menten constant for the transport process is 22 ± 2 µM for citrate and 33 ± 4 µM for succinate.

The transport process is obligatorily dependent on Na⁺ and the Na⁺-activation kinetics indicates that multiple Na⁺ ions are involved in the activation process. Extracellular pH has a differential effect on the transport function of mouse NaCT depending on whether the transported substrate is citrate or succinate. When examined in the *Xenopus laevis* oocyte expression system with the two-microelectrode voltage-clamp technique, the transport process mediated by mouse NaCT is electrogenic. The charge-to-substrate ratio is 1 for citrate and 2 for succinate. The most likely transport mechanism predicted by these studies involves the transport of citrate as a trivalent anion and succinate as a divalent anion with a fixed Na⁺:substrate stoichiometry of 4:1. This example provides the first unequivocal evidence for the electrogenic nature of mammalian NaCT, providing the first direct evidence that NaCT can transport its substrates in trivalent as well as divalent forms with a fixed Na⁺:substrate stoichiometry of 4:1.

### MATERIALS AND METHODS

Cloning of mouse NaCT cDNA. A search of GenBank database using the rat and human NaCT amino acid sequences as queries identified a sequence (Genbank Accession No. XM_137672), predicted from the NCBI (National Center for Biotechnology Information) contig NT_039520 by automated computational analysis using the gene prediction method GenomeScan, which is most likely the mouse ortholog of NaCT. This 1947 bp-long (open reading frame plus the termination codon) sequence codes for a putative protein consisting of 648 amino acids. This putative protein is 76 amino acids longer than the cloned rat NaCT and 80 amino acids longer than the cloned human NaCT. Since this is a theoretically predicted sequence of the coding region by computational analysis, it does not necessarily represent the sequence of the actual mRNA. A comparison of the nucleotide sequence of this putative mRNA with that of the mouse gene (contig NT_039520) led to the identification of the exons that code for the sequences containing the translational start codon and the translational termination codon.

In addition, a search of GenBank database revealed that the sequence containing the translational termination codon is located in two established sequence tags (ESTs) (gi10645928 and gi6101350). This information allowed the design of primers suitable for amplification of the entire coding region of mouse NaCT using mouse brain mRNA as the template. The upstream primer, containing the translational start codon (shown in bold), was 5'-GTCTCCCTTTCACGCGATGG-3' (SEQ ID NO:27) and the downstream primer, located just two nucleotides downstream of the translational termination codon, was 5'-TCGTCTAGAGCTTGTGCTCTTGCGGCTCT-3' (SEQ ID NO:28). The underlined sequence in the downstream primer is an *Xba*I site, added to the 5'-end of the primer for cloning purpose. RT-PCR with these primers and mouse brain mRNA as the template yielded an approximately 1.8 kb product. This product was subcloned into pGEM-T Easy vector. The insert was then released from the plasmid by digestion with *Eco*RI and *Xba*I and subcloned into the vector pGH19 at the *Eco*RI/*Xb*aI site. The pGH19 vector contains the 3'-untranslated region of the *Xenopus* β-globin gene downstream of the cloning site and has been shown to increase the expression levels of heterologous genes in oocytes (Liman et al., Neuron (1992);9: 861-871, Trudeau et al., Science (1995);269: 92-95). This vector also contains the T7 promoter upstream of the cloning site and thus is suitable for functional expression in mammalian cells using the vaccinia virus expression technique (Inoue et al., J. Biol. Chem. (2002);277: 39469-39476 and Inoue et al., Biochem. Biophys. Res. Commun. (2002);299: 465-471). The sense, as well as antisense, strands of the cDNA insert were sequenced by the *Taq* DyeDeoxy terminator cycle method using an automated Perkin-Elmer Applied Biosystems 377 Prism DNA sequencer. The sequence was analyzed using the NCBI server, available on the worldwide web at ncbi.nlm.nih.gov.

Functional expression of mouse NaCT cDNA in human retinal pigment epithelial (HRPE) cells. The cloned mouse NaCT cDNA was expressed functionally in HRPE cells using the vaccinia virus expression technique (Inoue et al., J. Biol. Chem. (2002);277: 39469-39476 and Inoue et al., Biochem. Biophys. Res. Commun. (2002);299: 465-471). Cells transfected with vector alone served as control for determination of endogenous transport activities. The transport of [¹⁴C]-citrate (sp. radioactivity, 55 mCi/mmol) and [³H]-succinate (sp. radioactivity, 40 Ci/mmol), both from Moravek Biochemicals (Brea, CA, USA), was measured in control cells and in cDNA-transfected cells in parallel. Based on time course studies, initial transport rates for citrate and succinate were measured using a 30-min incubation and a 15-min incubation, respectively. The transport buffer was 25 mM Hepes/Tris (pH 7.5) containing 140 mM NaCl, 5.4 mM KCl, 1.8 mM CaCl₂, 0.8 mM MgSO₄, and 5 mM glucose.

Interaction of various citric acid cycle intermediates and related compounds with the transporter was assessed by monitoring the ability of these compounds to compete with citrate and succinate for NaCT-mediated transport. The NaCT-specific transport was determined by subtracting the transport values measured in vector-transfected cells from the transport values measured in cDNA-transfected cells. Substrate saturation kinetics was analyzed by fitting the NaCT-specific transport data to the Michaelis-Menten equation. The kinetic constants (Michaelis-Menten constant, *Kₜ* and maximal velocity, *Vₘₐₓ*) were calculated by using non-linear as well as linear regression methods. The dependence of NaCT-mediated transport of citrate and succinate on Na⁺ was determined by comparing the transport values measured in the presence of varying concentrations of Na⁺ where NaCl was replaced isoosmotically with *N-*methyl-D-glucamine chloride. The Na⁺-activation kinetics was analyzed by fitting the NaCT-specific transport data to the Hill equation. The Hill coefficient (*n_{H}*, the number of Na⁺ involved in the activation process) was determined by using non-linear as well as linear regression methods.

Functional expression of mouse and rat NaCTs in *Xenopus laevis* oocytes. Capped cRNA from the cloned mouse NaCT cDNA was synthesized using the mMESSAGE mMACHINE kit (Ambion Inc., Austin, TX, USA). In some experiments, rat NaCT of Example 2 was used for functional expression. Mature oocytes from *Xenopus laevis* were isolated by treatment with collagenase A (1.6 mg/ml), manually defolliculated, and maintained at 18° C in modified Barth's medium supplemented with 10 mg/ml gentamycin as described previously (Hatanaka et al., J. Clin. Invest. (2001);107: 1035-1043 and Nakanishi et al., J. Physiol. (2001);532: 297-304). On the following day, oocytes were injected with 50 ng cRNA. Water-injected oocytes served as controls. The oocytes were used for electrophysiological studies 4-6 days after cRNA injection. Electrophysiological studies were performed by the two-microelectrode voltage-clamp method (Hatanaka et al., J. Clin. Invest. (2001);107: 1035-1043 and Nakanishi et al., J. Physiol. (2001);532: 297-304). Oocytes were perifused with a NaCl-containing buffer (100 mM NaCl, 2 mM KCl, 1 mM MgCl₂, 1 mM CaCl₂, 3 mM Hepes, 3 mM Mes, and 3 mM Tris, pH 7.5), followed by the same buffer containing citrate and succinate. The membrane potential was clamped at - 50 mV. The charge-to-substrate ratio was determined for citrate and succinate in three different oocytes. The oocytes were perifused with 50 µM [¹⁴C]-citrate or 50 µM succinate (unlabeled plus radiolabeled succinate) and inward currents were monitored over a period of 10 min. At the end of the experiment, the amount of citrate and succinate transported into the oocytes was calculated by measuring the radioactivity associated with the oocytes. The area within the curve describing the relationship between the time and inward current was integrated to calculate the charge transferred into the oocyte during incubation with citrate or succinate. The values for substrate transport and charge transfer were used to determine the charge-to-substrate ratio.

Data analysis. Experiments with HRPE cells were repeated three times with three independent transfections and transport measurements were made in duplicate in each experiment. Electrophysiological measurements of substrate-induced currents were repeated at least three times with separate oocytes. The data are presented as means ± S. E. of these replicates. The kinetic parameters were calculated using the commercially available computer program Sigma Plot, version 6.0 (SPSS, Inc., Chicago, IL, USA).

### RESULTS

Structural features of mouse NaCT. The coding region of mouse NaCT cDNA (SEQ ID NO:9) is 1719 bp long (including the termination codon) and the predicted protein coded by this cDNA consists of 572 amino acids (SEQ ID NO:10). See Fig. 27. This protein is 76 amino acids shorter than the protein predicted by computational analysis (GenBank Accession No. XM_137672). A comparison of the nucleotide sequence of this predicted mRNA with that of the mouse *nact* gene located in the contig NT_039520 shows that the extra 76 amino acids arise from intron 1 using alternative splice junctions as predicted by computational analysis. This sequence is however not found in the mRNA isolated from mouse brain. The mouse NaCT protein cloned from the brain shows 86% sequence identity (93% similarity) with rat NaCT and 74% sequence identity (85% similarity) with human NaCT (Fig. 28A). The amino acid sequence identity is much lower with other members of the gene family (SLC13), namely the low-affinity Na⁺-coupled dicarboxylate transporter NaDC1 (mouse NaCT versus mouse NaDC1, 50% identity), the high-affinity Na⁺-coupled dicarboxylate transporter NaDC3 (mouse NaCT versus mouse NaDC3, 44% identity), the Na⁺-coupled sulfate transporter NaSi1 (mouse NaCT versus mouse NaSi1, 40% identity) and the sulfate transporter SUT1 (mouse NaCT versus mouse SUT1, 39% identity).

NaCT represents the mammalian ortholog of *Drosophila* Indy with which the cloned mouse NaCT shares 33% identity and 57% similarity in amino acid sequence. Hydropathy analysis, according to the computer program Tmpred, available on the worldwide web at ch.embnet.org/cgi-bin/TMPRED_form_parser, predicts two alternative models for mouse NaCT. According to the strongly preferred model, mouse NaCT possesses 13 transmembrane domains with its amino terminus facing the cytoplasmic side of the membrane and C-terminus facing the exoplasmic side of the membrane. The alternative model predicts mouse NaCT as possessing 12 transmembrane domains with its N-terminus and C-terminus facing the exoplasmic side of the membrane. There are three putative *N*-glycosylation sites, Asn-179, Asn-382, and Asn-566. Of these, only Asn-566 is preserved in rat and human NaCT. In the topology model with 13 transmembrane domains, Asn-179 and Asn-382 are located in the cytoplasmic loops and Asn-566 lies on the exoplasmic side. In the topology model with 12 transmembrane domains, all three Asn residues are located on the exoplasmic side of the membrane.

Exon-intron organization of mouse *nact* gene. The mouse gene coding for NaCT is located on chromosome 11 and consists of 12 exons (Fig. 28B). Exon 1 codes for the translational start site and exon 12 codes for the translational termination site. The size of the gene is approximately 23 kb, excluding the promoter region. The exact lengths of individual exons and introns and the identity of the splice junctions are described in Table 9.

Functional features of mouse NaCT as assessed in a mammalian cell expression system. Fig. 29 shows the transport of succinate and citrate by mouse NaCT when expressed heterologously in HRPE cells. The transport of succinate (50 nM) is 7.8-fold higher in cells transfected with mouse NaCT cDNA than in cells transfected with vector alone (42.7 ± 4.6 fmol/10⁶ cells/min in cDNA-transfected cells and 5.5 ± 0.7 fmol/10⁶ cells/min in vector-transfected cells). The transport of citrate (20 µM) is 20.2-fold higher in cells transfected with mouse NaCT cDNA than in cells transfected with vector alone (48.4 ± 1.0 pmol/10⁶ cells/min in cDNA-transfected cells and 2.4 ± 0.1 pmol/10⁶ cells/min in vector-transfected cells). These data show that mouse NaCT is able to transport succinate as well as citrate and that the magnitude of citrate transport is much higher than that of succinate transport in terms of stimulation of transport induced by the cloned transporter. Kinetic analysis indicates that the transport of citrate and succinate mediated by mouse NaCT is saturable (Fig. 30). The Michaelis-Menten constant (*Kₜ*) is 22 ± 2 µM for citrate and 33 ± 4 µM for succinate. The corresponding values for the maximal velocity (*Vₘₐₓ*) are 118 ± 6 pmol/10⁶ cells/min (citrate) and 80 ± 5 pmol/10⁶ cells/min (succinate), respectively.

The transport of citrate and succinate via mouse NaCT is obligatorily dependent on the presence of Na⁺. Fig. 31 describes the Na⁺-activation kinetics for the transport of citrate and succinate. The relationship between the transport rate and Na⁺ concentration is sigmoidal for both substrates. Analysis of the data according to Hill equation shows that the Hill coefficient (*n_{H}*; the number of Na⁺ involved in the activation process) is 3.3 ± 0.6 for citrate and 2.0 ± 0.3 for succinate. Interestingly, the transport of citrate reaches saturation with a Na⁺ concentration of 120 mM and therefore the *K_{0.5}* for Na⁺ (i.e., the concentration of Na⁺ needed for half-maximal activation of the transport process) could be determined without ambiguity. The value is 72 ± 6 mM. In contrast, the transport of succinate does not reach saturation within the concentration range of Na⁺ tested (upto 140 mM) and therefore the *K_{0.5}* could not be reliably determined.

The substrate specificity of mouse NaCT was investigated by assessing the ability of various citric acid cycle intermediates and related compounds to compete with [¹⁴C]-citrate and [³H]-succinate for transport via mouse NaCT. These studies have shown that citrate, succinate, fumarate, malate, and 2-oxo-glutarate are recognized by the transporter as substrates. These five compounds effectively competed with radiolabeled citrate and succinate for transport via mouse NaCT (Table 10). The monocarboxylates pyruvate and lactate exhibit no or little inhibitory activity for the transport of citrate and succinate. Similarly, maleate, the *cis* isomer of fumarate, is also not effective as an inhibitor. When compared with citrate, the tricarboxylates isocitrate and cis-aconitate also do not interact with the transporter effectively.

Citrate is a tricarboxylate with pK values of 3.1, 4.8, and 6.4. Succinate is a dicarboxylate with pK values of 4.2 and 5.6. At pH 7.5, citrate exists about 90% as a trivalent anion and about 10% as a divalent anion. Succinate, on the other hand, exists almost completely as a divalent anion at pH 7.5. Therefore, it would be of interest to compare the influence of pH on the NaCT-mediated transport of citrate and succinate under identical conditions. The data, shown in Fig. 32, indicate that pH has differential influence on the transport of citrate (10 µM) and succinate (10 µM) via mouse NaCT. The transport of citrate exhibits a clear optimum pH at 7. The transport rate decreases significantly when the pH is made more alkaline or more acidic than 7. In contrast, the transport of succinate is maximal in the pH range 7.5-8.5 and decreases significantly when the pH is less than 7.5.

Functional features of mouse NaCT as assessed in the *Xenopus laevis* oocyte expression system. The studies of Examples 2 and 3 have indicated that mammalian NaCT is electrogenic, based on the influence of membrane depolarization of the transport activity. The present studies with mouse NaCT show that both succinate and citrate are transported via this transporter. Even though the data from the Na⁺-activation kinetics have shown that multiple Na⁺ ions are involved in the transport process, the participation of 2 Na⁺ ions in the activation of succinate transport and 3 Na⁺ ions in the activation of citrate transport does not necessarily predict the electrogenic nature of these transport processes. Therefore, the *Xenopus* oocyte expression system was chosen to analyze the electrogenicity of mouse NaCT.

Expression of mouse NaCT in oocytes led to a marked increase in the uptake of [¹⁴C]-citrate and [³H]-succinate, indicating functional expression of the transporter. These oocytes were then used for electrophysiological studies to determine if the perifusion of the oocytes in the presence of substrates leads to inward currents when monitored by the two-microelectrode voltage-clamp method. These studies have shown that exposure of the NaCT-expressing oocytes to citrate induces measurable inward currents (30 ± 7 nA at 0.5 mM citrate in three different oocytes) (Fig. 33). The citrate-induced current is obligatorily dependent on the presence of Na⁺. Isoosmotic replacement of Na⁺ with *N*-methyl-D-glucamine abolishes the citrate-induced currents almost completely. These currents are not dependent on Cl⁻ as isoosmotic replacement of Cl⁻ with gluconate does not affect the citrate-induced currents (Fig. 33). Exposure of the oocytes to succinate also induces inward currents in a Na⁺-dependent manner.

An analysis of the charge-to-substrate ratio for the transport process was motivated by the findings that the transport of citrate as well as succinate occurs via an electrogenic process. This raises three different possibilities in terms of transport mechanism. First, both citrate and succinate are transported as divalent anions and the Na⁺:substrate stoichiometry is 3:1. In this case, the charge-to-substrate ratio would be 1 for both substrates. Second, citrate is transported as a trivalent anion and succinate is transported as a divalent anion, but the Na⁺: substrate stoichiometry changes from 4:1 for citrate to 3:1 for succinate. In this case also, the charge-to-substrate ratio would remain as 1. Third, citrate is transported as a trivalent anion and succinate is transported as a divalent anion, and the Na⁺:substrate stoichiometry remains as 4:1 for both substrates. In this case, the charge-to-substrate ratio would vary depending on the substrate. This ratio would be 1 for citrate but 2 for succinate.

To differentiate among these three possibilities, the FetchEx method, in which the NaCT-expressing oocytes are perifused with radiolabeled citrate or succinate for a given time period in which the substrate-induced inward currents are monitored, was employed. At the end of the perifusion period, the amount of citrate or succinate transported into the oocytes is measured. The amount of charge transferred into the same oocyte can be calculated from the inward currents. This would allow the determination of the charge-to-substrate ratio. However, this requires a robust electrogenicity of the transport process so that radiolabeled substrate can be mixed with adequate amounts of unlabeled substrate to allow measurable inward currents for charge transfer calculations and, at the same time, to allow measurable transfer of radiolabel into the oocyte so that the amount of substrate transferred can be measured. Previous experience (Wang et al., Am. J. Physiol. (2000);278: C1019-C1030 and Fei et al., Biochim. Biophys. Acta (1999);1418: 344-351), has shown that the substrate-induced currents via mouse NaCT are not sufficient to determine the charge-to-substrate ratio using our electrophysiological set up.

Therefore rat NaCT was used, to see if this transporter is associated with a higher magnitude of substrate-induced currents compared to mouse NaCT. This indeed turned out to be the case. Perifusion of the oocytes, which expressed rat NaCT, to 0.5 mM citrate was found to induce 3-fold higher currents compared to mouse NaCT (Fig. 33). The ability of rat NaCT to transport other intermediates of the citric acid cycle was determine, by comparing the magnitude of inward currents induced by these compounds (Fig. 34). When the concentration of the substrate was kept constant at 0.5 mM, citrate induced the maximum current compared to various putative substrates tested. Fumarate, succinate, malate, and 2-oxo-glutarate, the dicarboxylate intermediates of the citric acid cycle, also induced appreciable currents, but the magnitude of the currents was significantly less that that induced by citrate (20-60% of citrate-induced currents). *Cis*-aconitate induced currents that amounted to approximately 10% of citrate-induced currents. Isocitrate, pyruvate, and lactate induced no or little currents. Since rat NaCT induced marked currents with citrate as well as succinate, the charge-to-substrate ratio for the transport process using rat NaCT rather than mouse NaCT was analyzed. Fig. 35 describes the data for the transport of citrate and succinate via rat NaCT. Even though the transport of both substrates via the transporter is electrogenic, the quantity of the charge transferred into the oocytes per a given amount of the substrate transfer is relatively higher for succinate than for citrate. The charge-to-substrate transfer ratio is 2 for succinate whereas the corresponding value is 1 for citrate, indicating that the Na⁺:substrate stoichiometry is 4:1 irrespective of whether transported substrate is citrate or succinate.

### DISCUSSION

The amino acid sequence of mouse NaCT cloned from the brain differs from that predicted from the mouse gene sequence by computational analysis by a stretch of 76 amino acids that is predicted by the GenomeScan but not found in the cloned NaCT. This additional sequence arises from the use of alternative splice junctions predicted by the GenomeScan in intron 1. The nucleotide sequence of the cloned NaCT cDNA (SEQ ID NO:9) shows that exon 1 is 102 bp-long (starting from the translational initiation codon ATG) and that exon 2 is 129 bp-long. However, according to the GenomeScan prediction, exon 1 contains an additional 194 bp sequence at its 3'-end and exon 2 contains an additional 34 bp sequence at its 5'-end. This stretch of 228 bp, predicted to be a part of the coding region, gives rise to the extra 76 amino acids. This sequence is however not found in the cDNA cloned from mouse brain. Whether these alternative splice junctions are used in tissues other than the brain is not known. NaCT has been cloned from rat brain and from a human liver cell line, but there is no evidence for the presence of alternative splice variants in these tissues. Therefore, it seems very unlikely that the stretch of the additional 76 amino acids predicted by the GenomeScan is actually found in NaCT expressed in any tissue.

Analysis of the amino acid sequence (SEQ ID NO:10) of the cloned mouse NaCT using the TMpred program predicts two different topology models, one with 13 transmembrane domains with its N-terminus placed on the cytoplasmic side of the membrane and C-terminus placed on the external surface of the membrane and the other with 12 transmembrane domains with its N-terminus and C-terminus placed on the external surface. This protein also possesses three putative *N*-glycosylation sites, of which Asn-566 is conserved in rat and human NaCTs. Additional evidence that the corresponding Asn residue in human NaCT (Asn-562) is most likely to be *N*-glycosylated is that treatment of HepG2 cells with tunicamycin, an inhibitor of *N*-linked glycosylation, leads to a significant inhibition of NaCT activity. These data suggest that Asn-566 in mouse NaCT is also likely to be *N*-glycosylated. Both topology models with either 12 or 13 transmembrane domains are in agreement with the potential *N-*glycosylation of Asn-566 because in both models this residue is located on the exoplasmic side of the membrane. Interestingly, analysis of the amino acid sequences of rat and human NaCTs using the same TMpred program indicates that these proteins are possess 12 transmembrane domains instead of 13. However, the program indicates that both N-terminus and C-terminus are most likely to be located on the external surface of the membrane, and places the conserved *N*-glycosylation site in the C-terminus tail in rat and human NaCTs on the external side of the membrane.

While Example 2 and Example 3 present the cloning and functional characteristics of rat and human NaCT, the present example provides important information, for the first time, on the transport mechanism of NaCT with respect to its Na⁺:substrate stoichiometry and electrogenicity. Mouse NaCT, when expressed in mammalian cells, transports not only citrate but also succinate. This is similar to rat NaCT, as shown in Example 2. In contrast, human NaCT exhibits very little ability to transport succinate (see Example 3). pH-dependence of the transport process mediated by mouse NaCT shows that the transport of succinate remains almost unaffected in the pH range 7.0-8.5 whereas the transport of citrate increases markedly when the pH is changed from 8.5 to 7.0. These data can be taken as evidence that citrate is recognized by NaCT in its protonated divalent anionic form because the fraction of the divalent form of citrate increases as the pH is changed from 8.5 to 7.0. The divalent form of succinate does not change significantly in the pH range 7.0-8.5.

Similar conclusions have been drawn in the case of NaDC1 and NaDC3 (Pajor, Annu. Rev. Physiol. (1999);61: 663-682 and Pajor, J. Membr. Biol. (2000);175: 1-8). Na⁺-activation kinetics of mouse NaCT shows that multiple Na⁺ ions are involved in the transport process. The Na⁺:substrate stoichiometry is 3:1 for citrate and 2:1 for succinate. These data, together with the results from the pH-dependence studies, would suggest that citrate transport is electrogenic whereas succinate transport is electroneutral. This however is not the case as evident from the oocyte expression system. In contrast to the studies with the mammalian cell expression system, electrophysiological studies with the oocyte expression system provide evidence for a completely different transport mechanism for mouse NaCT. The transport of citrate as well as succinate occurs via an electrogenic process. Exposure of NaCT-expressing oocytes to citrate or succinate is associated with inward currents in the presence of Na⁺, indicating that, for citrate as well as succinate, the transport process results in the transfer of net positive charge into the oocytes. But, the amount of positive charge transferred into the oocytes varies depending on whether the transported substrate is citrate or succinate. With citrate, the charge-to-substrate ratio is 1, indicating that citrate transport is associated with the transfer of one positive charge per citrate molecule. Therefore, the Na⁺:citrate stoichiometry should be 3:1 if citrate is recognized by the transporter as a divalent anion or 4:1 if the trivalent anionic form is recognized instead.

In contrast to citrate, the charge-to-substrate ratio is 2 for succinate that indicates that succinate transport is associated with the transfer of two positive charges per succinate molecule. Since succinate exists predominantly as a divalent anion under the experimental conditions, the Na⁺:succinate stoichiometry should be 4:1. Taken collectively, the data indicate that mouse NaCT transports succinate as well as citrate by an electrogenic process with a Na⁺:substrate stoichiometry of 4:1. Furthermore, the charge-to-substrate ratio of 2 for succinate and 1 for citrate strongly suggests that NaCT is capable of transporting citrate as a trivalent anion and succinate as a divalent anion. Thus, NaCT is a Na⁺-coupled transporter for dicarboxylates as well as tricarboxylates. This characteristic is distinct from that of NaDC1 and NaDC3 which recognize their substrates only in their divalent anionic form.

**TABLE 9**

| Exon-intron organization of mouse *nact* gene | | | | | | | | |
|---|---|---|---|---|---|---|---|---|
| 5' Exon | | | Intron | | | | 3' Exon | |
| No. | Size (bp) | Sequence | Donor | Size (bp) | No. | Acceptor | Sequence | No. |
| 1 | >118 | ...GACAAG | gtcagt... | 4312 | 1 | ...cttcag | TTTGCC... | 2 |
| 2 | 129 | ...AAGCAG | gtgagt... | 273 | 2 | ...ctccag | GTATGT... | 3 |
| 3 | 137 | ...CTCACG | gtaaac... | 1230 | 3 | ...ctgcag | GCTGAT... | 4 |
| 4 | 188 | ...TGCCAG | gtgcac... | 1068 | 4 | ...ccacag | GAAGCC... | 5 |
| 5 | 169 | ...GCAGGA | gtgagt... | 1475 | 5 | ...tttcag | ATTGTT... | 6 |
| 6 | 123 | ...ACACAA | gtaagt... | 3957 | 6 | ...gtctag | TTTAAA... | 7 |
| 7 | 219 | ...CACCGT | gtaagt... | 825 | 7 | ...tatcag | TCATAT... | 8 |
| 8 | 101 | ...AGGAAG | gtaagg... | 1413 | 8 | ...tcgcag | AAAGGA... | 9 |
| 9 | 119 | ...TGTGAG | gtactt... | 2897 | 9 | ...tcctag | ACGTCA... | 10 |
| 10 | 162 | ...TCCATG | gtaagt... | 2403 | 10 | ...ctctag | GCTCGT... | 11 |
| 11 | 138 | ...GACATG | gtaaca... | 1489 | 11 | ...ccctag | ATGAAA... | 12 |
| 12 | >132 | ...ACTTAG... | | | | | | |

**TABLE 10**

| Substrate specificity of mouse NaCT | | | | |
|---|---|---|---|---|
| Substrate analog | [¹⁴C]-Citrate transport | | [³H]-Succinate transport | |
| | pmol/10⁶ cells/min | % | pmol/10⁶ cells/min | % |
| Control | 38.4 ± 6.0 | 100 | 0.145 ± 0.010 | 100 |
| Citrate | 0.7 ± 0.1 | 2 | 0.004 ± 0.001 | 3 |
| Succinate | 0.1 ± 0.1 | 1 | 0.007 ± 0.003 | 5 |
| Fumarate | 1.6 ± 0.4 | 4 | 0.007 ± 0.002 | 5 |
| Malate | 0.2 ± 0.1 | 1 | 0.004 ± 0.001 | 3 |
| 2-Oxo-glutarate | 4.6 ± 0.5 | 12 | 0.018 ± 0.001 | 12 |
| Maleate | 31.2 ± 2.5 | 82 | 0.165 ± 0.014 | 113 |
| Isocitrate | 26.3 ± 1.1 | 69 | 0.125 ± 0.003 | 86 |
| *Cis*-Aconitate | 18.1 ± 1.8 | 47 | 0.062 ± 0.005 | 43 |
| Pyruvate | 32.3 ± 1.5 | 84 | 0.151 ± 0.013 | 104 |
| Lactate | 29.8 ± 3.4 | 78 | 0.162 ± 0.008 | 11 |

| | | | | |
|---|---|---|---|---|
| Transport of [¹⁴C]-citrate (10 µM) and [³H]-succinate (0.1 µM) was measured in parallel in vector-transfected cells and in cells transfected with mouse NaCT cDNA in the absence or presence of 2 mM various substrate analogs. NaCT-specific transport was calculated by subtracting the transport in vector-transfected cells from the transport in cDNA-transfected cells. Data (mean ± S.E.) represent only the NaCT-specific transport. | | | | |

The electrophysiological data from the oocyte expression studies demonstrate that the number of Na⁺ involved in the transport process is 4 irrespective of whether the transported substrate is citrate or succinate. In contrast, the Na⁺-activation kinetics in mammalian cell expression studies indicated that the number of Na⁺ ions involved in the transport process is 3 if the transported substrate is citrate and that the number changes to 2 if the transported substrate is succinate. However, since the oocyte expression studies demonstrate unequivocally the eletrogenic nature of the transport process for succinate which exists almost entirely as a divalent anion under the experimental conditions, the Na⁺:succinate stoichiometry of 2 cannot be correct.

Obviously, the value obtained for the number of Na⁺ ions involved in the transport of succinate in the mammalian cell expression system is an underestimate. The value of 3 for the Na⁺:citrate stoichiometry can be reconciled with the electrogenic nature of the transport process if one hypothesizes that citrate is transported as a divalent anion. But, the charge-to-substrate transfer ratios calculated from the oocyte expression studies suggest that citrate is recognized as a trivalent anion while succinate is recognized as a divalent anion. Therefore, the value obtained for the number of Na⁺ ions involved in the transport of citrate in the mammalian cell expression system is also likely to be an underestimate. There is precedence for such discrepancies between the Na⁺:substrate coupling ratio determined from Na⁺-activation kinetics (i.e., the Hill coefficient) and the actual coupling ratio (Pajor and Sun, Am. J. Physiol. (2000);279: F482-F490 and Pajor et al., Am. J. Physiol. (2001);280: C1215-C1223). For mouse NaDC1 and NaDC3, the Hill coefficient calculated from Na⁺-activation kinetics is 2 with succinate as the substrate while the transport process is undoubtedly electrogenic. The electrogenicity of the transport process mandates that the Na⁺-to-succinate coupling ratio should be at least 3. This apparent discrepancy arises because the Hill coefficient does not always represent the absolute value. The Hill coefficient represents the minimal number of Na⁺ binding sites involved in the activation process and the value for the Hill coefficient determined from Na⁺-activation kinetics is influenced significantly by the strength of cooperativity among the binding sites.

Another significant difference observed in the mammalian cell expression system is in the nature of Na⁺-activation kinetics between the transport of succinate and citrate. The transport of citrate saturates within the concentration range of Na⁺ tested whereas the transport of succinate does not saturate under identical conditions. Apparently, the cooperativity among the different Na⁺-binding sites differs depending on whether the transported substrate is succinate or citrate. Whether this is due to the difference in the ionic nature (i.e., divalent nature of succinate and the trivalent nature of citrate) or the bulkiness of these two substrates is not known.
The substrate specificity of mammalian NaCTs merits discussion. Human NaCT appears to be rather specific for citrate as its interaction with succinate and other dicarboxylates occurs with a very low affinity. In contrast, both rat and mouse NaCTs interact with citrate as well as succinate with comparable affinity. In this respect, the rodent NaCTs are similar to *Drosophila* NaCT (Indy). This functional difference can be used as a diagnostic criterion for the identification of the protein domains involved in substrate binding using human-mouse or human-rat chimeric NaCTs. A similar approach has been used to obtain valuable information on the substrate binding site of other transporters such as NaDC3 (Wang et al., Am. J. Physiol. (2000);278: C1019-C1030) and OCTN2 (Seth et al., J. Biol. Chem. (1999);274: 33388-33392).

### Example 6

### Zebrafish Na⁺-coupled Citrate Transporter (NaCT): Primary Structure and Transport Function

The zebrafish Na⁺-coupled citrate transporter (NaCT) has been cloned and functionally characterized. The nucleotide sequence of the full-length cDNA clone and translated amino acid sequence of the zebrafish Na⁺-coupled citrate transporter are SEQ ID NO:11 and SEQ ID NO:12, respectively, are shown in Fig. 36.

A comparison of the amino acid sequence of zebrafish NaCT (SEQ ID NO:12) with that of rat (SEQ ID NO:4), mouse (SEQ ID NO:10), and human (SEQ ID NO:6), carried out as described in Examples 1-5, is shown in Fig. 37. Zebrafish NaCT is 61% identical and 77% similar to human NaCT. Zebrafish NaCT is 57% identical and 72% similar to rat NaCT. Zebrafish NaCT is 57% identical and 74% similar to mouse NaCT.

Using the methods described in Examples 1-5, the zebrafish clone was determined to be fully functional. Fig. 38A shows a time course of citrate (2 µM) uptake in cells transfected with either vector alone or zebrafish NaCT cDNA and Fig. 38B demonstrates the influence of pH on citrate (1 µM) uptake mediated by zebrafish NaCT. Saturation kinetics (Fig. 39A) and Na⁺-activation kinetics (Fig. 39B) of citrate uptake mediated by zebrafish NaCT. The Michaelis constant for citrate uptake is 40 ± 4 µM. The value for Hill coefficient for the activation of uptake is 26 ± 0.2. Fig. 40A shows the inhibition of zebrafish NaCT-mediated [¹⁴C]-citrate (1 µM)uptake by various structural analogs (2mM). Fig. 40B demonstrates dose-response relationships for inhibition of zebrafish NaCT-mediated [¹⁴C]-citrate (1 µM) uptake by citrate, succinate, and cis-aconitate. The IC₅₀ values for the inhibition (i.e., the concentration of the inhibitor necessary for 50% inhibition) are 30 ± 4, 51 ± 9, and 624 ± 45 µM, respectively, for citrate, succinate, and cis-aconite.

### Example 7

### Human NaCT, the Ortholog of Drosophila Indy, as a Novel Target for Lithium Action

NaCT is a Na⁺-coupled citrate transporter recently identified in mammals that mediates the cellular uptake of citrate. It is expressed predominantly in the liver. NaCT is structurally and functionally related to the product of the *indy* gene in *Drosophila* whose dysfunction leads to lifespan extension. This example shows that NaCT mediates the utilization of extracellular citrate for fat synthesis in human liver cells and that the process is stimulated by lithium. The transport function of NaCT is enhanced by lithium at concentrations found in humans treated with lithium for bipolar disorder. Valproate and carbamazepine, two other drugs that are used for the treatment of bipolar disorder, do not affect the function of NaCT. The stimulatory effect of Li⁺ is specific for human NaCT as NaCTs from other animal species are either inhibited or unaffected by Li⁺. The data suggest that two of the four Na⁺-binding sites in human NaCT may become occupied by Li⁺ to produce the stimulatory effect. The stimulation of NaCT in humans by lithium at therapeutically relevant concentrations has potential clinical implications. It is also show here that a single base mutation in codon-500 (TTT→CTT) in human NaCT, leading to the substitution of Phe with Leu, stimulates the transport function and abolishes the stimulatory effect of lithium. This raises the possibility that genetic mutations in humans may lead to alterations in the constitutive activity of the transporter with associated clinical consequences.

As shown in Example 1, the *Indy* gene in *Drosophila* codes for a plasma membrane transporter that transports various dicarboxylates such as succinate as well as the tricarboxylate citrate. Dysfunction of this gene leads to lifespan extension in this organism (Rogina et al., Science (2000);290: 2137-2140). The search for the mammalian ortholog of *Drosophila* Indy has identified NaCT in rat (Example 2) and human (Example 3) tissues as the plasma membrane transporter with functional characteristics similar to those of Indy. NaCT belongs to the sodium dicarboxylate/sulfate cotransporter gene family (*SLC13*) (Pajor, J. Membr. Biol. (2000);175: 1-8). While the previously identified mammalian sodium dicarboxylate cotransporters NaDC1 and NaDC3 recognize only dicarboxylates as substrates, the newly identified NaCT accepts various dicarboxylates as well as the tricarboxylate citrate as substrates. In fact, human NaCT transports citrate much more effectively than succinate. The Na⁺:substrate stoichiometry for NaCT is 4:1 irrespective of whether the transported substrate is a dicarboxylate or a tricarboxylate. In contrast, the Na⁺:substrate stoichiometry for NaDC1 and NaDC3 is 3:1 (Pajor, J. Membr. Biol. (2000);175: 1-8). Earlier studies have shown that NaDC1 and NaDC3 are inhibited by lithium (Pajor, J. Membr. Biol. (2000);175: 1-8 and Wang et al., Am. J. Physiol. (2000);278: C1019-C1030). Urinary excretion of dicarboxylates is increased significantly in patients undergoing lithium therapy for affective disorders (Bond et al., Br. J. Pharmacol. (1972);46: 116-123) and inhibition of NaDC1 in the kidney by Li⁺ is believed to be the cause for this phenomenon (Wright et al., Proc. Natl. Acad. Sci. (1982);79: 7514-7517).

Since NaCT is structurally and functionally similar to NaDCs, this example investigated whether the transport function of NaCT is also inhibited by lithium. These studies have led to unexpected findings. While NaCTs from non-human organisms are either inhibited or unaffected by Li⁺, human NaCT is markedly stimulated by Li⁺. This example identifies human NaCT as a novel target for lithium action and have potential clinical implications for individuals who are on lithium therapy for affective disorders and also shows that NaCT facilitates the utilization of extracellular citrate for lipid synthesis in human liver cells and that Li⁺ stimulates this process. Furthermore, using rat/human chimeric NaCTs and site-directed mutagenesis, it is demonstrated here that a single base mutation in codon-500 (TTT→CTT) in human NaCT, leading to the substitution of Phe with Leu, stimulates the transport function and abolishes the stimulatory effect of lithium.

### MATERIALS AND METHODS

Heterologous expression of NaCTs. NaCT cDNAs cloned from human hepatocarcinoma cell line HepG2 (SEQ ID NO:5), rat brain (SEQ ID NO:3), mouse liver (SEQ ID NO:9), whole zebra fish (SEQ ID NO: 11), and whole *C. elegans* (SEQ ID NO:7) were expressed functionally in a human retinal pigment epithelial cell line (HRPE) using the vaccinia virus expression technique as described previously in Examples 3 and 5.

The same approach was also used to analyze the function of chimeric and mutant NaCTs. The transport function of NaCTs was monitored by measuring the uptake of [¹⁴C]citrate (Moravek Biochemicals, Brea, CA) using the uptake medium containing 140 mM NaCl, 5.4 mM KCl, 1.8 mM CaCl₂, 0.8 mM MgSO₄, and 5 mM glucose, buffered with 25 mM Hepes/Tris, pH 7.5. Uptake measurements were made in parallel in cDNA-transfected cells and in cells transfected with vector alone. The uptake in vector-transfected cells (endogenous activity) was subtracted from the uptake in cDNA-transfected cells to determine the cDNA-specific activity. When the effect of Li⁺ was studied, the uptake medium contained either LiCl or equimolar concentrations of *N-*methyl-D-glucamine chloride. The Na⁺ activation kinetics were analyzed by measuring the uptake of citrate at increasing concentrations of Na⁺, with the concentration of *N*-methyl-D-glucamine chloride adjusted appropriately to maintain the osmolality of the uptake medium. The Hill coefficient (n_{H}; the number of Na⁺ ions involved in the activation process) was determined by fitting the data to Hill equation. A 30-minute incubation was used in uptake measurements as these experimental conditions have been shown to be suitable for measurement of initial uptake rates for NaCTs.

Uptake measurement in human liver cell lines. The human liver cell lines (HepG2 and Huh-7) were cultured to confluency in 24-well culture plates using appropriate culture medium. The HepG2 cell line was obtained from American Type Culture Collection (Manasses, VA). The Huh-7 cell line (Bode et al., (2002) Am. J. Physiol. 283, G1062-G1073) was provided by Dr. Barrie P. Bode (St. Louis University, St. Louis, MO). Uptake measurements in monolayer cultures of these cells were measured as described above for the heterologously expressed NaCTs.

Measurement of incorporation of citrate and acetate into cellular lipids. Monolayer cultures of HepG2 cells were incubated with [¹⁴C]citrate (0.1 µCi) or [¹⁴C]acetate (0.1 µCi) (American Radiolabeled Chemicals, St. Louis, MO) for 24 hours in a NaCl-containing medium in the presence or absence of 2 or 10 mM Li⁺. The cellular lipids were then extracted by n-hexane/isopropanol (3:2, v/v) as described by Scharnagl et al. (Scharnagl et al., (2001) Biochem. Pharmacol. 62, 1545-1555). The radioactivity associated with the lipid fraction was quantified to calculate the extent of incorporation of extracellularly added [¹⁴C]citrate or [¹⁴C]acetate into lipids.

### RESULTS AND DISCUSSION

First, the influence of Li⁺ on the transport function of rat NaCT was tested. These studies showed that the activity was indeed inhibited by Li⁺ (Fig. 41). However, when the effect of Li⁺ on the activity of human NaCT was tested, the results were quite unexpected. The transport activity of human NaCT was not inhibited but stimulated by Li⁺ (Fig. 41). Li⁺ caused marked inhibition of citrate uptake via rat NaCT with an *IC₅₀* of 2.1 ± 0.8 mM. In contrast, Li⁺ stimulated citrate uptake via human NaCT and the concentration of Li⁺ needed for half-maximal stimulation was 2.1 ± 0.7 mM. The plasma concentrations of Li⁺ in patients treated with lithium are in the range of 0.8-2 mM (Sproule, Clin. Pharmacokinet. (2002);41: 639-660). At a concentration of 2.5 mM, Li⁺ stimulated human NaCT activity by 2.4-fold. The stimulatory effect of Li⁺ is associated with an increase in substrate affinity as well as with a decrease in maximal velocity (Fig. 42A). In the presence of 10 mM Li⁺, the affinity for citrate increased approximately 7-fold *(Kₜ* in the absence of Li⁺, 702 ± 55 µM; *Kₜ* in the presence of Li⁺, 95 ± 15 µM). The normal concentrations of citrate in human blood are in the range of 100-150 µM (Nordmann and Nordmann, Adv. Clin. Chem. (1961);4: 53-120). Therefore, under physiological conditions, the increase in substrate affinity is the primary effect of Li⁺ on cellular entry of citrate via NaCT. Li⁺ cannot support NaCT function in the absence of Na⁺. The stimulation of human NaCT function by Li⁺ was observed only in the presence of Na⁺. The stimulatory effect was greater at lower concentrations of Na⁺ and the effect decreased as the concentration of Na⁺ increased (Fig. 42B). The stimulation was 3-fold at 140 mM Na⁺, but the stimulation was 7-fold in the presence of 60 mM Na⁺. The Na⁺:citrate stoichiometry was also analyzed for the transport process in the presence and absence of Li⁺ by fitting the data from the Na⁺-activation kinetics to Hill equation. The Hill coefficient (n_{H}), which represents the number of Na⁺ ions involved in the activation process, was 4.5 ± 0.7 in the absence of Li⁺. This value changed to 1.8 ± 0.1 in the presence of 10 mM Li⁺. Measurements of citrate transfer and charge transfer in *X. laevis* oocytes expressing mammalian NaCT have shown that the Na⁺:citrate stoichiometry for NaCT is 4:1 and the Hill coefficient of 4.5 ± 0.7 determined in the present study is close to the actual value determined in oocytes. Interestingly, the Na⁺:citrate stoichiometry changes to 2:1 in the presence of Li⁺. These data suggest that two of the four Na⁺-binding sites in human NaCT may become occupied by Li⁺ during the stimulatory process.

These data show that there is a species-specific influence of Li⁺ on the activity of NaCT. To analyze this species-specific phenomenon in a broader scope, the NaCT orthologs from three additional species: mouse, zebra fish, and *C*. *elegans* were cloned. NaCTs from these species also mediate Na⁺-coupled transport of citrate. The influence of Li⁺ on the function of these NaCTs (Table 11) was then tested. The transport function of mouse and zebra fish NaCTs were inhibited by Li⁺ as was rat NaCT. In contrast, NaCT from *C*. *elegans* was not affected by Li⁺. To confirm the stimulatory effect of Li⁺ on human NaCT seen with the cloned transporter, the effect of Li⁺ on constitutively expressed NaCT in human liver cells was studied. For this purpose, two different human hepatoma cell lines (HepG2, and Huh-7) were used. In both cell lines, the constitutive expression of NaCT was detectable as measured by Na⁺-coupled uptake of citrate. Li⁺ stimulated this citrate uptake activity in both cell lines (Table 11).

In addition to lithium, two other drugs, valproate and carbamazepine, are currently used for effective treatment of bipolar disorder. The activity of human NaCT was not affected by therapeutically relevant concentrations of these two drugs at (valproate, 0.6 mM; carbamazepine, 50 µM) (Table 11). The therapeutic plasma levels for valproate and carbamazepine in humans are in the range of 0.3-0.6 mM and 20-50 µM, respectively (Williams et al. (2002) Nature 417, 292-295). Therefore, the concentrations of valproate and carbamazepine used in the present study are clinically relevant. The lack of effect of valproate and carbamazepine on human NaCT was also evident with the constitutively expressed NaCT in HepG2 cells. These drugs had no effect on not only human NaCT but also on NaCTs cloned from other species (Table 11). Thus, among the three widely used mood stabilizers, only lithium has the stimulatory effect on human NaCT.

NaCT is the first plasma membrane transporter identified in mammals that shows preference for citrate as a substrate and operates very effectively under physiological concentrations of citrate in the circulation. Citrate has wide biological functions. It is not only an intermediate in the citric acid cycle and hence a critical substrate for energy production, but also is a source of acetyl CoA in the cytoplasm for the synthesis of cholesterol and fatty acids. Citrate levels in the cytoplasm also signal the energy status of the cell by serving as a potent inhibitor of phosphofructokinase-1, the rate-limiting enzyme in glycolysis. Since the concentrations of citrate in blood are quite high, the presence of a plasma membrane transporter for citrate will provide the cells a continuous supply of an additional source of citrate for utilization in metabolic pathways. Therefore, the recent identification of NaCT as a plasma membrane energy-coupled uphill transporter for citrate in mammals is of great biological relevance. The functional characteristics and the tissue distribution pattern of NaCT support a critical role for this transporter in cellular metabolism. The affinity of human NaCT for citrate is ideal for its role in mediating the cellular entry of citrate from blood (*Kₜ* value, approximately 700 µM; citrate levels in human blood, approximately 135 µM). The transporter is expressed predominantly in the liver. It is also expressed in the testis and brain, but at much lower levels. Liver is metabolically very active, especially in the synthesis of cholesterol and fatty acids and in glucose homeostasis, the biochemical pathways in which citrate plays a critical role. *In situ* hybridization studies show that NaCT mRNA is expressed uniformly in perivenous hepatocytes as well as in periportal hepatocytes. These findings are of interest, considering the metabolic role of citrate in fatty acid synthesis, cholesterol synthesis, glycolysis, and gluconeogenesis. Citrate is not only a precursor for the synthesis of fatty acids and cholesterol but also a potent inhibitor of phosphofructokinase-1, one of the rate limiting enzymes in the glycolytic pathway. Therefore, citrate is an inhibitor of glycolysis and stimulator of gluconeogenesis. These four metabolic processes, namely fatty acid synthesis, cholesterol synthesis, glycolysis, and gluconeogenesis, exhibit zonal heterogeneity in the liver. Fatty acid synthesis and glycolysis occur predominantly in the perivenous hepatocytes whereas cholesterol synthesis and gluconeogenesis occur predominantly in the periportal hepatocytes (Jungermann and Katz, Physiol. Rev. (1989);69: 708-764). The observations that NaCT is expressed in perivenous as well as periportal hepatocytes suggest a role for this transporter in all of these metabolic processes irrespective of the zone-specific occurrence of these processes in the liver.

If NaCT plays a role in facilitating the hepatic utilization of extracellular citrate for the synthesis of fatty acids and cholesterol, the incorporation of extracellular citrate into lipids would be enhanced by Li⁺ in human liver cells. To test this, the incorporation of [¹⁴C]citrate from extracellular medium into lipid fraction that is extractable with n-hexane/isopropanol in HepG2 cells was monitored and the influence of Li⁺ on the process was assessed. The extracted lipid fraction contains triglycerides, cholesterol esters, and cholesterol. These studies have shown that Li⁺ markedly enhances the incorporation of citrate into this lipid fraction (Fig. 43). The stimulation was 2.6 ± 0.1-fold at therapeutically relevant concentrations of Li⁺ (2 mM). There are additional targets for Li⁺ in mammalian cells. Inositol monophosphatase, inositol polyphosphatase, and glycogen synthase kinase-3β are considered to be the primary targets for lithium action related to the therapeutic efficacy of lithium in the treatment of bipolar disorder (Williams et al., Nature (2002);417: 292-295; Williams and Harwood, Trends Pharmacol. Sci. (2000);21: 61-64; Phiel and Klein, Annu. Rev. Pharmacol. Toxicol. (2001);41: 789-813; and Li et al., Bipolar Disord. (2002);4: 137-144). Of these, glycogen synthase kinase-3β is involved in hepatic energy metabolism. Therefore, it is important to establish that the observed Li⁺-induced increase in the incorporation of extracellular citrate into lipids does not occur through targets other than NaCT. To provide evidence for the involvement of NaCT in this process, the incorporation of extracellular [¹⁴C]acetate into lipids in HepG2 cells was monitored and the influence of Li⁺ on this process was assessed (Fig. 43). NaCT has no role in the entry of acetate into hepatocytes. Extracellular acetate was incorporated into lipids extractable with n-hexane/isopropanol as expected, but Li⁺ did not have any effect on this process. These data show that Li⁺ at therapeutic concentrations enhances the utilization of extracellular citrate in human liver cells for the synthesis of fatty acids and cholesterol specifically via stimulation of NaCT.

To determine the domains in human NaCT that are responsible for the stimulation of transport function by Li⁺, 28 different chimeric transporters consisting of different regions of human NaCT and rat NaCT were made and compared their transport function with that of the parent rat and human NaCTs. The results of these studies have shown that replacement of a small region (amino acid position 496-516) in human NaCT with the corresponding region (amino acid position 500-520) in rat NaCT stimulated the transport function 5-fold and activity of this chimeric transporter is not affected by Li⁺ (Fig. 44A). There are only two amino acid differences between human and rat NaCTs in this region (Phe versus Leu and Thr versus Ala) (Fig. 44B). Codon-500 and codon-516 were individually mutated in human NaCT to generate the Phe→Leu and Thr→Ala mutants that match the sequence in rat NaCT in this region. The Thr→Ala mutation did not affect the transport activity nor did it interfere with the Li⁺-dependent stimulation. In contrast, the Phe→Leu mutation (TTT→CTT) led to a 3- to 4-fold stimulation of transport activity. Kinetic analysis of the wild type human NaCT and its Phe→Leu mutant showed that the stimulation of transport function seen with the mutant was due to an increase in substrate affinity with very little change in maximal velocity (Fig. 44C). The wild type human NaCT showed a *Kₜ* value of 585 ± 87 µM for citrate. The corresponding value for the mutant was 78 ± 8 µM. Thus, the Phe→Leu mutant showed a 7-fold increase in substrate affinity. Since a single base pair change in the codon can bring about this amino acid substitution (TTT→CTT), these findings suggest that genetic mutations in humans have potential to effect marked changes in the constitutive activity of NaCT and that such changes may influence the role of the transporter in hepatic synthesis of triglycerides and cholesterol. Interestingly, the Phe→Leu mutant did not exhibit Li⁺ sensitivity (Fig. 44D). The transport function of wild type human NaCT was stimulated by Li⁺ whereas the transport function of the mutant, which was about 4-fold higher than that of wild type NaCT, was not affected by Li⁺. Since rat NaCT, which possesses Leu at this site, is inhibited by Li⁺, the findings that activity of the Phe->Leu mutant of human NaCT is not inhibited by Li⁺ indicate that this region may not represent the Li⁺-binding site. Furthermore, Li⁺ affects the transport function of human NaCT not only by increasing the substrate affinity but also by decreasing the maximal velocity (Fig. 42A). In contrast, the influence of the Phe→Leu mutation is solely on substrate affinity. While the domain in human NaCT consisting of the amino acids 496-516 definitely plays a critical role in substrate binding, it may not have a direct role in Li⁺ binding. But, this domain appears to interact with the Li⁺-binding site, thus modifying the influence of Li⁺ on the transport function. Multiple alignments of amino acid sequences of NaCTs from different species using the program "T-coffee," available on the world-wide web at ch.embnet.org/software/TCoffee.html, show that the amino acid corresponding to Phe-500 in human NaCT is Leu in rat and mouse NaCTs. Interestingly, the corresponding amino acid in zebra fish and *C. elegans* NaCTs is Phe. Nonetheless, zebra fish NaCT was inhibited by Li⁺, whereas the *C. elegans* NaCT was not influenced by Li⁺. This agrees with the conclusion that the domain containing this amino acid does not represent the Li⁺-binding site.

This example shows that Li⁺ is a potent activator of NaCT, at concentrations found in patients treated with lithium for affective disorders, have important clinical implications. Interestingly, the activation of NaCT by Li⁺ is seen only with human NaCT. Patients with bipolar disorder are treated effectively with either lithium or other mood stabilizers such as valproate and carbamazepine. Present studies show that the activation of human NaCT is seen only with lithium and not with valproate and carbamazepine. Thus, only those patients who take lithium face the clinical consequences of activation of NaCT function. Such consequences include the enhanced synthesis of cholesterol and fatty acids and consequently an increased risk for hypercholesterolemia, hyperlipidemia, hyperglycemia, obesity, and insulin-resistant diabetes. It has been known for some time that lithium therapy is associated with significant increase in body weight (Chen and Silverstone, Int. Clin. Psychoharmacol. (1990);5: 217-225; Coxhead et al., Acta Psychiatr. Scand.(1992);85: 114-118; Price and Heninger, N. Engl. J. Med. (1994);331: 591-598; Baptista et al., Pharmacopsychiatry (1995);28: 35-44; Fagiolini et al., J. Clin. Psychiatry (2002);63: 528-533; and Atmaca et al., Neuropsycobiology (2002);46: 67-69).

Inositol monophosphatase, inositol polyphosphatase, and glycogen synthase kinase-3β, the previously known targets for Li⁺, are all enzymes and are all inhibited by Li⁺. Human NaCT is, in contrast, a plasma membrane transporter that is activated rather than inhibited by Li⁺. Human NaCT thus represents a new and novel target for Li⁺.

The data with the Phe→Leu mutant of human NaCT raise the possibility that genetic mutations in this amino acid position may alter the constitutive activity of the transporter in humans and consequently influence the hepatic utilization of extracellular citrate for fatty acid and cholesterol synthesis. Individuals with such mutations are likely to exhibit significant alterations in their blood lipid profile.

**TABLE 11**

| Differential effects of Li⁺, valproate, and carbamazepine on NaCT from different species and on NaCT in human liver cell lines. | | | | |
|---|---|---|---|---|
| | Control | Li⁺ (2 mM) | Valproate (0.6 µM) | Carbamazepine (50 µM) |
| *Citrate uptake (pmol*/*10⁶ cells*/*min)* | | | | |
| hNaCT | 51.8 ± 2.5 (100) | 105.2 ± 11.6 (203) | 57.3 ± 2.6 (111) | 54.1 ± 2.6 (105) |
| rNaCT | 148.8 ± 7.2 (100) | 92.3 ± 8.4 (62) | 142.4 ± 6.3 (96) | 133.5 ± 0.7 (90) |
| mNaCT | 42.5 ± 2.3 (100) | 29.4 ± 1.7 (69) | 42.5 ± 4.4 (100) | 43.7 ± 2.0 (103) |
| ceNaCT | 43.5 ± 2.1 (100) | 43.6 ± 0.8 (100) | 46.0 ± 3.6 (106) | 45.1 ± 1.5 (104) |
| zfNaCT | 485.8 ± 20.6 (100) | 298.0 ± 12.6 (61) | 453.0 ± 26.6 (93.2) | 441.0 ± 2.7 (91) |

| *Citrate uptake (pmol*/*min*/*mg protein)* | | | | |
|---|---|---|---|---|
| HepG2 cells | 9.5 ± 0.3 (100) | 21.2 ± 0.6 (222) | 9.9 ± 0.3 (105) | 10.0 ± 0.2 (106) |
| Huh-7 cells | 10.2 ± 0.2 (100) | 20.0 ± 0.5 (196) | ND | ND |

| | | | | |
|---|---|---|---|---|
| Values in parentheses represent percent of corresponding control. ND, not determined. | | | | |

### Example 8

### Inhibition of Citrate Transport via the NaCT Citrate Transporter by Hydroxycitrate

Human and rat NaCT cDNAs were independently expressed in HRPE cells and the transport function of the expressed transporter was assessed by the measurement of citrate into cells using the methods described in Example 2 and Example 3. The inhibitory potency of hydroxycitrate was monitored by determining citrate uptake into the cells in the absence (control) and presence of 0.1 mM hydroxycitrate. At this concentration, hydroxycitrate inhibited citrate uptake into the cells via NaCT by about 30-40%. These data show that hydroxycitrate is an inhibitor of the citrate transporter.

The complete disclosure of all patents, patent applications, and publications, and electronically available material (including, for instance, nucleotide sequence submissions in, e.g., GenBank and RefSeq, and amino acid sequence submissions in, e.g., SwissProt, PIR, PRF, PDB, and translations from annotated coding regions in GenBank and RefSeq) cited herein are incorporated by reference. The foregoing detailed description and examples have been given for clarity of understanding only. No unnecessary limitations are to be understood therefrom. The invention is not limited to the exact details shown and described, for variations obvious to one skilled in the art will be included within the invention defined by the claims.

All headings are for the convenience of the reader and should not be used to limit the meaning of the text that follows the heading, unless so specified.

### Sequence Listing Free Text

| | |
|---|---|
| SEQ ID NO:1 | cDNA of Drosophila drINDY |
| SEQ ID NO:2 | amino acid sequence of Drosophila drINDY |
| SEQ ID NO:3 | cDNA of rat NaCT |
| SEQ ID NO:4 | amino acid sequence of rat NaCT |
| SEQ ID NO:5 | cDNA human NaCT |
| SEQ ID NO:6 | amino acid sequence of human NaCT |
| SEQ ID NO:7 | cDNA of C. elegans NaCT |
| SEQ ID NO:8 | amino acid sequence of C. elegans NaCT |
| SEQ ID NO:9 | cDNA of mouse NaCT |
| SEQ ID NO:10 | amino acid sequence of mouse NaCT |
| SEQ ID NO:11 | cDNA of zebrafish NaCT |
| SEQ ID NO:12 | amino acid sequence of zebrafish NaCT |
| SEQ ID NO:13 | amino acid sequence of rat NaDC1 |
| SEQ ID NO:14 | amino acid sequence of rat NaDC3 |
| SEQ ID NO:15-28 | artificially synthesized oligonucleotide primers |
| SEQ ID NO:29 | peptide consensus sequence |

## Claims

1. An isolated polynucleotide that hybridizes to SEQ ID NO:5 under stringent hybridization conditions, wherein the polynucleotide encodes a polypeptide demonstrating Na⁺-dependent transmembrane transport of citrate.

2. The isolated polynucleotide of claim 1, wherein the polynucleotide comprises SEQ ID NO:5.

3. An isolated polynucleotide encoding a polypeptide having at least 35% sequence identity to SEQ ID NO:6, wherein the polynucleotide encodes a polypeptide demonstrating Na⁺-dependent transmembrane transport of citrate.

4. The isolated polynucleotide of claim 3, wherein the encoded Na⁺-dependent transmembrane transport of citrate is modulated by Li⁺.

5. The isolated polynucleotide of claim 3 or 4, wherein the polynucleotide comprises SEQ ID NO:5.

6. The isolated polynucleotide of anyone of claims 3 to 5, wherein the encoded polypeptide demonstrating Na⁺-dependent transmembrane transport of citrate demonstrates a requirement for multiple Na⁺ ions for transport coupling.

7. The isolated polynucleotide of anyone of claims 3 to 5, wherein the transmembrane transport of citrate is electrogenic.

8. A plasmid comprising the isolated polynucleotide of anyone of claims 3 to 7.

9. The plasmid of claim 8, wherein the plasmid comprises an expression vector.

10. An isolated host cell comprising the isolated polynucleotide of anyone of claims 3 to 7.

11. The isolated host cell of claim 10 demonstrating transient expression of the encoded Na⁺-dependent transmembrane citrate transporter.

12. The isolated host cell of claim 10 demonstrating stable expression of the encoded Na⁺-dependent transmembrane citrate transporter.

13. The isolated host cell of anyone of claims 10 to 12, wherein the Na⁺-dependent transmembrane transport of citrate is modulated by Li⁺.

14. The isolated host cell of anyone of claims 10 to 13, wherein the host cell is selected from the group consisting of human cells, insect cells, xenopus oocytes, and yeast cells.

15. An isolated polypeptide having at least 35% sequence identity to SEQ ID NO:6, wherein the polypeptide demonstrates Na⁺-dependent transmembrane transport of citrate.

16. An isolated polypeptide, wherein the polypeptide is encoded by a polynucleotide that hybridizes to SEQ ID NO:5 under stringent hybridization conditions and wherein the polypeptide demonstrates transmembrane transport of citrate.

17. An isolated polypeptide having at least 75% sequence identity to SEQ ID NO:6, wherein the polypeptide demonstrates Na⁺-dependent transmembrane transport of citrate.

18. The isolated polypeptide of any one of claims 15 to 17, wherein the encoded Na⁺-dependent transmembrane transport of citrate is modulated by Li⁺.

19. An isolated polypeptide comprising SEQ ID NO:6.

20. The isolated polypeptide of anyone of claims 15 to 19, wherein the polypeptide is a recombinant polypeptide.

21. An antibody that specifically binds to the isolated polypeptide of anyone of claims 15 to 20.

22. An in vitro method of identifying an agent that modifies transmembrane citrate transporter activity comprising:
contacting a host cell expressing a transmembrane citrate transporter polypeptide having at least 35% sequence identity to SEQ ID NO:6, wherein the transmembrane citrate transporter polypeptide demonstrates Na⁺- dependent transmembrane transport of citrate and wherein the encoded Na⁺- dependent transmembrane transport of citrate is stimulated by Li⁺ ;
measuring citrate transport into the host cell in the presence of agent;
and comparing citrate transport into the host cell in the presence of the agent to citrate transport into the host cell in the absence of the agent;
wherein a decreased transport of citrate into the host cell in the presence of the agent indicates the agent is an inhibitor of transmembrane citrate transporter activity;
wherein an increased transport of citrate into the host cell in the presence of the agent indicates the agent is a stimulator of transmembrane citrate transporter activity.

23. An in vitro method of identifying an agent that modifies Na⁺-dependent transmembrane citrate transporter activity comprising:
contacting a host cell expressing a Na⁺-dependent transmembrane citrate transporter comprising SEQ ID NO:6 with an agent;
measuring the citrate-induced inward electrical current into the host cell in the presence of agent; and
comparing the citrate-induced inward electrical current into the host cell in the presence of the agent to the citrate-induced inward electrical current into the host cell in the absence of the agent;
wherein a decrease in the inward electrical current into the host cell in the presence of the agent indicates the agent is a blocker of Na⁺-dependent transmembrane citrate transporter activity;
wherein an increase in the inward electrical current into the host cell in the presence of the agent indicates the agent is a stimulator of Na⁺-dependent transmembrane citrate transporter activity.

24. An in vitro method of identifying an agent that serves as a substrate of a Na⁺- dependent transmembrane citrate transporter comprising:
contacting a host cell expressing a Na⁺-dependent transmembrane citrate transporter comprising SEQ ID NO:6 with an agent; and
determining the entry of the agent into the cell via the Na⁺-dependent transmembrane citrate transporter in the presence of agent;
wherein entry of the agent via the Na⁺-dependent transmembrane citrate transporter indicates the agent is a substrate of a Na⁺-dependent transmembrane citrate transporter.

25. An isolated polypeptide consisting of SEQ ID NO:6, wherein amino acid positions 496 to 516 have been replaced with amino acid positions 500 to 520 of the rat NaCT transporter (SEQ ID NO:4).

26. An isolated polypeptide consisting SEQ ID NO:6, wherein the amino acid at codon 500 has been changed from a phenylalanine to a leucine.

27. A modifier of a transmembrane citrate transporter, the transmembrane citrate transporter comprising SEQ ID NO:6.

28. A composition comprising the modifier of claim 27.

29. A composition comprising the modifier of claim 27 and a pharmaceutically acceptable carrier.

30. The composition of claim 28 or 29 further comprising an additional therapeutic agent.

31. The composition of claim 30, wherein the additional therapeutic agent is lithium.


### Annex to the application documents - subsequently filed sequences listing

SEQUENCE LISTING

<110> MEDICAL COLLEGE OF GEORGIA RESEARCH INSTITUTE, INC.

<120> NaCT AS A TARGET FOR LIFESPAN EXPANSION AND WEIGHT REDUCTION

<130> L1699 EP/1

<140> 03 79 6430.1
<141> 2005-06-21

<150> 60/428,469
<151> 2002-11-22

<150> 60/459,441
<151> 2003-04-01

<160> 29

<170> PatentIn version 3.2

<210> 1
<211> 2602
<212> DNA
<213> Drosophila melanogaster

<400> 1
ttcaccgttt ccgaatcgga cgaaccgggc gtgattgctc tcctgctgct ttcgagatcg 60

gagtcccgat aaggatataa ctacaaccta aagaggaatc caagcctcct cctgccgcta 120

gtttcgaaaa gtaaatagag tacttgttat caactgggaa gcggagatac atagctccga 180

tattcctgtg aaagccagac aaacggatac caacgaacaa tcgccatatc tacacgccac 240

cgccactgga catcaaaatg gaaattgaaa ttggcgaaca accccagcct ccggtgaagt 300

gctccaactt cttcgctaac cactggaagg gattggttgt gttcctggtg ccgctgctat 360

gtctgcctgt tatgctgcta aacgaaggcg ccgaatttcg gtgcatgtac ctccttttgg 420

taatggccat attttgggtt acggaagcct tgcctctcta tgtgacgtcc atgataccga 480

ttgtggcctt cccaataatg ggtataatga gctcggatca gacttgccgc ttgtacttca 540

aggatacgct ggtgatgttc atgggcggca ttatggtcgc cctggctgtg gagtactgta 600

atctacacaa acgtcttgcc ttgagggtaa tccagatcgt gggctgcagt ccccgcagat 660

tacactttgg cctcatcatg gttacaatgt ttttgagcat gtggatttcg aacgccgcct 720

gtactgccat gatgtgtccg attatccaag ccgtgctgga ggagctgcag gctcagggtg 780

tctgcaaaat caaccatgag cctcaatacc aaatcgttgg aggcaacaag aaaaacaacg 840

aggatgagcc accatacccc accaagatca ctctgtgcta ctatctgggc attgcctacg 900

cctcctcgct gggtggctgt ggaaccatca tcggaactgc caccaatctt accttcaagg 960

gcatctacga ggctcgtttc aagaactcca ccgaacagat ggacttcccc accttcatgt 1020

tctactcggt gccatccatg ttggtctaca ccttgctgac attcgtgttc ctgcaatggc 1080

acttcatggg tctgtggcgt cccaagagca aggaggcaca ggaagtccag aggggacgag 1140

agggcgccga tgtcgccaaa aaggttatcg atcagcgcta caaggatctg ggtcccatgt 1200

ccattcacga gatccaagtg atgattctgt tcatttttat ggttgtgatg tacttcaccc 1260

gcaagcccgg catctttttg ggatgggccg atttgctgaa ttccaaggac attcgtaact 1320
ctatgcccac tatttttgtc gtcgtcatgt gcttcatgct gcccgccaat tatgctttcc 1380
tacgctactg caccagacgc ggtggtccag tgcccacggg tcccactcca tcgctgatca 1440
cctggaagtt catccagacc aaggtgccat ggggtctggt gttcctgctt ggcggtggct 1500
tcgctttggc cgaaggcagc aagcagagcg gcatggccaa gctgattggc aatgctctga 1560
ttggattgaa ggttctgccc aactctgtcc tcttactggt ggtcatcctg gtggctgtgt 1620
tcctgaccgc cttcagctcc aatgtggcga ttgccaacat tattattccc gttctggccg 1680
agatgtccct ggccattgag atccatcctc tgtacctgat cctgcccgct ggcttggcct 1740
gcagtatggc cttccacctg ccggttagta ctccgcccaa cgctttggtt gctggctatg 1800
ccaacattag gacgaaggac atggccattg ctggaatcgg tccgaccatc attaccatca 1860
tcaccctgtt tgttttctgc caaacctggg gcctggtcgt ctatccgaac cttaactcgt 1920
tccccgaatg ggctcagatt tatgccgcgg cagcactggg aaacaagacg cactagatag 1980
ttagtaatta gtgtaaataa ctaacatacc cgtcacagcg ataaagttga ggaaaattta 2040
gggaatttta aacgaaaagt gcctttgctg acagcgaaaa atgtgaaaaa tatttaacta 2100
tgtatacttg catttcagag ttgcgaaaag ttttgataca aaagcattac ctactgttta 2160
gaaaaatgtg ttaaaaaaaa aacgtatcgc aatatactgt taatcaggaa ttgaacacct 2220
ggtctacgca ctcagctaaa tatttaaata caaattaatg ttacttaatt gttgcattta 2280
gcataaaaat ggaaaagatt tggaaaagtt agaacagttt gttcaatggc agccctggcc 2340
tgctaatatt ttaaataact agactgagag aacttacata ttcatacatg tttttcaact 2400
tgtaaaaatt tttaaatgaa caactcactc aatacttcat tgcgaaccaa aatgaacaca 2460
caaatagcgg taggctaagc ttaaatgata ctgtgtacat tttcagatga tttatgtttt 2520
atatagtttg taaaaaatat taaataataa aaagctcaac cgccaataaa aaaaaaaaaa 2580
aaaaaaaaaa aaaaaaaaaa aa 2602

<210> 2
<211> 572
<212> PRT
<213> Drosophila melanogaster

<400> 2

Met Glu Ile Glu Ile Gly Glu Gln Pro Gln Pro Pro Val Lys Cys Ser
1               5                   10                  15

Asn Phe Phe Ala Asn His Trp Lys Gly Leu Val Val Phe Leu Val Pro
            20                  25                  30

Leu Leu Cys Leu Pro Val Met Leu Leu Asn Glu Gly Ala Glu Phe Arg
        35                  40                  45

Cys Met Tyr Leu Leu Leu Val Met Ala Ile Phe Trp Val Thr Glu Ala

50                  55                  60

Leu Pro Leu Tyr Val Thr Ser Met Ile Pro Ile Val Ala Phe Pro Ile
65                  70                  75                  80

Met Gly Ile Met Ser Ser Asp Gln Thr Cys Arg Leu Tyr Phe Lys Asp
                85                  90                  95

Thr Leu Val Met Phe Met Gly Gly Ile Met Val Ala Leu Ala Val Glu
            100                 105                 110

Tyr Cys Asn Leu His Lys Arg Leu Ala Leu Arg Val Ile Gln Ile Val
        115                 120                 125

Gly Cys Ser Pro Arg Arg Leu His Phe Gly Leu Ile Met Val Thr Met
    130                 135                 140

Phe Leu Ser Met Trp Ile Ser Asn Ala Ala Cys Thr Ala Met Met Cys
145                 150                 155                 160

Pro Ile Ile Gln Ala Val Leu Glu Glu Leu Gln Ala Gln Gly Val Cys
                165                 170                 175

Lys Ile Asn His Glu Pro Gln Tyr Gln Ile Val Gly Gly Asn Lys Lys
            180                 185                 190

Asn Asn Glu Asp Glu Pro Pro Tyr Pro Thr Lys Ile Thr Leu Cys Tyr
        195                 200                 205

Tyr Leu Gly Ile Ala Tyr Ala Ser Ser Leu Gly Gly Cys Gly Thr Ile
    210                 215                 220

Ile Gly Thr Ala Thr Asn Leu Thr Phe Lys Gly Ile Tyr Glu Ala Arg
225                 230                 235                 240

Phe Lys Asn Ser Thr Glu Gln Met Asp Phe Pro Thr Phe Met Phe Tyr
                245                 250                 255

Ser Val Pro Ser Met Leu Val Tyr Thr Leu Leu Thr Phe Val Phe Leu
            260                 265                 270

Gln Trp His Phe Met Gly Leu Trp Arg Pro Lys Ser Lys Glu Ala Gln
        275                 280                 285

Glu Val Gln Arg Gly Arg Glu Gly Ala Asp Val Ala Lys Lys Val Ile
    290                 295                 300

Asp Gln Arg Tyr Lys Asp Leu Gly Pro Met Ser Ile His Glu Ile Gln
305                 310                 315                 320

Val Met Ile Leu Phe Ile Phe Met Val Val Met Tyr Phe Thr Arg Lys

                325                 330                 335

Pro Gly Ile Phe Leu Gly Trp Ala Asp Leu Leu Asn Ser Lys Asp Ile
            340                 345                 350

Arg Asn Ser Met Pro Thr Ile Phe Val Val Val Met Cys Phe Met Leu
        355                 360                 365

Pro Ala Asn Tyr Ala Phe Leu Arg Tyr Cys Thr Arg Arg Gly Gly Pro
    370                 375                 380

Val Pro Thr Gly Pro Thr Pro Ser Leu Ile Thr Trp Lys Phe Ile Gln
385                 390                 395                 400

Thr Lys Val Pro Trp Gly Leu Val Phe Leu Leu Gly Gly Gly Phe Ala
                405                 410                 415

Leu Ala Glu Gly Ser Lys Gln Ser Gly Met Ala Lys Leu Ile Gly Asn
            420                 425                 430

Ala Leu Ile Gly Leu Lys Val Leu Pro Asn Ser Val Leu Leu Leu Val
        435                 440                 445

Val Ile Leu Val Ala Val Phe Leu Thr Ala Phe Ser Ser Asn Val Ala
    450                 455                 460

Ile Ala Asn Ile Ile Ile Pro Val Leu Ala Glu Met Ser Leu Ala Ile
465                 470                 475                 480

Glu Ile His Pro Leu Tyr Leu Ile Leu Pro Ala Gly Leu Ala Cys Ser
                485                 490                 495

Met Ala Phe His Leu Pro Val Ser Thr Pro Pro Asn Ala Leu Val Ala
            500                 505                 510

Gly Tyr Ala Asn Ile Arg Thr Lys Asp Met Ala Ile Ala Gly Ile Gly
        515                 520                 525

Pro Thr Ile Ile Thr Ile Ile Thr Leu Phe Val Phe Cys Gln Thr Trp
    530                 535                 540

Gly Leu Val Val Tyr Pro Asn Leu Asn Ser Phe Pro Glu Trp Ala Gln
545                 550                 555                 560

Ile Tyr Ala Ala Ala Ala Leu Gly Asn Lys Thr His
                565                 570

<210> 3
<211> 3254
<212> DNA
<213> rat NaCT

<400> 3
ccagtctgtc tccctttcac gcgatggctt cggcgaagac ttatgtgacc aagttcaagt 60
ccttcgtgat tttgttcttc gccccgatcc tgctgcttcc actcatcatt ctggtacctg 120
acaagtttgc caggtgtgcc tatgttataa tcctcatggc catctactgg tgcacagatg 180
tcatcccagt ggctatcact tccctcctgc ctgtcttact cttcccactt ttgaaggttc 240
tggactccaa gcaggtatgt gtccaataca tgacggacac caacatgctg ttcctgggca 300
gtctcattgt ggccacggct gtggaacgtt gggaacttca taagaggatt gctctgagaa 360
tgctactctt tgtggggacc aagccttcac ggctgatgct gggcttcatg ttcgtcacag 420
ccttcctgtc catgtggatc agcaatactg ccaccacggc catgatgata cccattgtgg 480
aggccatgct ggagcaaatg gtagccacaa atgtagcggt ggatgccagc cagaggacaa 540
tggaactgtt ggacaagaac aaggccagcg aattgccagg aagccaggtg gtatttgaag 600
accccagtgt gcagaagcag gaggatgaag aaacaaagaa tatgtacaag gctatgaacc 660
tatgtgtgtg ctacgcagcc agcatcgggg gtacagccac cttgaccggg acgggaccca 720
acgtggtgct cctgggccag atgcaggaat tgtttcctga cagtaaagac gtcatgaact 780
ttgcatcttg gtttgcattt gccctcccaa acatgctttt gatgctggtg atggcctggc 840
tgtggctcct gtgtttttac atgagaccca atttaaaaaa aacttgcatc tgctgtggga 900
ggaagaagaa ggacacggag aagattgcct ccaaggtgct gtatgaggag tacaggaagc 960
tggggccctt gagctacgct gaatgcaacg tgctcttttg cttcggcctg ctcatcatcc 1020
tgtggttctc ccgagacccc ggcttcatgc ctggctggct gtccatcgcc tggatcgagg 1080
gaaataccaa gcatgtcacg gacgccacag tggccatctt tgtggccatt ttgcttttca 1140
tcgtaccctc acaaaagccc aagttcaatt tcagccgcca gactgaggaa gaaaggaaaa 1200
ctcccttcta ccccccgcca ctgctgaatt ggaaagtcac ccaagagaaa gtgccctggg 1260
gcattgtgct gctcctgggg ggaggatttg ctatggccaa aggatgtgag acttcagggc 1320
tctctgagtg gatggcgaga cagatggagc ccttgagctc agtgagacct gctattatta 1380
ccttgatctt gtcctgtatt gttgcaatga ccacagagtg cacgagtaac gtggccacta 1440
ctaccctgtt cctgcctatc tttgcctcca tggctcgttc catcggcatc catccactgt 1500
atgtcatgat tccctgcacc ttgagtgcgt cacttgcctt catgttgcct gtggccaccc 1560
cacctaacgc catcgtgttt gcctacggac acctcaaagt tattgacatg gtaaaaacag 1620
gattggtaat gaacatcctt ggaattgcat ctgtgtttct gtcagtcaac acctggggac 1680
gggctgtgtt taacttggat aaattccctg actgggcaaa tttgacacat attaacactt 1740
aggagaacca caagagcaca ggcttgtccc ccaacccttt cgaggactgc gaaccttctg 1800
gcacaccttg cacagagcac tggtgctcat accccagtgt gacccaatga tgtcaacacc 1860
ccaagaagat ctaaccaact agccacctct tcctccaggc tcaggttcag agatggcaac 1920
gggcgatggg aagataggct cagaagggaa aggaaccttt gagaggtcgt gaggcccatc 1980
ttccctagga cccttccatc tcacctgggc aggaaacaga gggactgggg ctcaagtcct 2040

gtaccacgtg gctttgaaag acttctgact ccatgctggg ctctggttct cacatgcctg    2100

ttcccacggt ctccacatgg ggatcagatg accaggagac agcccctgtg cctcttctgg    2160

atgttcccag atcaccatct ctatcaccac gaaggaactt cctctccagg acagaactct    2220

gatcttgaac actttccact gccagagtta gagtggaaat cacggccccc tgaagacttt    2280

gactctacat ggtgccatct ccaaccactg ggcaacctga ggtgctaaca ttgaggcctt    2340

cctgctcacc cttggctgac ctgttcccta cttgccttat ctcttattag ttaacagttt    2400

gaggcccctt cccagctccc cagtgagact tcatcaactc ctagatgctc ctggctgagg    2460

ctccccagaa agtccctgtt tgattgttct ggggatgctc atcctgccag cctggagcag    2520

ctgggtcata catcagggat ggacaatggt gtctctttag gggatggtgt taaagtggga    2580

tacctaaacc ttccttcctt tggctggatc cagagcagat tcagcagctc gtttataagg    2640

gctgggccac cacagttggt cctgttggac tcttgagctc ctccagaagg ccttttcctt    2700

gctgtctgtg cactgtgggc aagcctggta gcaaccgggc atggttgaga tgagcgatgc    2760

ttgctggtgt ctgagcggac agaccctgcc agagtaagct gactaggtag agtgtccatg    2820

ggaacccatg ggaccactca catgaaaggg agacagaaga ggaagctctc ccttgtcctt    2880

cagggatgct ctcttccttg cttaatttgc tctgaaaaga agcatgagtg gggagataag    2940

atccgtggat gtcattttat ttttccaggc agaagacagt atgaggaccc aaggagtccc    3000

catcaaagac caagtatgtg tctggctcct tgggagggat ggctcctgac catactgtcg    3060

aaggcttgct cttgtcaaat actctgtctg ctatggaaag ttccagtgtg ctgactggtc    3120

tgtgatttcg tgccttgtga aggagacaga gggaggcaag atgaataaac agttgctctc    3180

tgtccacctc caaaaaaaaa aaaaaaaaaa aaaaaaaaaa aaaaaaaaaa aaaaaaaaaa    3240

aaaaaaaaaa aaaa                                                      3254

<210>  4
<211>  572
<212>  PRT
<213>  rat NaCT

<400>  4

Met Ala Ser Ala Lys Thr Tyr Val Thr Lys Phe Lys Ser Phe Val Ile
1               5                   10                  15

Leu Phe Phe Ala Pro Ile Leu Leu Leu Pro Leu Ile Ile Leu Val Pro
            20                  25                  30

Asp Lys Phe Ala Arg Cys Ala Tyr Val Ile Ile Leu Met Ala Ile Tyr
        35                  40                  45

Trp Cys Thr Asp Val Ile Pro Val Ala Ile Thr Ser Leu Leu Pro Val
    50                  55                  60

Leu Leu Phe Pro Leu Leu Lys Val Leu Asp Ser Lys Gln Val Cys Val

65                    70                   75                   80

Gln Tyr Met Thr Asp Thr Asn Met Leu Phe Leu Gly Ser Leu Ile Val
                85                  90                  95

Ala Thr Ala Val Glu Arg Trp Glu Leu His Lys Arg Ile Ala Leu Arg
            100                 105                 110

Met Leu Leu Phe Val Gly Thr Lys Pro Ser Arg Leu Met Leu Gly Phe
        115                 120                 125

Met Phe Val Thr Ala Phe Leu Ser Met Trp Ile Ser Asn Thr Ala Thr
    130                 135                 140

Thr Ala Met Met Ile Pro Ile Val Glu Ala Met Leu Glu Gln Met Val
145                 150                 155                 160

Ala Thr Asn Val Ala Val Asp Ala Ser Gln Arg Thr Met Glu Leu Leu
                165                 170                 175

Asp Lys Asn Lys Ala Ser Glu Leu Pro Gly Ser Gln Val Val Phe Glu
            180                 185                 190

Asp Pro Ser Val Gln Lys Gln Glu Asp Glu Glu Thr Lys Asn Met Tyr
        195                 200                 205

Lys Ala Met Asn Leu Cys Val Cys Tyr Ala Ala Ser Ile Gly Gly Thr
    210                 215                 220

Ala Thr Leu Thr Gly Thr Gly Pro Asn Val Val Leu Leu Gly Gln Met
225                 230                 235                 240

Gln Glu Leu Phe Pro Asp Ser Lys Asp Val Met Asn Phe Ala Ser Trp
                245                 250                 255

Phe Ala Phe Ala Leu Pro Asn Met Leu Leu Met Leu Val Met Ala Trp
            260                 265                 270

Leu Trp Leu Leu Cys Phe Tyr Met Arg Pro Asn Leu Lys Lys Thr Cys
        275                 280                 285

Ile Cys Cys Gly Arg Lys Lys Lys Asp Thr Glu Lys Ile Ala Ser Lys
    290                 295                 300

Val Leu Tyr Glu Glu Tyr Arg Lys Leu Gly Pro Leu Ser Tyr Ala Glu
305                 310                 315                 320

Cys Asn Val Leu Phe Cys Phe Gly Leu Leu Ile Ile Leu Trp Phe Ser
                325                 330                 335

Arg Asp Pro Gly Phe Met Pro Gly Trp Leu Ser Ile Ala Trp Ile Glu

            340                 345                 350

Gly Asn Thr Lys His Val Thr Asp Ala Thr Val Ala Ile Phe Val Ala
        355                 360                 365

Ile Leu Leu Phe Ile Val Pro Ser Gln Lys Pro Lys Phe Asn Phe Ser
    370                 375                 380

Arg Gln Thr Glu Glu Glu Arg Lys Thr Pro Phe Tyr Pro Pro Pro Leu
385                 390                 395                 400

Leu Asn Trp Lys Val Thr Gln Glu Lys Val Pro Trp Gly Ile Val Leu
                405                 410                 415

Leu Leu Gly Gly Gly Phe Ala Met Ala Lys Gly Cys Glu Thr Ser Gly
            420                 425                 430

Leu Ser Glu Trp Met Ala Arg Gln Met Glu Pro Leu Ser Ser Val Arg
        435                 440                 445

Pro Ala Ile Ile Thr Leu Ile Leu Ser Cys Ile Val Ala Met Thr Thr
    450                 455                 460

Glu Cys Thr Ser Asn Val Ala Thr Thr Thr Leu Phe Leu Pro Ile Phe
465                 470                 475                 480

Ala Ser Met Ala Arg Ser Ile Gly Ile His Pro Leu Tyr Val Met Ile
                485                 490                 495

Pro Cys Thr Leu Ser Ala Ser Leu Ala Phe Met Leu Pro Val Ala Thr
            500                 505                 510

Pro Pro Asn Ala Ile Val Phe Ala Tyr Gly His Leu Lys Val Ile Asp
        515                 520                 525

Met Val Lys Thr Gly Leu Val Met Asn Ile Leu Gly Ile Ala Ser Val
    530                 535                 540

Phe Leu Ser Val Asn Thr Trp Gly Arg Ala Val Phe Asn Leu Asp Lys
545                 550                 555                 560

Phe Pro Asp Trp Ala Asn Leu Thr His Ile Asn Thr
                565                 570

<210> 5
<211> 3248
<212> DNA
<213> human NaCT

<400> 5
ccctcccgcg cgatggcctc ggcgctgagc tatgtctcca agttcaagtc cttcgtgatc 60

ttgttcgtca ccccgctcct gctgctgcca ctcgtcattc tgatgcccgc caagtttgtc 120

aggtgtgcct acgtcatcat cctcatggcc atttactggt gcacagaagt catccctctg 180
gctgtcacct ctctcatgcc tgtcttgctt ttcccactct tccagattct ggactccagg 240
caggtgtgtg tccagtacat gaaggacacc aacatgctgt tcctgggcgg cctcatcgtg 300
gccgtggctg tggagcgctg gaacctgcac aagaggatcg ccctgcgcac gctcctctgg 360
gtgggggcca agcctgcacg gctgatgctg ggcttcatgg gcgtcacagc cctcctgtcc 420
atgtggatca gtaacacggc aaccacggcc atgatggtgc ccatcgtgga ggccatattg 480
cagcagatgg aagccacaag cgcagccacc gaggccggcc tggagctggt ggacaagggc 540
aaggccaagg agctgccagg gagtcaagtg atttttgaag gccccactct ggggcagcag 600
gaagaccaag agcggaagag gttgtgtaag gccatgaccc tgtgcatctg ctacgcggcc 660
agcatcgggg gcaccgccac cctgaccggg acgggaccca acgtggtgct cctgggccag 720
atgaacgagt tgtttcctga cagcaaggac ctcgtgaact ttgcttcctg gtttgcattt 780
gcctttccca acatgctggt gatgctgctg ttcgcctggc tgtggctcca gtttgtttac 840
atgagattca attttaaaaa gtcctggggc tgcgggctag agagcaagaa aaacgagaag 900
gctgccctca aggtgctgca ggaggagtac cggaagctgg ggcccttgtc cttcgcggag 960
atcaacgtgc tgatctgctt cttcctgctg gtcatcctgt ggttctcccg agaccctggc 1020
ttcatgcccg gctggctgac tgttgcctgg gtggagggtg agacaaagta tgtctccgat 1080
gccactgtgg ccatctttgt ggctaccctg ctattcattg tgccttcaca gaagcccaag 1140
tttaacttcc gcagccagac tgaggaagaa aggaaaactc cattttatcc ccctcccctg 1200
ctggattgga aggtaaccca ggagaaagtg ccctggggca tcgtgctgct actagggggc 1260
ggatttgctc tggctaaagg atccgaggcc tcggggctgt ccgtgtggat ggggaagcag 1320
atggagccct tgcacgcagt gcccccggca gccatcacct tgatcttgtc cttgctcgtt 1380
gccgtgttca ctgagtgcac aagcaacgtg gccaccacca ccttgttcct gcccatcttt 1440
gcctccatgt ctcgctccat cggcctcaat ccgctgtaca tcatgctgcc ctgtaccctg 1500
agtgcctcct ttgccttcat gttgcctgtg gccacccctc caaatgccat cgtgttcacc 1560
tatgggcacc tcaaggttgc tgacatggtg aaaacaggag tcataatgaa cataattgga 1620
gtcttctgtg tgtttttggc tgtcaacacc tggggacggg ccatatttga cttggatcat 1680
ttccctgact gggctaatgt gacacatatt gagacttagg aagagccaca agaccacaca 1740
cacagccctt accctcctca ggactaccga accttctggc acaccttgta cagagttttg 1800
gggttcacac cccaaaatga cccaacgatg tccacacacc accaaaaccc agccaatggg 1860
ccacctcttc ctccaagccc agatgcagag atggacatgg gcagctggag ggtaggctca 1920
gaaatgaagg gaacccctca gtgggctgct ggacccatct ttcccaagcc ttgccattat 1980
ctctgtgagg gaggccaggt agccgaggga tcaggatgca ggctgctgta cccgctctgc 2040
ctcaagcatc ccccacacag ggctctggtt ttcactcgct tcgtcctaga tagtttaaat 2100
gggaatcaga tcccctggtt gagagctaag acaaccacct accagtgccc atgtcccttc 2160

cagctcacct tgagcagcct cagatcatct ctgtcactct ggaagggaca ccccagccag 2220
ggacggaatg cctggtcttg agcaacctcc cactgctgga gtgcgagtgg gaatcagagc 2280
ctcctgaagc ctctgggaac tcctcctgtg gccaccacca aaggatgagg aatctgagtt 2340
gccaacttca ggacgacacc tggcttgcca cccacagtgc accacaggcc aacctacgcc 2400
cttcatcact tggttctgtt ttaatcgact ggccccctgt cccacctctc cagtgagcct 2460
ccttcaactc cttggtcccc tgttgtctgg gtcaacattt gccgagacgc cttggctggc 2520
accctctggg gtcccccttt tctcccaggc aggtcatctt ttctgggaga tgcttcccct 2580
gccatcccca aatagctagg atcacactcc aagtatgggc agtgatggcg ctctgggggc 2640
cacagtgggc tatctaggcc ctccctcacc tgaggcccag agtggacaca gctgttaatt 2700
tccactggct atgccacttc agagtctttc atgccagcgt ttgagctcct ctgggtaaaa 2760
tcttcccttt gttgactggc cttcacagcc atggctggtg acaacagagg atcgttgaga 2820
ttgagcagcg cttggtgatc tctcagcaaa caacccctgc ccgtgggcca atctacttga 2880
agttactcgg acaaagaccc caaagtgggg caacaactcc agagaggctg tgggaatctt 2940
cagaagcccc cctgtaagag acagacatga gagacaagca tcttctttcc cccgcaagtc 3000
cattttattt ccttcttgtg ctgctctgga agagaggcag tagcaaagag atgagctcct 3060
ggatggcatt ttccagggca ggagaaagta tgagagcctc aggaaacccc atcaaggacc 3120
gagtatgtgt ctggttcctt gggtgggacg attcctgacc acactgtcca gctcttgctc 3180
tcattaaatg ctctgtctcc cgcggaaaaa aaaaaaaaaa aaaaaaaaaa aaaaaaaaaa 3240
aaaaaaaa 3248

<210> 6
<211> 568
<212> PRT
<213> human NaCT

<400> 6

<210>  7
<211>  1747
<212>  DNA
<213>  C. elegans NaCT

<400>  7
gtttgagtgt taccactatc agacgaaggt atgaagccta gcccccagcg tacgttaata      60

aaaaagcttc tggtacttct tggaccactt gttgcagtac ctctactctt ttttggaccg     120

gagtacagat gtcttttctc aataatattt ttatcaacct attggattgg agaagcattt     180

cccattggtg tcacttctct ctttccattg gcactttatc caattcttca gattgttcca     240

tctaaacaaa ttagtccagt ttattttaaa gattcgatag ttctatttat gtgcacatta 300

agcatggcaa tggcggtaga agcaactgga ctccatcgaa gaatcgcact gaaattatta 360

acaaaagttg gagcaaagca accagtaatg ctgctgggtt tcatgtgcat cacgagtttc 420

atatcatttt tcgtttctga cacagcatgc acagctctta tgtgtccaac cgctgtggca 480

ctcctgatga gtatgtctga tgcagttcaa catttgaaag aagatcacag gaagccaaag 540

ccgccaccag atgatgctac tgttgctgaa aaaatgagaa tagatgatat gactcctcag 600

gatgctggtt tctgtaaagc attaattttg gcatgtgccc acgcatcgtt gatcggtggg 660

actgctatta tcacttcgac tgggccgaat ttagtttttc gagaaaatat tcacaagaga 720

taccccgagg gacaagtgac aatgacatac ctacaatgga tggttttcgc gataccacca 780

atgtttgtct accttcttgc atcttatatt attctggtgt gttatttcat gggcccctcg 840

acttttgctc gctggtttga aaggccttcc aaagaagaag ctcatttgaa aaaattaatt 900

gaaaagaata ttcagacaat gtatgaggat ttgggagatg tttcgtgggg cgaaaagtca 960

gtttttgtat tcttcattct cctaatcgga tcttggattt ctcgtgatcc cggattcaca 1020

cccggttggg gagatctttt accacacaga aacttcatat cagacagtgt ttctggagtc 1080

ttgatttctt gtattttatt tgtttggccc aaagatccat ttgatcctat tgatccaatg 1140

gctccgattc ttaaatggac cgacatgaaa agcaagtttt cctggtcgtg cacactttta 1200

atcggtgctg ggtatgctat ttcagaagga gttgataaat cgggattatc tagattgatt 1260

tcatgtggaa tgaaaaatat tttcgtgggg atgagctcac ttccattgca attaactgtg 1320

actacaatta ttgtgataat gacagagttt gcaagtaatg tgtccaccgg aagcattttc 1380

attccaattt ctttgggagt tgctgaatca atgggagttc atcctttata tcttgctctt 1440

ccaactactg tagcttgttc atttgctttt atgctaccaa tttcaacacc tccaaatgca 1500

gttgtatacg acactaaagt gatttcaatg gtggaaatga tagtctgtgg ttttctactc 1560

aatattgcgt gcatacttat cacatctctc aacatgaaca catggacata ttttattttt 1620

tcattgaata ttttccccga aaatattgta atatcttcag aaaactcttc atatccagtt 1680

tgctaatttt tgtacaaaat gtgtattgtc cgaatgaaac gtgtatttat taaaaaaaaa 1740

aaaaaaa 1747

<210> 8
<211> 551
<212> PRT
<213> C. elegans NaCT

<400> 8

Met Lys Pro Ser Pro Gln Arg Thr Leu Ile Lys Lys Leu Leu Val Leu
1               5                   10                  15

Leu Gly Pro Leu Val Ala Val Pro Leu Leu Phe Phe Gly Pro Glu Tyr
            20                  25                  30

Arg Cys Leu Phe Ser Ile Ile Phe Leu Ser Thr Tyr Trp Ile Gly Glu
        35                  40                  45

Ala Phe Pro Ile Gly Val Thr Ser Leu Phe Pro Leu Ala Leu Tyr Pro
    50                  55                  60

Ile Leu Gln Ile Val Pro Ser Lys Gln Ile Ser Pro Val Tyr Phe Lys
65                  70                  75                  80

Asp Ser Ile Val Leu Phe Met Cys Thr Leu Ser Met Ala Met Ala Val
                85                  90                  95

Glu Ala Thr Gly Leu His Arg Arg Ile Ala Leu Lys Leu Leu Thr Lys
            100                 105                 110

Val Gly Ala Lys Gln Pro Val Met Leu Leu Gly Phe Met Cys Ile Thr
        115                 120                 125

Ser Phe Ile Ser Phe Phe Val Ser Asp Thr Ala Cys Thr Ala Leu Met
    130                 135                 140

Cys Pro Thr Ala Val Ala Leu Leu Met Ser Met Ser Asp Ala Val Gln
145                 150                 155                 160

His Leu Lys Glu Asp His Arg Lys Pro Lys Pro Pro Pro Asp Asp Ala
                165                 170                 175

Thr Val Ala Glu Lys Met Arg Ile Asp Asp Met Thr Pro Gln Asp Ala
            180                 185                 190

Gly Phe Cys Lys Ala Leu Ile Leu Ala Cys Ala His Ala Ser Leu Ile
        195                 200                 205

Gly Gly Thr Ala Ile Ile Thr Ser Thr Gly Pro Asn Leu Val Phe Arg
    210                 215                 220

Glu Asn Ile His Lys Arg Tyr Pro Glu Gly Gln Val Thr Met Thr Tyr
225                 230                 235                 240

Leu Gln Trp Met Val Phe Ala Ile Pro Pro Met Phe Val Tyr Leu Leu
                245                 250                 255

Ala Ser Tyr Ile Ile Leu Val Cys Tyr Phe Met Gly Pro Ser Thr Phe
            260                 265                 270

Ala Arg Trp Phe Glu Arg Pro Ser Lys Glu Glu Ala His Leu Lys Lys
        275                 280                 285

Leu Ile Glu Lys Asn Ile Gln Thr Met Tyr Glu Asp Leu Gly Asp Val
    290                 295                 300

Ser Trp Gly Glu Lys Ser Val Phe Val Phe Phe Ile Leu Leu Ile Gly
305                 310                 315                 320

Ser Trp Ile Ser Arg Asp Pro Gly Phe Thr Pro Gly Trp Gly Asp Leu
                325                 330                 335

Leu Pro His Arg Asn Phe Ile Ser Asp Ser Val Ser Gly Val Leu Ile
            340                 345                 350

Ser Cys Ile Leu Phe Val Trp Pro Lys Asp Pro Phe Asp Pro Ile Asp
        355                 360                 365

Pro Met Ala Pro Ile Leu Lys Trp Thr Asp Met Lys Ser Lys Phe Ser
    370                 375                 380

Trp Ser Cys Thr Leu Leu Ile Gly Ala Gly Tyr Ala Ile Ser Glu Gly
385                 390                 395                 400

Val Asp Lys Ser Gly Leu Ser Arg Leu Ile Ser Cys Gly Met Lys Asn
                405                 410                 415

Ile Phe Val Gly Met Ser Ser Leu Pro Leu Gln Leu Thr Val Thr Thr
            420                 425                 430

Ile Ile Val Ile Met Thr Glu Phe Ala Ser Asn Val Ser Thr Gly Ser
        435                 440                 445

Ile Phe Ile Pro Ile Ser Leu Gly Val Ala Glu Ser Met Gly Val His
    450                 455                 460

Pro Leu Tyr Leu Ala Leu Pro Thr Thr Val Ala Cys Ser Phe Ala Phe
465                 470                 475                 480

Met Leu Pro Ile Ser Thr Pro Pro Asn Ala Val Val Tyr Asp Thr Lys
                485                 490                 495

Val Ile Ser Met Val Glu Met Ile Val Cys Gly Phe Leu Leu Asn Ile
            500                 505                 510

Ala Cys Ile Leu Ile Thr Ser Leu Asn Met Asn Thr Trp Thr Tyr Phe
        515                 520                 525

Ile Phe Ser Leu Asn Ile Phe Pro Glu Asn Ile Val Ile Ser Ser Glu
    530                 535                 540

Asn Ser Ser Tyr Pro Val Cys
545                 550

<210>  9
<211>  1735
<212>  DNA

<213>  mouse NaCT

<400>  9
gtctcccttt cacgcgatgg attcggcgaa gacttgtgtg accaagttca agtcctttgc      60

gattttgctc ttcaccccga tcctgatgct tccactcgtc attctgatac ctgacaagtt     120

tgccaggtgt gcctatgtta tagtcattat ggctgtctac tggtgcacag atgtcatccc     180

agtggctgtt acctccctcc tgcctgtctt actcttccca cttttgaagg ttctggactc     240

caagcaggta tgtatccaat acatgaagga caccaacatg ctgttcctgg gcagtctcat     300

tgtggctgtg gctgtggaac gctggaaact tcataagagg gttgccctga gaatgctgct     360

ctttgtgggg accaagccct cacggctgat gctgggcttt atgtttgtca cggccttcct     420

gtccatgtgg atcagcaata ctgccgccac agccatgatg atacccattg tggaggccat     480

gctgcagcaa atgatagccg ccaatacagc tgtggaggcc agcctgggga cactggagct     540

gctggacaag aacaagacca gcgagttgcc aggaagccag gtggtatttg aagaccccaa     600

tgtgcaggag caggaagacg aagaaacaaa gaatatgtac aaggctatgc acctatgtgt     660

ttgctattca gccagcattg ggggtacagc caccttgacc gggacgggac ccaacgtggt     720

gctcctgggc cagatgcagg aattgtttcc tgacagtaaa gatgtcctga actatgcatc     780

ttggtttgga tttgccttcc ccaacatggt gatgatgctg gtgctggcct ggctgtggct     840

ccagtgcttg tacatgagac acaatttaaa aaaaacttgc atctgctgtg gggagaagaa      900

gagggacacc gagaagattg cctacaaagt gctgaacgag gagtaccaga agctggggtc     960

cttgagctac cctgaatgca acgtgctctt ttgcttcacc ctacttgtca tcctgtggtt    1020

ctcccgagac cccggcttca tgcctggctg gctgtcattc gcctgggtcg agggaaacac    1080

cgttcatatc acagatgcca cagtggccat ctttgtggcc attttgcttt tcatcatacc    1140

ttcacaaaag cccaagttca acttcagcag ccagactgag gaagaaagga aaactccgtt    1200

ctacccccca gcactgctgg attggaaagt cgcccaagag aaagtgccct gggacatcgt    1260

gctgctcctg gggggagggt ttgctatggc aaaaggatgt gagacgtcag ggctctcgaa    1320

gtggatggca gcacagatgg aacccttgag attagtgaaa cctgctgtca ttaccttgat    1380

cttgtcctgt cttgttgcaa tgaccacaga gtgcacaagt aacgtggcca ctaccaccct    1440

gttcctgcct atctttgcct ccatggctcg ttccattggt atccatcctc tgtatgtcat    1500

gattccctgt accatgagtg cttcacttgc cttcatgttg cctgtggcca ccccaccgaa    1560

tgccatcgtg tttgcctacg gacacctcag agttgttgac atgatgaaaa caggattgat    1620

aatgaacttc gttggaatcc tatctgtgtt tctgtcagtc aacacctggg gtcgggctat    1680

gtttaacttg gataacttcc ccgactgggc aaattcaaca agtgttaaca cttag         1735

<210>  10
<211>  572
<212>  PRT
<213>  mouse NaCT

<400>  10

Met Asp Ser Ala Lys Thr Cys Val Thr Lys Phe Lys Ser Phe Ala Ile
1               5                   10                  15

Leu Leu Phe Thr Pro Ile Leu Met Leu Pro Leu Val Ile Leu Ile Pro
            20                  25                  30

Asp Lys Phe Ala Arg Cys Ala Tyr Val Ile Val Ile Met Ala Val Tyr
        35                  40                  45

Trp Cys Thr Asp Val Ile Pro Val Ala Val Thr Ser Leu Leu Pro Val
    50                  55                  60

Leu Leu Phe Pro Leu Leu Lys Val Leu Asp Ser Lys Gln Val Cys Ile
65                  70                  75                  80

Gln Tyr Met Lys Asp Thr Asn Met Leu Phe Leu Gly Ser Leu Ile Val
                85                  90                  95

Ala Val Ala Val Glu Arg Trp Lys Leu His Lys Arg Val Ala Leu Arg
            100                 105                 110

Met Leu Leu Phe Val Gly Thr Lys Pro Ser Arg Leu Met Leu Gly Phe
        115                 120                 125

Met Phe Val Thr Ala Phe Leu Ser Met Trp Ile Ser Asn Thr Ala Ala
    130                 135                 140

Thr Ala Met Met Ile Pro Ile Val Glu Ala Met Leu Gln Gln Met Ile
145                 150                 155                 160

Ala Ala Asn Thr Ala Val Glu Ala Ser Leu Gly Thr Leu Glu Leu Leu
                165                 170                 175

Asp Lys Asn Lys Thr Ser Glu Leu Pro Gly Ser Gln Val Val Phe Glu
            180                 185                 190

Asp Pro Asn Val Gln Glu Gln Glu Asp Glu Glu Thr Lys Asn Met Tyr
        195                 200                 205

Lys Ala Met His Leu Cys Val Cys Tyr Ser Ala Ser Ile Gly Gly Thr
    210                 215                 220

Ala Thr Leu Thr Gly Thr Gly Pro Asn Val Val Leu Leu Gly Gln Met
225                 230                 235                 240

Gln Glu Leu Phe Pro Asp Ser Lys Asp Val Leu Asn Tyr Ala Ser Trp
                245                 250                 255

Phe Gly Phe Ala Phe Pro Asn Met Val Met Met Leu Val Leu Ala Trp
            260                 265                 270

Leu Trp Leu Gln Cys Leu Tyr Met Arg His Asn Leu Lys Lys Thr Cys
        275                 280                 285

Ile Cys Cys Gly Glu Lys Lys Arg Asp Thr Glu Lys Ile Ala Tyr Lys
    290                 295                 300

Val Leu Asn Glu Glu Tyr Gln Lys Leu Gly Ser Leu Ser Tyr Pro Glu
305                 310                 315                 320

Cys Asn Val Leu Phe Cys Phe Thr Leu Leu Val Ile Leu Trp Phe Ser
                325                 330                 335

Arg Asp Pro Gly Phe Met Pro Gly Trp Leu Ser Phe Ala Trp Val Glu
            340                 345                 350

Gly Asn Thr Val His Ile Thr Asp Ala Thr Val Ala Ile Phe Val Ala
        355                 360                 365

Ile Leu Leu Phe Ile Ile Pro Ser Gln Lys Pro Lys Phe Asn Phe Ser
    370                 375                 380

Ser Gln Thr Glu Glu Glu Arg Lys Thr Pro Phe Tyr Pro Pro Ala Leu
385                 390                 395                 400

Leu Asp Trp Lys Val Ala Gln Glu Lys Val Pro Trp Asp Ile Val Leu
                405                 410                 415

Leu Leu Gly Gly Gly Phe Ala Met Ala Lys Gly Cys Glu Thr Ser Gly
            420                 425                 430

Leu Ser Lys Trp Met Ala Ala Gln Met Glu Pro Leu Arg Leu Val Lys
        435                 440                 445

Pro Ala Val Ile Thr Leu Ile Leu Ser Cys Leu Val Ala Met Thr Thr
    450                 455                 460

Glu Cys Thr Ser Asn Val Ala Thr Thr Thr Leu Phe Leu Pro Ile Phe
465                 470                 475                 480

Ala Ser Met Ala Arg Ser Ile Gly Ile His Pro Leu Tyr Val Met Ile
                485                 490                 495

Pro Cys Thr Met Ser Ala Ser Leu Ala Phe Met Leu Pro Val Ala Thr
            500                 505                 510

Pro Pro Asn Ala Ile Val Phe Ala Tyr Gly His Leu Arg Val Val Asp
        515                 520                 525

Met Met Lys Thr Gly Leu Ile Met Asn Phe Val Gly Ile Leu Ser Val
    530                 535                 540

Phe Leu Ser Val Asn Thr Trp Gly Arg Ala Met Phe Asn Leu Asp Asn
545                 550                 555                 560

Phe Pro Asp Trp Ala Asn Ser Thr Ser Val Asn Thr
                565                 570

<210>  11
<211>  2551
<212>  DNA
<213>  zebrafish NaCT

<400>  11
agagatcagc gcacagaagt tttgcgcagt ttctcaccgt ttggacattt cattgtaaag      60

ttatccaaag ccgaaatgat ggcttcacgt gcactcaaac tagtatggaa gatgaaaaat     120

acattgattc ttttttgcac tccatttctt ctgcttcctt tgccacttgt cattggatca     180

aaggaggctg gatgtgcata tgttgtggta ctgatggcag tttactggtg tacagaggtg     240

ctgccgctgg ctgtcactgc tctcctgccc gctgtgctct ttcccctctt cagaatcatg     300

gagtcccaag acgtatgtat gcagtacctt aaggacacta acatgctgtt tctgggtggc     360

ctgatggtgg ccgtggctgt cgaacactgg aatctgcaca agcggatcgc cctgcgggtg     420

ctgctccttg tgggggttcg accagctctg ttaatgttgg gcttcatggg tgtaacagct     480

ttcctctcca tgtggatcag taacacggcc acaacagcca tgatggtgcc catcgttcag     540

gcagttctcg agcagctcaa caacacagca caacaagaac aaagctccat acctgagacc     600

gaggaaaaga gcactgagaa acagcctgag agcccgggtg aggaaaaagt ggtactgaat     660

ggcgacaact tctcaatgga gtcagaccct gaagaacatt cacgagaagc agaggaaagg     720

ctgaagatgt ctaaaggcct gaccctgtgc gtgtgttatg ccgccagcat cggcggcaca     780

gccacactca caggcactgg accaaacctc gttcttatgg gacagatgag ccaactgttc     840

ccggacaacc ctgacatcat taactttgcg tcatggtttg gatttgcctt tccaaacatg     900

atcatcatgc tcacgctggc ctggctgtgg ctacagatcg tgtttctggg aataaacttt     960

aaaaagacat ggggctgtgg gacggtgaag acggagaagg agatcgcggc ctataatgtg    1020

attaaagagg agcaccgcag tctcggccct atgacctttg gggagctgag tgtccttgcc    1080

ctcttcatcc tcctggtggt gctttggttc actcgtgatc caggcttcgt ggacggctgg    1140

gcgacacgct tcttcaatgc tgacaaagag tttgtgacag atgccacggt tgcagtgttt    1200

gtggctgcgc tgctctttgt ctttccctct aaaccaccac gattgtgctt ctggagaaca    1260

gagagtttcg acacagtgcc ccagcaagaa agtggcccga ctccagcttt gctgacatgg    1320

aaagtgacac agaagaagat gccatggagt attatactgc tgctgggagg aggctttgcc    1380

ctggctaagg gcagtgagat ctcaggattg tccaagtggc ttggagatca gatgtctcct    1440

cttcaaagca ttcctccatg ggcaatagct attgtcatat gtttaatgat cgcaaccttc    1500

actgaatgca ccagtaatgt ggccacagct acattatttc tgcctatact ggcatctatg    1560

tctcagtcta taggtgtgaa tcctctgtat gttatggtgc cctgtaccct cagtgcatct    1620

tttgccttca tgctccctgt ggcaactcct ccaaacgcca tcgtcttctc atacggatac 1680
ctcaaagtct ctgacatggc caagactggg atcgtcatga acatcatcgg catcctctcc 1740
atcaccttag ccattaacag ctggggcaga gccatcttca gtttagacac gttccccagc 1800
tgggcaaaca ctactgatgt ctaagagaca cagagagccg gactgcccca ctcaccactt 1860
gtgaacttca gattgtttcc agttctcatg tgaacagaga aaaccgatta tgaccactgt 1920
ttagtcattt cacacattca tgtctatctt ttacaaaacc gtggtgttca ctacagtctg 1980
tggacattgt aacaccaata tttgaatgat tcataaaaga caaaaataga ttttacaaat 2040
catgattttt ctgtagcatt acagcatact gtgagcactg agcatatatt tggaccattg 2100
gttgttcgct tttggcttgt gaaagagcat ttggacgcag aaactcaaca tcagtcatat 2160
tgtccccttg tgtccagact cagagccagt ggccggtttc actggagatc aatactgtac 2220
tttgacggtt caaatcactt catgttcata tgcgtaattt aaagctgctt taaacacagc 2280
tacacaagag cacatgcaga aaaagctgaa aggctgaaat tgtgtaaata tcatctagat 2340
ttttaagcac aaaagtatat ttagagtata tatatatata tatgaataaa tttatacata 2400
cctcagatat gcagagctgc tttgacagag taatcatgta catttcaaaa ttaattcaaa 2460
ctctattttt aatgtacatt tattaaaatt atgtattttg ttcatattta ctaataaatt 2520
tttatttggt ttactcaaaa aaaaaaaaaa a 2551

<210> 12
<211> 581
<212> PRT
<213> zebrafish NaCT

<400> 12

Met Ala Ser Arg Ala Leu Lys Leu Val Trp Lys Met Lys Asn Thr Leu
1               5                   10                  15

Ile Leu Phe Cys Thr Pro Phe Leu Leu Leu Pro Leu Pro Leu Val Ile
            20                  25                  30

Gly Ser Lys Glu Ala Gly Cys Ala Tyr Val Val Val Leu Met Ala Val
        35                  40                  45

Tyr Trp Cys Thr Glu Val Leu Pro Leu Ala Val Thr Ala Leu Leu Pro
    50                  55                  60

Ala Val Leu Phe Pro Leu Phe Arg Ile Met Glu Ser Gln Asp Val Cys
65                  70                  75                  80

Met Gln Tyr Leu Lys Asp Thr Asn Met Leu Phe Leu Gly Gly Leu Met
                85                  90                  95

Val Ala Val Ala Val Glu His Trp Asn Leu His Lys Arg Ile Ala Leu
            100                 105                 110

Arg Val Leu Leu Leu Val Gly Val Arg Pro Ala Leu Leu Met Leu Gly
        115                 120                 125

Phe Met Gly Val Thr Ala Phe Leu Ser Met Trp Ile Ser Asn Thr Ala
    130                 135                 140

Thr Thr Ala Met Met Val Pro Ile Val Gln Ala Val Leu Glu Gln Leu
145                 150                 155                 160

Asn Asn Thr Ala Gln Gln Glu Gln Ser Ser Ile Pro Glu Thr Glu Glu
                165                 170                 175

Lys Ser Thr Glu Lys Gln Pro Glu Ser Pro Gly Glu Glu Lys Val Val
            180                 185                 190

Leu Asn Gly Asp Asn Phe Ser Met Glu Ser Asp Pro Glu Glu His Ser
        195                 200                 205

Arg Glu Ala Glu Glu Arg Leu Lys Met Ser Lys Gly Leu Thr Leu Cys
    210                 215                 220

Val Cys Tyr Ala Ala Ser Ile Gly Gly Thr Ala Thr Leu Thr Gly Thr
225                 230                 235                 240

Gly Pro Asn Leu Val Leu Met Gly Gln Met Ser Gln Leu Phe Pro Asp
                245                 250                 255

Asn Pro Asp Ile Ile Asn Phe Ala Ser Trp Phe Gly Phe Ala Phe Pro
            260                 265                 270

Asn Met Ile Ile Met Leu Thr Leu Ala Trp Leu Trp Leu Gln Ile Val
        275                 280                 285

Phe Leu Gly Ile Asn Phe Lys Lys Thr Trp Gly Cys Gly Thr Val Lys
    290                 295                 300

Thr Glu Lys Glu Ile Ala Ala Tyr Asn Val Ile Lys Glu Glu His Arg
305                 310                 315                 320

Ser Leu Gly Pro Met Thr Phe Gly Glu Leu Ser Val Leu Ala Leu Phe
                325                 330                 335

Ile Leu Leu Val Val Leu Trp Phe Thr Arg Asp Pro Gly Phe Val Asp
            340                 345                 350

Gly Trp Ala Thr Arg Phe Phe Asn Ala Asp Lys Glu Phe Val Thr Asp
        355                 360                 365

Ala Thr Val Ala Val Phe Val Ala Ala Leu Leu Phe Val Phe Pro Ser
    370                 375                 380

Lys Pro Pro Arg Leu Cys Phe Trp Arg Thr Glu Ser Phe Asp Thr Val
385                 390                 395                 400

Pro Gln Gln Glu Ser Gly Pro Thr Pro Ala Leu Leu Thr Trp Lys Val
                405                 410                 415

Thr Gln Lys Lys Met Pro Trp Ser Ile Ile Leu Leu Leu Gly Gly Gly
            420                 425                 430

Phe Ala Leu Ala Lys Gly Ser Glu Ile Ser Gly Leu Ser Lys Trp Leu
        435                 440                 445

Gly Asp Gln Met Ser Pro Leu Gln Ser Ile Pro Pro Trp Ala Ile Ala
    450                 455                 460

Ile Val Ile Cys Leu Met Ile Ala Thr Phe Thr Glu Cys Thr Ser Asn
465                 470                 475                 480

Val Ala Thr Ala Thr Leu Phe Leu Pro Ile Leu Ala Ser Met Ser Gln
                485                 490                 495

Ser Ile Gly Val Asn Pro Leu Tyr Val Met Val Pro Cys Thr Leu Ser
            500                 505                 510

Ala Ser Phe Ala Phe Met Leu Pro Val Ala Thr Pro Pro Asn Ala Ile
        515                 520                 525

Val Phe Ser Tyr Gly Tyr Leu Lys Val Ser Asp Met Ala Lys Thr Gly
    530                 535                 540

Ile Val Met Asn Ile Ile Gly Ile Leu Ser Ile Thr Leu Ala Ile Asn
545                 550                 555                 560

Ser Trp Gly Arg Ala Ile Phe Ser Leu Asp Thr Phe Pro Ser Trp Ala
                565                 570                 575

Asn Thr Thr Asp Val
            580

<210>  13
<211>  587
<212>  PRT
<213>  rat NaDC1

<400>  13

Met Ala Thr Cys Trp Pro Ala Leu Trp Ala Tyr Arg Phe Tyr Leu Ile
1               5                   10                  15

Val Leu Cys Leu Pro Ile Phe Leu Leu Pro Leu Pro Leu Ile Val Gln
            20                  25                  30

Thr Lys Glu Ala Tyr Cys Ala Tyr Ser Ile Ile Leu Met Ala Leu Leu
        35                  40                  45

Trp Cys Thr Glu Ala Leu Pro Leu Ala Val Thr Ala Leu Phe Pro Ile
    50                  55                  60

Val Leu Phe Pro Leu Met Gly Ile Met Asp Ala Ser Glu Val Cys Ile
65                  70                  75                  80

Glu Tyr Phe Lys Asp Thr Asn Ile Leu Phe Val Gly Gly Leu Met Val
                85                  90                  95

Ala Ile Ala Val Glu His Trp Asn Leu His Lys Arg Ile Ala Leu Gln
            100                 105                 110

Val Leu Leu Ile Ile Gly Val Arg Pro Ala Leu Leu Leu Leu Gly Phe
        115                 120                 125

Met Leu Val Thr Ala Phe Leu Ser Met Trp Ile Ser Asn Thr Ala Thr
    130                 135                 140

Thr Ala Met Met Val Pro Ile Gly His Ala Val Leu Glu Gln Leu Gln
145                 150                 155                 160

Gly Ser Lys Lys Asp Val Glu Gly Gly Asn Asn Asn Pro Thr Phe Glu
                165                 170                 175

Leu Gln Glu Glu Cys Pro Gln Lys Glu Val Thr Lys Leu Asp Asn Gly
            180                 185                 190

Gln Pro Val Ser Ala Pro Ser Glu Pro Arg Thr Gln Lys Thr Gln Glu
        195                 200                 205

His His Arg Phe Ser Gln Gly Leu Ser Leu Cys Ile Cys Tyr Ser Ala
    210                 215                 220

Ser Ile Gly Gly Ile Ala Thr Leu Thr Gly Thr Thr Pro Asn Leu Val
225                 230                 235                 240

Leu Gln Gly Gln Val Asn Ser Leu Phe Pro Gln Asn Gly Asn Val Val
                245                 250                 255

Asn Phe Ala Ser Trp Phe Gly Phe Ala Phe Pro Thr Met Ile Ile Leu
            260                 265                 270

Leu Leu Leu Ala Trp Leu Trp Leu Gln Val Leu Phe Leu Gly Val Asn
        275                 280                 285

Phe Arg Lys Asn Phe Gly Phe Gly Glu Gly Glu Glu Glu Arg Lys Gln
    290                 295                 300

Ala Ala Phe Gln Val Ile Lys Thr Gln Tyr Arg Leu Leu Gly Pro Met
305                 310                 315                 320

Ser Phe Ala Glu Lys Thr Val Thr Val Leu Phe Val Leu Leu Val Val
                325                 330                 335

Leu Trp Phe Thr Arg Glu Pro Gly Phe Phe Pro Gly Trp Gly Asp Thr
            340                 345                 350

Val Phe Ala Asn Glu Lys Gly Gln Ser Met Ala Ser Asp Gly Thr Val
        355                 360                 365

Ala Ile Phe Ile Ser Leu Val Met Phe Ile Ile Pro Ser Lys Ile Pro
    370                 375                 380

Gly Leu Met Gln Asp Pro Lys Lys Pro Gly Lys Leu Lys Ala Pro Pro
385                 390                 395                 400

Ala Ile Leu Thr Trp Lys Thr Val Asn Asp Lys Met Pro Trp Asn Ile
                405                 410                 415

Val Ile Leu Leu Gly Gly Gly Phe Ala Leu Ala Lys Gly Ser Glu Gln
            420                 425                 430

Ser Gly Leu Ser Glu Trp Leu Gly Asp Lys Leu Thr Pro Leu Gln His
        435                 440                 445

Ile Pro Pro Ser Ala Thr Ala Val Ile Leu Cys Leu Leu Ile Ala Ile
    450                 455                 460

Phe Thr Glu Cys Thr Ser Asn Val Ala Thr Thr Thr Leu Phe Leu Pro
465                 470                 475                 480

Ile Leu Ala Ser Met Ala Gln Ala Ile Cys Leu His Pro Leu Tyr Val
                485                 490                 495

Met Leu Pro Cys Thr Leu Ala Ala Ser Leu Ala Phe Met Leu Pro Val
            500                 505                 510

Ala Thr Pro Pro Asn Ala Ile Val Phe Ser Phe Gly Gly Leu Lys Val
        515                 520                 525

Ser Asp Met Ala Arg Ala Gly Phe Leu Leu Asn Ile Ile Gly Val Leu
    530                 535                 540

Ala Ile Thr Leu Ser Ile Asn Ser Trp Ser Ile Pro Ile Phe Lys Leu
545                 550                 555                 560

Asp Thr Phe Pro Ser Trp Ala His Ser Asn Thr Ser Gln Cys Leu Leu
                565                 570                 575

Asn Pro Ser Asn Ser Thr Val Pro Gly Gly Leu
            580                 585

<210>  14
<211>  600
<212>  PRT
<213>  rat NaDC3

<400>  14

Met Ala Ala Leu Ala Ala Leu Ala Lys Lys Val Trp Ser Ala Arg Arg
1               5                   10                  15

Leu Leu Val Leu Leu Leu Val Pro Leu Ala Leu Leu Pro Ile Leu Phe
            20                  25                  30

Ala Leu Pro Pro Lys Glu Gly Arg Cys Leu Tyr Val Ile Leu Leu Met
        35                  40                  45

Ala Val Tyr Trp Cys Thr Glu Ala Leu Pro Leu Ser Val Thr Ala Leu
    50                  55                  60

Leu Pro Ile Ile Leu Phe Pro Phe Met Gly Ile Leu Pro Ser Ser Lys
65                  70                  75                  80

Val Cys Pro Gln Tyr Phe Leu Asp Thr Asn Phe Leu Phe Leu Ser Gly
                85                  90                  95

Leu Ile Met Ala Ser Ala Ile Glu Glu Arg Asn Leu His Arg Arg Ile
            100                 105                 110

Ala Leu Lys Val Leu Met Leu Val Gly Val Gln Pro Ala Arg Leu Ile
        115                 120                 125

Leu Gly Met Met Val Thr Thr Ser Phe Leu Ser Met Trp Leu Ser Asn
    130                 135                 140

Thr Ala Ser Thr Ala Met Met Leu Pro Ile Ala Ser Ala Ile Leu Lys
145                 150                 155                 160

Ser Leu Phe Gly Gln Arg Asp Thr Arg Lys Asp Leu Pro Arg Glu Gly
                165                 170                 175

Glu Asp Ser Thr Ala Ala Val Arg Gly Asn Gly Leu Arg Thr Val Pro
            180                 185                 190

Thr Glu Met Gln Phe Leu Ala Ser Ser Glu Gly Gly His Ala Glu Asp
        195                 200                 205

Val Glu Ala Pro Leu Glu Leu Pro Asp Asp Ser Lys Glu Glu Glu His
    210                 215                 220

Arg Arg Asn Ile Trp Lys Gly Phe Leu Ile Ser Ile Pro Tyr Ser Ala

225                 230                 235                 240

Ser Ile Gly Gly Thr Ala Thr Leu Thr Gly Thr Ala Pro Asn Leu Ile
                245                 250                 255

Leu Leu Gly Gln Leu Lys Ser Phe Phe Pro Gln Cys Asp Val Val Asn
            260                 265                 270

Phe Gly Ser Trp Phe Ile Phe Ala Phe Pro Leu Met Leu Leu Phe Leu
        275                 280                 285

Leu Val Gly Trp Leu Trp Ile Ser Phe Leu Tyr Gly Gly Met Ser Trp
    290                 295                 300

Arg Gly Trp Arg Lys Lys Asn Ser Lys Leu Gln Asp Val Ala Glu Asp
305                 310                 315                 320

Lys Ala Lys Ala Val Ile Gln Glu Glu Phe Gln Asn Leu Gly Pro Ile
                325                 330                 335

Lys Phe Ala Glu Gln Ala Val Phe Ile Leu Phe Cys Leu Phe Ala Ile
            340                 345                 350

Leu Leu Phe Ser Arg Asp Pro Lys Phe Ile Pro Gly Trp Ala Ser Leu
        355                 360                 365

Phe Ala Pro Gly Phe Val Ser Asp Ala Val Thr Gly Val Ala Ile Val
    370                 375                 380

Thr Ile Leu Phe Phe Phe Pro Ser Gln Lys Pro Ser Leu Lys Trp Trp
385                 390                 395                 400

Phe Asp Phe Lys Ala Pro Asn Ser Glu Thr Glu Pro Leu Leu Ser Trp
                405                 410                 415

Lys Lys Ala Gln Glu Thr Val Pro Trp Asn Ile Ile Leu Leu Leu Gly
            420                 425                 430

Gly Gly Phe Ala Met Ala Lys Gly Cys Glu Glu Ser Gly Leu Ser Ala
        435                 440                 445

Trp Ile Gly Gly Gln Leu His Pro Leu Glu His Val Pro Pro Leu Leu
    450                 455                 460

Ala Val Leu Leu Ile Thr Val Val Ile Ala Phe Phe Thr Glu Phe Ala
465                 470                 475                 480

Ser Asn Thr Ala Thr Ile Ile Ile Phe Leu Pro Val Leu Ala Glu Leu
                485                 490                 495

Ala Ile Arg Leu His Val His Pro Leu Tyr Leu Met Ile Pro Gly Thr

500                 505                 510

Val Ser Cys Ser Tyr Ala Phe Met Leu Pro Val Ser Thr Pro Pro Asn
        515                 520                 525

Ser Ile Ala Phe Ser Thr Gly His Leu Leu Val Lys Asp Met Val Arg
    530                 535                 540

Thr Gly Leu Leu Met Asn Leu Met Gly Val Leu Leu Leu Ser Leu Ala
545                 550                 555                 560

Met Asn Thr Trp Ala Gln Ala Ile Phe Gln Leu Gly Thr Phe Pro Asp
                565                 570                 575

Trp Ala Asn Thr His Ala Ala Asn Val Thr Ala Leu Pro Pro Ala Leu
            580                 585                 590

Thr Asn Asn Thr Val Gln Thr Leu
        595                 600

<210>  15
<211>  20
<212>  DNA
<213>  Artificial

<220>
<223>  Artificially synthesized oligonucleotide primer

<400>  15
ctccaacttc ttcgctaacc                                                   20

<210>  16
<211>  19
<212>  DNA
<213>  Artificial

<220>
<223>  Artificially synthesized oligonucleotide primer

<400>  16
ctagtgcgtc ttgtttccc                                                    19

<210>  17
<211>  21
<212>  DNA
<213>  Artificial

<220>
<223>  Artificially synthesized oligonucleotide primer

<400>  17
tcttttctcc ctccagtcag t                                                 21

<210>  18
<211>  18
<212>  DNA
<213>  Artificial

<220>
<223> Artificially synthesized oligonucleotide primer

<400> 18
ggcaatcttc tcggtgtc 18

<210> 19
<211> 20
<212> DNA
<213> Artificial

<220>
<223> Artificially synthesized oligonucleotide primer

<400> 19
ctcggcgctg agctatgtct 20

<210> 20
<211> 17
<212> DNA
<213> Artificial

<220>
<223> Artificially synthesized oligonucleotide primer

<400> 20
gttgatctcc gcgaagg 17

<210> 21
<211> 19
<212> DNA
<213> Artificial

<220>
<223> Artificially synthesized oligonucleotide primer

<400> 21
ctccatcgaa gaatcgcac 19

<210> 22
<211> 20
<212> DNA
<213> Artificial

<220>
<223> Artificially synthesized oligonucleotide primer

<400> 22
gaaatagcat acccagcacc 20

<210> 23
<211> 40
<212> DNA
<213> Artificial

<220>
<223> Artificially synthesized oligonucleotide primer

<400> 23
cccgggtatg aagcctagcc cccagcgtac gttaataaaa 40

<210> 24

<211>  45
<212>  DNA
<213>  Artificial

<220>
<223>  Artificially synthesized oligonucleotide primer

<400>  24
gcggatccaa aaattagcaa actggatatg aagagttttc tgaag                    45


<210>  25
<211>  33
<212>  DNA
<213>  Artificial

<220>
<223>  Artificially synthesized oligonucleotide primer

<400>  25
gtcgacgagg tgttaaactg tatagtcgtg gtg                                 33


<210>  26
<211>  33
<212>  DNA
<213>  Artificial

<220>
<223>  Artificially synthesized oligonucleotide primer

<400>  26
gccggatcca agaagtacca gaagcttttt tat                                 33


<210>  27
<211>  20
<212>  DNA
<213>  Artificial

<220>
<223>  Artificially synthesized oligonucleotide primer

<400>  27
gtctcccttt cacgcgatgg                                                20


<210>  28
<211>  29
<212>  DNA
<213>  Artificial

<220>
<223>  Artificially synthesized oligonucleotide primer

<400>  28
tcgtctagag cttgtgctct tgcggctct                                      29


<210>  29
<211>  17
<212>  PRT
<213>  Artificial

<220>
<223>  peptide consensus sequence

<220>
<221> PEPTIDE
<222> (1)..(1)
<223> serine, alanine, cysteine, theonine or proline

<220>
<221> misc_feature
<222> (3)..(4)
<223> Xaa can be any naturally occurring amino acid

<220>
<221> misc_feature
<222> (6)..(7)
<223> Xaa can be any naturally occurring amino acid

<220>
<221> PEPTIDE
<222> (9)..(9)
<223> valine, leucine, isolecine, methionine

<220>
<221> PEPTIDE
<222> (10)..(10)
<223> glycine, serine, alanine

<220>
<221> misc_feature
<222> (11)..(13)
<223> Xaa can be any naturally occurring amino acid

<220>
<221> misc_feature
<222> (15)..(15)
<223> Xaa can be any naturally occurring amino acid

<220>
<221> PEPTIDE
<222> (16)..(16)
<223> isoleucine, leucine, valine, methionine

<400> 29

Ser Ser Xaa Xaa Phe Xaa Xaa Pro Val Gly Xaa Xaa Xaa Asn Xaa Ile
1               5                   10                  15

Val

SEQUENCE LISTING

<110> MEDICAL COLLEGE OF GEORGIA RESEARCH INSTITUTE, INC.

<120> NaCT AS A TARGET FOR LIFESPAN EXPANSION AND WEIGHT REDUCTION

<130> L1699 EP/1

<140> 07 00 0032.8
<141> 2007-01-02

<150> 60/428,469
<151> 2002-11-22

<150> 60/459,441
<151> 2003-04-01

<160> 29

<170> PatentIn version 3.2

<210> 1
<211> 2602
<212> DNA
<213> Drosophila melanogaster

<400> 1
ttcaccgttt ccgaatcgga cgaaccgggc gtgattgctc tcctgctgct ttcgagatcg 60

gagtcccgat aaggatataa ctacaaccta aagaggaatc caagcctcct cctgccgcta 120

gtttcgaaaa gtaaatagag tacttgttat caactgggaa gcggagatac atagctccga 180

tattcctgtg aaagccagac aaacggatac caacgaacaa tcgccatatc tacacgccac 240

cgccactgga catcaaaatg gaaattgaaa ttggcgaaca accccagcct ccggtgaagt 300

gctccaactt cttcgctaac cactggaagg gattggttgt gttcctggtg ccgctgctat 360

gtctgcctgt tatgctgcta aacgaaggcg ccgaatttcg gtgcatgtac ctccttttgg 420

taatggccat attttgggtt acggaagcct tgcctctcta tgtgacgtcc atgataccga 480

ttgtggcctt cccaataatg ggtataatga gctcggatca gacttgccgc ttgtacttca 540

aggatacgct ggtgatgttc atgggcggca ttatggtcgc cctggctgtg gagtactgta 600

atctacacaa acgtcttgcc ttgagggtaa tccagatcgt gggctgcagt ccccgcagat 660

tacactttgg cctcatcatg gttacaatgt ttttgagcat gtggatttcg aacgccgcct 720

gtactgccat gatgtgtccg attatccaag ccgtgctgga ggagctgcag gctcagggtg 780

tctgcaaaat caaccatgag cctcaatacc aaatcgttgg aggcaacaag aaaaacaacg 840

aggatgagcc accatacccc accaagatca ctctgtgcta ctatctgggc attgcctacg 900

cctcctcgct gggtggctgt ggaaccatca tcggaactgc caccaatctt accttcaagg 960

gcatctacga ggctcgtttc aagaactcca ccgaacagat ggacttcccc accttcatgt 1020

tctactcggt gccatccatg ttggtctaca ccttgctgac attcgtgttc ctgcaatggc 1080

acttcatggg tctgtggcgt cccaagagca aggaggcaca ggaagtccag aggggacgag 1140

agggcgccga tgtcgccaaa aaggttatcg atcagcgcta caaggatctg ggtcccatgt 1200

ccattcacga gatccaagtg atgattctgt tcatttttat ggttgtgatg tacttcaccc 1260

gcaagcccgg catctttttg ggatgggccg atttgctgaa ttccaaggac attcgtaact 1320

ctatgcccac tatttttgtc gtcgtcatgt gcttcatgct gcccgccaat tatgctttcc 1380

tacgctactg caccagacgc ggtggtccag tgcccacggg tcccactcca tcgctgatca 1440

cctggaagtt catccagacc aaggtgccat ggggtctggt gttcctgctt ggcggtggct 1500

tcgctttggc cgaaggcagc aagcagagcg gcatggccaa gctgattggc aatgctctga 1560

ttggattgaa ggttctgccc aactctgtcc tcttactggt ggtcatcctg gtggctgtgt 1620

tcctgaccgc cttcagctcc aatgtggcga ttgccaacat tattattccc gttctggccg 1680

agatgtccct ggccattgag atccatcctc tgtacctgat cctgcccgct ggcttggcct 1740

gcagtatggc cttccacctg ccggttagta ctccgcccaa cgctttggtt gctggctatg 1800

ccaacattag gacgaaggac atggccattg ctggaatcgg tccgaccatc attaccatca 1860

tcaccctgtt tgttttctgc caaacctggg gcctggtcgt ctatccgaac cttaactcgt 1920

tccccgaatg ggctcagatt tatgccgcgg cagcactggg aaacaagacg cactagatag 1980

ttagtaatta gtgtaaataa ctaacatacc cgtcacagcg ataaagttga ggaaaattta 2040

gggaatttta aacgaaaagt gcctttgctg acagcgaaaa atgtgaaaaa tatttaacta 2100

tgtatacttg catttcagag ttgcgaaaag ttttgataca aaagcattac ctactgttta 2160

gaaaaatgtg ttaaaaaaaa aacgtatcgc aatatactgt taatcaggaa ttgaacacct 2220

ggtctacgca ctcagctaaa tatttaaata caaattaatg ttacttaatt gttgcattta 2280

gcataaaaat ggaaaagatt tggaaaagtt agaacagttt gttcaatggc agccctggcc 2340

tgctaatatt ttaaataact agactgagag aacttacata ttcatacatg tttttcaact 2400

tgtaaaaatt tttaaatgaa caactcactc aatacttcat tgcgaaccaa aatgaacaca 2460

caaatagcgg taggctaagc ttaaatgata ctgtgtacat tttcagatga tttatgtttt 2520

atatagtttg taaaaaatat taaataataa aaagctcaac cgccaataaa aaaaaaaaaa 2580

aaaaaaaaaa aaaaaaaaaa aa 2602

<210> 2
<211> 572
<212> PRT
<213> Drosophila melanogaster

<400> 2

Met Glu Ile Glu Ile Gly Glu Gln Pro Gln Pro Pro Val Lys Cys Ser
1               5                   10                  15

Asn Phe Phe Ala Asn His Trp Lys Gly Leu Val Val Phe Leu Val Pro

20                  25                  30

Leu Leu Cys Leu Pro Val Met Leu Leu Asn Glu Gly Ala Glu Phe Arg
        35                  40                  45

Cys Met Tyr Leu Leu Leu Val Met Ala Ile Phe Trp Val Thr Glu Ala
    50                  55                  60

Leu Pro Leu Tyr Val Thr Ser Met Ile Pro Ile Val Ala Phe Pro Ile
65                  70                  75                  80

Met Gly Ile Met Ser Ser Asp Gln Thr Cys Arg Leu Tyr Phe Lys Asp
                85                  90                  95

Thr Leu Val Met Phe Met Gly Gly Ile Met Val Ala Leu Ala Val Glu
            100                 105                 110

Tyr Cys Asn Leu His Lys Arg Leu Ala Leu Arg Val Ile Gln Ile Val
        115                 120                 125

Gly Cys Ser Pro Arg Arg Leu His Phe Gly Leu Ile Met Val Thr Met
    130                 135                 140

Phe Leu Ser Met Trp Ile Ser Asn Ala Ala Cys Thr Ala Met Met Cys
145                 150                 155                 160

Pro Ile Ile Gln Ala Val Leu Glu Glu Leu Gln Ala Gln Gly Val Cys
                165                 170                 175

Lys Ile Asn His Glu Pro Gln Tyr Gln Ile Val Gly Gly Asn Lys Lys
            180                 185                 190

Asn Asn Glu Asp Glu Pro Pro Tyr Pro Thr Lys Ile Thr Leu Cys Tyr
        195                 200                 205

Tyr Leu Gly Ile Ala Tyr Ala Ser Ser Leu Gly Gly Cys Gly Thr Ile
    210                 215                 220

Ile Gly Thr Ala Thr Asn Leu Thr Phe Lys Gly Ile Tyr Glu Ala Arg
225                 230                 235                 240

Phe Lys Asn Ser Thr Glu Gln Met Asp Phe Pro Thr Phe Met Phe Tyr
                245                 250                 255

Ser Val Pro Ser Met Leu Val Tyr Thr Leu Leu Thr Phe Val Phe Leu
            260                 265                 270

Gln Trp His Phe Met Gly Leu Trp Arg Pro Lys Ser Lys Glu Ala Gln

        275                 280                 285

Glu Val Gln Arg Gly Arg Glu Gly Ala Asp Val Ala Lys Lys Val Ile
    290                 295                 300

Asp Gln Arg Tyr Lys Asp Leu Gly Pro Met Ser Ile His Glu Ile Gln
305                 310                 315                 320

Val Met Ile Leu Phe Ile Phe Met Val Val Met Tyr Phe Thr Arg Lys
                325                 330                 335

Pro Gly Ile Phe Leu Gly Trp Ala Asp Leu Leu Asn Ser Lys Asp Ile
            340                 345                 350

Arg Asn Ser Met Pro Thr Ile Phe Val Val Val Met Cys Phe Met Leu
        355                 360                 365

Pro Ala Asn Tyr Ala Phe Leu Arg Tyr Cys Thr Arg Arg Gly Gly Pro
    370                 375                 380

Val Pro Thr Gly Pro Thr Pro Ser Leu Ile Thr Trp Lys Phe Ile Gln
385                 390                 395                 400

Thr Lys Val Pro Trp Gly Leu Val Phe Leu Leu Gly Gly Gly Phe Ala
                405                 410                 415

Leu Ala Glu Gly Ser Lys Gln Ser Gly Met Ala Lys Leu Ile Gly Asn
            420                 425                 430

Ala Leu Ile Gly Leu Lys Val Leu Pro Asn Ser Val Leu Leu Leu Val
        435                 440                 445

Val Ile Leu Val Ala Val Phe Leu Thr Ala Phe Ser Ser Asn Val Ala
    450                 455                 460

Ile Ala Asn Ile Ile Ile Pro Val Leu Ala Glu Met Ser Leu Ala Ile
465                 470                 475                 480

Glu Ile His Pro Leu Tyr Leu Ile Leu Pro Ala Gly Leu Ala Cys Ser
                485                 490                 495

Met Ala Phe His Leu Pro Val Ser Thr Pro Pro Asn Ala Leu Val Ala
            500                 505                 510

Gly Tyr Ala Asn Ile Arg Thr Lys Asp Met Ala Ile Ala Gly Ile Gly
        515                 520                 525

Pro Thr Ile Ile Thr Ile Ile Thr Leu Phe Val Phe Cys Gln Thr Trp

530                 535                 540

Gly Leu Val Val Tyr Pro Asn Leu Asn Ser Phe Pro Glu Trp Ala Gln
545                 550                 555                 560

Ile Tyr Ala Ala Ala Ala Leu Gly Asn Lys Thr His
                565                 570

<210>  3
<211>  3254
<212>  DNA
<213>  Rattus norvegicus

<400>  3
ccagtctgtc tccctttcac gcgatggctt cggcgaagac ttatgtgacc aagttcaagt      60

ccttcgtgat tttgttcttc gccccgatcc tgctgcttcc actcatcatt ctggtacctg     120

acaagtttgc caggtgtgcc tatgttataa tcctcatggc catctactgg tgcacagatg     180

tcatcccagt ggctatcact tccctcctgc ctgtcttact cttcccactt ttgaaggttc     240

tggactccaa gcaggtatgt gtccaataca tgacggacac caacatgctg ttcctgggca     300

gtctcattgt ggccacggct gtggaacgtt gggaacttca taagaggatt gctctgagaa     360

tgctactctt tgtggggacc aagccttcac ggctgatgct gggcttcatg ttcgtcacag     420

ccttcctgtc catgtggatc agcaatactg ccaccacggc catgatgata cccattgtgg     480

aggccatgct ggagcaaatg gtagccacaa atgtagcggt ggatgccagc cagaggacaa     540

tggaactgtt ggacaagaac aaggccagcg aattgccagg aagccaggtg gtatttgaag     600

accccagtgt gcagaagcag gaggatgaag aaacaaagaa tatgtacaag gctatgaacc     660

tatgtgtgtg ctacgcagcc agcatcgggg gtacagccac cttgaccggg acgggaccca     720

acgtggtgct cctgggccag atgcaggaat tgtttcctga cagtaaagac gtcatgaact     780

ttgcatcttg gtttgcattt gccctcccaa acatgctttt gatgctggtg atggcctggc     840

tgtggctcct gtgtttttac atgagaccca atttaaaaaa aacttgcatc tgctgtggga     900

ggaagaagaa ggacacggag aagattgcct ccaaggtgct gtatgaggag tacaggaagc     960

tggggccctt gagctacgct gaatgcaacg tgctcttttg cttcggcctg ctcatcatcc    1020

tgtggttctc ccgagacccc ggcttcatgc ctggctggct gtccatcgcc tggatcgagg    1080

gaaataccaa gcatgtcacg gacgccacag tggccatctt tgtggccatt ttgcttttca    1140

tcgtaccctc acaaaagccc aagttcaatt tcagccgcca gactgaggaa gaaaggaaaa    1200

ctcccttcta ccccccgcca ctgctgaatt ggaaagtcac ccaagagaaa gtgccctggg    1260

gcattgtgct gctcctgggg ggaggatttg ctatggccaa aggatgtgag acttcagggc    1320

tctctgagtg gatggcgaga cagatggagc ccttgagctc agtgagacct gctattatta    1380

ccttgatctt gtcctgtatt gttgcaatga ccacagagtg cacgagtaac gtggccacta    1440

ctaccctgtt cctgcctatc tttgcctcca tggctcgttc catcggcatc catccactgt 1500
atgtcatgat tccctgcacc ttgagtgcgt cacttgcctt catgttgcct gtggccaccc 1560
cacctaacgc catcgtgttt gcctacggac acctcaaagt tattgacatg gtaaaaacag 1620
gattggtaat gaacatcctt ggaattgcat ctgtgtttct gtcagtcaac acctggggac 1680
gggctgtgtt taacttggat aaattccctg actgggcaaa tttgacacat attaacactt 1740
aggagaacca caagagcaca ggcttgtccc ccaacccttt cgaggactgc gaaccttctg 1800
gcacaccttg cacagagcac tggtgctcat accccagtgt gacccaatga tgtcaacacc 1860
ccaagaagat ctaaccaact agccacctct tcctccaggc tcaggttcag agatggcaac 1920
gggcgatggg aagataggct cagaagggaa aggaaccttt gagaggtcgt gaggcccatc 1980
ttccctagga cccttccatc tcacctgggc aggaaacaga gggactgggg ctcaagtcct 2040
gtaccacgtg gctttgaaag acttctgact ccatgctggg ctctggttct cacatgcctg 2100
ttcccacggt ctccacatgg ggatcagatg accaggagac agcccctgtg cctcttctgg 2160
atgttcccag atcaccatct ctatcaccac gaaggaactt cctctccagg acagaactct 2220
gatcttgaac actttccact gccagagtta gagtggaaat cacggccccc tgaagacttt 2280
gactctacat ggtgccatct ccaaccactg ggcaacctga ggtgctaaca ttgaggcctt 2340
cctgctcacc cttggctgac ctgttcccta cttgccttat ctcttattag ttaacagttt 2400
gaggcccctt cccagctccc cagtgagact tcatcaactc ctagatgctc ctggctgagg 2460
ctccccagaa agtccctgtt tgattgttct ggggatgctc atcctgccag cctggagcag 2520
ctgggtcata catcagggat ggacaatggt gtctctttag gggatggtgt taaagtggga 2580
tacctaaacc ttccttcctt tggctggatc cagagcagat tcagcagctc gtttataagg 2640
gctgggccac cacagttggt cctgttggac tcttgagctc ctccagaagg ccttttcctt 2700
gctgtctgtg cactgtgggc aagcctggta gcaaccgggc atggttgaga tgagcgatgc 2760
ttgctggtgt ctgagcggac agaccctgcc agagtaagct gactaggtag agtgtccatg 2820
ggaacccatg ggaccactca catgaaaggg agacagaaga ggaagctctc ccttgtcctt 2880
cagggatgct ctcttccttg cttaatttgc tctgaaaaga agcatgagtg gggagataag 2940
atccgtggat gtcattttat ttttccaggc agaagacagt atgaggaccc aaggagtccc 3000
catcaaagac caagtatgtg tctggctcct tgggagggat ggctcctgac catactgtcg 3060
aaggcttgct cttgtcaaat actctgtctg ctatggaaag ttccagtgtg ctgactggtc 3120
tgtgatttcg tgccttgtga aggagacaga gggaggcaag atgaataaac agttgctctc 3180
tgtccacctc caaaaaaaaa aaaaaaaaaa aaaaaaaaaa aaaaaaaaaa aaaaaaaaaa 3240
aaaaaaaaaa aaaa 3254

<210>  4
<211>  572
<212>  PRT
<213>  Rattus norvegicus

<400>  4

Met Ala Ser Ala Lys Thr Tyr Val Thr Lys Phe Lys Ser Phe Val Ile
1               5                   10                  15

Leu Phe Phe Ala Pro Ile Leu Leu Leu Pro Leu Ile Ile Leu Val Pro
            20                  25                  30

Asp Lys Phe Ala Arg Cys Ala Tyr Val Ile Ile Leu Met Ala Ile Tyr
        35                  40                  45

Trp Cys Thr Asp Val Ile Pro Val Ala Ile Thr Ser Leu Leu Pro Val
    50                  55                  60

Leu Leu Phe Pro Leu Leu Lys Val Leu Asp Ser Lys Gln Val Cys Val
65                  70                  75                  80

Gln Tyr Met Thr Asp Thr Asn Met Leu Phe Leu Gly Ser Leu Ile Val
                85                  90                  95

Ala Thr Ala Val Glu Arg Trp Glu Leu His Lys Arg Ile Ala Leu Arg
            100                 105                 110

Met Leu Leu Phe Val Gly Thr Lys Pro Ser Arg Leu Met Leu Gly Phe
        115                 120                 125

Met Phe Val Thr Ala Phe Leu Ser Met Trp Ile Ser Asn Thr Ala Thr
    130                 135                 140

Thr Ala Met Met Ile Pro Ile Val Glu Ala Met Leu Glu Gln Met Val
145                 150                 155                 160

Ala Thr Asn Val Ala Val Asp Ala Ser Gln Arg Thr Met Glu Leu Leu
                165                 170                 175

Asp Lys Asn Lys Ala Ser Glu Leu Pro Gly Ser Gln Val Val Phe Glu
            180                 185                 190

Asp Pro Ser Val Gln Lys Gln Glu Asp Glu Glu Thr Lys Asn Met Tyr
        195                 200                 205

Lys Ala Met Asn Leu Cys Val Cys Tyr Ala Ala Ser Ile Gly Gly Thr
    210                 215                 220

Ala Thr Leu Thr Gly Thr Gly Pro Asn Val Val Leu Leu Gly Gln Met

225                 230                 235                 240

Gln Glu Leu Phe Pro Asp Ser Lys Asp Val Met Asn Phe Ala Ser Trp
                245                 250                 255

Phe Ala Phe Ala Leu Pro Asn Met Leu Leu Met Leu Val Met Ala Trp
            260                 265                 270

Leu Trp Leu Leu Cys Phe Tyr Met Arg Pro Asn Leu Lys Lys Thr Cys
        275                 280                 285

Ile Cys Cys Gly Arg Lys Lys Lys Asp Thr Glu Lys Ile Ala Ser Lys
    290                 295                 300

Val Leu Tyr Glu Glu Tyr Arg Lys Leu Gly Pro Leu Ser Tyr Ala Glu
305                 310                 315                 320

Cys Asn Val Leu Phe Cys Phe Gly Leu Leu Ile Ile Leu Trp Phe Ser
                325                 330                 335

Arg Asp Pro Gly Phe Met Pro Gly Trp Leu Ser Ile Ala Trp Ile Glu
            340                 345                 350

Gly Asn Thr Lys His Val Thr Asp Ala Thr Val Ala Ile Phe Val Ala
        355                 360                 365

Ile Leu Leu Phe Ile Val Pro Ser Gln Lys Pro Lys Phe Asn Phe Ser
    370                 375                 380

Arg Gln Thr Glu Glu Glu Arg Lys Thr Pro Phe Tyr Pro Pro Pro Leu
385                 390                 395                 400

Leu Asn Trp Lys Val Thr Gln Glu Lys Val Pro Trp Gly Ile Val Leu
                405                 410                 415

Leu Leu Gly Gly Gly Phe Ala Met Ala Lys Gly Cys Glu Thr Ser Gly
            420                 425                 430

Leu Ser Glu Trp Met Ala Arg Gln Met Glu Pro Leu Ser Ser Val Arg
        435                 440                 445

Pro Ala Ile Ile Thr Leu Ile Leu Ser Cys Ile Val Ala Met Thr Thr
    450                 455                 460

Glu Cys Thr Ser Asn Val Ala Thr Thr Thr Leu Phe Leu Pro Ile Phe
465                 470                 475                 480

Ala Ser Met Ala Arg Ser Ile Gly Ile His Pro Leu Tyr Val Met Ile

485                 490                 495

Pro Cys Thr Leu Ser Ala Ser Leu Ala Phe Met Leu Pro Val Ala Thr
            500                 505                 510

Pro Pro Asn Ala Ile Val Phe Ala Tyr Gly His Leu Lys Val Ile Asp
        515                 520                 525

Met Val Lys Thr Gly Leu Val Met Asn Ile Leu Gly Ile Ala Ser Val
    530                 535                 540

Phe Leu Ser Val Asn Thr Trp Gly Arg Ala Val Phe Asn Leu Asp Lys
545                 550                 555                 560

Phe Pro Asp Trp Ala Asn Leu Thr His Ile Asn Thr
                565                 570

<210>  5
<211>  3248
<212>  DNA
<213>  Homo sapiens

<400>  5
   ccctcccgcg cgatggcctc ggcgctgagc tatgtctcca agttcaagtc cttcgtgatc      60
   ttgttcgtca ccccgctcct gctgctgcca ctcgtcattc tgatgcccgc caagtttgtc     120
   aggtgtgcct acgtcatcat cctcatggcc atttactggt gcacagaagt catccctctg     180
   gctgtcacct ctctcatgcc tgtcttgctt ttcccactct tccagattct ggactccagg     240
   caggtgtgtg tccagtacat gaaggacacc aacatgctgt tcctgggcgg cctcatcgtg     300
   gccgtggctg tggagcgctg gaacctgcac aagaggatcg ccctgcgcac gctcctctgg     360
   gtgggggcca agcctgcacg gctgatgctg ggcttcatgg gcgtcacagc cctcctgtcc     420
   atgtggatca gtaacacggc aaccacggcc atgatggtgc ccatcgtgga ggccatattg     480
   cagcagatgg aagccacaag cgcagccacc gaggccggcc tggagctggt ggacaagggc     540
   aaggccaagg agctgccagg gagtcaagtg atttttgaag gccccactct ggggcagcag     600
   gaagaccaag agcggaagag gttgtgtaag gccatgaccc tgtgcatctg ctacgcggcc     660
   agcatcgggg gcaccgccac cctgaccggg acgggaccca acgtggtgct cctgggccag     720
   atgaacgagt tgtttcctga cagcaaggac ctcgtgaact ttgcttcctg gtttgcattt     780
   gcctttccca acatgctggt gatgctgctg ttcgcctggc tgtggctcca gtttgtttac     840
   atgagattca attttaaaaa gtcctggggc tgcgggctag agagcaagaa aaacgagaag     900
   gctgccctca aggtgctgca ggaggagtac cggaagctgg ggcccttgtc cttcgcggag     960
   atcaacgtgc tgatctgctt cttcctgctg gtcatcctgt ggttctcccg agaccctggc    1020
   ttcatgcccg gctggctgac tgttgcctgg gtggagggtg agacaaagta tgtctccgat    1080
   gccactgtgg ccatctttgt ggctaccctg ctattcattg tgccttcaca gaagcccaag 1140
   tttaacttcc gcagccagac tgaggaagaa aggaaaactc cattttatcc ccctcccctg 1200
   ctggattgga aggtaaccca ggagaaagtg ccctggggca tcgtgctgct actagggggc 1260
   ggatttgctc tggctaaagg atccgaggcc tcggggctgt ccgtgtggat ggggaagcag 1320
   atggagccct tgcacgcagt gcccccggca gccatcacct tgatcttgtc cttgctcgtt 1380
   gccgtgttca ctgagtgcac aagcaacgtg gccaccacca ccttgttcct gcccatcttt 1440
   gcctccatgt ctcgctccat cggcctcaat ccgctgtaca tcatgctgcc ctgtaccctg 1500
   agtgcctcct ttgccttcat gttgcctgtg gccacccctc caaatgccat cgtgttcacc 1560
   tatgggcacc tcaaggttgc tgacatggtg aaaacaggag tcataatgaa cataattgga 1620
   gtcttctgtg tgtttttggc tgtcaacacc tggggacggg ccatatttga cttggatcat 1680
   ttccctgact gggctaatgt gacacatatt gagacttagg aagagccaca agaccacaca 1740
   cacagccctt accctcctca ggactaccga accttctggc acaccttgta cagagttttg 1800
   gggttcacac cccaaaatga cccaacgatg tccacacacc accaaaaccc agccaatggg 1860
   ccacctcttc ctccaagccc agatgcagag atggacatgg gcagctggag ggtaggctca 1920
   gaaatgaagg gaacccctca gtgggctgct ggacccatct ttcccaagcc ttgccattat 1980
   ctctgtgagg gaggccaggt agccgaggga tcaggatgca ggctgctgta cccgctctgc 2040
   ctcaagcatc ccccacacag ggctctggtt ttcactcgct tcgtcctaga tagtttaaat 2100
   gggaatcaga tcccctggtt gagagctaag acaaccacct accagtgccc atgtcccttc 2160
   cagctcacct tgagcagcct cagatcatct ctgtcactct ggaagggaca ccccagccag 2220
   ggacggaatg cctggtcttg agcaacctcc cactgctgga gtgcgagtgg gaatcagagc 2280
   ctcctgaagc ctctgggaac tcctcctgtg gccaccacca aaggatgagg aatctgagtt 2340
   gccaacttca ggacgacacc tggcttgcca cccacagtgc accacaggcc aacctacgcc 2400
   cttcatcact tggttctgtt ttaatcgact ggccccctgt cccacctctc cagtgagcct 2460
   ccttcaactc cttggtcccc tgttgtctgg gtcaacattt gccgagacgc cttggctggc 2520
   accctctggg gtcccccttt tctcccaggc aggtcatctt ttctgggaga tgcttcccct 2580
   gccatcccca aatagctagg atcacactcc aagtatgggc agtgatggcg ctctgggggc 2640
   cacagtgggc tatctaggcc ctccctcacc tgaggcccag agtggacaca gctgttaatt 2700
   tccactggct atgccacttc agagtctttc atgccagcgt ttgagctcct ctgggtaaaa 2760
   tcttcccttt gttgactggc cttcacagcc atggctggtg acaacagagg atcgttgaga 2820
   ttgagcagcg cttggtgatc tctcagcaaa caacccctgc ccgtgggcca atctacttga 2880
   agttactcgg acaaagaccc caaagtgggg caacaactcc agagaggctg tgggaatctt 2940
   cagaagcccc cctgtaagag acagacatga gagacaagca tcttctttcc cccgcaagtc 3000
   cattttattt ccttcttgtg ctgctctgga agagaggcag tagcaaagag atgagctcct 3060
   ggatggcatt ttccagggca ggagaaagta tgagagcctc aggaaacccc atcaaggacc 3120
   gagtatgtgt ctggttcctt gggtgggacg attcctgacc acactgtcca gctcttgctc 3180
   tcattaaatg ctctgtctcc cgcggaaaaa aaaaaaaaaa aaaaaaaaaa aaaaaaaaaa 3240
   aaaaaaaa 3248

<210> 6
<211> 568
<212> PRT
<213> Homo sapiens

<400> 6

<210>  7
<211>  1747
<212>  DNA
<213>  Caenohabditis elegans

<400>  7
gtttgagtgt taccactatc agacgaaggt atgaagccta gcccccagcg tacgttaata      60

aaaaagcttc tggtacttct tggaccactt gttgcagtac ctctactctt ttttggaccg     120

gagtacagat gtcttttctc aataatattt ttatcaacct attggattgg agaagcattt     180

cccattggtg tcacttctct ctttccattg gcactttatc caattcttca gattgttcca     240

tctaaacaaa ttagtccagt ttattttaaa gattcgatag ttctatttat gtgcacatta     300

agcatggcaa tggcggtaga agcaactgga ctccatcgaa gaatcgcact gaaattatta     360

acaaaagttg gagcaaagca accagtaatg ctgctgggtt tcatgtgcat cacgagtttc     420

atatcatttt tcgtttctga cacagcatgc acagctctta tgtgtccaac cgctgtggca     480

ctcctgatga gtatgtctga tgcagttcaa catttgaaag aagatcacag gaagccaaag     540

ccgccaccag atgatgctac tgttgctgaa aaaatgagaa tagatgatat gactcctcag     600

gatgctggtt tctgtaaagc attaattttg gcatgtgccc acgcatcgtt gatcggtggg     660

actgctatta tcacttcgac tgggccgaat ttagtttttc gagaaaatat tcacaagaga     720

taccccgagg gacaagtgac aatgacatac ctacaatgga tggttttcgc gataccacca 780

atgtttgtct accttcttgc atcttatatt attctggtgt gttatttcat gggcccctcg 840

acttttgctc gctggtttga aaggccttcc aaagaagaag ctcatttgaa aaaattaatt 900

gaaaagaata ttcagacaat gtatgaggat ttgggagatg tttcgtgggg cgaaaagtca 960

gtttttgtat tcttcattct cctaatcgga tcttggattt ctcgtgatcc cggattcaca 1020

cccggttggg gagatctttt accacacaga aacttcatat cagacagtgt ttctggagtc 1080

ttgatttctt gtattttatt tgtttggccc aaagatccat ttgatcctat tgatccaatg 1140

gctccgattc ttaaatggac cgacatgaaa agcaagtttt cctggtcgtg cacactttta 1200

atcggtgctg ggtatgctat ttcagaagga gttgataaat cgggattatc tagattgatt 1260

tcatgtggaa tgaaaaatat tttcgtgggg atgagctcac ttccattgca attaactgtg 1320

actacaatta ttgtgataat gacagagttt gcaagtaatg tgtccaccgg aagcattttc 1380

attccaattt ctttgggagt tgctgaatca atgggagttc atcctttata tcttgctctt 1440

ccaactactg tagcttgttc atttgctttt atgctaccaa tttcaacacc tccaaatgca 1500

gttgtatacg acactaaagt gatttcaatg gtggaaatga tagtctgtgg ttttctactc 1560

aatattgcgt gcatacttat cacatctctc aacatgaaca catggacata ttttattttt 1620

tcattgaata ttttccccga aaatattgta atatcttcag aaaactcttc atatccagtt 1680

tgctaatttt tgtacaaaat gtgtattgtc cgaatgaaac gtgtatttat taaaaaaaaa 1740

aaaaaaa 1747

<210> 8
<211> 551
<212> PRT
<213> Caenohabditis elegans

<400> 8

Met Lys Pro Ser Pro Gln Arg Thr Leu Ile Lys Lys Leu Leu Val Leu
1               5                   10                  15

Leu Gly Pro Leu Val Ala Val Pro Leu Leu Phe Phe Gly Pro Glu Tyr
            20                  25                  30

Arg Cys Leu Phe Ser Ile Ile Phe Leu Ser Thr Tyr Trp Ile Gly Glu
        35                  40                  45

Ala Phe Pro Ile Gly Val Thr Ser Leu Phe Pro Leu Ala Leu Tyr Pro
    50                  55                  60

Ile Leu Gln Ile Val Pro Ser Lys Gln Ile Ser Pro Val Tyr Phe Lys
65                  70                  75                  80

Asp Ser Ile Val Leu Phe Met Cys Thr Leu Ser Met Ala Met Ala Val
                85                  90                  95

Glu Ala Thr Gly Leu His Arg Arg Ile Ala Leu Lys Leu Leu Thr Lys
            100                 105                 110

Val Gly Ala Lys Gln Pro Val Met Leu Leu Gly Phe Met Cys Ile Thr
        115                 120                 125

Ser Phe Ile Ser Phe Phe Val Ser Asp Thr Ala Cys Thr Ala Leu Met
    130                 135                 140

Cys Pro Thr Ala Val Ala Leu Leu Met Ser Met Ser Asp Ala Val Gln
145                 150                 155                 160

His Leu Lys Glu Asp His Arg Lys Pro Lys Pro Pro Pro Asp Asp Ala
                165                 170                 175

Thr Val Ala Glu Lys Met Arg Ile Asp Asp Met Thr Pro Gln Asp Ala
            180                 185                 190

Gly Phe Cys Lys Ala Leu Ile Leu Ala Cys Ala His Ala Ser Leu Ile
        195                 200                 205

Gly Gly Thr Ala Ile Ile Thr Ser Thr Gly Pro Asn Leu Val Phe Arg
    210                 215                 220

Glu Asn Ile His Lys Arg Tyr Pro Glu Gly Gln Val Thr Met Thr Tyr
225                 230                 235                 240

Leu Gln Trp Met Val Phe Ala Ile Pro Pro Met Phe Val Tyr Leu Leu
                245                 250                 255

Ala Ser Tyr Ile Ile Leu Val Cys Tyr Phe Met Gly Pro Ser Thr Phe
            260                 265                 270

Ala Arg Trp Phe Glu Arg Pro Ser Lys Glu Glu Ala His Leu Lys Lys
        275                 280                 285

Leu Ile Glu Lys Asn Ile Gln Thr Met Tyr Glu Asp Leu Gly Asp Val
    290                 295                 300

Ser Trp Gly Glu Lys Ser Val Phe Val Phe Phe Ile Leu Leu Ile Gly
305                 310                 315                 320

Ser Trp Ile Ser Arg Asp Pro Gly Phe Thr Pro Gly Trp Gly Asp Leu
                325                 330                 335

Leu Pro His Arg Asn Phe Ile Ser Asp Ser Val Ser Gly Val Leu Ile
            340                 345                 350

Ser Cys Ile Leu Phe Val Trp Pro Lys Asp Pro Phe Asp Pro Ile Asp
        355                 360                 365

Pro Met Ala Pro Ile Leu Lys Trp Thr Asp Met Lys Ser Lys Phe Ser
    370                 375                 380

Trp Ser Cys Thr Leu Leu Ile Gly Ala Gly Tyr Ala Ile Ser Glu Gly
385                 390                 395                 400

Val Asp Lys Ser Gly Leu Ser Arg Leu Ile Ser Cys Gly Met Lys Asn
                405                 410                 415

Ile Phe Val Gly Met Ser Ser Leu Pro Leu Gln Leu Thr Val Thr Thr
            420                 425                 430

Ile Ile Val Ile Met Thr Glu Phe Ala Ser Asn Val Ser Thr Gly Ser
        435                 440                 445

Ile Phe Ile Pro Ile Ser Leu Gly Val Ala Glu Ser Met Gly Val His
    450                 455                 460

Pro Leu Tyr Leu Ala Leu Pro Thr Thr Val Ala Cys Ser Phe Ala Phe
465                 470                 475                 480

Met Leu Pro Ile Ser Thr Pro Pro Asn Ala Val Val Tyr Asp Thr Lys
                485                 490                 495

Val Ile Ser Met Val Glu Met Ile Val Cys Gly Phe Leu Leu Asn Ile
            500                 505                 510

Ala Cys Ile Leu Ile Thr Ser Leu Asn Met Asn Thr Trp Thr Tyr Phe
        515                 520                 525

Ile Phe Ser Leu Asn Ile Phe Pro Glu Asn Ile Val Ile Ser Ser Glu
    530                 535                 540

Asn Ser Ser Tyr Pro Val Cys
545                 550

<210> 9
<211> 1735
<212> DNA
<213> Mus musculus

<400> 9
gtctcccttt cacgcgatgg attcggcgaa gacttgtgtg accaagttca agtcctttgc 60

gattttgctc ttcaccccga tcctgatgct tccactcgtc attctgatac ctgacaagtt 120
tgccaggtgt gcctatgtta tagtcattat ggctgtctac tggtgcacag atgtcatccc 180
agtggctgtt acctccctcc tgcctgtctt actcttccca cttttgaagg ttctggactc 240
caagcaggta tgtatccaat acatgaagga caccaacatg ctgttcctgg gcagtctcat 300
tgtggctgtg gctgtggaac gctggaaact tcataagagg gttgccctga gaatgctgct 360
ctttgtgggg accaagccct cacggctgat gctgggcttt atgtttgtca cggccttcct 420
gtccatgtgg atcagcaata ctgccgccac agccatgatg atacccattg tggaggccat 480
gctgcagcaa atgatagccg ccaatacagc tgtggaggcc agcctgggga cactggagct 540
gctggacaag aacaagacca gcgagttgcc aggaagccag gtggtatttg aagaccccaa 600
tgtgcaggag caggaagacg aagaaacaaa gaatatgtac aaggctatgc acctatgtgt 660
ttgctattca gccagcattg gggtacagcc caccttgacc gggacgggac ccaacgtggt 720
gctcctgggc cagatgcagg aattgtttcc tgacagtaaa gatgtcctga actatgcatc 780
ttggtttgga tttgccttcc ccaacatggt gatgatgctg gtgctggcct ggctgtggct 840
ccagtgcttg tacatgagac acaatttaaa aaaaacttgc atctgctgtg gggagaagaa 900
gagggacacc gagaagattg cctacaaagt gctgaacgag gagtaccaga agctggggtc 960
cttgagctac cctgaatgca acgtgctctt ttgcttcacc ctacttgtca tcctgtggtt 1020
ctcccgagac cccggcttca tgcctggctg gctgtcattc gcctgggtcg agggaaacac 1080
cgttcatatc acagatgcca cagtggccat ctttgtggcc attttgcttt tcatcatacc 1140
ttcacaaaag cccaagttca acttcagcag ccagactgag gaagaaagga aaactccgtt 1200
ctacccccca gcactgctgg attggaaagt cgcccaagag aaagtgccct gggacatcgt 1260
gctgctcctg gggggagggt ttgctatggc aaaaggatgt gagacgtcag ggctctcgaa 1320
gtggatggca gcacagatgg aacccttgag attagtgaaa cctgctgtca ttaccttgat 1380
cttgtcctgt cttgttgcaa tgaccacaga gtgcacaagt aacgtggcca ctaccaccct 1440
gttcctgcct atctttgcct ccatggctcg ttccattggt atccatcctc tgtatgtcat 1500
gattccctgt accatgagtg cttcacttgc cttcatgttg cctgtggcca ccccaccgaa 1560
tgccatcgtg tttgcctacg gacacctcag agttgttgac atgatgaaaa caggattgat 1620
aatgaacttc gttggaatcc tatctgtgtt tctgtcagtc aacacctggg gtcgggctat 1680
gtttaacttg gataacttcc ccgactgggc aaattcaaca agtgttaaca cttag 1735

<210> 10
<211> 572
<212> PRT
<213> Mus musculus

<400> 10

Met Asp Ser Ala Lys Thr Cys Val Thr Lys Phe Lys Ser Phe Ala Ile
1               5                   10                  15

Leu Leu Phe Thr Pro Ile Leu Met Leu Pro Leu Val Ile Leu Ile Pro
            20                  25                  30

Asp Lys Phe Ala Arg Cys Ala Tyr Val Ile Val Ile Met Ala Val Tyr
        35                  40                  45

Trp Cys Thr Asp Val Ile Pro Val Ala Val Thr Ser Leu Leu Pro Val
    50                  55                  60

Leu Leu Phe Pro Leu Leu Lys Val Leu Asp Ser Lys Gln Val Cys Ile
65                  70                  75                  80

Gln Tyr Met Lys Asp Thr Asn Met Leu Phe Leu Gly Ser Leu Ile Val
                85                  90                  95

Ala Val Ala Val Glu Arg Trp Lys Leu His Lys Arg Val Ala Leu Arg
            100                 105                 110

Met Leu Leu Phe Val Gly Thr Lys Pro Ser Arg Leu Met Leu Gly Phe
        115                 120                 125

Met Phe Val Thr Ala Phe Leu Ser Met Trp Ile Ser Asn Thr Ala Ala
    130                 135                 140

Thr Ala Met Met Ile Pro Ile Val Glu Ala Met Leu Gln Gln Met Ile
145                 150                 155                 160

Ala Ala Asn Thr Ala Val Glu Ala Ser Leu Gly Thr Leu Glu Leu Leu
                165                 170                 175

Asp Lys Asn Lys Thr Ser Glu Leu Pro Gly Ser Gln Val Val Phe Glu
            180                 185                 190

Asp Pro Asn Val Gln Glu Gln Glu Asp Glu Glu Thr Lys Asn Met Tyr
        195                 200                 205

Lys Ala Met His Leu Cys Val Cys Tyr Ser Ala Ser Ile Gly Gly Thr
    210                 215                 220

Ala Thr Leu Thr Gly Thr Gly Pro Asn Val Val Leu Leu Gly Gln Met
225                 230                 235                 240

Gln Glu Leu Phe Pro Asp Ser Lys Asp Val Leu Asn Tyr Ala Ser Trp
                245                 250                 255

Phe Gly Phe Ala Phe Pro Asn Met Val Met Met Leu Val Leu Ala Trp
            260                 265                 270

Leu Trp Leu Gln Cys Leu Tyr Met Arg His Asn Leu Lys Lys Thr Cys
        275                 280                 285

Ile Cys Cys Gly Glu Lys Lys Arg Asp Thr Glu Lys Ile Ala Tyr Lys
    290                 295                 300

Val Leu Asn Glu Glu Tyr Gln Lys Leu Gly Ser Leu Ser Tyr Pro Glu
305                 310                 315                 320

Cys Asn Val Leu Phe Cys Phe Thr Leu Leu Val Ile Leu Trp Phe Ser
                325                 330                 335

Arg Asp Pro Gly Phe Met Pro Gly Trp Leu Ser Phe Ala Trp Val Glu
            340                 345                 350

Gly Asn Thr Val His Ile Thr Asp Ala Thr Val Ala Ile Phe Val Ala
        355                 360                 365

Ile Leu Leu Phe Ile Ile Pro Ser Gln Lys Pro Lys Phe Asn Phe Ser
    370                 375                 380

Ser Gln Thr Glu Glu Glu Arg Lys Thr Pro Phe Tyr Pro Pro Ala Leu
385                 390                 395                 400

Leu Asp Trp Lys Val Ala Gln Glu Lys Val Pro Trp Asp Ile Val Leu
                405                 410                 415

Leu Leu Gly Gly Gly Phe Ala Met Ala Lys Gly Cys Glu Thr Ser Gly
            420                 425                 430

Leu Ser Lys Trp Met Ala Ala Gln Met Glu Pro Leu Arg Leu Val Lys
        435                 440                 445

Pro Ala Val Ile Thr Leu Ile Leu Ser Cys Leu Val Ala Met Thr Thr
    450                 455                 460

Glu Cys Thr Ser Asn Val Ala Thr Thr Thr Leu Phe Leu Pro Ile Phe
465                 470                 475                 480

Ala Ser Met Ala Arg Ser Ile Gly Ile His Pro Leu Tyr Val Met Ile
                485                 490                 495

Pro Cys Thr Met Ser Ala Ser Leu Ala Phe Met Leu Pro Val Ala Thr
            500                 505                 510

Pro Pro Asn Ala Ile Val Phe Ala Tyr Gly His Leu Arg Val Val Asp
        515                 520                 525

Met Met Lys Thr Gly Leu Ile Met Asn Phe Val Gly Ile Leu Ser Val
    530                 535                 540

Phe Leu Ser Val Asn Thr Trp Gly Arg Ala Met Phe Asn Leu Asp Asn
545                 550                 555                 560

Phe Pro Asp Trp Ala Asn Ser Thr Ser Val Asn Thr
                565                 570

<210>  11
<211>  2551
<212>  DNA
<213>  Danio rerio

<400>  11
agagatcagc gcacagaagt tttgcgcagt ttctcaccgt ttggacattt cattgtaaag      60

ttatccaaag ccgaaatgat ggcttcacgt gcactcaaac tagtatggaa gatgaaaaat     120

acattgattc tttttgcac tccatttctt ctgcttcctt tgccacttgt cattggatca     180

aaggaggctg gatgtgcata tgttgtggta ctgatggcag tttactggtg tacagaggtg     240

ctgccgctgg ctgtcactgc tctcctgccc gctgtgctct ttcccctctt cagaatcatg     300

gagtcccaag acgtatgtat gcagtacctt aaggacacta acatgctgtt tctgggtggc     360

ctgatggtgg ccgtggctgt cgaacactgg aatctgcaca agcggatcgc cctgcgggtg     420

ctgctccttg tgggggttcg accagctctg ttaatgttgg gcttcatggg tgtaacagct     480

ttcctctcca tgtggatcag taacacggcc acaacagcca tgatggtgcc catcgttcag     540

gcagttctcg agcagctcaa caacacagca caacaagaac aaagctccat acctgagacc     600

gaggaaaaga gcactgagaa acagcctgag agcccgggtg aggaaaaagt ggtactgaat     660

ggcgacaact tctcaatgga gtcagaccct gaagaacatt cacgagaagc agaggaaagg     720

ctgaagatgt ctaaaggcct gaccctgtgc gtgtgttatg ccgccagcat cggcggcaca     780

gccacactca caggcactgg accaaacctc gttcttatgg gacagatgag ccaactgttc     840

ccggacaacc ctgacatcat taactttgcg tcatggtttg gatttgcctt tccaaacatg     900

atcatcatgc tcacgctggc ctggctgtgg ctacagatcg tgtttctggg aataaacttt     960

aaaaagacat ggggctgtgg gacggtgaag acggagaagg agatcgcggc ctataatgtg    1020

attaaagagg agcaccgcag tctcggccct atgacctttg gggagctgag tgtccttgcc    1080

ctcttcatcc tcctggtggt gctttggttc actcgtgatc caggcttcgt ggacggctgg    1140

gcgacacgct tcttcaatgc tgacaaagag tttgtgacag atgccacggt tgcagtgttt    1200

gtggctgcgc tgctctttgt ctttccctct aaaccaccac gattgtgctt ctggagaaca    1260

gagagtttcg acacagtgcc ccagcaagaa agtggcccga ctccagcttt gctgacatgg 1320

aaagtgacac agaagaagat gccatggagt attatactgc tgctgggagg aggctttgcc 1380

ctggctaagg gcagtgagat ctcaggattg tccaagtggc ttggagatca gatgtctcct 1440

cttcaaagca ttcctccatg ggcaatagct attgtcatat gtttaatgat cgcaaccttc 1500

actgaatgca ccagtaatgt ggccacagct acattatttc tgcctatact ggcatctatg 1560

tctcagtcta taggtgtgaa tcctctgtat gttatggtgc cctgtaccct cagtgcatct 1620

tttgccttca tgctccctgt ggcaactcct ccaaacgcca tcgtcttctc atacggatac 1680

ctcaaagtct ctgacatggc caagactggg atcgtcatga acatcatcgg catcctctcc 1740

atcaccttag ccattaacag ctggggcaga gccatcttca gtttagacac gttccccagc 1800

tgggcaaaca ctactgatgt ctaagagaca cagagagccg gactgcccca ctcaccactt 1860

gtgaacttca gattgtttcc agttctcatg tgaacagaga aaaccgatta tgaccactgt 1920

ttagtcattt cacacattca tgtctatctt ttacaaaacc gtggtgttca ctacagtctg 1980

tggacattgt aacaccaata tttgaatgat tcataaaaga caaaaataga ttttacaaat 2040

catgattttt ctgtagcatt acagcatact gtgagcactg agcatatatt tggaccattg 2100

gttgttcgct tttggcttgt gaaagagcat ttggacgcag aaactcaaca tcagtcatat 2160

tgtccccttg tgtccagact cagagccagt ggccggtttc actggagatc aatactgtac 2220

tttgacggtt caaatcactt catgttcata tgcgtaattt aaagctgctt taaacacagc 2280

tacacaagag cacatgcaga aaaagctgaa aggctgaaat tgtgtaaata tcatctagat 2340

ttttaagcac aaaagtatat ttagagtata tatatatata tatgaataaa tttatacata 2400

cctcagatat gcagagctgc tttgacagag taatcatgta catttcaaaa ttaattcaaa 2460

ctctattttt aatgtacatt tattaaaatt atgtattttg ttcatattta ctaataaatt 2520

tttatttggt ttactcaaaa aaaaaaaaaa a 2551

<210> 12
<211> 581
<212> PRT
<213> Danio rerio

<400> 12

Met Ala Ser Arg Ala Leu Lys Leu Val Trp Lys Met Lys Asn Thr Leu
1               5                   10                  15

Ile Leu Phe Cys Thr Pro Phe Leu Leu Leu Pro Leu Pro Leu Val Ile
            20                  25                  30

Gly Ser Lys Glu Ala Gly Cys Ala Tyr Val Val Val Leu Met Ala Val
        35                  40                  45

Tyr Trp Cys Thr Glu Val Leu Pro Leu Ala Val Thr Ala Leu Leu Pro
    50                  55                  60

Ala Val Leu Phe Pro Leu Phe Arg Ile Met Glu Ser Gln Asp Val Cys
65                  70                  75                  80

Met Gln Tyr Leu Lys Asp Thr Asn Met Leu Phe Leu Gly Gly Leu Met
                85                  90                  95

Val Ala Val Ala Val Glu His Trp Asn Leu His Lys Arg Ile Ala Leu
            100                 105                 110

Arg Val Leu Leu Leu Val Gly Val Arg Pro Ala Leu Leu Met Leu Gly
        115                 120                 125

Phe Met Gly Val Thr Ala Phe Leu Ser Met Trp Ile Ser Asn Thr Ala
    130                 135                 140

Thr Thr Ala Met Met Val Pro Ile Val Gln Ala Val Leu Glu Gln Leu
145                 150                 155                 160

Asn Asn Thr Ala Gln Gln Glu Gln Ser Ser Ile Pro Glu Thr Glu Glu
                165                 170                 175

Lys Ser Thr Glu Lys Gln Pro Glu Ser Pro Gly Glu Glu Lys Val Val
            180                 185                 190

Leu Asn Gly Asp Asn Phe Ser Met Glu Ser Asp Pro Glu Glu His Ser
        195                 200                 205

Arg Glu Ala Glu Glu Arg Leu Lys Met Ser Lys Gly Leu Thr Leu Cys
    210                 215                 220

Val Cys Tyr Ala Ala Ser Ile Gly Gly Thr Ala Thr Leu Thr Gly Thr
225                 230                 235                 240

Gly Pro Asn Leu Val Leu Met Gly Gln Met Ser Gln Leu Phe Pro Asp
                245                 250                 255

Asn Pro Asp Ile Ile Asn Phe Ala Ser Trp Phe Gly Phe Ala Phe Pro
            260                 265                 270

Asn Met Ile Ile Met Leu Thr Leu Ala Trp Leu Trp Leu Gln Ile Val
        275                 280                 285

Phe Leu Gly Ile Asn Phe Lys Lys Thr Trp Gly Cys Gly Thr Val Lys
    290                 295                 300

Thr Glu Lys Glu Ile Ala Ala Tyr Asn Val Ile Lys Glu Glu His Arg
305                 310                 315                 320

Ser Leu Gly Pro Met Thr Phe Gly Glu Leu Ser Val Leu Ala Leu Phe
                325                 330                 335

Ile Leu Leu Val Val Leu Trp Phe Thr Arg Asp Pro Gly Phe Val Asp
            340                 345                 350

Gly Trp Ala Thr Arg Phe Phe Asn Ala Asp Lys Glu Phe Val Thr Asp
        355                 360                 365

Ala Thr Val Ala Val Phe Val Ala Ala Leu Leu Phe Val Phe Pro Ser
    370                 375                 380

Lys Pro Pro Arg Leu Cys Phe Trp Arg Thr Glu Ser Phe Asp Thr Val
385                 390                 395                 400

Pro Gln Gln Glu Ser Gly Pro Thr Pro Ala Leu Leu Thr Trp Lys Val
                405                 410                 415

Thr Gln Lys Lys Met Pro Trp Ser Ile Ile Leu Leu Leu Gly Gly Gly
            420                 425                 430

Phe Ala Leu Ala Lys Gly Ser Glu Ile Ser Gly Leu Ser Lys Trp Leu
        435                 440                 445

Gly Asp Gln Met Ser Pro Leu Gln Ser Ile Pro Pro Trp Ala Ile Ala
    450                 455                 460

Ile Val Ile Cys Leu Met Ile Ala Thr Phe Thr Glu Cys Thr Ser Asn
465                 470                 475                 480

Val Ala Thr Ala Thr Leu Phe Leu Pro Ile Leu Ala Ser Met Ser Gln
                485                 490                 495

Ser Ile Gly Val Asn Pro Leu Tyr Val Met Val Pro Cys Thr Leu Ser
            500                 505                 510

Ala Ser Phe Ala Phe Met Leu Pro Val Ala Thr Pro Pro Asn Ala Ile
        515                 520                 525

Val Phe Ser Tyr Gly Tyr Leu Lys Val Ser Asp Met Ala Lys Thr Gly
    530                 535                 540

Ile Val Met Asn Ile Ile Gly Ile Leu Ser Ile Thr Leu Ala Ile Asn
545                 550                 555                 560

Ser Trp Gly Arg Ala Ile Phe Ser Leu Asp Thr Phe Pro Ser Trp Ala
                565                 570                 575

Asn Thr Thr Asp Val
            580

<210>  13
<211>  587
<212>  PRT
<213>  Rattus norvegicus

<400>  13

Met Ala Thr Cys Trp Pro Ala Leu Trp Ala Tyr Arg Phe Tyr Leu Ile
1               5                   10                  15

Val Leu Cys Leu Pro Ile Phe Leu Leu Pro Leu Pro Leu Ile Val Gln
            20                  25                  30

Thr Lys Glu Ala Tyr Cys Ala Tyr Ser Ile Ile Leu Met Ala Leu Leu
        35                  40                  45

Trp Cys Thr Glu Ala Leu Pro Leu Ala Val Thr Ala Leu Phe Pro Ile
    50                  55                  60

Val Leu Phe Pro Leu Met Gly Ile Met Asp Ala Ser Glu Val Cys Ile
65                  70                  75                  80

Glu Tyr Phe Lys Asp Thr Asn Ile Leu Phe Val Gly Gly Leu Met Val
                85                  90                  95

Ala Ile Ala Val Glu His Trp Asn Leu His Lys Arg Ile Ala Leu Gln
            100                 105                 110

Val Leu Leu Ile Ile Gly Val Arg Pro Ala Leu Leu Leu Leu Gly Phe
        115                 120                 125

Met Leu Val Thr Ala Phe Leu Ser Met Trp Ile Ser Asn Thr Ala Thr
    130                 135                 140

Thr Ala Met Met Val Pro Ile Gly His Ala Val Leu Glu Gln Leu Gln
145                 150                 155                 160

Gly Ser Lys Lys Asp Val Glu Gly Gly Asn Asn Asn Pro Thr Phe Glu
                165                 170                 175

Leu Gln Glu Glu Cys Pro Gln Lys Glu Val Thr Lys Leu Asp Asn Gly
            180                 185                 190

Gln Pro Val Ser Ala Pro Ser Glu Pro Arg Thr Gln Lys Thr Gln Glu
        195                 200                 205

His His Arg Phe Ser Gln Gly Leu Ser Leu Cys Ile Cys Tyr Ser Ala
    210                 215                 220

Ser Ile Gly Gly Ile Ala Thr Leu Thr Gly Thr Thr Pro Asn Leu Val
225                 230                 235                 240

Leu Gln Gly Gln Val Asn Ser Leu Phe Pro Gln Asn Gly Asn Val Val
                245                 250                 255

Asn Phe Ala Ser Trp Phe Gly Phe Ala Phe Pro Thr Met Ile Ile Leu
            260                 265                 270

Leu Leu Leu Ala Trp Leu Trp Leu Gln Val Leu Phe Leu Gly Val Asn
        275                 280                 285

Phe Arg Lys Asn Phe Gly Phe Gly Glu Gly Glu Glu Glu Arg Lys Gln
    290                 295                 300

Ala Ala Phe Gln Val Ile Lys Thr Gln Tyr Arg Leu Leu Gly Pro Met
305                 310                 315                 320

Ser Phe Ala Glu Lys Thr Val Thr Val Leu Phe Val Leu Leu Val Val
                325                 330                 335

Leu Trp Phe Thr Arg Glu Pro Gly Phe Phe Pro Gly Trp Gly Asp Thr
            340                 345                 350

Val Phe Ala Asn Glu Lys Gly Gln Ser Met Ala Ser Asp Gly Thr Val
        355                 360                 365

Ala Ile Phe Ile Ser Leu Val Met Phe Ile Ile Pro Ser Lys Ile Pro
    370                 375                 380

Gly Leu Met Gln Asp Pro Lys Lys Pro Gly Lys Leu Lys Ala Pro Pro
385                 390                 395                 400

Ala Ile Leu Thr Trp Lys Thr Val Asn Asp Lys Met Pro Trp Asn Ile
                405                 410                 415

Val Ile Leu Leu Gly Gly Gly Phe Ala Leu Ala Lys Gly Ser Glu Gln
            420                 425                 430

Ser Gly Leu Ser Glu Trp Leu Gly Asp Lys Leu Thr Pro Leu Gln His
        435                 440                 445

Ile Pro Pro Ser Ala Thr Ala Val Ile Leu Cys Leu Leu Ile Ala Ile
    450                 455                 460

Phe Thr Glu Cys Thr Ser Asn Val Ala Thr Thr Thr Leu Phe Leu Pro
465                 470                 475                 480

Ile Leu Ala Ser Met Ala Gln Ala Ile Cys Leu His Pro Leu Tyr Val
                485                 490                 495

Met Leu Pro Cys Thr Leu Ala Ala Ser Leu Ala Phe Met Leu Pro Val
            500                 505                 510

Ala Thr Pro Pro Asn Ala Ile Val Phe Ser Phe Gly Gly Leu Lys Val
        515                 520                 525

Ser Asp Met Ala Arg Ala Gly Phe Leu Leu Asn Ile Ile Gly Val Leu
    530                 535                 540

Ala Ile Thr Leu Ser Ile Asn Ser Trp Ser Ile Pro Ile Phe Lys Leu
545                 550                 555                 560

Asp Thr Phe Pro Ser Trp Ala His Ser Asn Thr Ser Gln Cys Leu Leu
                565                 570                 575

Asn Pro Ser Asn Ser Thr Val Pro Gly Gly Leu
            580                 585

<210>  14
<211>  600
<212>  PRT
<213>  Rattus norvegicus

<400>  14

Met Ala Ala Leu Ala Ala Leu Ala Lys Lys Val Trp Ser Ala Arg Arg
1               5                   10                  15

Leu Leu Val Leu Leu Leu Val Pro Leu Ala Leu Leu Pro Ile Leu Phe
            20                  25                  30

Ala Leu Pro Pro Lys Glu Gly Arg Cys Leu Tyr Val Ile Leu Leu Met
        35                  40                  45

Ala Val Tyr Trp Cys Thr Glu Ala Leu Pro Leu Ser Val Thr Ala Leu
    50                  55                  60

Leu Pro Ile Ile Leu Phe Pro Phe Met Gly Ile Leu Pro Ser Ser Lys
65                  70                  75                  80

Val Cys Pro Gln Tyr Phe Leu Asp Thr Asn Phe Leu Phe Leu Ser Gly

                85                  90                  95

Leu Ile Met Ala Ser Ala Ile Glu Glu Arg Asn Leu His Arg Arg Ile
            100                 105                 110

Ala Leu Lys Val Leu Met Leu Val Gly Val Gln Pro Ala Arg Leu Ile
        115                 120                 125

Leu Gly Met Met Val Thr Thr Ser Phe Leu Ser Met Trp Leu Ser Asn
    130                 135                 140

Thr Ala Ser Thr Ala Met Met Leu Pro Ile Ala Ser Ala Ile Leu Lys
145                 150                 155                 160

Ser Leu Phe Gly Gln Arg Asp Thr Arg Lys Asp Leu Pro Arg Glu Gly
                165                 170                 175

Glu Asp Ser Thr Ala Ala Val Arg Gly Asn Gly Leu Arg Thr Val Pro
            180                 185                 190

Thr Glu Met Gln Phe Leu Ala Ser Ser Glu Gly Gly His Ala Glu Asp
        195                 200                 205

Val Glu Ala Pro Leu Glu Leu Pro Asp Asp Ser Lys Glu Glu Glu His
    210                 215                 220

Arg Arg Asn Ile Trp Lys Gly Phe Leu Ile Ser Ile Pro Tyr Ser Ala
225                 230                 235                 240

Ser Ile Gly Gly Thr Ala Thr Leu Thr Gly Thr Ala Pro Asn Leu Ile
                245                 250                 255

Leu Leu Gly Gln Leu Lys Ser Phe Phe Pro Gln Cys Asp Val Val Asn
            260                 265                 270

Phe Gly Ser Trp Phe Ile Phe Ala Phe Pro Leu Met Leu Leu Phe Leu
        275                 280                 285

Leu Val Gly Trp Leu Trp Ile Ser Phe Leu Tyr Gly Gly Met Ser Trp
    290                 295                 300

Arg Gly Trp Arg Lys Lys Asn Ser Lys Leu Gln Asp Val Ala Glu Asp
305                 310                 315                 320

Lys Ala Lys Ala Val Ile Gln Glu Glu Phe Gln Asn Leu Gly Pro Ile
                325                 330                 335

Lys Phe Ala Glu Gln Ala Val Phe Ile Leu Phe Cys Leu Phe Ala Ile

340                 345                 350

Leu Leu Phe Ser Arg Asp Pro Lys Phe Ile Pro Gly Trp Ala Ser Leu
        355                 360                 365

Phe Ala Pro Gly Phe Val Ser Asp Ala Val Thr Gly Val Ala Ile Val
    370                 375                 380

Thr Ile Leu Phe Phe Phe Pro Ser Gln Lys Pro Ser Leu Lys Trp Trp
385                 390                 395                 400

Phe Asp Phe Lys Ala Pro Asn Ser Glu Thr Glu Pro Leu Leu Ser Trp
                405                 410                 415

Lys Lys Ala Gln Glu Thr Val Pro Trp Asn Ile Ile Leu Leu Leu Gly
            420                 425                 430

Gly Gly Phe Ala Met Ala Lys Gly Cys Glu Glu Ser Gly Leu Ser Ala
        435                 440                 445

Trp Ile Gly Gly Gln Leu His Pro Leu Glu His Val Pro Pro Leu Leu
    450                 455                 460

Ala Val Leu Leu Ile Thr Val Val Ile Ala Phe Phe Thr Glu Phe Ala
465                 470                 475                 480

Ser Asn Thr Ala Thr Ile Ile Ile Phe Leu Pro Val Leu Ala Glu Leu
                485                 490                 495

Ala Ile Arg Leu His Val His Pro Leu Tyr Leu Met Ile Pro Gly Thr
            500                 505                 510

Val Ser Cys Ser Tyr Ala Phe Met Leu Pro Val Ser Thr Pro Pro Asn
        515                 520                 525

Ser Ile Ala Phe Ser Thr Gly His Leu Leu Val Lys Asp Met Val Arg
    530                 535                 540

Thr Gly Leu Leu Met Asn Leu Met Gly Val Leu Leu Leu Ser Leu Ala
545                 550                 555                 560

Met Asn Thr Trp Ala Gln Ala Ile Phe Gln Leu Gly Thr Phe Pro Asp
                565                 570                 575

Trp Ala Asn Thr His Ala Ala Asn Val Thr Ala Leu Pro Pro Ala Leu
            580                 585                 590

Thr Asn Asn Thr Val Gln Thr Leu

        595                 600

<210> 15
<211> 20
<212> DNA
<213> Artificial sequence

<220>
<221> source
<223> /note="Description of artifical sequence:Artificially synthesized
oligonucleotide primer"

<400> 15
ctccaacttc ttcgctaacc 20


<210> 16
<211> 19
<212> DNA
<213> Artificial sequence

<220>
<221> source
<223> /note="Description of artifical sequence:Artificially synthesized
oligonucleotide primer
"
<400> 16
ctagtgcgtc ttgtttccc 19


<210> 17
<211> 21
<212> DNA
<213> Artificial sequence

<220>
<221> source
<223> /note="Description of artifical sequence:Artificially synthesized
oligonucleotide primer"

<400> 17
tcttttctcc ctccagtcag t 21


<210> 18
<211> 18
<212> DNA
<213> Artificial sequence

<220>
<221> source
<223> /note="Description of artifical sequence:Artificially synthesized
oligonucleotide primer"

<400> 18
ggcaatcttc tcggtgtc 18


<210> 19
<211> 20
<212> DNA
<213> Artificial sequence

<220>
<221> source
<223> /note="Description of artifical sequence:Artificially synthesized
oligonucleotide primer"

<400> 19
ctcggcgctg agctatgtct 20

<210> 20
<211> 17
<212> DNA
<213> Artificial sequence

<220>
<221> source
<223> /note="Description of artifical sequence:Artificially synthesized
oligonucleotide primer"

<400> 20
gttgatctcc gcgaagg 17

<210> 21
<211> 19
<212> DNA
<213> Artificial sequence

<220>
<221> source
<223> /note="Description of artifical sequence:Artificially synthesized
oligonucleotide primer"

<400> 21
ctccatcgaa gaatcgcac 19

<210> 22
<211> 20
<212> DNA
<213> Artificial sequence

<220>
<221> source
<223> /note="Description of artifical sequence:Artificially synthesized
oligonucleotide primer"

<400> 22
gaaatagcat acccagcacc 20

<210> 23
<211> 40
<212> DNA
<213> Artificial sequence

<220>
<221> source
<223> /note="Description of artifical sequence:Artificially synthesized
oligonucleotide primer"

<400> 23
cccgggtatg aagcctagcc cccagcgtac gttaataaaa 40

<210>  24
<211>  45
<212>  DNA
<213>  Artificial sequence

<220>
<221>  source
<223>  /note="Description of artifical sequence:Artificially synthesized
oligonucleotide primer"

<400>  24
gcggatccaa aaattagcaa actggatatg aagagttttc tgaag                    45


<210>  25
<211>  33
<212>  DNA
<213>  Artificial sequence

<220>
<221>  source
<223>  /note="Description of artifical sequence:Artificially synthesized
oligonucleotide primer"

<400>  25
gtcgacgagg tgttaaactg tatagtcgtg gtg                                 33


<210>  26
<211>  33
<212>  DNA
<213>  Artificial sequence

<220>
<221>  source
<223>  /note="Description of artifical sequence:Artificially synthesized
oligonucleotide primer"

<400>  26
gccggatcca agaagtacca gaagcttttt tat                                 33


<210>  27
<211>  20
<212>  DNA
<213>  Artificial sequence

<220>
<221>  source
<223>  /note="Description of artifical sequence:Artificially synthesized
oligonucleotide primer"

<400>  27
gtctcccttt cacgcgatgg                                                20


<210>  28
<211>  29
<212>  DNA
<213>  Artificial sequence

<220>
<221>  artificial sequence

<223>  /note="Description of artifical sequence:Artificially synthesized
oligonucleotide primer"

<400>  28
tcgtctagag cttgtgctct tgcggctct                                        29


<210>  29
<211>  17
<212>  PRT
<213>  Artificial sequence

<220>
<221>  source
<223>  /note="Description of artifical sequence:peptide consensus sequence"


<220>
<221>  VARIANT
<222>  1
<223>  /replace="Ala"
       /replace="Pro"
       /replace="Cys"
       /replace="Thr"

<220>
<221>  VARIANT
<222>  3
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"
       /replace="Pro"
       /replace="Ser"
       /replace="Thr"
       /replace="Trp"
       /replace="Tyr"
       /replace="Val"

<220>
<221>  VARIANT
<222>  4
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"

/replace="Pro"
/replace="Ser"
/replace="Thr"
/replace="Trp"
/replace="Tyr"
/replace="Val"

<220>
<221>  VARIANT
<222>  6
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"
       /replace="Pro"
       /replace="Ser"
       /replace="Thr"
       /replace="Trp"
       /replace="Tyr"
       /replace="Val"

<220>
<221>  VARIANT
<222>  7
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"
       /replace="Pro"
       /replace="Ser"
       /replace="Thr"
       /replace="Trp"
       /replace="Tyr"
       /replace="Val"

<220>
<221>  VARIANT
<222>  9
<223>  /replace="Leu"
       /replace="Ile"
       /replace="Met"

<220>
<221>  VARIANT
<222>  10
<223>  /replace="Ser"

/replace="Ala"

<220>
<221>  VARIANT
<222>  11
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"
       /replace="Pro"
       /replace="Ser"
       /replace="Thr"
       /replace="Trp"
       /replace="Tyr"
       /replace="Val"

<220>
<221>  VARIANT
<222>  12
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"
       /replace="Pro"
       /replace="Ser"
       /replace="Thr"
       /replace="Trp"
       /replace="Tyr"
       /replace="Val"

<220>
<221>  VARIANT
<222>  13
<223>  /replace="Arg"
       /replace="Asn"
       /replace="Asp"
       /replace="Cys"
       /replace="Glu"
       /replace="Gln"
       /replace="Gly"
       /replace="His"
       /replace="Ile"
       /replace="Leu"
       /replace="Lys"
       /replace="Met"
       /replace="Phe"

/replace="Pro"
/replace="Ser"
/replace="Thr"
/replace="Trp"
/replace="Tyr"
/replace="Val"

<220>
<221> VARIANT
<222> 15
<223> /replace="Arg"
/replace="Asn"
/replace="Asp"
/replace="Cys"
/replace="Glu"
/replace="Gln"
/replace="Gly"
/replace="His"
/replace="Ile"
/replace="Leu"
/replace="Lys"
/replace="Met"
/replace="Phe"
/replace="Pro"
/replace="Ser"
/replace="Thr"
/replace="Trp"
/replace="Tyr"
/replace="Val"
<220>
<221> VARIANT
<222> 16
<223> /replace="Leu"
/replace="Val"
/replace="met"

<400> 29

Ser Ser Ala Ala Phe Ala Ala Pro Val Gly Ala Ala Ala Asn Ala Ile
1               5                   10                  15

Val